# EUROPEAN PATENT APPLICATION

(11) **EP 4 831 165 A1**
(43) Date of publication of application: **28.10.2026**
(21) Application number: 24907495.6
(22) Date of filing: 19.12.2024
(51) Int. Cl.: C07D 311/14

(54) **NOVEL SULFONAMIDE DERIVATIVE**

(30) Priority: 19.12.2023 JP 2023213851
(71) Applicant: Wakunaga Pharmaceutical Co., Ltd., Osaka-shi Osaka 532-0003 (JP)
(72) Inventor: SAKATA, Kotaro, Takata-shi, Hiroshima 739-1195 (JP); YAMAGUCHI, Tomonori, Takata-shi, Hiroshima 739-1195 (JP); IKEDA, Masaki, Takata-shi, Hiroshima 739-1195 (JP); KAWAKUBO, Yohei, Takata-shi, Hiroshima 739-1195 (JP); UMETSU, Mina, Takata-shi, Hiroshima 739-1195 (JP); NAKANO, Shun-ichi, Takata-shi, Hiroshima 739-1195 (JP)
(74) Representative: Wächtershäuser & Hartz Patentanwaltspartnerschaft mbB
(86) International application number: PCT/JP2024/044954
(87) International publication number: WO 2025/135111

(57) **Abstract**

Provided is a novel compound which inhibits TGF-β signaling and is useful for treating or preventing fibrosis, obesity, and diabetes, suppressing cancer metastasis, and enhancing anti-tumor immunity.

A compound represented by the following formula (1), or a salt thereof: 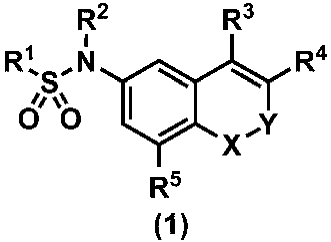 [wherein X is an oxygen atom, a sulfur atom or the like; Y is a carbonyl group, a thiocarbonyl group, a sulfoxide group or a sulfonyl group; R¹ is a lower alkyl group or the like which may have a substituent; R² is a hydrogen atom or the like; R³ is a hydrogen atom or the like; R⁴ is a hydrogen atom or the like; and R⁵ is a hydrogen atom or the like].

## Description

### Field of the Invention

The present invention relates to compounds that have TGF-β signaling inhibitory activity and are useful for treating or preventing fibrosis and the like, and uses thereof.

### Background of the Invention

Transforming growth factor-β (TGF-β) is a cytokine with a wide range of physiological activities, such as regulating cell proliferation and differentiation, repair and regeneration after tissue damage, and regulating the immune system and angiogenesis.

TGF-β signal transduction is thought to occur via the following pathways 1) to 3). 1) After binding to type II and type I TGF-β receptors on the cell surface, TGF-β activates activin receptor-like kinase 5 (ALK5), which is a serine/threonine kinase domain of the type I receptor, and phosphorylates Smad2/3 in the cytoplasm. 2) Phosphorylated Smad2/3 forms a complex with Smad4 (phosphorylated Smad), which translocates into the nucleus and then binds to specific response elements on DNA, where it acts as a transcriptional activator. 3) As a result, physiological effects are exerted by inducing the expression of various response genes such as α-smooth muscle actin (α-SMA), collagen, tissue inhibitor of metalloprotease (TIMP), and connective tissue growth factor (CTGF) (Non-Patent Literature 1). Here, nuclear localization of Smad2/3 is an essential process for TGF-β signal transduction, since TGF-β signaling is suppressed when a mutation is introduced into the nuclear localization signal of Smad3 (Non-Patent Literature 2).

Abnormalities in the TGF-β signaling pathway are known to be involved in the onset and progression of various diseases, including organ fibrosis, obesity, diabetes, cancer metastasis, and suppression of anti-tumor immunity.

Organ fibrosis is a disease in which excessive extracellular matrix accumulates in tissues due to chronic inflammation, etc., resulting in the replacement of normal tissue with the increased fibrous connective tissue, resulting in organ dysfunction. Progression of organ fibrosis can eventually lead to organ failure and death. Examples of organ fibrosis include liver fibrosis, pulmonary fibrosis, kidney fibrosis, skin fibrosis, cardiac fibrosis, and pancreatic fibrosis.

The common cause of organ fibrosis is thought to be an excessive repair response associated with chronic inflammation and tissue injury. In other words, various physiologically active substances are secreted from immune cells and other cells mobilized to damaged tissues, and TGF-β in particular promotes the proliferation of fibroblasts or hepatic stellate cells and their transformation into myofibroblast-like cells, as well as the production of large amounts of extracellular matrix such as collagen, leading to the deposition of fibers. At the same time, fibrolysis is suppressed by the production of TIMPs and other substances that inhibit proteases that degrade the extracellular matrix, which is thought to result in the progression of fibrosis (Non-Patent Literature 3).

Furthermore, it has been reported that TGF-β levels are elevated in patients with liver fibrosis, renal fibrosis, and pulmonary fibrosis compared to healthy individuals (Non-Patent Literature 4 to 6), and that enhanced fibrosis has also been reported in animal models in which TGF-β is artificially overexpressed (Non-Patent Literature 7). Thus, enhanced TGF-β signaling is closely related to organ fibrosis.

Regarding the relationship between TGF-β and obesity or diabetes, it has been reported that, for example, the amount of TGF-β in serum is positively correlated with body mass index (Non-Patent Literature 8), and that in high-fat diet-fed mice in which TGF-β signaling is blocked by deleting the Smad3 gene, body fat is reduced, glucose tolerance is improved, and fat accumulation in the liver is suppressed (Non-Patent Literature 9).

Regarding the relationship between TGF-β and cancer metastasis, it has been reported that, for example, TGF-β acts on epithelial cancer cells and induces the expression of Snail and ZEB through the activation of Smad2/3, thereby causing the cells to acquire mesenchymal traits with high motility and invasiveness, promoting metastasis from the primary lesion to other organs, and at the same time increasing resistance to anticancer drugs (Non-Patent Literature 10).

Regarding the relationship between TGF-β and anti-tumor immunity, it is known that TGF-β acts on receptors on naive T cells and induces Foxp3 expression via activation of Smad2/3, thereby promoting differentiation into regulatory T cells (Non-Patent Literature 11). It has been reported that these regulatory T cells suppress anti-tumor immunity mediated by cytotoxic T cells and the like in malignant tumors, promoting the progression of cancer (Non-Patent Literature 12).

Therefore, it is believed that the above diseases can be prevented and treated by inhibiting the TGF-β signaling pathway, and it has been reported that TGF-β neutralizing antibodies and ALK5 inhibitors have the effect of improving fibrosis (Non-Patent Literature 13-14), that when an ALK5 inhibitor is administered to a mouse model transplanted with highly metastatic breast cancer cells, the migration and infiltration of cancer cells as well as metastasis to the lungs is suppressed (Non-Patent Literature 15), and that when an immune checkpoint inhibitor and a TGF-β inhibitor antibody are administered in combination to a mouse model transplanted with malignant melanoma, urothelial cancer, or breast cancer cells, the anti-tumor immune response is enhanced and the tumors regress (Non-Patent Literature 16-17).

It has also been reported that certain sulfonamide derivatives are effective against fibrosis (Patent Literature 1 and 2) and have a TGF-β receptor binding inhibitory effect (Patent Literature 3).

(Patent Literature 1) WO 2015/141775
(Patent Literature 2) WO 2017/146309
(Patent Literature 3) WO 2006/070984

(Non-patent Literature 1) Cell 113(6), 685-700, 2003
(Non-patent Literature 2) Proc. Natl. Acad. Sci. U. S. A.97(14), 7853-7858, 2000
(Non-patent Literature 3) Liver Int. 26(1), 8-22, 2006
(Non-patent Literature 4) Hepatology 14, 269-273, 1991
(Non-patent Literature 5) Curr. Opin. Nephrol. Hypertens.3, 446-452, 1994
(Non-patent Literature 6) Arch. Bronconeumol. 42, 380-383, 2006
(Non-patent Literature 7) Am. J. Physiol. 276(4), G1059-68, 1999
(Non-patent Literature 8) Dis. Markers. 2009, 27, 23
(Non-patent Literature 9) Cell Metab. 2011, 14, 67
(Non-patent Literature 10) Ups. J. Med. Sci. 2012, 117, 143
(Non-patent Literature 11) J. Exp. Med. 2003, 198, 1875
(Non-patent Literature 12) Int. Immunol. 2016. 28, 401
(Non-patent Literature 13) Proc. Natl. Acad. Sci. U. S. A. 97(14), 8015-8020, 2000
(Non-patent Literature 14) Br J Pharmacol. 145(2), 166-177, 2005
(Non-patent Literature 15) Cancer Lett. 2014, 351, 72
(Non-patent Literature 16) Cancer Immunol. Res. 2018, 6, 1459
(Non-patent Literature 17) Nature 2018, 554, 544

### Summary of the Invention

The present invention relates to providing novel compounds that inhibit TGF-β signaling and are useful for treating or preventing fibrosis, obesity, and diabetes, inhibiting cancer metastasis, and enhancing anti-tumor immunity.

As a result of extensive research aimed at solving the above problems, the present inventors have found that a compound represented by the following formula (1) having a sulfonamide group at the 6-position potently inhibits TGF-β signaling by suppressing the nuclear localization of Smad2/3, thereby completing the present invention.

That is, the present invention relates to the following [1] to [16].
[1] A compound represented by the following formula (1), or a salt thereof: 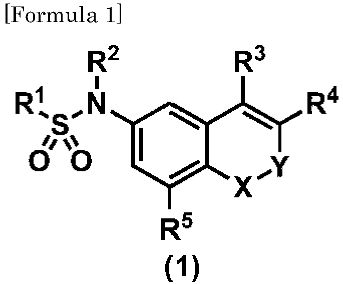
   [In the formula, X represents an oxygen atom, a sulfur atom, or a nitrogen atom which may have a substituent;
   Y represents a carbonyl group, a thiocarbonyl group, a sulfoxide group, or a sulfonyl group;
   R¹ represents an optionally substituted lower alkyl group, an optionally substituted aryl group, an optionally substituted 5- or 6-membered heteroaryl group, or an optionally substituted aralkyl group;
   R² represents a hydrogen atom, an optionally substituted lower alkyl group, a lower cycloalkyl group, a lower heterocycloalkyl group, an optionally substituted lower alkynyl group, an optionally substituted aralkyl group, or any of the groups shown in 1) to 4) below:
      1) The following formula (a): 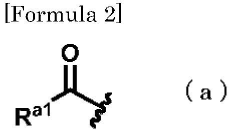 (In the formula, R^{a1} represents an optionally substituted lower alkyl group, a lower cycloalkyl group, an optionally substituted lower alkenyl group, an optionally substituted lower alkoxy group, an optionally substituted aralkyloxy group, or an optionally substituted aryl group, and the wavy line represents the bonding site.)
      2) The following formula (b): 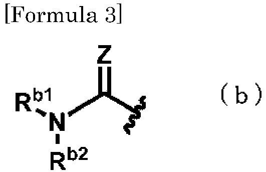 (In the formula, Z represents an oxygen atom or a sulfur atom; R^{b1} and R^{b2} may be the same or different and represent a hydrogen atom, an optionally substituted lower alkyl group, a lower cycloalkyl group, an optionally substituted aryl group, an optionally substituted 5- or 6-membered heteroaryl group, an optionally substituted aralkyl group or an optionally substituted heteroaralkyl group; or R^{b1} and R^{b2} together with the adjacent nitrogen atom form a 4- to 7-membered cyclic amino group which may contain a heteroatom; and the wavy line represents a bonding site.)
      3) The following formula (c): 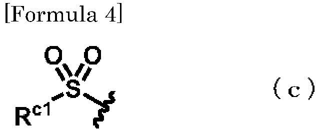 (In the formula, R^{c1} represents an optionally substituted lower alkyl group, a lower cycloalkyl group, a lower heterocycloalkyl group, an optionally substituted lower alkenyl group, an optionally substituted aryl group, an optionally substituted 5- or 6-membered heteroaryl group, an optionally substituted aralkyl group, or an optionally substituted heteroaralkyl group, and the wavy line represents the bonding site.)
      4) The following formula (d): 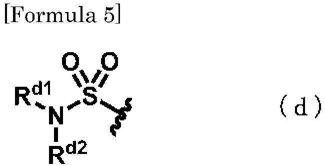 (In the formula, R^{d1} and R^{d2} may be the same or different and represent a hydrogen atom, an optionally substituted lower alkyl group, a lower cycloalkyl group, or an optionally substituted aralkyl group, or R^{d1} and R^{d2} together with the adjacent nitrogen atom form a 4- to 7-membered cyclic amino group which may contain a heteroatom, and the wavy line represents the bonding site.)
         R³ represents a hydrogen atom, a lower alkyl group, a lower cycloalkyl group, or an aryl group;
         R⁴ represents a hydrogen atom, a halogen atom, an optionally substituted lower alkyl group, a lower cycloalkyl group, an optionally substituted lower alkenyl group, an optionally substituted lower alkynyl group, a hydroxy group, an optionally substituted arylsulfinyl group, an optionally substituted lower alkoxy group, an amino group, a lower alkylamino group, a 4- to 7-membered cyclic amino group, a lower alkanoylamino group, a lower alkanoyl-lower alkylamino group, a lower alkoxycarbonylamino group, an aralkyloxycarbonylamino group, a lower alkoxycarbonyl group, a nitrile group, a carboxy group, a formyl group, an optionally substituted aryl group, an optionally substituted 5-or 6-membered heteroaryl group, an optionally substituted aralkyl group or an optionally substituted heteroaralkyl group;
         R⁵ represents a hydrogen atom, a halogen atom or a lower alkoxy group].
[2] The compound or salt thereof according to [1], wherein X is an oxygen atom, Y is a carbonyl group or a sulfonyl group, R¹ is an optionally substituted phenyl group, R² is a group represented by formula (c), R^{c1} is an optionally substituted phenyl group, R³ is a hydrogen atom, R⁴ is an optionally substituted phenyl group, and R⁵ is a hydrogen atom or a fluorine atom.
[3] The compound or salt thereof according to [2], wherein R¹ is a phenyl group or a phenyl group substituted with a C₁₋₇ alkyl group, and R^{c1} in formula (c) represented by R² is a phenyl group substituted with a C₁₋₇ alkyl group.
[4] A medicament containing the compound according to [1] or a salt thereof and a pharmaceutically acceptable carrier.
[5] A preventive or therapeutic agent for fibrosis, comprising the compound according to [1] or a salt thereof as an active ingredient.
[6] A TGF-β signal inhibitor comprising the compound according to [1] or a salt thereof as an active ingredient.
[7] An agent for suppressing nuclear localization of Smad2/3, comprising the compound according to [1] or a salt thereof as an active ingredient.
[8] Use of the compound according to [1] or a salt thereof for the production of an agent for preventing or treating fibrosis.
[9] Use of the compound according to [1] or a salt thereof for the production of a TGF-β signal inhibitor.
[10] Use of the compound according to [1] or a salt thereof for producing an inhibitor of nuclear localization of Smad2/3.
[11] The compound or salt thereof according to [1], which is used for preventing or treating fibrosis.
[12] The compound according to [1] or a salt thereof, which is used to inhibit TGF-β signaling.
[13] The compound or a salt thereof according to [1], which is used to inhibit the nuclear localization of Smad2/3.
[14] A method for preventing or treating fibrosis, which comprises administering the compound of [1] or a salt thereof to a subject.
[15] A method for inhibiting TGF-β signaling, which comprises administering the compound according to [1] or a salt thereof to a subject.
[16] A method for inhibiting nuclear localization of Smad2/3, which comprises administering the compound of [1] or a salt thereof to a subject.

### Advantageous Effects of Invention

The compound of the present invention has a strong TGF-β signal inhibitory effect by suppressing the nuclear localization of Smad2/3, and can be used as a preventive or therapeutic drug for fibrosis in the liver, kidney, lung, skin, etc.

### Brief Description of the Drawing

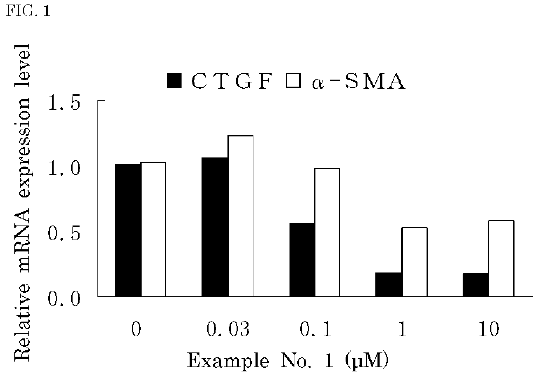

Fig. 1 is a graph showing the inhibitory effect of the compound of the present invention on Smad2/3 nuclear localization.

### Detailed Description of the Invention

The present invention will be described in detail below.

In this specification, unless otherwise specified, the term "lower" means that the number of carbon atoms in the hydrocarbon portion of the group to which this term is attached is 1 to 12 if the hydrocarbon portion is linear, or 3 to 12 if the hydrocarbon portion is cyclic, and that the linear hydrocarbon portion may be either a straight chain or a branched chain.

In this specification, the number of carbon atoms (x to y) in a hydrocarbon moiety is abbreviated as "C_{x-y}".

Furthermore, the phrase "optionally have a substituent" means that a hydrogen atom of the target group may be substituted with another group, and the number of the substituents may be one or more. When the target group has two or more substituents, the substituents may be the same or different.

Furthermore, the phrase "the ring may contain a heteroatom" means that one methylene chain on the ring may be replaced with a heteroatom.

The symbols used in formula (1) are explained below.

The "lower alkyl group" represented by R¹ is preferably a C₁₋₇ alkyl group, more preferably a C₁₋₄ alkyl group such as a methyl group, an ethyl group, a propyl group, an isopropyl group, a butyl group, an isobutyl group, or a tert-butyl group, and even more preferably a methyl group, an ethyl group, an isopropyl group, or a butyl group.

Examples of groups which can substitute on the lower alkyl group include amino groups, C₁₋₃ alkylamino groups (e.g., methylamino, ethylamino, dimethylamino), and C₁₋₃ alkanoylamino groups (e.g., acetylamino, ethylcarbonylamino).

The "aryl group" represented by R¹ includes C₆₋₁₀ aryl groups such as phenyl group and naphthyl group, and preferably a phenyl group.

Examples of substituents on the aryl group include a halogen atom (e.g., a fluorine atom), a hydroxy group, a C₁₋₁₂ alkyl group (e.g., a methyl group, an ethyl group, an isopropyl group, a butyl group), a halo C₁₋₃ alkyl group (e.g., a difluoromethyl group, a trifluoromethyl group, a 2,2,2-trifluoroethyl group), a C₁₋₁₂ alkoxy group (e.g., a methoxy group, an ethoxy group, an isopropoxy group, a butoxy group), and a nitro group.

Examples of the "5- or 6-membered heteroaryl group" represented by R¹ include a pyrrolyl group, a furyl group, a thienyl group, a pyrazolyl group, an imidazolyl group, a triazolyl group, an oxazolyl group, an isoxazolyl group, a thiazolyl group, a thiadiazolyl group, a pyridyl group, a pyrimidinyl group, and a 2,4-dioxo-1,2,3,4-tetrahydropyrimidinyl group. Of these, a pyridyl group is preferred, and a (2-, 3-, or 4-)pyridyl group is more preferred.

Examples of substituents on the heteroaryl group include C₁₋₁₂ alkyl groups (e.g., methyl, ethyl, isopropyl, butyl), halo C₁₋₃ alkyl groups (e.g., difluoromethyl, trifluoromethyl, 2,2,2-trifluoroethyl), C₁₋₁₂ alkoxy groups (e.g., methoxy, ethoxy, isopropoxy, butoxy), and C₁₋₁₂ alkylamino groups (e.g., methylamino, ethylamino, dimethylamino).

The "aralkyl group" represented by R¹ is preferably a phenyl C₁₋₃ alkyl group in which the aryl moiety is a benzene ring and the alkyl moiety is an alkyl group having 1 to 3 carbon atoms, such as a benzyl group, a 1-phenylethyl group, a 2-phenylethyl group, a 1-phenylisopropyl group, a 2-phenylisopropyl group, etc., and more preferably a benzyl group.

Examples of groups that can substitute on the aralkyl group include groups that can substitute on the aryl moiety of an aralkyl group, such as a halogen atom (e.g., a fluorine atom), a C₁₋₄ alkyl group (e.g., a methyl group, an ethyl group, an isopropyl group, a butyl group), and a halo C₁₋₃ alkyl group (e.g., a difluoromethyl group, a trifluoromethyl group, a 2,2,2-trifluoroethyl group).

The "lower alkyl group" represented by R² is preferably a C₁₋₇ alkyl group, more preferably a C₁₋₄ alkyl group such as a methyl group, an ethyl group, a propyl group, an isopropyl group, a butyl group, an isobutyl group, or a tert-butyl group, and even more preferably a methyl group, an ethyl group, an isopropyl group, or a butyl group.

Examples of groups that can substitute on the lower alkyl group include a halogen atom (e.g., a fluorine atom), a hydroxy group, a C₁₋₃ alkoxy group (e.g., a methoxy group, an ethoxy group), a C₃₋₇ cycloalkyl group (e.g., a cyclopropyl group, a cyclohexyl group), an amino group, a C₁₋₃ alkylamino group (e.g., a methylamino group, an ethylamino group, a dimethylamino group), a C₁₋₃ alkanoylamino group (e.g., an acetylamino group, an ethylcarbonylamino group), a 4- to 6-membered cyclic ether group (e.g., an oxetanyl group, a tetrahydrofuranyl group, a tetrahydropyranyl group, a dioxanyl group), a carboxy group, a C₁₋₄ alkoxycarbonyl group (e.g., a methoxycarbonyl group, an ethoxycarbonyl group), and a nitrile group.

The "lower cycloalkyl group" represented by R² is preferably a C₃₋₇ cycloalkyl group, more preferably a cyclopropyl group, a cyclobutyl group, a cyclopentyl group or a cyclohexyl group.

The "lower heterocycloalkyl group" represented by R² is preferably a C₂₋₆ heterocycloalkyl having one or more heteroatoms such as a nitrogen atom or an oxygen atom in the ring, and examples thereof include an azetidinyl group, an oxetanyl group, a pyrrolidinyl group, a piperidinyl group, etc., and preferably a 3-azetidinyl group, a 3-oxetanyl group, etc.

The "lower alkynyl group" represented by R² is preferably a C₂₋₄ alkynyl group, more preferably an ethynyl group.

Examples of groups that can substitute on the lower alkynyl group include C₁₋₇ alkyl groups (e.g., methyl, ethyl, n-propyl, n-butyl), alkoxycarbonyl groups (e.g., methoxycarbonyl, ethoxycarbonyl), carboxy groups, phenyl groups, and triisopropylsilyl groups.

The "aralkyl group" represented by R² is preferably a phenyl C₁₋₃ alkyl group in which the aryl moiety is a benzene ring and the alkyl moiety is an alkyl group having 1 to 3 carbon atoms, and is preferably a benzyl group.

Examples of groups that can substitute on the aralkyl group include groups that can substitute on the aryl moiety of an aralkyl group, such as a halogen atom (e.g., a fluorine atom), a C₁₋₄ alkyl group (e.g., a methyl group, an ethyl group, an isopropyl group, a butyl group), and a halo C₁₋₃ alkyl group (e.g., a difluoromethyl group, a trifluoromethyl group, a 2,2,2-trifluoroethyl group).

In formula (a), the "lower alkyl group" represented by R^{a1} is preferably a C₁₋₇ alkyl group, more preferably a C₁₋₄ alkyl group such as a methyl group, an ethyl group, a propyl group, an isopropyl group, a butyl group, an isobutyl group, or a tert-butyl group, and even more preferably a methyl group, an ethyl group, or an isopropyl group.

Examples of groups that can substitute on the lower alkyl group include halogen atoms (e.g., fluorine atom), hydroxy groups, C₁₋₄ alkoxy groups (e.g., methoxy, ethoxy), C₃₋₇ cycloalkyl groups (e.g., cyclopropyl, cyclohexyl), amino groups, C₁₋₄ alkylamino groups (e.g., methylamino, ethylamino, dimethylamino), C₁₋₃ alkanoylamino groups (e.g., acetylamino, ethylcarbonylamino), and 4- to 6-membered cyclic ether groups (e.g., oxetanyl, tetrahydrofuranyl, tetrahydropyranyl, dioxanyl).

The "lower cycloalkyl group" represented by R^{a1} is preferably a C₃₋₇ cycloalkyl group, more preferably a cyclopropyl group, a cyclobutyl group, a cyclopentyl group or a cyclohexyl group.

The "lower alkenyl group" represented by R^{a1} includes an aliphatic unsaturated hydrocarbon group having at least one carbon-carbon double bond, preferably a C₂₋₇ alkenyl group, more preferably a vinyl group, a 1-propenyl group, a 2-methyl-1-propenyl group, and a 1-methyl-1-propenyl group.

Examples of substituents on the lower alkenyl group include a halogen atom (e.g., a fluorine atom), a hydroxy group, a halo C₁₋₃ alkyl group (e.g., a difluoromethyl group, a trifluoromethyl group, a 2,2,2-trifluoroethyl group), and a phenyl group.

The "lower alkoxy group" represented by R^{a1} is preferably a C₁₋₇ alkoxy group, more preferably a C₁₋₄ alkoxy group such as a methoxy group, an ethoxy group, a propoxy group, an isopropoxy group, a butoxy group, an isobutoxy group, or a tert-butoxy group, and even more preferably a methoxy group, an ethoxy group, an isopropoxy group, or a tert-butoxy group.

Examples of substituents on the lower alkoxy group include a halogen atom (e.g., a fluorine atom), a hydroxy group, and a C₁₋₃ alkoxy group (e.g., a methoxy group, an ethoxy group, a methoxymethoxy group).

The "aralkyloxy group" represented by R^{a1} is preferably a phenyl C₁₋₃ alkoxy group in which the aryl moiety is a benzene ring and the alkoxy moiety is a C₁₋₃ alkoxy group, and is preferably a benzyloxy group.

Examples of groups that can substitute on the aralkyloxy group include groups that can substitute on the aryl moiety of an aralkyloxy group, such as a halogen atom (e.g., a fluorine atom), a hydroxy group, and a C₁₋₇ alkyl group (e.g., a methyl group, an ethyl group, an isopropyl group, a butyl group).

The "aryl group" represented by R^{a1} includes C₆₋₁₀ aryl groups such as phenyl group and naphthyl group, and preferably a phenyl group.

Examples of substituents on the aryl group include halogen atoms (e.g., fluorine atoms), hydroxy groups, C₁₋₇ alkyl groups (e.g., methyl groups, ethyl groups, isopropyl groups, butyl groups), halo C₁₋₃ alkyl groups (e.g., difluoromethyl groups, trifluoromethyl groups, 2,2,2-trifluoroethyl groups), and C₁₋₇ alkoxy groups (e.g., methoxy groups, ethoxy groups, isopropoxy groups, butoxy groups).

In formula (b), the "lower alkyl group" represented by R^{b1} and R^{b2} includes the same as those represented by R¹ , and examples of substituents on the lower alkyl group include a halogen atom (e.g., a fluorine atom), a C₁₋₃ alkoxy group (e.g., a methoxy group, an ethoxy group), a C₃₋₇ cycloalkyl group (e.g., a cyclopropyl group, a cyclohexyl group), an amino group, a C₁₋₃ alkylamino group (e.g., a methylamino group, an ethylamino group, a dimethylamino group), a C₁₋₃ alkanoylamino group (e.g., an acetylamino group, an ethylcarbonylamino group), a carboxy group, and a C₁₋₃ alkoxycarbonyl group (e.g., a methoxycarbonyl group, an ethoxycarbonyl group).

The "lower cycloalkyl group" represented by R^{b1} and R^{b2} includes the same groups as those represented by R^{a1}.

Examples of the "optionally substituted aryl group", "optionally substituted 5-or 6-membered heteroaryl group", and "optionally substituted aralkyl group" represented by R^{b1} and R^{b2} include the same as those represented by R¹.

The "heteroaralkyl group" represented by R^{b1} and R^{b2} is preferably a 5- or 6-membered heteroaryl C₁₋₂ alkyl group in which the heteroaryl moiety is a 5- or 6-membered heteroaryl and the alkyl moiety is an alkyl group having 1 to 2 carbon atoms, and as the 5- or 6-membered heteroaryl, a pyridyl group is preferable, and a (2-, 3-, or 4-)pyridyl group is more preferable.

Examples of groups that can substitute on the heteroaralkyl group include groups that can substitute on the heteroaryl moiety of a heteroaralkyl group, such as C₁₋₄ alkyl groups (e.g., methyl, ethyl, isopropyl, butyl) and halo C₁₋₃ alkyl groups.

Examples of the 4- to 7-membered cyclic amino group which may contain a heteroatom and which is formed by R^{b1} and R^{b2} together with the adjacent nitrogen atom include an azetidino group, a pyrrolidino group, a piperidino group, a piperazino group, and a morpholino group, and a morpholino group is preferred.

R^{b1} and R^{b2} are preferably an optionally substituted lower alkyl group, a lower cycloalkyl group, an optionally substituted aryl group, an optionally substituted 5- or 6-membered heteroaryl group, or a 4- to 7-membered cyclic amino group which may contain a heteroatom and which is formed by R^{b1} and R^{b2} together with the adjacent nitrogen atom; more preferably, either R^{b1} or R^{b2} is a C₁₋₃ alkyl group or a phenyl group, or R^{b1} and R^{b2} together form a morpholino group.

In formula (c), the "lower alkyl group" represented by R^{c1} is preferably a C₁₋₇ alkyl group, more preferably a C₁₋₄ alkyl group such as a methyl group, an ethyl group, a propyl group, an isopropyl group, a butyl group, an isobutyl group, or a tert-butyl group, and even more preferably a methyl group, an ethyl group, or an isopropyl group.

Examples of groups that can substitute on the lower alkyl group include a halogen atom (e.g., a fluorine atom), a hydroxy group, a C₁₋₃ alkoxy group (e.g., a methoxy group, an ethoxy group), a C₃₋₇ cycloalkyl group (e.g., a cyclopropyl group, a cyclohexyl group), an amino group, a C₁₋₃ alkylamino group (e.g., a methylamino group, an ethylamino group, a dimethylamino group, a trimethylammonium group), a C₁₋₃ alkanoylamino group (e.g., an acetylamino group, an ethylcarbonylamino group), a 4- to 6-membered cyclic ether group (e.g., an oxetanyl group, a tetrahydrofuranyl group, a tetrahydropyranyl group, a dioxanyl group), a carboxy group, a C₁₋₃ alkoxycarbonyl group (e.g., a methoxycarbonyl group, or an ethoxycarbonyl group), a 1,3-dioxoisoindolin-2-yl group, and a sulfonyl group (e.g., a methanesulfonyl group, a toluenesulfonyl group).

The "lower cycloalkyl group" represented by R^{c1} is preferably a C₃₋₇ cycloalkyl group, more preferably a cyclopropyl group, a cyclobutyl group, a cyclopentyl group or a cyclohexyl group.

The "lower heterocycloalkyl" represented by R^{c1} is preferably a C₂₋₆ heterocycloalkyl having one or more heteroatoms such as a nitrogen atom or an oxygen atom in the ring, and examples thereof include an azetidinyl group, an oxetanyl group, a pyrrolidinyl group, and a piperidinyl group.

The "lower alkenyl group" represented by R^{c1} is preferably a C₂₋₇ alkenyl group, more preferably a vinyl group, a 1-propenyl group, an isopropenyl group, a 2-methyl-1-propenyl group, a 1-methyl-1-propenyl group, and the like.

Examples of substituents on the lower alkenyl group include a halogen atom (e.g., a fluorine atom), a hydroxy group, a halo C₁₋₃ alkyl group (e.g., a difluoromethyl group, a trifluoromethyl group, a 2,2,2-trifluoroethyl group), and a phenyl group.

The "aryl group" represented by R^{c1} includes C₆₋₁₀ aryl groups such as phenyl and naphthyl groups, and preferably a phenyl group.

Examples of substituents on the aryl group include a halogen atom (e.g., a fluorine atom), a hydroxy group, a C₁₋₁₂ alkyl group (e.g., a methyl group, an ethyl group, an isopropyl group, a butyl group), a halo C₁₋₃ alkyl group (e.g., a difluoromethyl group, a trifluoromethyl group, a 2,2,2-trifluoroethyl group), a C₁₋₄ alkyl group having as a substituent a group selected from a methylamino group, a dimethylamino group, a trimethylammonium group, an N,O-dimethylamino group, an N-methyl-N-hydroxyamino group, a 1-piperidinyl group, a 4-methylpiperazinyl group, a 1-morpholino group, and a methanesulfonyl group, a vinyl group having as a substituent a C₁₋₃ alkylsulfonyl group (e.g., a methanesulfonylethenyl group), a C₁₋₁₂ alkoxy group (e.g., a methoxy group, an ethoxy group, an isopropoxy group, a butoxy group, a butan-2-yloxy group, a 2-methylpropyloxy group), a C₁₋₃ alkoxy group having as a substituent a group selected from an amino group, a C₁₋₄ alkanoylamino group (e.g., an acetylamino group), a 4-oxo-4-[2-(2-methoxyethoxy)ethylamino]butanoylamino group, 1 to 3 fluorine atoms, a C₁₋₄ alkoxy group (e.g., a methoxy group), a 4-[2,2-dimethyl-1,3-dioxolan-4-yl] group, 1 to 3 hydroxy groups, a C₁₋₄ alkylamino group (e.g., a methylamino group), a 4-[2-(2-{5-[(3aR,6aS)-2-oxo-hexahydro-1H-thieno[3,4-d]imidazolidin-4-yl]pentanamido}ethoxy)] group, and a 4-(2-{2-[({3',6'-dihydroxy-3-oxo-3H-spiro[2-benzofuran-1,9'-xanthen]-5-yl}carbamothioyl)amino]ethoxy}) group, an amino group, a C₁₋₁₂ alkylamino group (e.g., a methylamino group, an ethylamino group, a dimethylamino group), an N-C₁₋₃ alkyl-C₁₋₅ alkoxycarbonylamino group (e.g., an N-methylmethoxycarbonylamino group, an N-methyl-(2R,3R,4S,5R)-3,4,5,6-tetrahydroxyoxan-2-ylmethoxycarbonylamino group, an N-methyl-(2S)-2-amino-2-carboxy-n-butoxycarbonylamino group, an N-methyl-2-{[2-(2-methoxy)ethoxy]ethoxy}ethoxycarbonylamino group, an N-methyl-(5-methyl-2-oxo-2H-1,3-dioxol-4-yl)methoxycarbonylamino group), an N-(C₁₋₃ alkyl)aryloxycarbonylamino group (e.g., an N-methyl-3-fluorophenoxycarbonylamino group, a (2S)-2-amino-2-carboxyethylphenoxycarbonylamino group), a ureido group having as a substituent a 3,3-di(C₁₋₃ alkyl)-1-C₁₋₃ alkyl group (e.g., a 3,3-diethyl-1-methylureido group), an N-(C₁₋₃ alkyl)formylamino group (e.g., an N-methylformylamino group), a phosphorylamino group having as a substituent a group selected from an N-(C₁₋₃ alkyl) group and an N-(C₁₋₃ alkyl)-O-di(C₁₋₃ alkyl) group (e.g., an N-methylphosphorylamino group, an N-methyl-diethylphosphorylamino group), a C₁₋₃ alkanoylamino group (e.g., an acetylamino group, an ethylcarbonylamino group), a nitrile group, a nitro group, a carboxy group, a formyl group, a hydroxyimino group, an imino group having as a substituent a C₁₋₃ alkoxy group (e.g., a methoxyimino group), a C₁₋₄ alkoxycarbonyl group (e.g., a methoxycarbonyl group, an ethoxycarbonyl group), an aminocarbonyl group having as a substituent a group selected from an N-(ethynyl C₁₋₃ alkyl) group, an N-(hydroxy C₁₋₃ alkyl) group, and an N,N-di C₁₋₃ alkyl group (e.g., a propargylaminocarbonyl group, an N,N-diethylaminocarbonyl group, a 2-hydroxyethylaminocarbonyl group), and a 1-morpholinocarbonyl group.

Examples of the "5- or 6-membered heteroaryl group" represented by R^{c1} include a pyrrolyl group, a furyl group, a thienyl group, a pyrazolyl group, an imidazolyl group, a triazolyl group, an oxazolyl group, an isoxazolyl group, a thiazolyl group, a thiadiazolyl group, a pyridyl group, a pyrimidinyl group, and a 2,4-dioxo-1,2,3,4-tetrahydropyrimidinyl group. Of these, a 5- or 6-membered nitrogen-containing heteroaryl group such as a pyrazolyl group, an imidazolyl group, a pyridyl group, a pyrimidinyl group, or a 2,4-dioxo-1,2,3,4-tetrahydropyrimidinyl group is preferred, and a (3- or 4-)pyrazolyl group or a (2-, 3-, or 4-)pyridyl group is more preferred.

Examples of substituents on the 5- or 6-membered heteroaryl group include a halogen atom (e.g., a fluorine atom, a chlorine atom), a hydroxy group, a C₁₋₁₂ alkyl group (e.g., a methyl group, an ethyl group, an isopropyl group, a butyl group), a halo C₁₋₃ alkyl group (e.g., a difluoromethyl group, a trifluoromethyl group, a 2,2,2-trifluoroethyl group), a C₁₋₁₂ alkoxy group (e.g., a methoxy group, an ethoxy group, an isopropoxy group, a butoxy group), an amino group, a C₁₋₁₂ alkylamino group (e.g., a methylamino group, an ethylamino group, a dimethylamino group), an amino group optionally having as a substituent a group selected from a hydroxy group, a C₁₋₃ alkyl group, and a C₁₋₄ alkoxy group, a 5- or 6-membered cyclic amino group optionally having as a substituent a group selected from a C₁₋₃ alkoxy group and a C₁₋₃ alkylamino group (e.g., a (3R)-methoxy-(4R)-dimethylaminopyrrolidinyl group), a nitrile group, a nitro group, a carboxy group, a formyl group, and a C₁₋₄ alkoxycarbonyl group (e.g., a methoxycarbonyl group, an ethoxycarbonyl group).

The "aralkyl group" represented by R^{c1} is preferably a phenyl C₁₋₃ alkyl group in which the aryl moiety is a benzene ring and the alkyl moiety is an alkyl group having 1 to 3 carbon atoms, such as a benzyl group, a 1-phenylethyl group, a 2-phenylethyl group, a 1-phenylisopropyl group, a 2-phenylisopropyl group, etc., and more preferably a benzyl group.

The "heteroaralkyl group" represented by R^{c1} is preferably a 5- or 6-membered heteroaryl C₁₋₂ alkyl group in which the heteroaryl moiety is a 5- or 6-membered heteroaryl and the alkyl moiety is an alkyl group having 1 to 2 carbon atoms, and the 5-or 6-membered heteroaryl is more preferably a (2-, 3-, or 4-)pyridyl group.

Examples of groups that can substitute on the aralkyl group or heteroaralkyl group include groups that can substitute on the aryl moiety of an aralkyl group or heteroaralkyl group, such as a halogen atom (e.g., a fluorine atom), a C₁₋₃ alkyl group (e.g., a methyl group, an ethyl group), a halo C₁₋₃ alkyl group (e.g., a difluoromethyl group, a trifluoromethyl group), a C₁₋₃ alkoxy group (e.g., a methoxy group, an ethoxy group, an isopropoxy group), a halo C₁₋₃ alkoxy group (e.g., a difluoromethoxy group, a trifluoromethoxy group), a hydroxyl group, a nitrile group, a nitro group, and an amino group.

R^{c1} is preferably an optionally substituted lower alkyl group, a lower cycloalkyl group, or an optionally substituted aryl group, more preferably an optionally substituted phenyl group, and even more preferably a phenyl group substituted with a C₁₋₇ alkyl group.

In formula (d), examples of the "lower alkyl group" represented by R^{d1} and R^{d2} include the same as those represented by R¹, and examples of substituents on the lower alkyl group include a halogen atom (e.g., a fluorine atom), a C₁₋₃ alkoxy group (e.g., a methoxy group, an ethoxy group), a C₃₋₇ cycloalkyl group (e.g., a cyclopropyl group, a cyclohexyl group), a carboxy group, a C₁₋₃ alkoxycarbonyl group (e.g., a methoxycarbonyl group, an ethoxycarbonyl group). A halogen atom and a C₁₋₃ alkoxy group are preferred.

The "lower cycloalkyl group" represented by R^{d1} and R^{d2} includes the same as those represented by R², with a cyclopropyl group being more preferred.

The "aralkyl group" represented by R^{d1} and R^{d2} is preferably a phenyl C₁₋₃ alkyl group in which the aryl moiety is a benzene ring and the alkyl moiety is an alkyl group having 1 to 3 carbon atoms, more preferably a 2-phenethyl group.

Examples of groups that can substitute on the aralkyl group include groups that can substitute on the aryl moiety of an aralkyl group, such as a halogen atom (e.g., a fluorine atom), a C₁₋₄ alkyl group (e.g., a methyl group, an ethyl group, an isopropyl group, a butyl group), and a halo C₁₋₃ alkyl group (e.g., a difluoromethyl group, a trifluoromethyl group, a 2,2,2-trifluoroethyl group).

Examples of the 4- to 7-membered cyclic amino group optionally containing a heteroatom, which may be formed by R^{d1} and R^{d2} together with the adjacent nitrogen atom, include an azetidino group, a pyrrolidino group, a piperidino group, a piperazino group, and a morpholino group.

R^{d1} and R^{d2} are preferably a lower alkyl group or a lower cycloalkyl group which may have a substituent, or a 4- to 7-membered cyclic amino group which may contain a heteroatom, formed together with the adjacent nitrogen atom, and more preferably a C₁₋₃ alkyl group, or a morpholino group formed together with the adjacent nitrogen atom.

The "lower alkyl group" represented by R³ is preferably a C₁₋₇ alkyl group, more preferably a C₁₋₄ alkyl group such as a methyl group, an ethyl group, a propyl group, an isopropyl group, a butyl group, an isobutyl group, or a tert-butyl group, and even more preferably a methyl group.

The "lower cycloalkyl group" represented by R³ is preferably a C₃₋₇ cycloalkyl group, including a cyclopropyl group, a cyclobutyl group, a cyclopentyl group, and a cyclohexyl group, with a cyclopropyl group being more preferred.

The "aryl group" represented by R³ includes C₆₋₁₀ aryl groups such as phenyl group and naphthyl group, and preferably a phenyl group.

R³ is preferably a hydrogen atom or a C₁₋₃ alkyl group, more preferably a hydrogen atom or a methyl group.

The "halogen atom" represented by R⁴ includes fluorine, chlorine, bromine and iodine.

The "lower alkyl group" represented by R⁴ is preferably a C₁₋₇ alkyl group, more preferably a C₁₋₄ alkyl group such as a methyl group, an ethyl group, a propyl group, an isopropyl group, a butyl group, an isobutyl group, or a tert-butyl group, and even more preferably a methyl group, an ethyl group, or an isopropyl group.

Examples of substituents on the lower alkyl group include a halogen atom (e.g., a fluorine atom), a hydroxy group, a C₁₋₃ alkoxy group (e.g., a methoxy group, an ethoxy group), a C₃₋₇ cycloalkyl group (e.g., a cyclopropyl group, a cyclohexyl group), an amino group, a C₁₋₃ alkylamino group (e.g., a methylamino group, an ethylamino group, a dimethylamino group), a C₁₋₃ alkanoylamino group (e.g., an acetylamino group, an ethylcarbonylamino group), and a 4- to 6-membered cyclic ether group (e.g., an oxetanyl group, a tetrahydrofuranyl group, a tetrahydropyranyl group, a dioxanyl group).

The "lower cycloalkyl group" represented by R⁴ is preferably a C₃₋₇ cycloalkyl group, more preferably a cyclopropyl group, a cyclobutyl group, a cyclopentyl group or a cyclohexyl group.

The "lower alkenyl group" represented by R⁴ is preferably a C₂₋₄ alkenyl group, more preferably a vinyl group, an allyl group, or an isopropenyl group.

Examples of groups that can substitute on the lower alkenyl group include C₁₋₃ alkyl groups (e.g., methyl, ethyl), alkoxycarbonyl groups (e.g., methoxycarbonyl, ethoxycarbonyl, tert-butoxycarbonyl), carboxy groups, and phenyl groups.

The "lower alkynyl group" represented by R⁴ is preferably a C₂₋₄ alkynyl group, more preferably an ethynyl group.

Examples of groups that can substitute on the lower alkynyl group include C₁₋₇ alkyl groups (e.g., methyl, ethyl, n-propyl, n-butyl), alkoxycarbonyl groups (e.g., methoxycarbonyl, ethoxycarbonyl), carboxy groups, phenyl groups, and triisopropylsilyl groups.

The "aryl group" of the substituted arylsulfinyl group represented by R⁴ includes C₆₋₁₀ aryl groups such as phenyl and naphthyl, and preferably a phenyl.

Examples of substituents on the aryl group include C₁₋₃ alkyl groups (e.g., methyl groups).

The "lower alkoxy group" represented by R⁴ includes a C₁₋₄ alkoxy group, more preferably a methoxy group, an ethoxy group, an isopropoxy group, or a tert-butoxy group.

Examples of substituents on the lower alkoxy group include a halogen atom (e.g., a fluorine atom), a hydroxy group, and a C₁₋₃ alkoxy group (e.g., a methoxy group, an ethoxy group, a methoxymethoxy group).

The "lower alkylamino group" represented by R⁴ is preferably a mono- or di- C₁₋₃ alkylamino group, such as a methylamino group, a dimethylamino group, a methylethylamino group, or an isopropylamino group.

Examples of the "4- to 7-membered cyclic amino group" represented by R⁴ include 4- to 7-membered saturated cyclic amino groups such as azetidino, pyrrolidino, piperidino, hexahydroazepino, morpholino, and piperazino.

The "lower alkanoylamino group" represented by R⁴ is preferably a C₁₋₃ alkanoylamino group, more preferably an acetylamino group.

The "lower alkanoyl-lower alkylamino group" represented by R⁴ is preferably an N-C₁₋₃ alkanoyl-N-C₁₋₃ alkylamino group, more preferably an N-acetyl-N-methylamino group.

The "lower alkoxycarbonylamino group" represented by R⁴ is preferably a C₁₋₄ alkoxycarbonylamino group, more preferably a tert-butoxycarbonylamino group.

The "aralkyloxycarbonylamino group" represented by R⁴ is preferably an aralkyloxycarbonylamino group in which the aryl moiety is a benzene ring and the alkyl moiety has 1 to 3 carbon atoms (phenyl C₁₋₃ alkoxycarbonylamino group), and is preferably a benzyloxycarbonylamino group.

The "lower alkoxycarbonyl group" represented by R⁴ is preferably a C₁₋₃ alkoxycarbonyl group, more preferably a methoxycarbonyl group or an ethoxycarbonyl group.

The "aryl group" represented by R⁴ includes C₆₋₁₀ aryl groups such as phenyl group and naphthyl group, and preferably a phenyl group.

Examples of the "heteroaryl group" represented by R⁴ include 5- or 6-membered heteroaryl groups, such as a furyl group, a thienyl group, a pyrazolyl group, an oxazolyl group, an isoxazolyl group, a thiazolyl group, and a pyridyl group, and among these, a 3-pyrazolyl group, a 2-furyl group, or a (2-, 3-, or 4-)pyridyl group is more preferred.

Examples of substituents on the aryl group or heteroaryl group include a halogen atom (e.g., a fluorine atom, a chlorine atom), a hydroxy group, C₁₋₃ alkyl group (e.g., a methyl group, an ethyl group, a propyl group, an isopropyl group), a halo C₁₋₃ alkyl group (e.g., a difluoromethyl group, a trifluoromethyl group), a C₁₋₃ alkylamino C₁₋₃ alkyl group (e.g., a methylaminomethyl group, a dimethylaminomethyl group), a C₁₋₄ alkoxy group (e.g., a methoxy group, an ethoxy group, an isopropoxy group, an n-butoxy group), a C₁₋₃ alkoxy group having as a substituent a group selected from an amino group, a C₁₋₃ alkanoylamino group (e.g., an acetylamino group), an N-methyl C₁₋₃ alkanoylamino group (e.g., an N-methylacetylamino group), a C₁₋₃ alkylamino group (e.g., a methylamino group), a 4-oxo-4-[2-(2-methoxyethoxy)ethylamino]butanoylamino group, 1 to 3 fluorine atoms, a C₁₋₃ alkoxy group (e.g., a methoxy group), a 2,2-dimethyl-1,3-dioxolan-2-yl group, and a hydroxy group (e.g., a 2,3-dihydroxypropyloxy group), an amino group, a C₁₋₃ alkylamino group (e.g., a methylamino group, an ethylamino group, a dimethylamino group), a C₁₋₁₈ alkanoylamino group (e.g., an acetylamino group, an ethylcarbonylamino group, an octadecanoylamino group), an N-(C₁₋₃ alkyl)-C₁₋₄ alkyloxycarbonylamino group optionally having a 5-methyl-2-oxo-2H-1,3-dioxol-4-yl group as a substituent, a nitrile group, a nitro group, a carboxy group, a C₁₋₄ alkoxycarbonyl group (e.g., a methoxycarbonyl group, an ethoxycarbonyl group), an N,N-di(C₁₋₃ alkyl)aminocarbonyl group (e.g., an N,N-diethylaminocarbonyl group), a C₁₋₃ alkylaminocarbonyl group optionally having a hydroxy group as a substituent (e.g., a 2-hydroxyethylaminocarbonyl group), an aminocarbonyl group, a 1-morpholinocarbonyl group, a C₁₋₃ alkanoyloxy group (e.g., an acetoxy group), and an aralkyloxy group (e.g., a benzyloxy group).

The "aralkyl group" represented by R⁴ is preferably a phenyl C₁₋₃ alkyl group in which the aryl moiety is a benzene ring and the alkyl moiety is an alkyl group having 1 to 3 carbon atoms, such as a benzyl group, a 2-phenylpropan-2-yl group, a 1-phenylethyl group, a 2-phenylethyl group, a 1-phenylisopropyl group, a 2-phenylisopropyl group, etc., and more preferably a benzyl group or a 2-phenylethyl group.

The "heteroaralkyl group" represented by R⁴ is preferably a 5- or 6-membered heteroaryl C₁₋₂ alkyl group in which the heteroaryl moiety is a 5- or 6-membered heteroaryl and the alkyl moiety is an alkyl group having 1 or 2 carbon atoms, more preferably a (2-, 3-, or 4-)pyridyl group.

Examples of substituents on the aryl or heteroaryl group include a halogen atom (e.g., a fluorine atom), a C₁₋₃ alkyl group (e.g., methyl, ethyl), a hydroxy group, a C₁₋₁₀ alkanoyloxy group (e.g., acetyl, n-propanoyloxy, n-octanoyloxy), a C₁₋₄ alkanoyloxy group having as a substituent a C₃₋₇ alkoxy group having an ether bond (e.g., -O(CH₂)₂OCH₃, - O(CH₂)₂O(CH₂)₂OCH₃, -O(CH₂)₂O(CH₂)₂O(CH₂)₂OCH₃, -O(CH₂)₃OCH₃, - O(CH₂)₃O(CH₂)₃OCH₃, -OCH₂OCH₃), a C₁₋₃ alkoxy group (e.g., a methoxy group, an ethoxy group, an n-butoxy group), a phosphonooxymethoxy group, and a phosphonooxy group.

R⁴ is preferably a lower alkyl group which may have a substituent, a C₆₋₁₀ aryl group or an aralkyl group, and more preferably a phenyl group.

The lower alkoxy group represented by R⁵ is preferably a C₁₋₃ alkoxy group, such as a methoxy group, an ethoxy group, or an n-propoxy group.

Of the compounds represented by formula (1) shown above, it is preferable that X is an oxygen atom and Y is a carbonyl group or a sulfonyl group, and furthermore, it is preferable that R¹ is an optionally substituted phenyl group, preferably a phenyl group which may have a C₁₋₇ alkyl group, more preferably a phenyl group, R² is a group represented by formula (c), and preferably, in formula (c), R^{c1} is a C₆₋₁₀ aryl group which may have a substituent, preferably an optionally substituted phenyl group, more preferably a phenyl group which may have a C₁₋₇ alkyl group, R³ is a hydrogen atom or a methyl group, preferably a hydrogen atom, R⁴ is an optionally substituted phenyl group, and R⁵ is a hydrogen atom or a fluorine atom.

The compound of the present invention represented by formula (1) can form a salt with an acidic or basic compound, and these salts are also included in the compounds of the present invention.

The compounds of formula (1) of the present invention can form base addition salts with acidic moieties (such as, but not limited to, carboxylic acids) in the compounds. Examples of base addition salts include (A) salts with alkali metals such as sodium and potassium, (B) salts with alkaline earth metals such as calcium and magnesium, (C) ammonium salts, (D) salts with nitrogen-containing organic bases such as triethylamine, pyridine, N-methylmorpholine, dimethylamine, 2-aminoethane-1-ol, diisopropylethylamine, and 1,1,3,3-tetramethylguanidine, and (E) salts with basic amino acids such as arginine and lysine.

The compounds of formula (1) of the present invention can form acid addition salts with basic moieties in the compounds (such as, but not limited to, pyridine or amino groups). Examples of acid addition salts include (A) salts with inorganic acids such as hydrochloric acid, sulfuric acid, and phosphoric acid; (B) salts with carboxylic acids such as acetic acid, citric acid, and benzoic acid; (C) salts with vinylic carboxylic acids such as Meldrum's acid and ascorbic acid; (D) salts with sulfonic acids such as paratoluenesulfonic acid, 10-camphorsulfonic acid, and trifluoromethanesulfonic acid; and (E) salts with acidic amino acids such as aspartic acid and glutamic acid.

The compounds of formula (1) of the present invention can exist not only in unsolvated form but also as hydrates or solvates. Therefore, all crystalline forms and hydrates or solvates thereof are included as part of the present invention.

The compounds of the present invention represented by formula (1) may exist in their tautomeric forms (e.g., keto-enol tautomers), and all tautomeric forms are encompassed within the scope of the present invention.

The compound of the present invention represented by formula (1) can exist as a stereoisomer (e g., enantiomer or diastereomer). Therefore, all stereoisomers are included as part of the present invention. Furthermore, each stereoisomer of the sulfonamide derivative of the present invention may be present either substantially pure, for example free from other isomers, or as a mixture, for example, a racemate.

The compound of the present invention represented by formula (1) can exist as a geometric isomer (E or Z), and both geometric isomers are encompassed in the compound of the present invention.

The compound of the present invention represented by formula (1) can basically be produced by the following methods of steps 1 to 8, and can be produced by any method suitable for the type of substituents, etc., after appropriate modification.

### (Step 1)

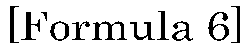

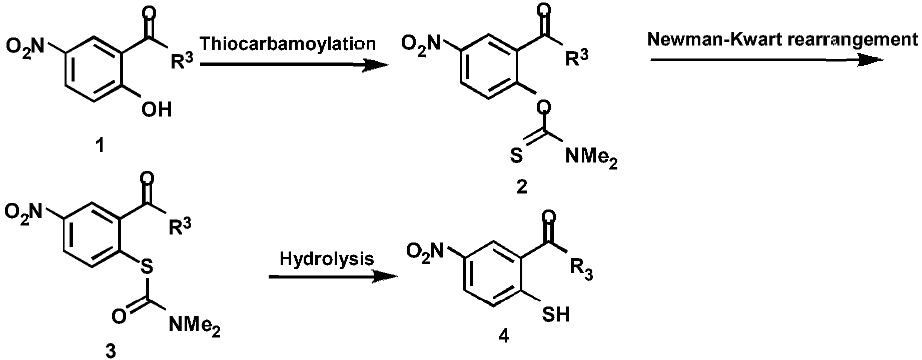

That is, compound 1 is carbamoylated to give compound 2, which is then converted to compound 3 by Newman-Kwart rearrangement, and then the aldehyde 4 can be obtained by hydrolysis.

Compound 1 is converted to compound 2 with the corresponding thiocarbonyl chloride or the like in the presence of a basic compound in an appropriate solvent.

The solvent used for the thiocarbamoylation may be any solvent that does not affect the reaction, and examples thereof include esters (e.g., ethyl acetate, butyl acetate), ethers (e.g., diethyl ether, tetrahydrofuran), halogenated hydrocarbons (e.g., dichloromethane, chloroform, 1,2-dichloroethane), alcohols (e.g., methanol, ethanol), and water, or a mixed solvent thereof. Examples of the basic compound used in this reaction include organic bases such as triethylamine, pyridine, N-methylpyrrolidine, 1,8-diazabicyclo[5.4.0]undec-7-ene (DBU), and 1,4-diazabicyclo[2.2.2]octane (DABCO), among which DABCO and triethylamine are more preferred.

The reaction is generally carried out at a temperature of from -20°C to 50°C, preferably from 0°C to 20°C, and the reaction time is generally from several minutes to 48 hours, preferably from 1 to 24 hours.

Compound 2 is converted to compound 3 by heating in a suitable solvent or in the absence of a solvent. This Newman-Kwart rearrangement can be carried out with reference to J. Org. Chem. 1966, 31, 12, 3980-3984. That is, any solvent may be used as long as it does not affect the reaction, and examples thereof include polar solvents such as N,N-dimethylformamide, N,N-dimethylacetamide, and N-methylpyrrolidone, and aromatic hydrocarbons such as toluene and xylene. The reaction is generally carried out at 100°C. to 300°C., preferably 100°C. to 200°C., and the reaction time is generally 1 hour to 48 hours, preferably 12 to 24 hours.

Compound 3 is converted to compound 4 by alkaline hydrolysis in a suitable solvent. A commonly used hydrolysis reaction can be applied; for example, it can be carried out using an inorganic base such as sodium hydroxide or potassium hydroxide in an alcohol solution. This reaction is usually carried out at 0 to 150°C, preferably room temperature to 100°C, and the reaction time is usually 10 minutes to 48 hours.

### (Step 2)

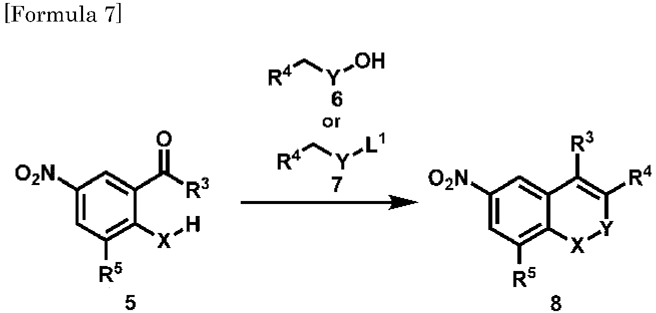

[In the formula, X represents an oxygen atom or a sulfur atom, Y represents a carbonyl group or a sulfonyl group, L¹ represents a functional group that easily leaves such as a halogen atom, and R³, R⁴, and R⁵ are the same as those defined above.]

That is, compound 5 can be condensed with compound 6 and then subjected to cyclization conditions to give compound 8.

Alternatively, compound 5 can be reacted with compound 7 and then subjected to cyclization conditions to give compound 8.

Compound 5 can be converted to compound 8 by the method described in the following literature or a method similar thereto.
1) J. Med. Chem. 1964, 7, 446.
2) Synth. Commun. 1999, 29, 1451.
3) J. Chem. Res. 2006, 688.
4) Tetrahedron Lett. 2007, 48, 5077.
5) Tetrahedron Lett. 2012, 53, 4422.
6) Synlett 2017, 28, 825.
7) Bioorg. Med. Chem. 2015, 23, 12, 2895.
8) Tetrahedron, 2015, 71, 5, 863.
9) Tetrahedron, 2012, 68, 5541.
10) ChemMedChem 2017, 12, 1578.
11) Lett. Drug Des. Discov. 2013, 10, 410.
12) Bioorg. Med. Chem. 2020, 28.
13) J. Med. Chem. 2015, 58, 3975.

### (Step 3)

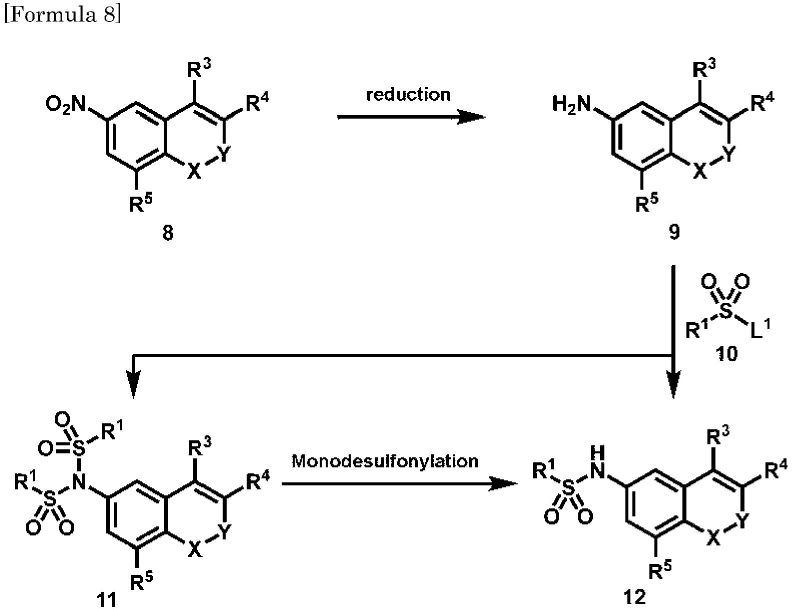

[In the formula, L¹, R¹, R³ and R⁴ are the same as defined above.]

That is, compound 8 is subjected to reduction conditions to give compound 9, which is then reacted with sulfonyl halides 10 to give compound 12 in one step. Alternatively, compound 9 can be reacted with two equivalents of sulfonyl halide 10 to give compound 11, which can then be monodesulfonylated to give compound 12.

Compound 8 is converted to compound 9 by catalytic reduction with a heterogeneous catalyst or reduction with a metal and an acid in a suitable solvent.

The solvent used in the catalytic reduction using a heterogeneous catalyst may be any solvent that does not affect the reaction, and examples thereof include esters (e.g., ethyl acetate, butyl acetate), ethers (e.g., diethyl ether, tetrahydrofuran), halogenated hydrocarbons (e.g., dichloromethane, chloroform, 1,2-dichloroethane), alcohols (e.g., methanol, ethanol), and water, or a mixed solvent thereof may also be used. Examples of the metal catalyst that can be used include palladium, platinum, nickel, and rhodium, with palladium being preferred. The reaction is generally carried out at room temperature to 80°C, preferably room temperature to 50°C, and the reaction time is generally from several minutes to 48 hours, preferably from 1 to 24 hours. This reaction may also be carried out in the presence of an acid such as hydrochloric acid or a carboxylic acid.

The solvent used in the reduction with a metal and an acid may be any solvent that does not affect the reaction, and examples thereof include ethers (e.g., diethyl ether, tetrahydrofuran), alcohols (e.g., methanol, ethanol), and water, or a mixed solvent thereof. Examples of the metal that can be used include iron, tin, and zinc, with iron being preferred. The acid to be used includes hydrochloric acid, acetic acid, and ammonium chloride. The reaction is generally carried out at room temperature to 100°C, preferably 50°C to 80°C, and the reaction time is generally from several minutes to 24 hours, preferably 30 minutes to 12 hours.

The synthesis of compound 12 by monosulfonylation of compound 9 is carried out in the presence of a basic compound in a suitable solvent. The solvent used in this reaction may be any solvent that does not affect the reaction, and examples thereof include aromatic hydrocarbons (e.g., toluene, xylene), ethers (e.g., diethyl ether, tetrahydrofuran), and halogenated hydrocarbons (e.g., dichloromethane, chloroform, 1,2-dichloroethane), with halogenated hydrocarbons being preferred. Examples of the basic compound used in this reaction include organic bases such as triethylamine, pyridine, N-methylpyrrolidine, 1,8-diazabicyclo[5.4.0]undec-7-ene (DBU), 1,4-diazabicyclo[2.2.2]octane (DABCO), etc., with pyridine being more preferred.

The amount of compound 10 used is preferably equimolar or more, particularly about 1 to 3 times the molar amount of compound 9.

The reaction is generally carried out at 0 to 80°C, preferably room temperature to 50°C, and the reaction time is generally from several minutes to 48 hours, preferably from 1 to 24 hours.

The synthesis of compound 11 by sulfonylation of compound 9 is carried out in the presence of a basic compound in a suitable solvent. The solvent used in this reaction may be any solvent that does not affect the reaction, and examples thereof include aromatic hydrocarbons (e.g., toluene, xylene), ethers (e.g., diethyl ether, tetrahydrofuran), and halogenated hydrocarbons (e.g., dichloromethane, chloroform, 1,2-dichloroethane), with halogenated hydrocarbons being preferred. Examples of the basic compound used in this reaction include organic bases such as triethylamine, pyridine, N-methylpyrrolidine, 1,8-diazabicyclo[5.4.0]undec-7-ene (DBU), 1,4-diazabicyclo[2.2.2]octane (DABCO), etc., and triethylamine is more preferred.

The amount of compound 10 used is preferably 2 times or more, particularly about 2 to 5 times the amount of compound 9 by mole.

The reaction is generally carried out at 0 to 100°C, preferably room temperature to 80°C, for a reaction time of several minutes to 48 hours, preferably 1 to 24 hours. This reaction may also be carried out in the presence of an activator such as N,N-dimethyl-4-aminopyridine.

Monodesulfonylation of compound 11 is carried out in the presence of tetrabutylammonium fluoride in a suitable solvent according to the report by Yasuhara et al. (Chem. Pharm. Bull. 1999, 47, 809.). The solvent used in this reaction may be any solvent that does not affect the reaction, and examples thereof include ethers (e.g., diethyl ether, tetrahydrofuran) and halogenated hydrocarbons (e.g., dichloromethane, chloroform, 1,2-dichloroethane), with ethers being preferred.

The amount of the solvent used is not particularly limited, but may be 1 to 300 times (v/w) the amount of Compound 11.

The reaction is generally carried out at 0 to 80°C, preferably room temperature to 50°C, and the reaction time is generally from several minutes to 24 hours, preferably 30 minutes to 12 hours.

### (Step 4)

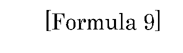

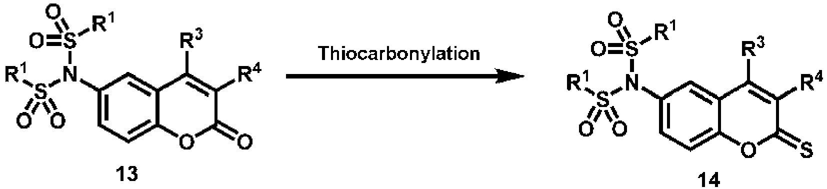

### [In the formula, R¹, R³ and R⁴ are the same as defined above.]

Thiocarbonylation of compound 13 is carried out in the presence of Lawesson's reagent in a suitable solvent. The solvent used in this reaction may be any solvent that does not affect the reaction, and examples thereof include ethers (e.g., diethyl ether, tetrahydrofuran), halogenated hydrocarbons (e.g., dichloromethane, chloroform, 1,2-dichloroethane), benzene, and toluene, with toluene being preferred.

The amount of the solvent used is not particularly limited, but may be 1 to 300 times (v/w) the amount of Compound 13.

The reaction is generally carried out at 0 to 150°C, preferably 80 to 120°C, for a reaction time of several minutes to 3 days, preferably 12 hours to 1 day.

### (Step 5)

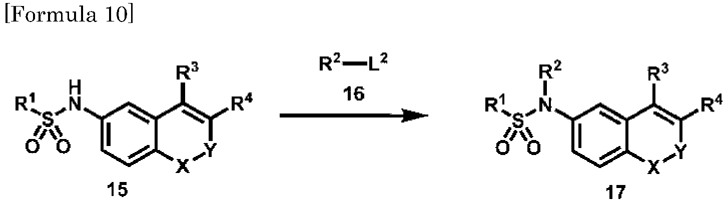

[In the formula, L² represents a halogen atom, a mesylate group, a tosylate group, a triflate group, a lower alkoxy group, or an aryloxy group, and R¹, R², R³, and R⁴ are the same as those defined above.]

That is, compound 17 can be obtained by carrying out a substitution reaction between compound 15 and compound 16.

Here, the "halogen atom" represented by L² is preferably a chlorine atom, a bromine atom, or an iodine atom. The "lower alkoxy group" is preferably a tert-butoxy group. The "aryloxy group" is preferably a pentafluorophenyloxy group.

The solvent used in this reaction may be any solvent that does not affect the reaction, and preferred are ethers (e.g., diethyl ether, tetrahydrofuran), halogenated hydrocarbons (e.g., dichloromethane, chloroform, 1,2-dichloroethane), and aprotic polar solvents (e.g., N,N-dimethylformamide, dimethyl sulfoxide, acetonitrile).

In this reaction, it is preferable to add a basic compound.

Examples of the basic compound include metal hydrides such as sodium hydride and potassium hydride; inorganic bases such as sodium hydroxide and potassium carbonate; alkoxides such as sodium methoxide and potassium tert-butoxide; and organic bases such as triethylamine, pyridine, N-methylpyrrolidine, 1,8-diazabicyclo[5.4.0]undec-7-ene (DBU), and 1,4-diazabicyclo[2.2.2]octane (DABCO), among which potassium carbonate or triethylamine is preferred.

The amount of compound 16 used is preferably at least equimolar to compound 15, particularly about 1 to 5 times the molar amount, and the amount of the basic compound used is preferably at least equimolar to compound 15, particularly about 1 to 10 times the molar amount.

This reaction is usually carried out at 0 to 100°C, preferably room temperature to 80°C, and the reaction time is usually from several minutes to 48 hours, preferably from 1 to 24 hours. This reaction may also be carried out in the presence of an activator such as N,N-dimethyl-4-aminopyridine or tetrabutylammonium iodide.

Furthermore, when R² of Compound 17 is a group represented by formula (b), it can be produced by any known method, and one example is as follows.

### (Step 6)

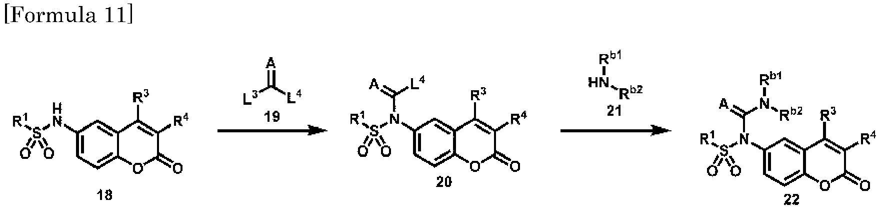

[In the formula, A represents an oxygen atom or a sulfur atom; L³ and L⁴ may be the same or different and represent a halogen atom, a halo-lower alkoxy group, an aryloxy group, or an imidazolyl group; and R¹, R³, R^{b1}, and R^{b2} are the same as those defined above.]

That is, after preparing the reaction intermediate 20 from the compound 18 and the compound 19, the compound 22 can be obtained by a substitution reaction with the compound 21 without purification or isolation. The reaction intermediate 20 can also be purified and isolated.

Here, the "halogen atom" represented by L³ and L⁴ is preferably a chlorine atom.

As the "halo lower alkoxy group", a trichloromethyl group or a 1,1,1-trichloroethyl group is preferred. As the "aryloxy group", a 4-nitrophenyloxy group is preferred.

The solvent used in this reaction may be any solvent that does not affect the reaction, and examples thereof include aromatic hydrocarbons (e.g., toluene, xylene), ethers (e.g., diethyl ether, tetrahydrofuran), halogenated hydrocarbons (e.g., dichloromethane, chloroform, 1,2-dichloroethane), and aprotic polar solvents (e.g., N,N-dimethylformamide, dimethyl sulfoxide, and acetonitrile).

In this reaction, it is preferable to add a basic compound.

Examples of the basic compound include metal hydrides such as sodium hydride and potassium hydride; inorganic bases such as sodium hydroxide and potassium carbonate; alkoxides such as sodium methoxide and potassium tert-butoxide; and organic bases such as triethylamine, pyridine, N-methylpyrrolidine, 1,8-diazabicyclo[5.4.0]undec-7-ene (DBU), and 1,4-diazabicyclo[2.2.2]octane (DABCO), among which sodium hydride, potassium carbonate, triethylamine, and DBU are preferred.

The amount of compound 19 used is preferably at least equimolar to compound 18, particularly about 1 to 5 times the molar amount, and the amount of the basic compound used is preferably at least equimolar to compound 18, particularly about 2 to 10 times the molar amount.

This reaction is usually carried out at -20 to 120°C, preferably 0 to 80°C, and the reaction time is usually from several minutes to 48 hours, preferably 1 to 24 hours.

### (Step 7)

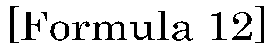

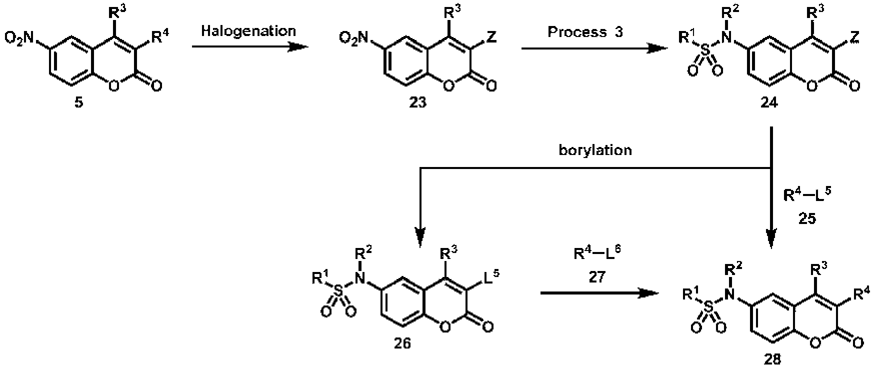

[In the formula, Z represents a halogen atom, L⁵ represents a hydrogen atom, a halogen atom or a boryl group, preferably a boryl group, L⁶ represents a halogen atom or a triflate group, R⁴ represents a hydrogen atom, and R¹, R² and R³ are the same as those defined above.]

That is, compound 8a is halogenated to give compound 23, and then compound 24 is obtained from this in accordance with steps 3-1 and 3-3. Compound 24 is then coupled with compound 25 in the presence of a transition metal catalyst to give compound 28. In addition, as a separate report, compound 24 is borylated to obtain compound 26, which is then subjected to a coupling reaction with compound 27 in the presence of a transition metal catalyst to obtain compound 28.

Here, the "halogen atom" represented by Z is preferably a bromine or iodine atom. The "boryl group" represented by L⁵ is preferably a dihydroxyboryl group, a pinacolboryl group, or a catecholboryl group. The "halogen atom" represented by L⁶ includes a chlorine atom, a bromine atom and an iodine atom.

The solvent used in the halogenation of compound 8a may be any solvent that does not affect the reaction, and aprotic polar solvents (e.g., N,N-dimethylformamide, acetonitrile) and halogenated hydrocarbons (e.g., dichloromethane, chloroform, 1,2-dichloroethane) are preferred.

Examples of halogenating agents include halogen molecules, a combination of N-halosuccinimide and a strong organic acid such as paratoluenesulfonic acid, and a combination of hydrohalic acid and Oxone (registered trademark of Du Pont), with the combination of hydrohalic acid and Oxone (registered trademark of Du Pont) being preferred.

The amount of the halogenating agent used is preferably at least equimolar, particularly about 1 to 5 times the molar amount of Compound 8a.

This reaction is usually carried out at 0 to 100°C, preferably room temperature to 80°C, and the reaction time is usually from several minutes to 24 hours, preferably 30 minutes to 12 hours.

Compound 23 can be converted to compound 24 by following step 3 above.

For the synthesis of compound 28 by the coupling reaction of compound 24, Suzuki-Miyaura cross-coupling reaction, Heck reaction, Sonogashira coupling reaction, Buchwald-Hartwig cross-coupling reaction, etc. may be used.

The solvent used in this reaction may be any solvent that does not affect the reaction, and examples thereof include aromatic hydrocarbons (e.g., toluene, xylene), ethers (e.g., tetrahydrofuran, 1,4-dioxane), aprotic polar solvents (e.g., N,N-dimethylformamide, acetonitrile), and water, or a mixture of these solvents.

Examples of the transition metal catalyst include palladium catalysts such as palladium chloride, palladium acetate, tetrakis(triphenylphosphine)palladium(0), tris(dibenzylideneacetone)dipalladium(0), bis(tert-butylphosphine)palladium(0), and bis(diphenylphosphinoferrocene)dichloropalladium(II), and copper catalysts such as copper(I) iodide. These may be used alone or in combination of two or more. In the case of the Sonogashira reaction, the synthesis may also be carried out in the presence of copper(I) iodide.

The amount of the transition metal catalyst used may be 0.005 to 1 mole per mole of compound 24.

This reaction may be carried out in the presence of a phosphine compound.

Examples of the phosphine compound include tri(tert-butyl)phosphine, tri(cyclohexyl)phosphine, tri(2-furyl)phosphine, and diphenylphosphinoferrocene, with tri(tert-butyl)phosphine being preferred.

The amount of the phosphine compound used may be 0.01 to 1 mole per mole of Compound 27.

In this reaction, it is preferable to add a basic compound.

Examples of the basic compound include inorganic bases such as potassium carbonate, cesium carbonate, and potassium phosphate; alkoxides such as sodium methoxide and potassium tert-butoxide; metal fluorides such as sodium fluoride and cesium fluoride; and organic bases such as triethylamine and 1,8-diazabicyclo[5.4.0]undec-7-ene, of which potassium carbonate, potassium tert-butoxide, cesium fluoride, and triethylamine are preferred.

The amount of the basic compound used is preferably at least equimolar to Compound 24, particularly about 1 to 5 times the molar amount.

This reaction is usually carried out at 0 to 150°C, preferably room temperature to 120°C, and the reaction time is usually from several minutes to 48 hours, preferably from 1 to 24 hours. This reaction is preferably carried out in an atmosphere of an inert gas such as nitrogen or argon.

The solvent used in the synthesis of compound 26 by borylation of compound 24 may be any solvent that does not affect the reaction, and examples thereof include aromatic hydrocarbons (e.g., toluene, xylene), ethers (e.g., tetrahydrofuran, 1,4-dioxane), aprotic polar solvents (e.g., N,N-dimethylformamide, acetonitrile), and water, or a mixture thereof.

In this reaction, it is desirable to use tetrahydroxydiboron or a diboronic acid ester as the borylation agent. Examples of the diboronic acid ester that can be used include bis(pinacolato)diboron and bis(catecholato)diboron. The amount of the borylation agent used is preferably at least equimolar to compound 24, particularly about 1 to 5 times the molar amount.

This reaction is preferably carried out using a transition metal catalyst. Examples of the transition metal catalyst include palladium catalysts such as palladium chloride, palladium acetate, tetrakis(triphenylphosphine)palladium(0), tris(dibenzylideneacetone)dipalladium(0), bis(tert-butylphosphine)palladium(0), and bis(diphenylphosphinoferrocene)dichloropalladium(II), and these may be used alone or in combination of two or more. The amount of the transition metal catalyst used may be 0.005 to 1 mole per mole of compound 24.

This reaction may be carried out in the presence of a phosphine compound. Examples of the phosphine compound include tri(tert-butyl)phosphine, tri(cyclohexyl)phosphine, tri(2-furyl)phosphine, and diphenylphosphinoferrocene, with tri(tert-butyl)phosphine being preferred.

The amount of the phosphine compound used may be 0.01 to 1 mole per mole of Compound 24.

In this reaction, it is preferable to add a basic compound.

Examples of the basic compound include inorganic bases such as potassium carbonate, cesium carbonate, and potassium phosphate; alkoxides such as sodium methoxide and potassium tert-butoxide; metal fluorides such as sodium fluoride and cesium fluoride; and organic bases such as triethylamine and 1,8-diazabicyclo[5.4.0]undec-7-ene, of which potassium carbonate, potassium tert-butoxide, cesium fluoride, and triethylamine are preferred.

The amount of the basic compound used is preferably at least equimolar to Compound 24, particularly about 1 to 5 times the molar amount.

This reaction is usually carried out at 0 to 150°C, preferably room temperature to 120°C, and the reaction time is usually from several minutes to 48 hours, preferably from 1 to 24 hours. This reaction is preferably carried out in an atmosphere of an inert gas such as nitrogen or argon.

As an alternative to this borylation, compound 26 may be synthesized by reacting with a boronic acid ester or diboronic acid ester in the presence of a strong base compound.

The solvent to be used may be any solvent that does not affect the reaction, and examples thereof include hydrocarbons (e.g., hexane) and ethers (e.g., tetrahydrofuran, 1,4-dioxane), or a mixture thereof.

The strong basic compound to be used may be an organometallic base reagent such as a lithium species or magnesium species, and n-butyllithium or s-butyllithium is more preferred.

The amount of the basic compound used is preferably equimolar or more, particularly equimolar, to compound 24.

Examples of the boronic acid ester or diboronic acid ester that can be used include bis(pinacolato)diboron, bis(catecholato)diboron, isopropoxyboronic acid pinacol ester, and trimethoxyborane. The amount of the boronic acid ester or diboronic acid ester used is preferably equimolar or more, particularly about 1 to 5 times the molar amount of compound 24.

This reaction is usually carried out at -100°C to 50°C, preferably -78°C to 0°C, and the reaction time is usually from several minutes to 48 hours, preferably from 1 to 24 hours. This reaction is preferably carried out in an atmosphere of an inert gas such as nitrogen or argon.

The solvent used in the synthesis of compound 28 by the reaction of compound 26 with compound 27 may be any solvent that does not affect the reaction, and examples thereof include aromatic hydrocarbons (e.g., toluene, xylene), ethers (e.g., tetrahydrofuran, 1,4-dioxane), aprotic polar solvents (e.g., N,N-dimethylformamide, acetonitrile), and water, or a mixture thereof.

This reaction uses a transition metal catalyst.

Examples of the transition metal catalyst to be used include palladium catalysts such as palladium chloride, palladium acetate, tetrakis(triphenylphosphine)palladium(0), tris(dibenzylideneacetone)dipalladium(0), bis(tert-butylphosphine)palladium(0), and bis(diphenylphosphinoferrocene)dichloropalladium(II), and copper catalysts such as copper(I) iodide. These may be used alone or in combination of two or more.

The amount of the transition metal catalyst used may be 0.005 to 1 mole per mole of compound 26.

This reaction may be carried out in the presence of a phosphine compound.

Examples of the phosphine compound include tri(tert-butyl)phosphine, tri(cyclohexyl)phosphine, tri(2-furyl)phosphine, and diphenylphosphinoferrocene, with tri(tert-butyl)phosphine being preferred.

The amount of the phosphine compound used may be 0.01 to 1 mole per mole of compound 26.

In this reaction, it is preferable to add a basic compound.

Examples of the basic compound include inorganic bases such as potassium carbonate, cesium carbonate, and potassium phosphate; alkoxides such as sodium methoxide and potassium tert-butoxide; metal fluorides such as sodium fluoride and cesium fluoride; and organic bases such as triethylamine and 1,8-diazabicyclo[5.4.0]undec-7-ene, of which potassium carbonate, potassium tert-butoxide, cesium fluoride, and triethylamine are preferred.

The amount of the basic compound used is preferably equimolar or more, particularly about 1 to 5 times the molar amount of compound 26.

This reaction is usually carried out at 0 to 150°C, preferably room temperature to 120°C, and the reaction time is usually from several minutes to 48 hours, preferably from 1 to 24 hours. This reaction is preferably carried out in an atmosphere of an inert gas such as nitrogen or argon.

### (Step 8)

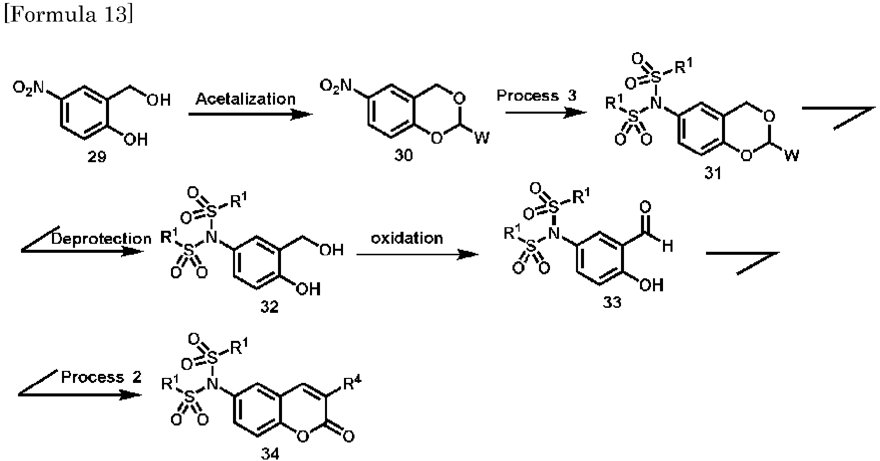

[In the formula, W represents a C₁ to C₄ lower alkyl group or an aryl group, and R¹ and R⁴ are the same as those defined above.]

That is, compound 29 is acetalized to give compound 30, which is then used to obtain compound 31 according to the above step 3, and compound 32 can then be obtained by deprotecting the acetal. The obtained 32 is oxidized to give 33, and then compound 34 can be obtained according to step 2 above.

Here, the "alkyl group" represented by W is preferably a C₁₋₄ lower alkyl group such as a methyl group, an ethyl group, a propyl group, etc. The "aryl group" includes a C₆₋₁₀ aryl group such as a phenyl group, a naphthyl group, etc., and is preferably a phenyl group.

The acetalization of compound 29 is carried out in a solvent using an acid catalyst. The solvent used in this acetalization may be any solvent that does not affect the reaction, and aprotic polar solvents (e.g., N,N-dimethylformamide, acetonitrile) and aromatic hydrocarbons (e.g., toluene, xylene) are preferred.

The acid catalyst used in this reaction includes, for example, p-toluenesulfonic acid, camphorsulfonic acid, etc. The amount of the acid catalyst used is preferably 0.01 equivalent or more, particularly about 0.03 to 0.1 equivalent, relative to compound 29.

This reaction is usually carried out at 0 to 100°C, preferably room temperature to 50°C, and the reaction time is usually from several minutes to 24 hours, preferably 30 minutes to 12 hours.

Compound 30 can be converted to compound 31 by following step 3 above.

Deprotection of compound 31 is carried out using an acid catalyst in a solvent. The solvent used in this deacetalization may be any solvent that does not affect the reaction, and protic polar solvents (e.g., water, methanol) and aromatic hydrocarbons (e.g., toluene, xylene) are preferred.

The acid catalyst used in this reaction includes, for example, p-toluenesulfonic acid, camphorsulfonic acid, etc. The amount of the acid catalyst used is preferably 0.01 equivalent or more, particularly about 0.03 to 0.1 equivalent, relative to compound 30.

This reaction is usually carried out at 0 to 100°C, preferably room temperature to 50°C, and the reaction time is usually from several minutes to 24 hours, preferably 30 minutes to 12 hours.

In this reaction, when the group represented by W is an aryl group, it is converted to compound 32 by catalytic reduction with a heterogeneous catalyst or reduction with a metal and an acid in an appropriate solvent.

The solvent used in the catalytic reduction using a heterogeneous catalyst may be any solvent that does not affect the reaction, and examples thereof include esters (e.g., ethyl acetate, butyl acetate), ethers (e.g., diethyl ether, tetrahydrofuran), halogenated hydrocarbons (e.g., dichloromethane, chloroform, 1,2-dichloroethane), alcohols (e.g., methanol, ethanol), and water, or a mixed solvent thereof may also be used. Examples of the metal catalyst that can be used include palladium, platinum, nickel, and rhodium, with palladium being preferred. The reaction is generally carried out at room temperature to 80°C, preferably room temperature to 50°C, and the reaction time is generally from several minutes to 48 hours, preferably from 1 to 24 hours.

The oxidation of compound 32 is carried out using a suitable oxidizing agent in a solvent.

Examples of oxidizing agents that can be used to synthesize compound 33 by oxidation of compound 32 include chromium (IV)-based oxidants such as pyridinium chromate (PCC) and pyridinium dichromate (PDC), manganese (IV)-based oxidants such as manganese dioxide, ruthenium (VII)-co-oxidant based oxidants such as tetrapropylammonium perruthenate (TPAP) and N-methylmorpholine-N-oxide as a co-oxidant, hypervalent iodine-based oxidants such as Dess-Martin periodinane, and DMSO-based oxidants such as dimethyl sulfoxide (DMSO), oxalyl dichloride, and triethylamine (Swern oxidation).

Any solvent may be used as long as it does not affect the reaction. For example, when manganese dioxide (IV) is used, aliphatic hydrocarbons (e.g., pentane, hexane), halogenated hydrocarbons (e.g., dichloromethane, chloroform, 1,2-dichloroethane), and aprotic polar solvents (e.g., N,N-dimethylformamide, acetonitrile) are preferred. The reaction is generally carried out at 0°C to 80°C, preferably room temperature to 50°C, and the reaction time is generally from several minutes to 48 hours, preferably from 1 to 24 hours.

Compound 33 can be converted to compound 34 by following step 2 above.

Compounds 11 to 14, compound 17, compound 22, compound 28, compound 34 or each synthetic intermediate compound is isolated and purified as a free compound, a salt thereof, a hydrate, a solvate, or a crystalline polymorphic substance.

Salts of Compounds 11 to 14, Compound 17, Compound 22, Compound 28, Compound 34 or the respective synthetic intermediate compounds can also be prepared by subjecting them to a conventional salt formation reaction.

Isolation and purification are carried out by applying conventional chemical procedures such as extraction, fractional crystallization, various fractional chromatography, evaporation, drying, filtration, centrifugation, and the like.

Various stereoisomers can be produced by selecting appropriate starting compounds, or can be separated by taking advantage of the differences in physicochemical properties between the isomers. For example, optical isomers can be obtained by a general optical resolution method for a racemate (e.g., fractional crystallization leading to a diastereomeric salt with an optically active base or acid, chromatography using a chiral column), or can also be produced from an appropriate optically active starting compound.

The compound represented by formula (1) of the present invention or a salt thereof thus obtained has the effect of suppressing the nuclear localization of Smad2/3 in human-derived hepatic stellate cells, and has the effect of suppressing the mRNA expression of connective tissue growth factor (CTGF) and α-smooth muscle actin (α-SMA), as shown in the test examples described below.

SMAD2/3 is a major transcription factor in the TGF-β signal transduction pathway, and by binding to various cofactors, is involved in the regulation of the expression of a wide variety of genes, including CTGF and α-SMA. CTGF is a secretory protein that promotes the proliferation of fibroblasts, including hepatic stellate cells, and also acts cooperatively with TGF-β to induce transformation into myofibroblast-like cells that produce large amounts of collagen (Lab. Invest. 2002, 82, 767; FASEB. J. 2004, 18, 469).

α-SMA is a marker molecule that indicates that fibroblasts, including hepatic stellate cells, have been transformed into myofibroblast-like cells that produce large amounts of collagen (Physiol. Rev. 2008, 88, 125.).

That is, the sulfonamide derivative or a salt thereof of the present invention inhibits the TGF-β signaling by suppressing the nuclear localization of Smad2/3, thereby making it possible to prevent, treat, or ameliorate diseases or pathological conditions induced by abnormalities in the signal transduction system. Examples of the diseases and pathologies include fibrosis (organ fibrosis) such as liver fibrosis, pulmonary fibrosis, renal fibrosis, skin fibrosis, cardiac fibrosis, and pancreatic fibrosis, as well as obesity and diabetes. Other examples include cancer metastasis and suppression of anti-tumor immunity.

Therefore, the compound represented by formula (1) of the present invention or a salt thereof can serve as an inhibitor of nuclear localization of Smad2/3, a TGF-β signal inhibitor, and further as a preventive or therapeutic agent for fibrosis, a preventive or therapeutic agent for obesity, a preventive or therapeutic agent for diabetes, a cancer metastasis inhibitor, or an anti-tumor immunity enhancer.

Here, "inhibition of nuclear localization of Smad2/3" means inhibiting the nuclear translocation of phosphorylated Smad including Smad2/3, or degrading or transferring to the cytoplasm the phosphorylated Smad including Smad2/3 that have translocated to the nucleus.

"TGF-β signaling inhibition" means that the nuclear localization of Smad2/3 is inhibited, resulting in the suppression of expression of response genes whose transcription is induced by Smad2/3.

"Fibrosis" includes organ fibrosis such as liver fibrosis, pulmonary fibrosis, kidney fibrosis, skin fibrosis, cardiac fibrosis, and pancreatic fibrosis. Symptoms associated with fibrosis, such as inflammation and atrophy, are observed depending on the organ in which fibrosis has progressed and the degree of progression. Therefore, the present invention also encompasses the treatment of symptoms associated with fibrosis.

Pulmonary fibrosis includes not only idiopathic pulmonary fibrosis but also pulmonary fibrosis conditions including coexistence with interstitial pneumonia. That is, pulmonary fibrosis of the present invention includes interstitial pneumonia that may be accompanied by pulmonary fibrosis.

When the compound represented by formula (1) of the present invention or a salt thereof is used as a pharmaceutical, it can be formulated as a composition (pharmaceutical composition) together with a pharmaceutically acceptable carrier for parenteral administration such as injection or rectal administration, and oral administration in solid, semi-solid or liquid form, or the like.

The form of the composition for injection may be pharmaceutically acceptable sterile aqueous solution, non-aqueous solution, suspension or emulsion. Examples of suitable non-aqueous carriers, diluents, solvents or vehicles include propylene glycol, polyethylene glycol, vegetable oils such as olive oil, and injectable organic esters such as ethyl oleate. Such compositions may also contain adjuvants such as preservatives, wetting agents, emulsifying agents, dispersing agents and the like. These compositions may be sterilized, for example, by filtration through a bacterial-retaining filter, or by incorporating a sterilizing agent in the form of sterile solid compositions which can be dissolved in sterile water, or some other sterile injectable medium immediately before use.

Solid preparations for oral administration include capsules, tablets, pills, troches, powders, and granules. In preparing such solid preparations, the compound of the present invention is generally mixed with at least one inert diluent such as sucrose, lactose, starch, and the like. The formulation may also include additional substances other than inert diluents as in normal formulations, such as lubricating agents (e.g., magnesium stearate). In the case of capsules, tablets and pills, buffering agents may also be included. Tablets and pills may further be provided with an enteric coating.

Liquid preparations for oral administration include pharmaceutically acceptable emulsions, solutions, suspensions, syrups, and elixirs, containing inert diluents commonly used by those skilled in the art, such as water. In addition to such inert diluents, the composition may also contain adjuvants such as wetting agents, emulsifying agents, suspending agents, sweetening agents, seasoning agents, and flavoring agents. In the case of a preparation for rectal administration, it is preferable to contain excipients such as cacao butter, suppository wax, etc. in addition to the compound of the present invention.

The dose of the medicament of the present invention is determined depending on conditions such as the age, sex, body weight, symptoms, and administration route of the subject to be administered, for example, a patient with fibrosis or suspected fibrosis, and is generally in the range of 0.001 mg/kg or more and 1000 mg/kg or less, preferably 0.01 mg/kg or more and 100 mg/kg or less, and more preferably 0.1 mg/kg or more and 10 mg/kg or less, per day for an adult, as the compound represented by formula (1) or a salt thereof. The medicament of the present invention can be administered daily or at intervals.

### Examples

The present invention will be explained in more detail below by way of Reference Examples and Examples, but these Examples are intended to provide a better understanding of the present invention and are not intended to limit the scope of the present invention. For the sake of convenience, compounds having tautomeric groups are shown using the structural formula of one of the tautomers.

### Reference example 1

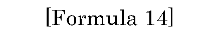

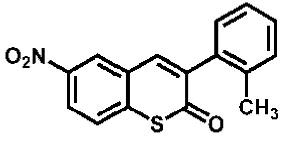

### 3-(2-methylphenyl)-6-nitro-2H-thiochromen-2-one

(1) 5-Nitrosalicylaldehyde (840 mg) and 1,4-diazabicyclo[2.2.2]octane (DABCO) were dissolved in dichloromethane (30 mL), and dimethylthiocarbamoyl chloride (930 mg) was added at room temperature. The mixture was then stirred for one day. Water (50 mL) was added to the reaction mixture, which was then extracted with ethyl acetate (200 mL). The extract was washed with 1M aqueous HCl solution (50 mL), 1M aqueous NaOH solution (50 mL), and saturated brine (50 mL). The organic layer was dried over sodium sulfate and concentrated to give 1-(2-formyl-4-nitrophenoxy)-N,N-dimethylmethanethioamide (860 mg) as a yellow solid.
(2) 1-(2-formyl-4-nitrophenoxy)-N,N-dimethylmethanethioamide (860 mg) obtained in the previous section was dissolved in toluene (34 mL) and stirred under heating and reflux for one day. After the reaction, the resulting precipitate was collected by filtration to obtain a crude product of 1-[(2-formyl-4-nitrophenyl)sulfanyl]-N,N-dimethylformamide.
(3) To a solution of the crude product obtained in the previous section in methanol (27 mL), 3M aqueous NaOH solution (13 mL) was added, and the mixture was stirred under heating and reflux for 4 hours. The reaction mixture was acidified with 1M aqueous HCl solution and then extracted with ethyl acetate (100 mL). The extract was washed with water (30 mL) and saturated brine (30 mL). The organic layer was dried over sodium sulfate and then concentrated, and the residue was purified by column chromatography (ethyl acetate/hexane=0%-100%) to obtain 5-nitro-2-sulfanylbenzaldehyde.
(4) The 5-nitro-2-sulfanylbenzaldehyde obtained in the previous section, 2-methylphenylacetic acid (610 mg), and pyridine (0.68 mL) were dissolved in acetic anhydride (4 mL), and the solution was stirred at 135°C for 3 days. The reaction mixture was neutralized with an aqueous solution of sodium carbonate and then extracted with ethyl acetate (100 mL). The extract was washed with 1M aqueous HCl solution (30 mL), saturated aqueous sodium hydrogen carbonate solution (30 mL), water (30 mL), and saturated brine (30 mL). The organic layer was dried over sodium sulfate and concentrated, and the residue was purified by column chromatography (ethyl acetate/hexane=0%-20%) to obtain the title compound (0.310 g).

¹H NMR(500MHz,CDCl₃): δ8.50(d,J=2.2Hz,1H),8.33(dd,J=8.8,2.4Hz,1H),7.78(s,1H),7.66(d,J=9.0Hz,1H),7.37-7.33(m,1H),7.31-7.27(m,2H),7.22-7.19(m,1H),2.25(s,3H)

### Reference example 2

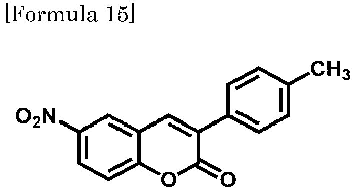

### 3-(4-methylphenyl)-6-nitro-2H-chromen-2-one

To a solution of triphenylphosphine (0.790 g) and iodine (0.760 g) in dichloromethane (20 mL) were added 5-nitrosalicylaldehyde (0.340 g), 4-methylphenylacetic acid (0.370 g), and triethylamine (1.4 mL) at 0°C, and the mixture was stirred at room temperature for one day. A saturated aqueous solution of sodium hydrogen carbonate (20 mL) was added to the reaction mixture, which was then extracted with dichloromethane (100 mL). The extract was washed with water (20 mL). The organic layer was dried over sodium sulfate and concentrated, and the residue was purified by column chromatography (ethyl acetate/hexane=66%) to obtain the title compound (0.340 g).

¹H NMR(500MHz,CDCl₃): δ8.48(d,J=2.6Hz,1H),8.38(dd,J=9.0,2.6Hz,1H),7.85(s,1H),7.62(d,J=8.2Hz,2H),7.48(d,J=9 .0Hz,1H),7.30(d,J=8.2Hz,2H),2.43(s,3H)

### Reference example 3

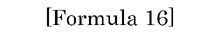

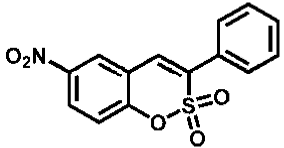

### 6-nitro-3-phenyl-1,2-benzoxathiin-2,2-dioxide

(1) To a solution of 5-nitrosalicylaldehyde (840 mg) in dichloromethane (25 mL) was added triethylamine (2.8 mL), and then benzylsulfonyl chloride was added, followed by stirring at room temperature for 3 days. Water (50 mL) was added to the reaction mixture, which was then extracted with dichloromethane (100 mL x 2). The extract was dried over sodium sulfate and concentrated, and the resulting residue was purified by column chromatography (ethyl acetate/hexane=25%) to give 2-formyl-4-nitrophenyl benzylsulfonate (1.1 g).
(2) To a solution of the above 2-formyl-4-nitrophenyl benzylsulfonate (0.300 g) in dichloromethane, 1,8-diazabicyclo[5.4.0]undec-7-ene (0.140 mL) was added at 0°C, and the mixture was stirred for 3 hours. The reaction mixture was quenched with 1M aqueous HCl solution, and then extracted with a 1:1 mixture of diethyl ether and ethyl acetate (100 mL). The extract was washed with water (30 mL). The organic layer was dried over sodium sulfate and then concentrated, and the residue was suspended in diethyl ether. The resulting solid was collected by filtration to obtain the title compound (130 mg) as a white solid.

¹H NMR(500MHz,CDCl₃): δ8.42(d,J=2.7Hz,1H),8.36(dd,J=9.0,2.7Hz,1H),7.70-7.67(m,2H),7.56-7.50(m,3H),7.48(d,J=9.0Hz,1H),7.30(s,1H).

### Reference example 4

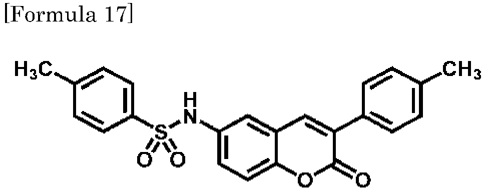

### 4-methyl-N-[3-(4-methylphenyl)-2-oxo-2H-chromen-6-yl]benzene-1-sulfonamide

(1) A mixture of iron powder (140 mg) and ammonium chloride (13 mg) in ethanol/water (10 mL/5 mL) was stirred at 70°C for 30 minutes, and then a solution of 3-(4-methylphenyl)-6-nitro-2H-chromen-2-one (140 mg) obtained in Reference Example 2 or a method analogous thereto in tetrahydrofuran (5 mL) was added, followed by stirring at 70°C for 2 hours. The reaction mixture was filtered through Celite, and the filtrate was extracted with ethyl acetate. The organic layer was washed with water and saturated brine, dried over sodium sulfate, and concentrated to give 6-amino-3-(4-methylphenyl)-2H-chromen-2-one (126 mg) as a yellow solid.
   ¹H NMR(500MHz,CDCl₃): δ7.65(s,1H),7.59(d,J=8.3Hz,2H),7.27-7.25(m,2H),7.18(d,J=8.8Hz,1H),6.86(dd,J=8.8,2.7Hz,1H),6.77(d,J=2.7Hz,1H),3.72(brs,2 H),2.40(s,3H)
(2) To a solution of 6-amino-3-(4-methylphenyl)-2H-chromen-2-one (126 mg) obtained in the previous section in dichloromethane (5 mL), triethylamine (350 µL) and p-toluenesulfonyl chloride (240 mg) were added at room temperature, and the mixture was stirred at the same temperature for 2 days. Water was added to the reaction mixture, which was then extracted with dichloromethane. The organic layer was washed with water and saturated brine, dried over sodium sulfate, and then concentrated. The residue was purified by silica gel column chromatography (ethyl acetate/hexane=30%) to give 4-methyl-N-(4-methylbenzenesulfonyl)-N-[3-(4-methylphenyl)-2-oxo-2H-chromen-6-yl]benzene-1-sulfonamide (120 mg) as a white solid.
   ¹H NMR(500MHz,CDCl₃): δ7.83-7.80(m,4H),7.67(s,1H),7.58(d,J=8.2Hz,2H),7.38-7.34(m,4H),7.30(d,J=8.8Hz,1H),7.28-7.25(m,3H),7.10(dd,J=8.8,2.5Hz,1H),2.49(s,6H),2.41(s,3H)
(3) To a solution of 4-methyl-N-(4-methylbenzenesulfonyl)-N-[3-(4-methylphenyl)-2-oxo-2H-chromen-6-yl]benzene-1-sulfonamide (100 mg) obtained in the previous section in tetrahydrofuran (3 mL), tetrabutylammonium fluoride (1 mol/L tetrahydrofuran solution; 240 µL) was added at room temperature, and the mixture was stirred at the same temperature for 2 hours. Water was added to the reaction mixture, which was then extracted with ethyl acetate. The organic layer was washed with water and saturated brine, dried over sodium sulfate, and then concentrated. The residue was purified by silica gel column chromatography (ethyl acetate/hexane 33%) to obtain the title compound (41 mg) as a white solid.
   ¹H NMR(500MHz,CDCl₃): δ7.70(s,1H),7.62(d,J=8.3Hz,2H),7.58(d,J=8.2Hz,2H),7.37(d,J=2.6Hz,1H),7.27-7.21(m,5H),7.08(dd,J=8.8,2.6Hz,1H),6.48(brs,1H),2.40(s,3H),2.39(s,3H)

### Reference example 5

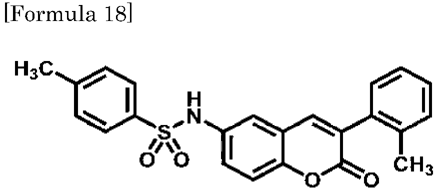

### 4-methyl-N-[3-(2-methylphenyl)-2-oxo-2H-chromen-6-yl]benzene-1-sulfonamide

(1) 3-(2-methylphenyl)-6-nitro-2H-chromen-2-one synthesized by Reference Example 2 or a method analogous thereto was used to obtain 6-amino-3-(2-methylphenyl)-2H-chromen-2-one by the method described in Reference Example 4-(1) or a method analogous thereto.
(2) To a solution of the compound obtained above (950 mg) in dichloromethane (40 mL) were added pyridine (1.2 mL) and p-toluenesulfonyl chloride (1.1 g) at room temperature, and the mixture was stirred at the same temperature for 1 day. Water was added to the reaction mixture, which was then extracted with dichloromethane. The organic layer was washed with 1 mol/L hydrochloric acid, dried over sodium sulfate, and then concentrated. The residue was purified by silica gel column chromatography (ethyl acetate/hexane=40%) to obtain the title compound (1.4 g) as a pale yellow solid.
   ¹H NMR(500MHz,CDCl₃): δ7.66-7.63(m,2H),7.55(s,1H),7.35-7.31(m,2H),7.29-7.20(m,6H),7.11(dd,J=8.8,2.5Hz,1H),6.51(brs,1H),2.40(s,3H),2.28(s,3H)

### Reference example 6

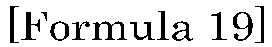

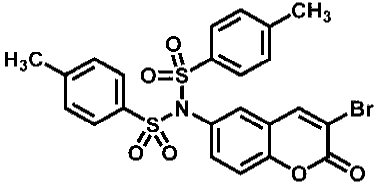

### N-(3-bromo-2-oxo-2H-chromen-6-yl)-4-methyl-N-(4-methylbenzenesulfonyl)benzene-1-sulfonamide

(1) To a solution of 6-nitro-2H-chromen-2-one (1.0 g) and Oxone (registered trademark; 3.9 g) in dichloromethane (20 mL) was added dropwise 2 mol/L hydrobromic acid (6.2 mL) at room temperature, and the mixture was stirred at the same temperature for 1 day. Triethylamine (4.2 mL) was added to the reaction mixture, and the mixture was further stirred at room temperature for 4 hours. The reaction mixture was diluted with ethyl acetate, washed with saturated aqueous sodium hydrogen carbonate solution, water and saturated brine, dried over sodium sulfate, and then concentrated. The residue was purified by silica gel column chromatography (ethyl acetate/hexane=20%) to give 3-bromo-6-nitro-2H-chromen-2-one (940 mg) as a brown solid.
(2) Using 3-bromo-6-nitro-2H-chromen-2-one (935 mg) obtained in the previous section, 6-amino-3-bromo-2H-chromen-2-one (760 mg) was obtained as a brown solid by the method described in Reference Example 4-(1) or a method analogous thereto.
(3) Using 6-amino-3-bromo-2H-chromen-2-one (760 mg) obtained in the previous section and according to the method of Reference Example 4-(2) or a method analogous thereto, the title compound (1.7 g) was obtained as a brown solid.
   ¹H NMR(500MHz,CDCl₃): δ7.99(s,1H),7.79(d,J=8.4Hz,4H),7.36(d,J=8.4Hz,4H),7.30(d,J=8.8Hz,1H),7.18(d,J=2.5Hz ,1H),7.15(dd,J=8.8,2.5Hz,1H),2.49(s,6H)

### Reference example 7

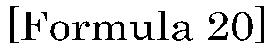

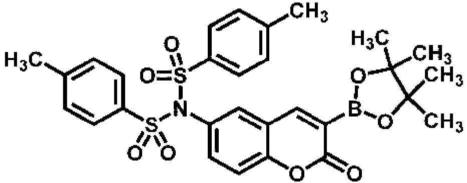

### 4-methyl-N-(4-methylbenzenesulfonyl)-N-[2-oxo-3-(tetramethyl-1,3,2-dioxaborolan-2-yl)-2H-chromen-6-yl]benzene-1-sulfonamide

N-(3-bromo-2-oxo-2H-chromen-6-yl)-4-methyl-N-(4-methylbenzenesulfonyl)benzene-1-sulfonamide (0.165 g) obtained by the method described in Reference Example 6, bis(pinacolato)diboron (0.114 g), potassium acetate (88 mg), and [1,1'-bis(diphenylphosphino)ferrocene]dichloropalladium(II) dichloromethane complex (24 mg) were suspended in 1,4-dioxane and stirred at 100°C for 2 hours under a nitrogen atmosphere. A saturated aqueous solution of ammonium chloride (15 mL) was added to the reaction mixture, which was then extracted with ethyl acetate (90 mL). The extract was washed with water (20 mL) and saturated brine (20 mL), dried over sodium sulfate, and concentrated to give the title compound.

¹H NMR(500MHz,CDCl₃): δ8.48(s,1H),7.76(d,J=2.6Hz,1H),7.71-7.68(m,4H),7.50-7.48(m,5H),7.38(d,J=8.8Hz,1H),7.12(dd,J=8.8,2.6Hz,1H),2.46(s,6H),1.30(s,12H).

### Reference example 8

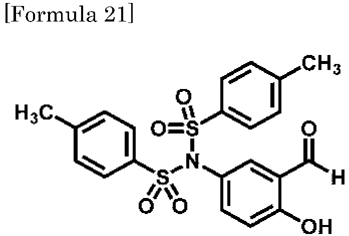

### 5-[N,N-bis(4-methylbenzenesulfonyl)amino]salicylaldehyde

(1) To a solution of 2-hydroxy-5-nitrobenzyl alcohol (1.7 g) in acetonitrile (100 mL) were added benzaldehyde dimethyl acetal (1.9 mL) and p-toluenesulfonic acid monohydrate (57 mg), and the mixture was stirred at room temperature for 1 day. The reaction mixture was diluted with dichloromethane (300 mL) and then washed with saturated aqueous sodium hydrogen carbonate solution (100 mL x 2) and water (100 mL). The organic layer was dried over sodium sulfate and concentrated. The solid residue was collected by filtration with ethyl acetate/hexane to give 6-nitro-2-phenyl-2,4-dihydro-1,3-benzodioxine (1.2 g) as a yellow solid.
(2) Using the compound obtained above, 6-amino-2-phenyl-2,4-dihydro-1,3-benzodioxine was obtained by the method described in Reference Example 4-(1) or a method analogous thereto, and this compound was then subjected to the method described in Reference Example 4-(2) or a method analogous thereto to obtain 6-[bis(4-methylbenzenesulfonyl)amino]-2-phenyl-2,4-dihydro-1,3-benzodioxine (2.0 g) as a white solid.
(3) To a solution of the compound (2.0 g) obtained above in a dichloromethane/methanol (2.4/1) mixture (40 mL), (+)-camphorsulfonic acid (0.860 g) was added, and the mixture was stirred at room temperature for 3.5 hours. The reaction mixture was neutralized with triethylamine and then concentrated under reduced pressure. The residue was purified by silica gel column chromatography (ethyl acetate/hexane=60-80%) to give 5-[N,N-bis(4-methylbenzenesulfonyl)amino]-2-hydroxybenzyl alcohol (1.5 g) as a white solid.
(4) Manganese dioxide (2.9 g) was added to a solution of the compound (1.5 g) obtained above in dichloroethane (34 mL), and the mixture was stirred at room temperature for one day. The reaction mixture was diluted with dichloromethane and ethyl acetate and filtered through Celite. The filtrate was concentrated and the solid residue was collected by filtration with dichloromethane to obtain the title compound (1.3 g) as a white solid.

¹H NMR(500MHz,CDCl₃): δ11.22(s,1H),9.77(s,1H),7.83-7.79(m,4H),7.38-7.34(m,4H),7.27-7.26(m,1H),7.13(dd,J=8.9,2.6Hz,1H),6.95(d,J=8.9Hz,1H),2.49(s,6H).

### Examples 1 to 13

The compounds shown in Tables 1-1 to 1-4 were synthesized using 6-amino-3-(2-methylphenyl)-2H-chromen-2-one described in Reference Example 5-(1) by the method described in Reference Example 4-(2) or a method similar thereto.

### Example 14

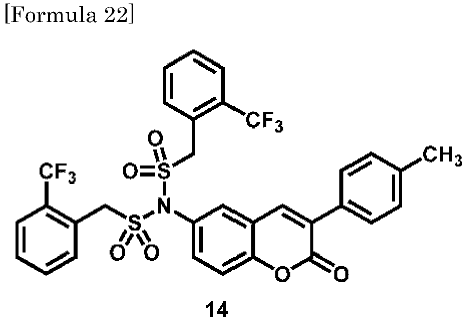

### N-[3-(4-methylphenyl)-2-oxo-2H-chromen-6-yl]-1-[2-(trifluoromethyl)phenyl]-N-{[4-(trifluoromethyl)phenyl]methanesulfonyl}methanesulfonamide

The title compound was obtained by the method of Reference Example (4)-(2) or a method analogous thereto and using 6-amino-3-(4-methylphenyl)-2H-chromen-2-one obtained in Reference Example 4-(1).

¹H NMR(500MHz,CDCl₃): δ7.77-7.68(m,6H),7.58-7.51(m,4H),7.37(s,1H),7.29-7.26(m,2H),7.07(d,J=8.8Hz,1H),6.50(dd,J=8.8,2.5Hz,1H),6.42(d,J=2.5Hz,1H),5.00-4.98(m,4H),2.41(s,3H)

### Example 15

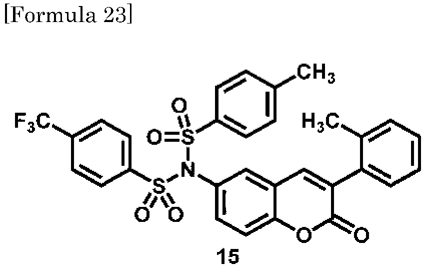

### N-(4-methylbenzenesulfonyl)-N-[3-(2-methylphenyl)-2-oxo-2H-chromen-6-yl]-4-(trifluoromethyl)benzene-1-sulfonamide

To a solution of 4-methyl-N-[3-(2-methylphenyl)-2-oxo-2H-chromen-6-yl]benzene-1-sulfonamide (20 mg) obtained in Reference Example 5 in dichloromethane (1 mL) were added triethylamine (35 µL) and 4-(trifluoromethyl)benzenesulfonyl chloride (25 mg) at room temperature, and the mixture was stirred at the same temperature for 2 days. Water was added to the reaction mixture, which was then extracted with dichloromethane. The organic layer was dried over sodium sulfate and then concentrated. The residue was purified by silica gel column chromatography (ethyl acetate/hexane=20%) to obtain the title compound (16 mg) as a white solid.

¹H NMR(500MHz,CDCl₃): δ8.12(d,J=8.3Hz,2H),7.86(d,J=8.3Hz,2H),7.83-7.80(m,2H),7.55(s,1H),7.39-7.33(m,4H),7.31-7.22(m,4H),7.13(dd,J=8.8,2.5Hz,1H),2.50(s,3H),2.31(s,3H)

### Examples 16 to 55

The compounds shown in Tables 2-1 to 2-10 were synthesized by the method described in Example 15 or a method similar thereto.

### Example 56

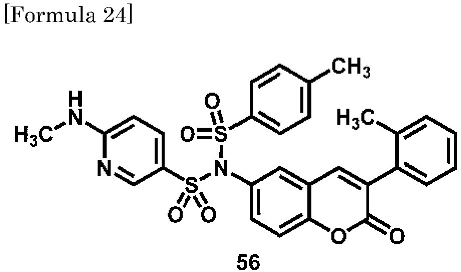

### 6-(methylamino)-N-(4-methylbenzenesulfonyl)-N-[3-(2-methylphenyl)-2-oxo-2H-chromen-6-yl]pyridine-3-sulfonamide

To a solution of 6-chloro-N-(4-methylbenzenesulfonyl)-N-[3-(2-methylphenyl)-2-oxo-2H-chromen-6-yl]pyridine-3-sulfonamide (40 mg) obtained in Example 50 in 1,4-dioxane (1 mL), N,N-diisopropylethylamine (24 µL) and methylamine hydrochloride (7 mg) were added at room temperature, and the mixture was stirred at 80°C for 24 hours. The reaction mixture was concentrated, and the residue was purified by silica gel column chromatography (ethyl acetate/hexane=0-60%) to obtain the title compound (4 mg) as a white solid.

¹H NMR(500MHz,DMSO-d₆): δ8.30-8.26(m,1H),8.09(s,1H),7.93-7.87(m,1H),7.75-7.72(m,2H),7.67(d,J=2.6Hz,1H),7.62-7.56(m,1H),7.52-7.47(m,3H),7.36-7.26(m,4H),7.16(dd,J=8.7,2.6Hz,1H),6.58(d,J=9.2Hz,1H),2.87(brs,3H),2.45(s,3H),2.23(s, 3H)

### Examples 57 to 63

The compounds shown in Tables 3-1 and 3-2 were synthesized by the method described in Example 56 or a method similar thereto.

### Example 64

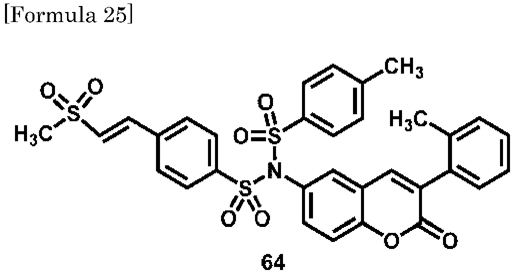

### 4-[(E)-2-methanesulfonylethenyl)-N-(4-methylbenzenesulfonyl)-N-[3-(2-methylphenyl)-2-oxo-2H-chromen-6-yl]benzene-1-sulfonamide

To a solution of 4-iodo-N-(4-methylbenzenesulfonyl)-N-[3-(2-methylphenyl)-2-oxo-2H-chromen-6-yl]benzene-1-sulfonamide (0.026 mL) obtained in Example 54 in acetonitrile (1 mL) were added methyl vinyl sulfone (7 mg), triethylamine (24 µL), tris(p-tolyl)phosphine (5 mg), and palladium(II) acetate (3 mg) at room temperature, and the mixture was stirred at 90°C under a nitrogen atmosphere for 4 hours. A saturated aqueous solution of ammonium chloride (10 mL) was added to the reaction mixture, and the mixture was extracted with ethyl acetate (30 mL). The extract was washed with water (10 mL) and saturated brine (10 mL). The organic layer was dried over sodium sulfate and concentrated. The residue was purified by silica gel column chromatography (ethyl acetate/hexane=0-30%) to obtain the title compound (8 mg) as a white solid.

¹H NMR(500MHz,DMSO-d₆): δ8.08(s,1H),8.04-8.02(m,2H),7.90-7.88(m,2H),7.77(d,J=15.5Hz,1H),7.75-7.73(m,2H),7.67(d,J=2.6Hz,1H),7.64(d,J=15.5Hz,1H),7.52-7.50(m,3H),7.36-7.28(m,4H),7.16(dd,J=8.7,2.6Hz,1H),3.15(s,3H),2.46(s,6H),2.23(s,3H)

### Example 65

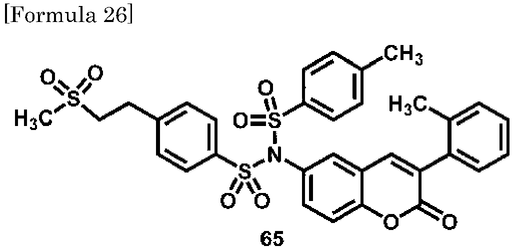

### 4-(2-methanesulfonylethyl)-N-(4-methylbenzenesulfonyl)-N-[3-(2-methylphenyl)-2-oxo-2H-chromen-6-yl]benzene-1-sulfonamide

To a solution of 4-[(E)-2-methanesulfonylethenyl)-N-(4-methylbenzenesulfonyl)-N-[3-(2-methylphenyl)-2-oxo-2H-chromen-6-yl]benzene-1-sulfonamide (6 mg) obtained in Example 64 in methanol (3 mL), 10% palladium carbon (40 mg) was added, and the mixture was stirred at room temperature under a hydrogen atmosphere for 1 day. The reaction mixture was filtered through a membrane filter, and the filtrate was concentrated to obtain the title compound (2 mg).

¹H NMR(500MHz,DMSO-d₆): δ8.07(s,1H),7.79-7.77(m,2H),7.71-7.69(m,2H),7.66-7.62(m,3H),7.50-7.49(m,3H),7.36-7.25(m,4H),7.11(dd,J=8.8,2.6Hz,1H),3.56-3.50(m,2H),3.20-3.16(m,2H),3.01(s,3H),2.46(s,6H),2.23(s,3H)

### Example 66

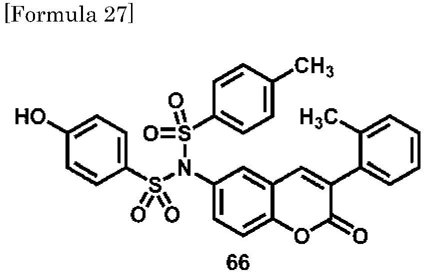

### 4-Hydroxy-N-(4-methylbenzenesulfonyl)-N-[3-(2-methylphenyl)-2-oxo-2H-chromen-6-yl]benzene-1-sulfonamide

To a solution of 4-methoxy-N-(4-methylbenzenesulfonyl)-N-[3-(2-methylphenyl)-2-oxo-2H-chromen-6-yl]benzene-1-sulfonamide (90 mg) obtained in Example 17 in dichloromethane (2.0 mL), a 1 M solution of boron tribromide in dichloromethane (0.470 mL) was added at -20°C, and the mixture was stirred at room temperature for 1 day. Methanol (1 mL) was added to the reaction mixture, followed by the addition of ethyl acetate (50 mL). This solution was washed with water (10 mL) and saturated brine (10 mL). The organic layer was dried over sodium sulfate and then concentrated. The residue was purified by silica gel column chromatography (ethyl acetate/hexane=45%) to obtain the title compound (20 mg) as a white solid.

¹H NMR(500MHz:CDCl₃): δ7.85-7.81(m,4H),7.54(s,1H),7.38-7.32(m, 4H),7.31-7.21(m, 4H),7.12(dd,J=8.8, 2.5Hz,1H), 6.97-6.94(m, 2H),5.91(brs,1H),2.48(s,3H),2.30(s,3H)

### Example 67

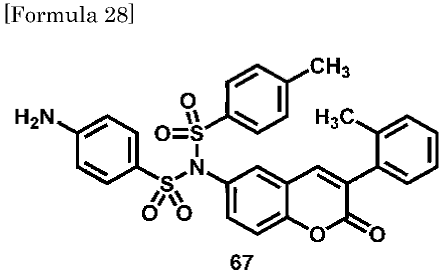

### 4-amino-N-(4-methylbenzenesulfonyl)-N-[3-(2-methylphenyl)-2-oxo-2H-chromen-6-yl]benzene-1-sulfonamide

N-(4-methylbenzenesulfonyl)-N-[3-(2-methylphenyl)-2-oxo-2H-chromen-6-yl]-4-nitrobenzene-1-sulfonamide obtained in Example 19 was used. The title compound (1.3 g) was obtained as a pale yellow solid by the method described in Reference Example 4-(1) or a method analogous thereto.

¹H NMR(500MHz,DMSO-d₆): δ8.08(s,1H),7.73-7.69(m,2H),7.62(d,J=2.6Hz,1H),7.49-7.46(m,3H),7.37-7.25(m,6H),7.06(dd,J=8.8,2.6Hz,1H),6.63-6.60(m,2H),6.44(brs,2H),2.45(s,3H),2.23(s,3H)

### Example 68

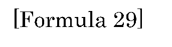

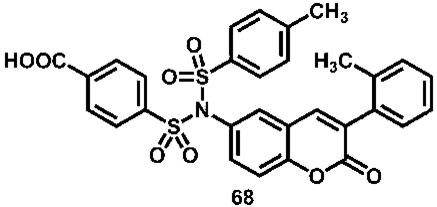

### 4-[(4-methylbenzenesulfonyl)[3-(2-methylphenyl)-2-oxo-2H-chromen-6-yl]sulfamoyl]benzoic acid

To a solution of methyl 4-({N-[3-(2-methylphenyl)-2-oxo-2H-chromen-6-yl]-4-methylbenzenesulfonamido}sulfonyl)benzoate (50 mg) obtained in Example 21 in acetic acid (0.5 mL), a 6 M hydrochloric acid solution (0.500 mL) was added, and the mixture was stirred at 100°C for 2 hours. The reaction mixture was concentrated under reduced pressure, the residue was suspended in water, and the precipitated solid was collected by filtration to obtain the title compound (43 mg) as a white solid.

¹H NMR(500MHz,CDCl₃): δ8.30-8.26(m,2H),8.10-8.06(m,2H),7.84-7.81(m,2H),7.54(s,1H),7.40-7.33(m,4H),7.31-7.22(m,4H),7.13(dd,J=8.8,2.5Hz,1H),2.50(s,3H),2.31(s,3H)

### Example 69

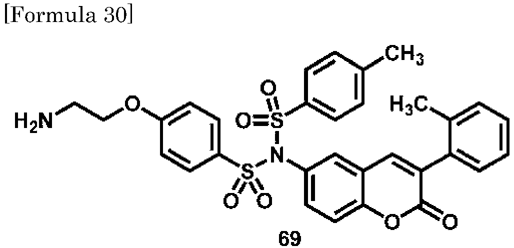

### 4-(2-aminoethoxy)-N-(4-methylbenzenesulfonyl)-N-[3-(2-methylphenyl)-2-oxo-2H-chromen-6-yl]benzene-1-sulfonamide

(1) To asolution of 4-hydroxy-N-(4-methylbenzenesulfonyl)-N-[3-(2-methylphenyl)-2-oxo-2H-chromen-6-yl]benzene-1-sulfonamide (76 mg) obtained in Example 66, tert-butyl N-(2-hydroxyethyl)carbamate (45 mg), and triphenylphosphine (110 mg) in tetrahydrofuran (1.5 mL), di-tert-butyl azodicarboxylate (65 mg) was added at 0°C, and the mixture was stirred at room temperature for 2 hours. A saturated aqueous solution of sodium hydrogen carbonate was added to the reaction mixture, and the mixture was extracted with ethyl acetate. The organic layer was washed with water and saturated brine, dried over sodium sulfate, and then concentrated. The residue was purified by silica gel column chromatography (ethyl acetate/hexane=25%) to obtain tert-butyl N-{2-[4-({N-[3-(2-methylphenyl)-2-oxo-2H-chromen-6-yl]-4-methylbenzenesulfonamido}sulfonyl)phenoxy]ethyl}carbamate as a white solid.
   ¹H NMR(500MHz,CDCl₃): δ7.90-7.86(m,2H),7.85-7.81(m,2H),7.54(s,1H),7.38-7.32(m,5H),7.31-7.21(m,3H),7.12(dd,J=8.8,2.5Hz,1H),7.03-6.99(m,2H),4.96(brs,1H),4.13(t,J=5.2Hz,2H),3.61-3.59(m,2H),2.49(s,3H),2.30(s,3H),1.46(s,9H)
(2) Trifluoroacetic acid (1 mL) was added to a solution of tert-butyl N-{2-[4-({N-[3-(2-methylphenyl)-2-oxo-2H-chromen-6-yl]-4-methylbenzenesulfonamido}sulfonyl)phenoxy]ethyl}carbamate (63 mg) obtained in the previous section in dichloromethane (2 mL), and the mixture was stirred at the same temperature for 4 hours. The reaction mixture was concentrated, neutralized with saturated sodium bicarbonate, and extracted with ethyl acetate. The organic layer was dried over sodium sulfate and then concentrated. The residue was dispersed in a hexane/dichloromethane (5:1) solution and then collected by filtration to obtain the title compound (37 mg) as a white solid.
   ¹H NMR (500MHz,DMSO-d₆): δ8.08(s,1H),7.74-7.71(m,4H),7.64(d,J=2.6Hz,1H),7.51-7.48(m,3H),7.37-7.25(m,4H),7.20-7.17(m,2H),7.11(dd,J=8.8,2.6Hz,1H),4.06(t,J=5.7Hz,2H),2.92(t,J=5.7Hz,2H),2.46(s,3H), 2.23(s,3H)

### Example 70

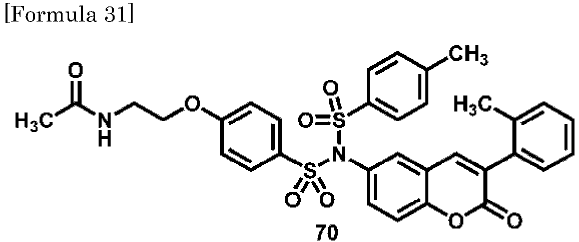

### N-(2-{4-[(4-methylbenzenesulfonyl)[3-(2-methylphenyl)-2-oxo-2H-chromen-6-yl]sulfamoyl]phenoxy}ethyl)acetamide

To a solution of 4-(2-aminoethoxy)-N-(4-methylbenzenesulfonyl)-N-[3-(2-methylphenyl)-2-oxo-2H-chromen-6-yl]benzene-1-sulfonamide (12 mg) obtained in Example 69 in dichloromethane (500 µL), triethylamine (8 µL) and acetyl chloride (2 µL) were added at room temperature, and the mixture was stirred at the same temperature for 5 days. Water was added to the reaction mixture, and the resulting solid was collected by filtration to obtain the title compound (9 mg) as a white solid.

¹H NMR(500MHz,CDCl₃): δ7.91-7.87(m,2H),7.85-7.81(m,2H),7.54(s,1H),7.38-7.32(m,4H),7.31-7.22(m,4H),7.12(dd,J=8.8,2.5Hz,1H),7.03-7.00(m,2H),5.92-5.86(m,1H),4.15(t,J=5.2Hz,2H),3.69(dt,J=5.2,5.2Hz,2H),2.49(s,3H),2.30(s,3H),2.04(s,3H )

### Example 71

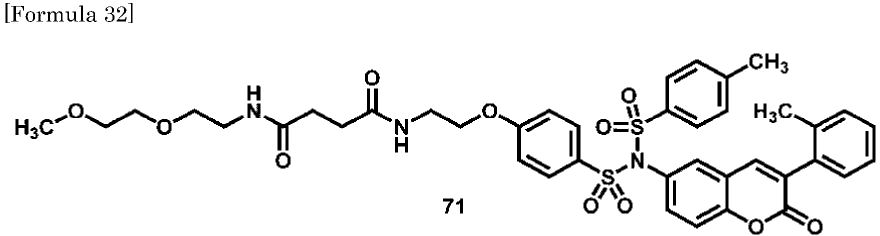

### N-[2-(2-methoxyethoxy)ethyl]-N'-(2-{4-[(4-methylbenzenesulfonyl)[3-(2-methylphenyl)-2-oxo-2H-chromen-6-yl]sulfamoyl]phenoxy}ethyl)butanediamide

The title compound was obtained using 4-hydroxy-N-(4-methylbenzenesulfonyl)-N-[3-(2-methylphenyl)-2-oxo-2H-chromen-6-yl]benzene-1-sulfonamide obtained in Example 66 and according to the method described in Example 69-(1) or a method analogous thereto.

¹H NMR(500MHz,CDCl₃): δ7.89-7.85(m,2H),7.84-7.81(m,2H),7.55(s,1H),7.38-7.32(m,4H),7.31-7.22(m,4H),7.12(dd,J=8.8,2.5Hz,1H),7.03-7.00(m,2H),6.62(t,J=6.0Hz,1H),6.27-6.22(brs,1H),4.14(t,J=5.2Hz,2H),3.69(q,J=5.2Hz,2H),3.62-3.59(m,2H),3.56-3.52(m,4H),3.43(q,J=5.2Hz,2H),3.39(s,3H),2.54(s,4H),2.49(s,3H),2.30(s,3H)

### Example 72

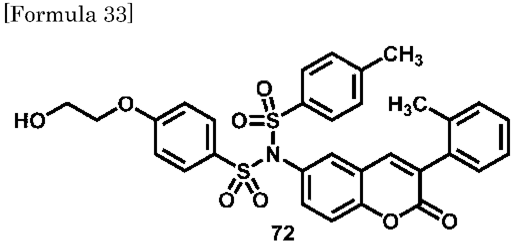

### 4-(2-hydroxyethoxy)-N-(4-methylbenzenesulfonyl)-N-[3-(2-methylphenyl)-2-oxo-2H-chromen-6-yl]benzene-1-sulfonamide

(1) To a solution of 4-hydroxy-N-(4-methylbenzenesulfonyl)-N-[3-(2-methylphenyl)-2-oxo-2H-chromen-6-yl]benzene-1-sulfonamide (56 mg) obtained in Example 66 in N,N-dimethylformamide (1 mL) were added potassium carbonate (69 mg) and O-trityloxyethyl bromide (73 mg), and the mixture was stirred at room temperature for one day. A saturated aqueous solution of ammonium chloride (10 mL) was added to the reaction mixture, and the mixture was extracted with ethyl acetate (30 mL). The organic layer was washed with water (20 mL x 3) and saturated brine (20 mL), dried over sodium sulfate, and then concentrated. The residue was purified by silica gel column chromatography (ethyl acetate/hexane=0-50%) to give 4-(2-trityloxyethoxy)-N-(4-methylbenzenesulfonyl)-N-[3-(2-methylphenyl)-2-oxo-2H-chromen-6-yl]benzene-1-sulfonamide (20 mg) as a white solid.
(2) The compound obtained in the previous section was treated as in Example 69-(2) or a method analogous thereto to give the title compound (3 mg) as a white solid.
   ¹H NMR(500MHz,CDCl₃): δ7.93-7.89(m,2H),7.84-7.81(m,2H),7.54(s,1H),7.37-7.32(m,4H),7.30-7.22(m,4H),7.11(dd,J=8.7,2.5Hz,1H),7.04-7.02(m,2H),4.76-4.74(m,2H),4.39-4,37(m,2H),3.50(s,1H),2.48(s,3H),2.30(s,3H)

### Example 73

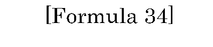

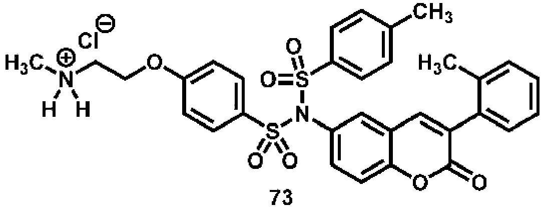

### 4-[2-(methylamino)ethoxy]-N-(4-methylbenzenesulfonyl)-N-[3-(2-methylphenyl)-2-oxo-2H-chromen-6-yl]benzene-1-sulfonamide hydrochloride

(1) Using 4-hydroxy-N-(4-methylbenzenesulfonyl)-N-[3-(2-methylphenyl)-2-oxo-2H-chromen-6-yl]benzene-1-sulfonamide obtained in Example 66, 4-[2-(N-methyl)-(N-tert-butoxycarbonyl)aminoethoxy]-N-(4-methylbenzenesulfonyl)-N-[3-(2-methylphenyl)-2-oxo-2H-chromen-6-yl]benzene-1-sulfonamide (37 mg) was obtained according to the method described in Example 69-(1) or a method analogous thereto.
(2) The target compound (30 mg) was obtained as a white solid by the method described in Example 69-(2) or a method analogous thereto.
   ¹H NMR(500MHz: DMSO-d₆): δ8.80(brs,2H),8.08(s,1H),7.84-7.80(m,2H),7.75-7.71(m,2H),7.62(d,J=2.6Hz,1H),7.53-7.49(m,3H),7.38-7.23(m,6H),7.10(dd,J=8.8,2.6Hz,1H),4.39(dd,J=4.9,4.9Hz,2H),3.42-3.37(m,2H),2.65(t,J=5.3Hz,3H),2.46(s,3H),2.23(s,3H)

### Example 74

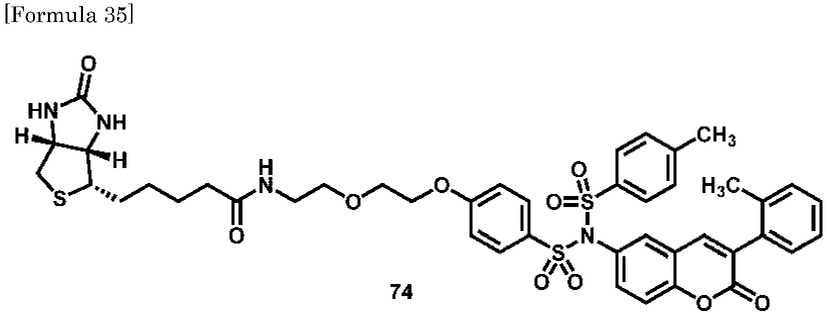

### 5-[(3aS,4S,6aS)-2-oxo-hexahydro-1H-thieno[3,4-d]imidazolidin-4-yl]-N-[2-(2-{4-[(4-methylbenzenesulfonyl)[3-(2-methylphenyl)-2-oxo-2H-chromen-6-yl]sulfamoyl]phenoxy}ethoxy)ethyl]pentanamide

(1) Using 4-hydroxy-N-(4-methylbenzenesulfonyl)-N-[3-(2-methylphenyl)-2-oxo-2H-chromen-6-yl]benzene-1-sulfonamide obtained in Example 66, N-(4-{N'-[3-(2-methylphenyl)-2-oxo-2H-chromen-6-yl]-N'-(4-methylbenzenesulfonyl)aminosulfonyl}phenoxyethoxyethyl)carbamic acid tert-butyl ester was obtained according to the method described in Example 69-(1) or a method analogous thereto.
(2) Using the compound obtained in the previous section, 4-[2-aminoethylethoxy]-N-(4-methylbenzenesulfonyl)-N-[3-(2methylphenyl)-2-oxo-2H-chromen-6-yl]benzenesulfonamide hydrochloride (30 mg) was obtained as a white solid according to the method described in Example 69-(2) or a method analogous thereto.
(3) To a solution of the compound obtained above in N,N-dimethylformamide (1 mL), triethylamine (0.024 mL) and biotin N-succinimide ester (30 mg) were added, and the mixture was stirred at room temperature for 3 days. The reaction mixture was diluted with ethyl acetate (50 mL) and then washed with water (10 mL x 4). The organic layer was dried over sodium sulfate and then concentrated. The residue was purified by silica gel column chromatography (methanol/dichloromethane=2-8%) to obtain the title compound (31 mg) as an orange solid.
   ¹H NMR(500MHz,DMSO-d₆): δ8.08(s,1H),7.86(t,J=5.7Hz,1H),7.75-7.71(m,4H),7.64(d,J=2.6Hz,1H),7.52-7.48(m,3H),7.37-7.25(m,4H),7.22-7.18(m,2H),7.10(dd,J=8.8,2.6Hz,1H),6.41(brs,1H),6.35(brs,1H),4.31-4.27(m,1H),4.26-4.22(m,2H),4.13-4.09(m,1H),3.79-3.75(m,2H),3.48(t,J=5.9Hz,2H),3.22(dd,J=11.7,5.9Hz,2H),3.11-3.05(m,1H),2.80(dd,J=12.4,5.1Hz,1H),2.57(d,J=12.4Hz,1H),2.46(s,3H),2.23(s,3H),2.06(t, J=7.4Hz,1H),1.69-1.40(m,5H),1.36-1.26(m,1H)

### Example 75

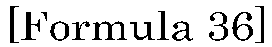

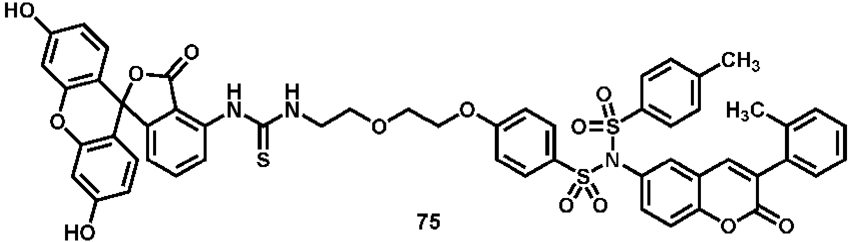

### 3-{3',6'-dihydroxy-3-oxo-3H-spiro[2-benzofuran-1,9'-xanthen]-5-yl}-1-[2-(2-{4-methylbenzenesulfonyl)[3-(2-methylphenyl)-2-oxo-2H-chromen-6-yl]sulfamoyl]phenoxy}ethoxy)ethyl]thiourea

(1) To a solution of 4-[2-(methylamino)ethoxy]-N-(4-methylbenzenesulfonyl)-N-[3-(2-methylphenyl)-2-oxo-2H-chromen-6-yl]benzene-1-sulfonamide hydrochloride (60 mg) obtained in Example 74-(2) in dichloromethane/methanol (2/1) (1.5 mL), fluorescein isothiocyanate (34 mg) was added, followed by the addition of triethylamine (0.024 mL) and stirring at room temperature for 2 hours. The reaction mixture was concentrated, and the residue was purified by silica gel column chromatography (methanol/dichloromethane=2-8%) to obtain the title compound (31 mg) as an orange solid.
   ¹H NMR(500MHz,DMSO-d₆): δ10.11(s,2H),10.02(brs,1H),8.26(s,1H),8.13(brs,1H),8.08(s,1H),7.74-7.70(m,5H),7.63(d,J=2.6Hz,1H),7.51-7.48(m,3H),7.36-7.25(m,4H),7.23-7.19(m,2H),7.18(d,J=8.4Hz,1H),7.10(dd,J=8.8,2.6Hz,1H),6.67(d,J=2.3Hz,1H),7.59(d,J=8 .7Hz,1H),6.55(dd,J=8.7,2.3Hz,1H),4.32-4.28(m,2H),3.88-3.84(m2H),3.77-3.68(m,4H),2.45(s,3H),2.23(s,3H)

### Examples 76-80

The compounds shown in Table 4-1 and Table 4-2 were obtained using 4-hydroxy-N-(4-methylbenzenesulfonyl)-N-[3-(2-methylphenyl)-2-oxo-2H-chromen-6-yl]benzene-1-sulfonamide obtained in Example 66 and according to the method described in Example 69-(1) or a method analogous thereto.

### Example 81

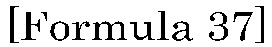

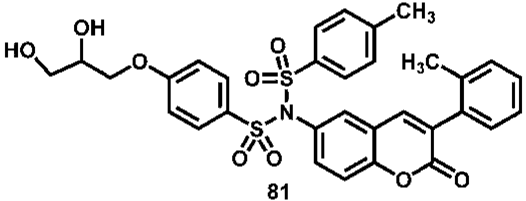

### 4-(Dihydroxypropyl)-N-(4-methylbenzenesulfonyl)-N-[3-(2-methylphenyl)-2-oxo-2H-chromen-6-yl]benzene-1-sulfonamide

To a solution of 4-[(2,2-dimethyl-1,3-dioxolan-4-yl)methoxy]-N-(4-methylbenzenesulfonyl)-N-[3-(2-methylphenyl)-2-oxo-2H-chromen-6-yl]benzene-1-sulfonamide (25 mg) obtained in Example 77 in dichloromethane/methanol (5/1) (5 mL), (+)-camphorsulfonic acid (5 mg) was added and the mixture was stirred at room temperature for 5 hours. The reaction mixture was neutralized with triethylamine and then concentrated under reduced pressure. The residue was purified by silica gel column chromatography (ethyl acetate/hexane=25%) to obtain the title compound (17 mg) as a white solid.

¹H NMR(500MHz,CDCl₃): δ7.91-7.87(m,2H),7.85-7.81(m,2H),7.54(s,1H),7.38-7.32(m,4H),7.31-7.22(m,4H),7.12(dd,J=8.8, 2.4Hz,1H),7.06-7.03(m,2H),4.21-4.14(m,3H),3.91-3.86(m,1H),3.81-3.76(m,1H),2.50(d,J=4.4Hz,1H),3.49(s,3H),2.49(s,3H),2.30(s,3H),1.89(d,J=5.9Hz,1H)

### Example 82

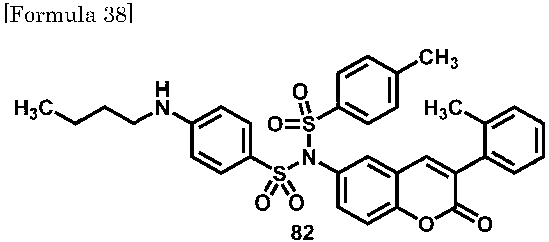

### 4-butylamino-N-(4-methylbenzenesulfonyl)-N-[3-(2-methylphenyl)-2-oxo-2H-chromen-6-yl]benzene-1-sulfonamide

To a solution of 4-amino-N-(4-methylbenzenesulfonyl)-N-[3-(2-methylphenyl)-2-oxo-2H-chromen-6-yl]benzene-1-sulfonamide (56 mg) obtained in Example 67 in 1,2-dichloroethane (1 mL), n-butylaldehyde (12 µL) was added, followed by the addition of sodium triacetoxyborohydride (55 mg), and the mixture was stirred at 60°C for 24 hours. Water (10 mL) was added to the reaction mixture, which was then extracted with dichloromethane (30 mL). The organic layer was dried over sodium sulfate and concentrated. The residue was purified by silica gel column chromatography (ethyl acetate/hexane=80%) to obtain the title compound (5 mg) as a white solid.

¹H NMR(500MHz,DMSO-d₆): δ8.07(s,1H),7.71-7.69(m,2H),7.60(d,J=2.6Hz,1H),7.48-7.46(m,3H),7.40-7.38(m,2H),7.33-7.28(m,4H),7.07(dd,J=8.8,2.6Hz,1H),6.98-6.96(m,1H),6.64-6.63(m,2H),3.12-3.08(m,2H),2.45(s,3H),2.23(s,3H),1.58-1.50(m,2H),1.42-1.36(m,2H),0.91(t,J=7.4Hz,3H)

### Example 83

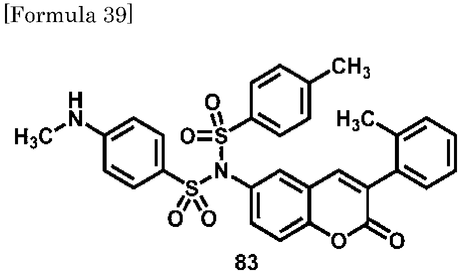

### 4-butylamino-N-(4-methylbenzenesulfonyl)-N-[3-(2-methylphenyl)-2-oxo-2H-chromen-6-yl]benzene-1-sulfonamide

To a solution of 4-amino-N-(4-methylbenzenesulfonyl)-N-[3-(2-methylphenyl)-2-oxo-2H-chromen-6-yl]benzene-1-sulfonamide (10 mg) obtained in Example 67 in dichloromethane (0.1 mL), diisopropylethylamine (6 µL) was added, followed by the addition of trimethyloxonium tetrafluoroborate (3 mg), and the mixture was stirred at room temperature for 4 hours. The reaction mixture was concentrated, and the residue was then purified by silica gel column chromatography (ethyl acetate/hexane=0-50%) to obtain the title compound (5 mg) as a white solid.

¹H NMR(500MHz,DMSO-d₆): δ8.07(s,1H),7.72-7.70(m,2H),7.49-7.45(m,2H),7.36-7.25(m,4H),7.06(dd,J=8.8,2.6Hz,1H),7.06(dd,J=8.8,2.6Hz,1H),7.02(d,J=4.9Hz,1H),6.64-6.59(m,2H),2.77(d,J=4.8Hz,3H),2.45(s,3H),2.23(s,3H)

### Example 84

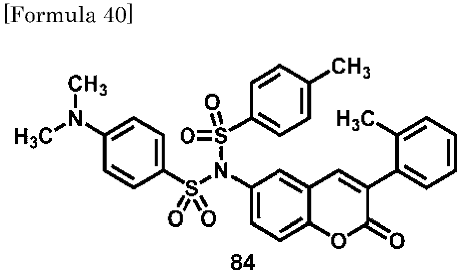

### 4-(dimethylamino)-N-(4-methylbenzenesulfonyl)-N-[3-(2-methylphenyl)-2-oxo-2H-chromen-6-yl]benzene-1-sulfonamide

To a solution of 4-amino-N-(4-methylbenzenesulfonyl)-N-[3-(2-methylphenyl)-2-oxo-2H-chromen-6-yl]benzene-1-sulfonamide (0.30 g) obtained in Example 67 in dichloromethane (9 mL), diisopropylethylamine (0.2 mL) was added, followed by the addition of trimethyloxonium tetrafluoroborate (87 mg), and the mixture was stirred at room temperature for 4 hours. The reaction mixture was concentrated, and the residue was then purified by silica gel column chromatography (ethyl acetate/hexane=0-50%) to obtain the title compound (43 mg) as a white solid.

¹H NMR(500MHz,DMSO-d₆): δ8.08(s,1H),7.72-7.70(m,2H),7.62(d,J=2.6Hz,1H),7.51-7.46(m,5H),7.36-7.25(m,4H),7.06(dd,J=8.8,2.6Hz,1H),6.80-6.78(m,2H),3.06(s,6H),2.45(s,3H),2.23(s,3H).

### Example 85

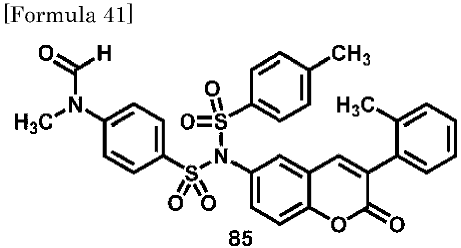

### N-methyl-N-[4-{N-[3-(2-methylphenyl)-2-oxo-2H-chromen-6-yl]4-methylbenzenesulfonamido}sulfonyl]phenyl]formamide

(1) To a solution of 4-(methylamino)-N-(4-methylbenzenesulfonyl)-N-[3-(2-methylphenyl)-2-oxo-2H-chromen-6-yl]benzene-1-sulfonamide (57 mg) obtained in Example 83 in dichloromethane (1 mL), triphosgene (45 mg) was added, and then triethylamine (0.025 mL) was added at 0°C, and the mixture was stirred at room temperature for 24 hours. The reaction mixture was concentrated, and the residue was then purified by silica gel column chromatography (ethyl acetate/hexane=0-50%) to give N-methyl-N-{4-[(4-methylbenzenesulfonyl)[3-(2-methylphenyl)-2-oxo-2H-chromen-6-yl]sulfamoyl]phenyl}carbamoyl chloride (51 mg) as a white solid.
(2) To a solution of the acid chloride (51 mg) obtained above in dichloromethane (1 mL), 2,3,4,6-tetra-O-benzyl-β-D-glucose (48 mg) was added, followed by the addition of triethylamine (0.022 mL), and the mixture was stirred at room temperature for 24 hours. The reaction solution was concentrated, and then the residue was purified by silica gel column chromatography (ethyl acetate/hexane=0-50%) to give N-methyl-N-{4-[(4-methylbenzenesulfonyl)[3-(2-methylphenyl)-2-oxo-2H-chromen-6-yl]sulfamoyl]phenyl}carbamic acid [3,4,5,6-tetrakis(benzyloxy)oxan-2-yl]methyl ester (51 mg) as a white solid.
(3) To a solution of the compound (58 mg) obtained above in ethyl acetate (10 mL), 10% Pd-C (40 mg) was added and the mixture was stirred at room temperature under a hydrogen atmosphere for 24 hours. The reaction mixture was filtered, and the filtrate was concentrated to obtain the title compound (14 mg) as a white solid.
   ¹H NMR(500MHz,DMSO-d₆): δ8.89(s,1H),8.08(s,1H),7.84-7.83(m,2H),7.74-7.72(m,2H),7.67-7.63(m,3H),7.52-7.50(m,3H),7.34-7.27(m,6H),7.16(dd,J=8.8,2.6Hz,1H),3.29(s,3H),2.46(s,3H),2.23(s,3H)

### Example 86

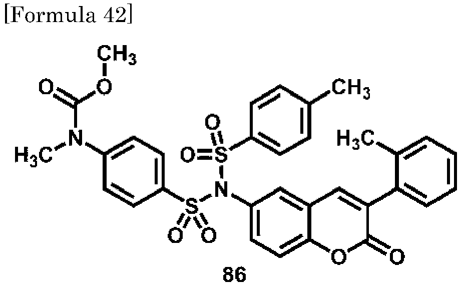

### N-methyl-N-{4-[(4-methylbenzenesulfonyl)[3-(2-methylphenyl)-2-oxo-2H-chromen-6-yl]sulfamoyl]phenyl]carbamic acid methyl ester

N-methyl-N-{4-[(4-methylbenzenesulfonyl)[3-(2-methylphenyl)-2-oxo-2H-chromen-6-yl]sulfamoyl]phenyl}carbamoyl chloride (20 mg) obtained in Example 85-(1) was dissolved in methanol (0.5 mL) and stirred at 50°C for 24 hours. The reaction solution was concentrated, and then the residue was purified by silica gel column chromatography (ethyl acetate/hexane=0-50%) to obtain N-methyl-N-{4-[(4-methylbenzenesulfonyl)[3-(2-methylphenyl)-2-oxo-2H-chromen-6-yl]sulfamoyl]phenyl}carbamoyl chloride (8 mg) as a white solid.

¹H NMR(500MHz,DMSO-d₆): δ8.06(s,1H),7.83-7.79(m,2H),7.72-7.68(m,2H),7.68-7.65(m,2H),7.64(dd,J=2.6Hz,1H),7.52-7.47(m,3H),7.37-7.25(m,4H),7.15(dd,J=8.8,2.6Hz,1H),3.70(s,3H),3.34(s,3H),2.45(s,3H),2.23(s,3H)

### Example 87

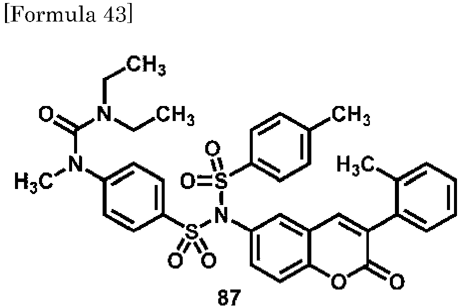

### 3,3-diethyl-1-methyl-1-[4-({N-[3-(2-methylphenyl)-2-oxo-2H-chromen-6-yl]-N-(4-methylbenzenesulfonyl)amino}sulfonyl)phenyl]urea

To a solution of N-methyl-N-{4-[(4-methylbenzenesulfonyl)[3-(2-methylphenyl)-2-oxo-2H-chromen-6-yl]sulfamoyl]phenyl}carbamoyl chloride (20 mg) obtained in Example 85-(1) in dichloromethane (0.5 mL), 1,2:3,4-di-O-isopropylidene-α-D-galactopyranose (13 mg) and triethylamine (0.014 mL) were added, and the mixture was stirred at room temperature for 20 hours and at 40°C for 24 hours. Further, 1,2-dichloroethane (0.5 mL) was added and the mixture was stirred at 70°C for 24 hours. The reaction mixture was concentrated, and the residue was purified by silica gel column chromatography (ethyl acetate/hexane=0-35%) to obtain the title compound (8 mg).

¹H NMR(500MHz,CDCl₃): δ7.84-7.82(m,2H),7.82-7.81(m,2H),7.54(s,1H),7.38-7.32(m,4H),7.30-7.24(m,3H),7.12(dd,J=8.7,2.5Hz,1H),7.04-7.02(m,2H),3.28(q,J=7.2Hz,4H),3.24(s,3),2.48(s,3H),2.30(s,3H),1.14(t,J=7.2Hz, 6H)

### Example 88

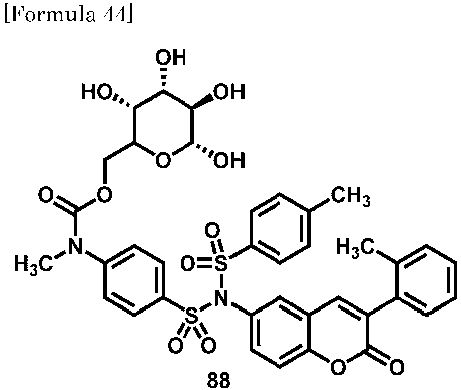

### N-methyl-N-(4-{(4-methylbenzenesulfonyl)[3-(2-methylphenyl)-2-oxo-2H-chromen-6-yl]sulfamoyl}phenyl)carbamic acid [(3R,4S,5R,6R)-3,4,5,6-tetrahydroxyoxan-2-yl]methyl ester

To a solution of 1,2:3,4-di-O-isopropylidene-α-D-galactopyranose (25 mg) in N,N-dimethylformamide (0.5 mL) was added 60% sodium hydride (3.3 mg) at 0°C, and then a solution of N-methyl-N-{4-[(4-methylbenzenesulfonyl)[3-(2-methylphenyl)-2-oxo-2H-chromen-6-yl]sulfamoyl]phenyl}carbamoyl chloride (40 mg) obtained in Example 85-(1) in N,N-dimethylformamide (0.5 mL) was added at 0°C, and the mixture was stirred at 0°C for 30 minutes. Water (10 mL) was added to the reaction mixture, which was then extracted with ethyl acetate (30 mL). The extract was washed with water (10 mL) and saturated brine (10 mL). The organic layer was dried over sodium sulfate and then concentrated. The residue was purified by silica gel column chromatography (ethyl acetate/hexane=50%). The obtained solid was dissolved in dichloromethane (0.2 mL), and water (0.2 mL) and trifluoroacetic acid (0.8 mL) were added at 0°C, followed by stirring at room temperature for 7 hours. The reaction mixture was concentrated, and the residue was suspended in water. The precipitate was collected by filtration to obtain the title compound (15 mg) as a white solid.

¹H NMR(500MHz,MeCN-d₆): δ7.81-7.78(m,0.8H),7.73-7.70(m,1.2H),7.60-7.58(m,0.8H),7.44-7.43(m,0.8H),7.40(d,J=8.8Hz,0.4H),7.36-7.30(m, 1.2H),7.28-7.27(m,0.8H),7.20(dd,J=8.8,2.6Hz,0.4H),5.10(d,J=3.7Hz,0.4H),4.25-4.23(m,0.4H),4.16-4.12(m,0.4H),3.80(d,J=3.4Hz,0.4H),3.65(dd,J=9.8,3.2Hz,0.4H),3.58(dd,J=9.8,3.6Hz,0.4H ),3.36(s,1.2H),2.47(s,1.2H),2.26(s,1.2H).

### Example 89

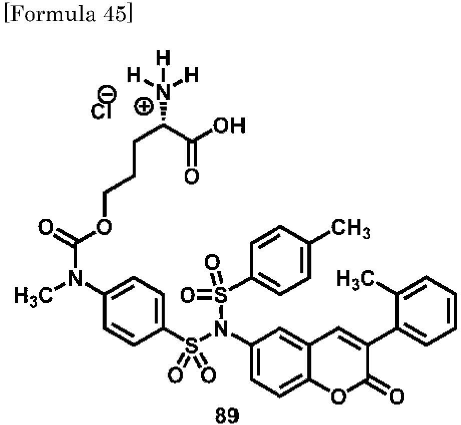

### (2S)-2-Amino-5-{[methyl({4-[(4-methylbenzenesulfonyl)[3-(2-methylphenyl)-2-oxo-2H-chromen-6-yl]sulfamoyl]phenyl})carbamoyl]oxy}pentanoic acid hydrochloride

(1) Using N-methyl-N-{4-[(4-methylbenzenesulfonyl)[3-(2-methylphenyl)-2-oxo-2H-chromen-6-yl]sulfamoyl]phenyl}carbamoyl chloride obtained in Example 85-(1), and in a manner described in Example 86 or a method analogous thereto, (2S)-2-{[(tert-butoxy)carbonyl]amino}-5-{[methyl({4-[(4-methylbenzenesulfonyl)[3-(2-methylphenyl)-2-oxo-2H-chromen-6-yl]sulfamoyl]phenyl})carbamoyl]oxy}pentanoic acid tert-butyl ester (34 mg) was obtained.
(2) The compound obtained above was treated as in Example 69-(2) or a method analogous thereto to give the title compound (6 mg).
   ¹H NMR(500MHz,DMSO-d₆): δ8.10(s,1H),7.83-7.79(m,2H),7.73-7.66(m,5H),7.52(d,J=8.6Hz,1H),7.50-7.48(m,2H),7.36-7.26(m,4H),7.15(dd,J=8.7,2.6Hz,1H),4.12-4.09(m,2H),3.38-3.37(m,1H),3.35(s,3H),3.10-2.91(m,2H),2.45(s,3H),2.23(s,3H),1.74-1.70(m,2H).

### Example 90

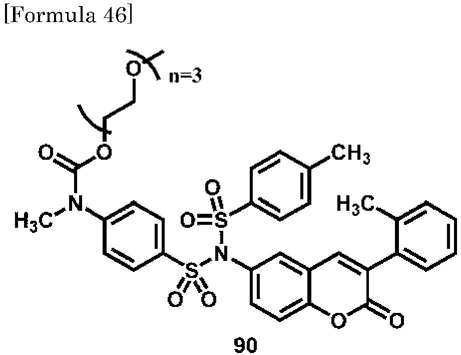

### N-methyl-N-{4-[(4-methylbenzenesulfonyl)[3-(2-methylphenyl)-2-oxo-2H-chromen-6-yl]sulfamoyl]phenyl}carbamic acid 2-[2-(2-methoxyethoxy)ethoxy]ethyl ester

The title compound (15 mg) was obtained using N-methyl-N-{4-[(4-methylbenzenesulfonyl)[3-(2-methylphenyl)-2-oxo-2H-chromen-6-yl]sulfamoyl]phenyl}carbamoyl chloride obtained in Example 85-(1), and according to a method in Example 86 or in a similar manner thereto.

¹H NMR(500MHz,DMSO-d₆): δ8.07(s,1H),7.82-7.79(m, 2H),7.73-7.66(m,4H),7.64(d,J=2.5Hz,1H),7.53-7.48(m,3H),7.37-7.26(m,4H),7.14(dd,J=8.7,2.6Hz,1H),4.23-4.22(m,2H),3.66-3.63(m,2H),3.54-3.47(m,6H),3.39-3.38(m,2H),3.35(s,3H),3.19(s, 3H),2.46(s,3H),2.23(s,3H)

### Example 91

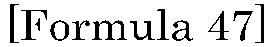

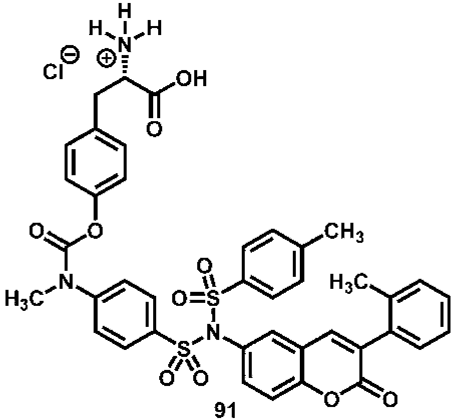

### (2S)-2-Amino-3(4-{[N-methyl({4-[(4-methylbenzenesulfonyl)[3-(2-methylphenyl)-2-oxo-2H-chromen-6-yl]sulfamoyl]phenyl})carbamoyl]oxy}phenyl)propionic acid hydrochloride

The title compound (23 mg) was obtained using N-methyl-N-{4-[(4-methylbenzenesulfonyl)[3-(2-methylphenyl)-2-oxo-2H-chromen-6-yl]sulfamoyl]phenyl}carbamoyl chloride obtained in Example 85-(1), and according to a method in Example 86 or in a similar manner thereto.

¹H NMR(500MHz,DMSO-d₆): δ8.29(brs,4H),8.07(s,1H),7.90-7.88(m,2H),7.82-7.79(m,2H),7.69-7.68(m,2H),7.66(d,J=2.6Hz,1H),7.51-7.47(m,3H),7.35-7.27(m,6H),7.22-7.20(m,2H),7.15(dd,J=8.6,2.6Hz,1H),4.42-4.40(m,1H),3.56(s,3H),3.47(s,2H),2.44(s,3H),2.23(s,3H)

### Example 92

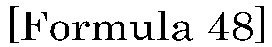

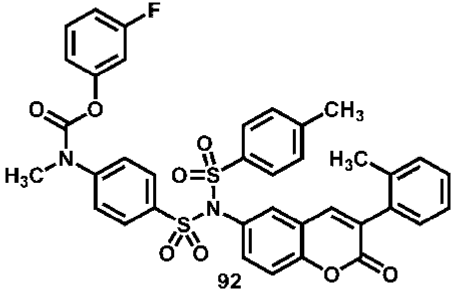

### N-methyl-N-{4-[(4-methylbenzenesulfonyl)[3-(2-methylphenyl)-2-oxo-2H-chromen-6-yl]sulfamoyl]phenyl}carbamic acid 3-fluorophenyl ester

The title compound (10 mg) was obtained using N-methyl-N-{4-[(4-methylbenzenesulfonyl)[3-(2-methylphenyl)-2-oxo-2H-chromen-6-yl]sulfamoyl]phenyl}carbamoyl chloride obtained in Example 85-(1), and according to the method of Example 86 or in a similar manner thereto..

¹H NMR(500MHz,DMSO-d₆): δ8.06(s,1H),7.89-7.87(m,2H),7.82-7.80(m,2H),7.68-7.66(m,3H),7.51(d,J=9.0Hz,1H),7.48-7.46(m,2H),7.36-7.27(m,5H),7.25-7.23(m,1H),7.15(dd,J=8.7,2.6Hz,1H),7.14-7.10(m,2H),3.48(s,3H),2.43(s,3H),2.22(s,3H)

### Example 93

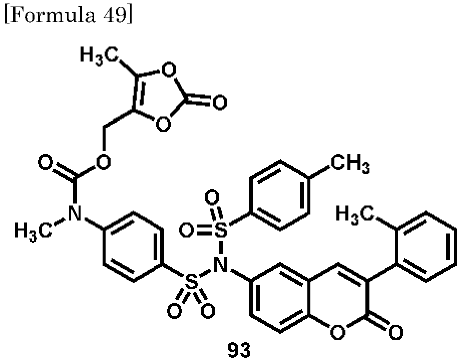

### N-methyl-N-{4-[(4-methylbenzenesulfonyl)[3-(2-methylphenyl)-2-oxo-2H-chromen-6-yl]sulfamoyl]phenyl}carbamic acid (5-methyl-2-oxo-2H-1,3-dioxol-4-yl)methyl ester

The title compound (1.4 mg) was obtained using N-methyl-N-{4-[(4-methylbenzenesulfonyl)[3-(2-methylphenyl)-2-oxo-2H-chromen-6-yl]sulfamoyl]phenyl}carbamoyl chloride obtained in Example 85-(1), and according to a method in Example 86 or in a similar manner thereto.

¹H NMR(500MHz,DMSO-d₆): δ8.07(s,1H),7.84-7.82(m,2H),7.70-7.65(m,5H),7.52-7.48(m,3H),7.38-7.26(m,4H),7.16(dd,J=8.7,2.6Hz,1H),5.04(s,2H),2.45(s,3H),2.23(s,3H),2.17(s,3H)

### Example 94

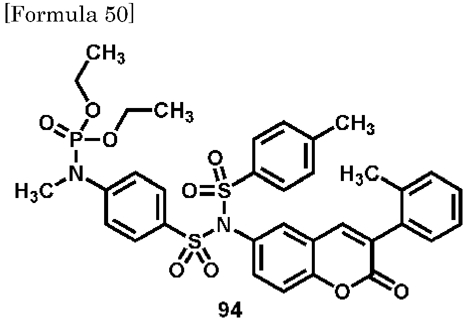

### 4-[N-(diethylphosphoryl)-N-(methyl)amino]-N-(4-methylbenzenesulfonyl)-N-[3-(2-methylphenyl)-2-oxo-2H-chromen-6-yl]benzene-1-sulfonamide

To a solution of 4-(methylamino)-N-(4-methylbenzenesulfonyl)-N-[3-(2-methylphenyl)-2-oxo-2H-chromen-6-yl]benzene-1-sulfonamide (0.150 g) obtained in Example 83 in tetrahydrofuran (2.6 mL), diethylphosphoryl chloride (0.076 mL) and a 1 M THF solution (0.52 mL) of potassium hexamethyldisilazide were added at 0°C, and the mixture was stirred at the same temperature for 2 hours and then at room temperature for 4 hours. Water (20 mL) was added to the reaction mixture, which was then extracted with ethyl acetate (50 mL). The organic layer was dried over sodium sulfate and concentrated. The residue was purified by silica gel column chromatography (ethyl acetate/hexane=0-50%) to obtain the title compound (36 mg) as a white solid.

¹H NMR(500MHz,DMSO-d₆): δ8.05(s,1H),7.76-7.74(m,2H),7.72-7.69(m,2H),7.63(d,J=2.5Hz,1H),7.55-7.48(m,5H),7.37-7.25(m,4H),7.13(dd,J=8.8, 2.6Hz,1H),4.10-3.98(m,4H),3.22(d,J=8.2Hz,3H),2.46(s,3H),2.23(s,3H),1.26-1.20(m,6H)

### Example 95

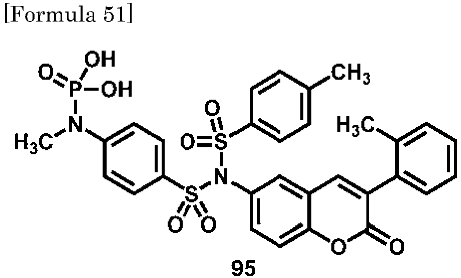

### 4-[N-(phosphono)-N-(methyl)amino]-N-(4-methylbenzenesulfonyl)-N-[3-(2-methylphenyl)-2-oxo-2H-chromen-6-yl]benzene-1-sulfonamide

Trimethylsilyl bromide (0.015 mL) was added to a solution of 4-[N-(diethylphosphoryl)-N-(methyl)amino]-N-(4-methylbenzenesulfonyl)-N-[3-(2-methylphenyl)-2-oxo-2H-chromen-6-yl]benzene-1-sulfonamide (10 mg) obtained in Example 94 in acetonitrile (0.66 mL) at 0°C, and the mixture was stirred at 50°C for 50 hours. The reaction mixture was purified by preparative thin-layer chromatography (methanol/ethyl acetate=25%) to obtain the title compound (2.5 mg).

¹H NMR(500MHz,DMSO-d₆): δ8.06(s,1H),7.73-7.70(m,4H),7.68-7.67(m,1H),7.51-7.49(m,3H),7.47-7.44(m,2H),7.35-7.29(m,4H),7.16(dd,J=8.6,2.6Hz,1H),2.45(s,3H),2.23(s,3H)

### Example 96

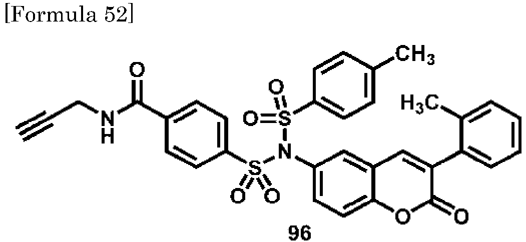

### 4-({N-[3-(2-methylphenyl)-2-oxo-2H-chromen-6-yl]-4-methylbenzenesulfonamide}sulfonyl)-N-(prop-2-yn-1-yl)benzamide

To a solution of 4-[(4-methylbenzenesulfonyl)[3-(2-methylphenyl)-2-oxo-2H-chromen-6-yl]sulfamoyl]benzoic acid (15 mg) obtained in Example 68 in dichloromethane (500 µL), prop-2-yn-1-amine (2 µL), 1-hydroxybenzotriazole (5 mg), and 1-ethyl-3-(3-dimethylaminopropyl)carbodiimide hydrochloride (2 mg) were added at room temperature, and the mixture was stirred at the same temperature for 1 day. A saturated aqueous solution of sodium bicarbonate was added to the reaction mixture, followed by extraction with ethyl acetate. The organic layer was washed with saturated aqueous ammonium chloride solution, water and saturated brine, dried over sodium sulfate, and then concentrated. The residue was purified by silica gel column chromatography (ethyl acetate/hexane=33%) to obtain the title compound (9 mg) as a white solid.

¹H NMR(500MHz,CDCl₃): δ8.07-8.04(m,2H),7.98-7.95(m,2H),7.83-7.80(m,2H),7.54(s,1H),7.39-7.32(m,4H),7.31-7.22(m,4H),7.11(dd,J=8.8,2.5Hz,1H),6.33(t,J=5.4Hz,1H),4.31-4.29(m,2H),2.50(s,3H),2.33(t,J=2.6Hz,1H),2.30(s,3H)

### Examples 97 to 99

The compounds shown in Table 5 were synthesized by the method described in Example 96 or a method similar thereto.

### Example 100

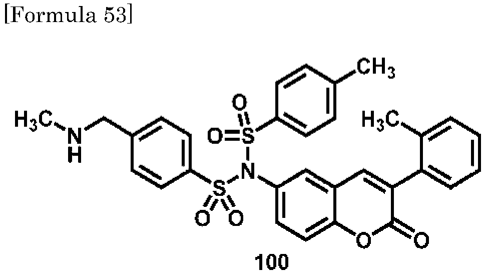

### 4-[(methylamino)methyl]-N-(4-methylbenzenesulfonyl)-N-[3-(2-methylphenyl)-2-oxo-2H-chromen-6-yl]benzene-1-sulfonamide

(1) Using 4-methyl-N-[3-(2-methylphenyl)-2-oxo-2H-chromen-6-yl]benzene-1-sulfonamide obtained in Reference Example 5, 4-formyl-N-(4-methylbenzenesulfonyl)-N-[3-(2-methylphenyl)-2-oxo-2H-chromen-6-yl]benzene-1-sulfonamide was obtained in accordance with the method of Example 15 or a method analogous thereto.
(2) The compound obtained above was treated in the same manner as in Example 82 or a method analogous thereto to obtain the title compound.
   ¹H NMR(500MHz,CDCl₃): δ8.07-8.04(m,2H),7.98-7.95(m,2H),7.83-7.80(m,2H),7.54(s,1H),7.39-7.32(m,4H),7.31-7.22(m,4H),7.11(dd,J=8.8,2.5Hz,1H),6.33(t,J=5.4Hz,1H),4.31-4.29(m,2H),2.50(s,3H),2.33(t,J=2.6Hz,1H),2.30(s,3H)

### Example 101

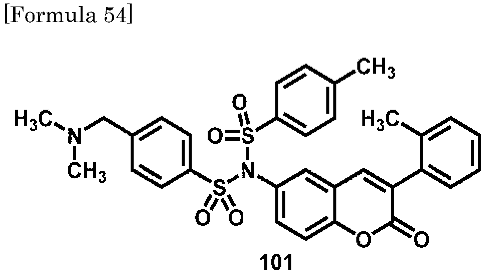

### 4-[(dimethylamino)methyl]-N-(4-methylbenzenesulfonyl)-N-[3-(2-methylphenyl)-2-oxo-2H-chromen-6-yl]benzene-1-sulfonamide

To a solution of 4-[(methylamino)methyl]-N-(4-methylbenzenesulfonyl)-N-[3-(2-methylphenyl)-2-oxo-2H-chromen-6-yl]benzene-1-sulfonamide (26 mg) obtained in Example 100 in N,N-dimethylformamide (1 mL), potassium carbonate (18 mg) and methyl iodide (4 µL) were added and stirred at room temperature for 3 hours. Water (10 mL) was added to the reaction mixture, which was then extracted with ethyl acetate (30 mL). The extract was washed with water (10 mL x 2), dried over sodium sulfate, and concentrated. The residue was purified by silica gel column chromatography (hexane:ethyl acetate=0-100%, methanol/ethyl acetate=100%) to obtain the title compound (24 mg) as a white solid.

¹H NMR(500MHz,DMSO-d₆): δ8.05(s,1H),7.78-7.76(m,2H),7.71-7.69(m,2H),7.63(d,J=2.6Hz,1H),7.60-7.58(m,2H),7.51-7.48(m,3H),7.36-7.29(m,4H),7.13(dd,J=8.8,2.6Hz,1H),3.53(s,2H),2.45(s,3H),2.22(s,3H),2.18(s,6H)

### Example 102

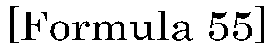

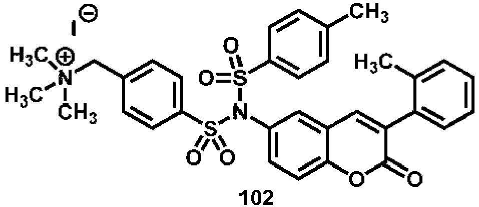

### Trimethyl({4-[(4-methylbenzenesulfonyl)[3-(2-methylphenyl)-2-oxo-2H-chromen-6-yl]sulfamoyl]phenyl}methyl)ammonium iodide

The title compound was obtained using 4-[(dimethylamino)methyl]-N-(4-methylbenzenesulfonyl)-N-[3-(2-methylphenyl)-2-oxo-2H-chromen-6-yl]benzene-1-sulfonamide obtained in Example 101, and according to the method of Example 101 or in a method analogous thereto.

¹H NMR(500MHz,DMSO-d₆): δ8.05(s,1H),7.98-7.96(m,2H),7.84-7.83(m,2H),7.77-7.76(m,2H),7.66(d,J=2.6Hz,1H),7.53-7.50(m,3H),7.38-7.28(m,4H),7.20(dd,J=8.8,2.6Hz,1H),4.67(s,2H),3.08(s,9H),2.47(s,3H),2.23(s,3H)

### Examples 103-109

The compounds shown in Table 6-1 and Table 6-2 were obtained using 4-formyl-N-(4-methylbenzenesulfonyl)-N-[3-(2-methylphenyl)-2-oxo-2H-chromen-6-yl]benzene-1-sulfonamide obtained in Example 100-(1) and according to the method of Example 82 or a method analogous thereto.

### Example 110

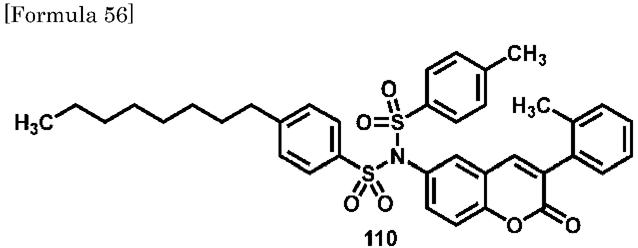

### 4-Octyl-N-(4-methylbenzenesulfonyl)-N-[3-(2-methylphenyl)-2-oxo-2H-chromen-6-yl]benzene-1-sulfonamide

To a suspension of n-heptylphosphonium bromide (88 mg) in THF (1 mL) was added a 1.56 M n-butyllithium hexane solution (0.12 mL) at 0°C, and the mixture was stirred at the same temperature for 30 minutes. To the reaction mixture was added a solution of 4-formyl-N-(4-methylbenzenesulfonyl)-N-[3-(2-methylphenyl)-2-oxo-2H-chromen-6-yl]benzene-1-sulfonamide (57 mg) obtained in Example 100-(1) in THF (0.5 mL) at 0°C, and the mixture was stirred at the same temperature for 1 hour. A saturated aqueous solution of ammonium chloride (15 mL) was added to the reaction mixture, which was then extracted with ethyl acetate (40 mL). The extract was washed with water (10 mL) and saturated brine (10 mL), dried over sodium sulfate, and then concentrated. The residue was purified by silica gel column chromatography (ethyl acetate/hexane=0-50%). The obtained compound was treated according to the method of Example 65 or a method analogous thereto to give the title compound (34 mg) as a white solid.

### ¹H NMR(500MHz,DMSO-d₆): δ8.05(s,1H),7.73-7.71(m,2H),7.70-7.68(m,2H),7.62(d,J=2.6Hz,1H),7.51-7.47(m,5H),7.37-7.26(m,4H),7.11(dd,J=8.7,2.6Hz,1H),2.71(t,J=7.8Hz,2H),2.45(s,3H),2.23(s,3H),1.64-1.58(m,2H),1.32-1.20(m,10H),0.85(t,J=7.1Hz,3H)

### Example 111

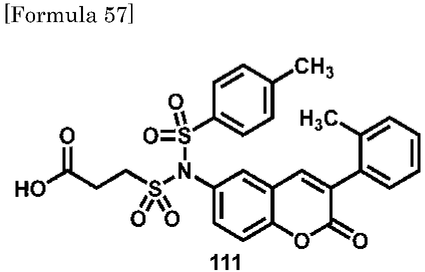

### 3-({N-[3-(2-methylphenyl)-2-oxo-2H-chromen-6-yl]-4-methylbenzenesulfonamide}sulfonyl)propionic acid

(1) 4-methyl-N-[3-(2-methylphenyl)-2-oxo-2H-chromen-6-yl]benzene-1-sulfonamide obtained in Reference Example 5 was treated according to the method of Example 15 or a method analogous thereto to give 3-({N-[3-(2-methylphenyl)-2-oxo-2H-chromen-6-yl]methylbenzenesulfonamide}sulfonyl)propionic acid methyl ester.
(2) To a solution of the compound (15 mg) obtained above in acetic acid/1,4-dioxane (1/1) was added 6M hydrochloric acid (0.5 mL), and the mixture was stirred at 100°C for 2 hours. The reaction mixture was concentrated to obtain the title compound (5.2 mg) as a pale brown solid.
   ¹H NMR(500MHz,CDCl₃): δ7.75-7.70(m,2H),7.57(s,1H),7.38-7.17(m,9H),4.07(t,J=7.4Hz,2H),3.06(d,J=7.6Hz,2H),2.47(s,3H),2.29(s,3H)

### Example 112

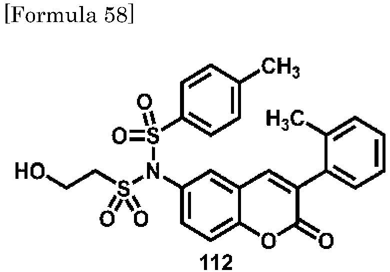

### N-(2-hydroxyethylsulfonyl)-4-methyl-N-[3-(2-methylphenyl)-2-oxo-2H-chromen-6-yl]benzene-1-sulfonamide

(1) 4-methyl-N-[3-(2-methylphenyl)-2-oxo-2H-chromen-6-yl]benzene-1-sulfonamide obtained in Reference Example 5 was treated according to the method of Example 15 or a method analogous thereto to give N-(2-benzyloxyethylsulfonyl)-4-methyl-N-[3-(2-methylphenyl)-2-oxo-2H-chromen-6-yl]benzene-1-sulfonamide.
(2) The compound obtained above was treated in the same manner as in Example 65 or a method analogous thereto to give the title compound (8 mg) as a white solid.
   ¹H NMR(500MHz,CDCl₃): δ7.77-7.74(m,2H),7.57(s,1H),7.41(d,J=2.5Hz,1H),7.38-7.32(m,4H),7.31-7.28(m,1H),7.27-7.21(m,3H),4.28-4.23(m,2H),3.96-3.91(m,2H),2.48(s,3H),2.44(t,J=6.7Hz,1H),2.30(s,3H)

### Example 113

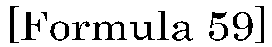

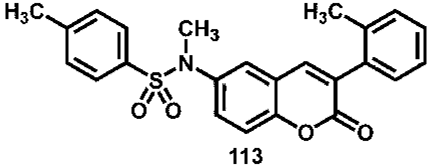

### N,4-dimethyl-N-[3-(2-methylphenyl)-2-oxo-2H-chromen-6-yl]benzene-1-sulfonamide

Using 4-methyl-N-[3-(2-methylphenyl)-2-oxo-2H-chromen-6-yl]benzene-1-sulfonamide (40 mg) obtained in Reference Example 5 and according to the method of Example 101 or a method analogous thereto, the title compound (41 mg) was obtained as a white solid.

¹H NMR(500MHz,CDCl3): δ7.58(s,1H),7.46(d,J=8.3Hz,2H),7.39(d,J=2.6Hz,1H),7.33(ddd,J=7.7,6.9,1.9Hz,1H),7.29-7.24(m,5H),7.23(dd,J=7.7,1.8Hz,1H),7.14(dd,J=8.9,2.6Hz,1H),3.19(s,3H),2.44(s,3H),2.30 (s,3H)

### Examples 114 to 122

The compounds shown in Tables 7-1 to 7-3 were synthesized according to the method of Example 113.

### Example 123

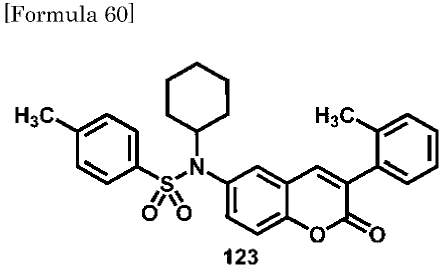

### N-cyclohexyl-4-methyl-N-[3-(2-methylphenyl)-2-oxo-2H-chromen-8-yl]benzene-1-sulfonamide

To a solution of 4-methyl-N-[3-(2-methylphenyl)-2-oxo-2H-chromen-6-yl]benzene-1-sulfonamide (80 mg) obtained in Reference Example 5 in dichloromethane (2 mL), cyclohexene (0.08 mL), potassium carbonate (16 mg), and boron trifluoride diethyl ether complex (0.150 mL) were added, and the mixture was stirred at 80°C for 22 hours. The reaction mixture was diluted with ethyl acetate (30 mL), washed with water (20 mL) and saturated brine (20 mL), dried over sodium sulfate, and then concentrated. The residue was purified by silica gel column chromatography (ethyl acetate/hexane=17%) to obtain the title compound (8 mg) as a yellow solid.

¹H NMR(500MHz,CDCls): δ7.63(d,J=8.3Hz,2H),7.57(s,1H),7.35-7.28(m,5H),7.24-7.23(m,1H),7.09(dd,J=8.7,2.4Hz,1H),4.18(tt,J=12.0,3.6Hz,1H),2.43(s,3H),2.30(s,3H),1.8 7-1.85(m,2H),1.74-1.72(m,2H),1.57-1.46(m,1H),1.43-1.32(m,2H),1.05(dddd,J=12.5,12.5,12.5,3.5Hz,2H),0.81(tt,J=13.2,3.6Hz,1H)

### Example 124

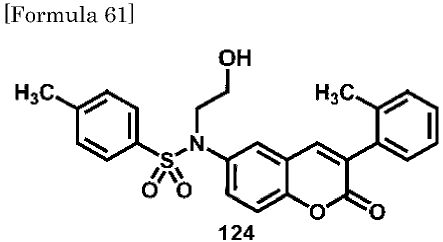

### N-(2-hydroxyethyl)-N-[3-(2-methylphenyl)-2-oxo-2H-chromen-6-yl]-4-methylbenzenesulfonamide

Using 4-methyl-N-[3-(2-methylphenyl)-2-oxo-2H-chromen-6-yl]benzene-1-sulfonamide obtained in Reference Example 5 and according to the method of Example 101 or a method analogous thereto, N-(2-methoxymethoxyethyl)-N-[3-(2-methylphenyl)-2-oxo-2H-chromen-6-yl]-4-methylbenzenesulfonamide was obtained. To a solution of the obtained compound in dichloromethane (1 mL) was added a 10% solution of hydrogen chloride in methanol (1 mL), and the mixture was stirred at room temperature for one day. The reaction mixture was concentrated, and the residue was then purified by silica gel column chromatography (ethyl acetate/hexane=40%) to obtain the title compound (11 mg) as a white solid.

¹H NMR(500MHz,CDCls): δ7.59(s,1H),7.56-7.53(m,2H),7.44(d,J=2.5Hz,1H),7.37-7.33(m,1H),7.33-7.29(m,4H),7.28-7.23(m,2H),7.09(dd,J=8.8,2.5Hz,1H),3.76-3.68(m,4H),2.46(s,3H),2.31(s,3H),1.93(t,J=5.2Hz,1H)

### Example 125

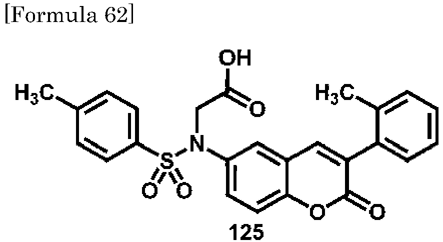

### 2-{N-[3-(2-methylphenyl)-2-oxo-2H-chromen-6-yl]}-N-(4-methylbenzenesulfonyl)aminoacetic acid

(1) Using 4-methyl-N-[3-(2-methylphenyl)-2-oxo-2H-chromen-6-yl]benzene-1-sulfonamide obtained in Reference Example 5, 2-{N-[3-(2-methylphenyl)-2-oxo-2H-chromen-6-yl]}-N-(4-methylbenzenesulfonyl)aminoacetic acid tert-butyl ester was obtained in accordance with the method of Example 101 or a method analogous thereto.
(2) The 2-{N-[3-(2-methylphenyl)-2-oxo-2H-chromen-6-yl]}-N-(4-methylbenzenesulfonyl)aminoacetic acid tert-butyl ester obtained above was treated by the method of Example 69-(2) or a method analogous thereto to give the title compound (13 mg).
   ¹H NMR(500MHz,CDCl₃): δ7.59-7.54(m,4H),7.36-7.32(m,1H),7.31-7.19(m,7H),4.46(s,2H),2.44(s,3H),2.29(s,3H)

### Example 126

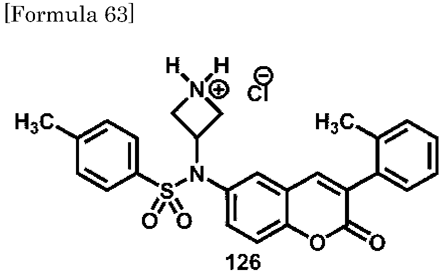

### N-(azetidin-3-yl)-4-methyl-N-[(3-methylphenyl)-2-oxo-2H-chromen-6-yl]benzene-1-sulfonamide hydrochloride

(1) Using 4-methyl-N-[3-(2-methylphenyl)-2-oxo-2H-chromen-6-yl]benzene-1-sulfonamide obtained in Reference Example 5, N-(1-benzhydrylazetidin-3-yl)-4-methyl-N-[(3-methylphenyl)-2-oxo-2H-chromen-6-yl]benzene-1-sulfonamide was obtained according to the method of Example 101 or a method analogous thereto. To the obtained compound was added 4M HCl/AcOEt (1 mL), and the mixture was stirred at room temperature for 30 minutes. The precipitated solid was collected by filtration and converted into the hydrochloride salt.
(2) The compound obtained above was treated in the same manner as in Example 65 or a method analogous thereto to give the title compound (13 mg) as a pale brown solid.
   ¹H NMR(500MHz: DMSO-d₆): δ 9.01(brs,1H),8.67(brs,1H),8.02(s,1H),7.57(d,J=2.6Hz,1H),7.46(d,J=8.8Hz,1H),7.44(s,4H) ,7.38-7.25(m,4H),7.08(dd,J=8.8,2.6Hz,1H),4.79-4.72(m,1H),4.14-4.06(m,2H),3.90-3.81(m,2H),2.42(s,3H),2.22(s,3H)

### Example 127

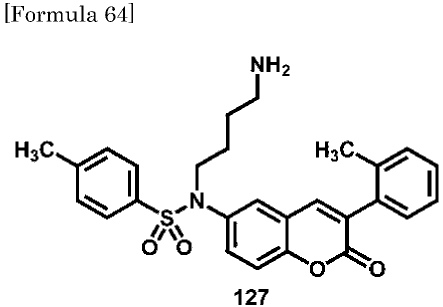

### N-(4-aminobutyl)-4-methyl-N-[3-(2-methylphenyl)-2-oxo-2H-chromen-6-yl]benzene-1-sulfonamide

(1) Using 4-methyl-N-[3-(2-methylphenyl)-2-oxo-2H-chromen-6-yl]benzene-1-sulfonamide obtained in Reference Example 5, N-[4-(1,3-dioxo-2,3-dihydro-1H-isoindol-2-yl)butyl]-4-methyl-N-[3-(2-methylphenyl)-2-oxo-2H-chromen-6-yl]benzene-1-sulfonamide was obtained according to the method of Example 101 or a method analogous thereto.
(2) To a solution of the compound obtained above (35 mg) in dichloromethane/methanol (1/1, 1 mL) was added hydrazine monohydrate (0.014 mL), and the mixture was stirred at room temperature for two days. The reaction mixture was concentrated, the residue was diluted with dichloromethane, and insoluble materials were removed by filtration. The filtrate was concentrated to give the title compound (27 mg).
   ¹H NMR(500MHz,CDCl₃): δ7.59 (s,1H),7.51-7.48(m,2H),7.38(d,J=2.5Hz,1H),7.37-7.31(m,1H),7.32-7.23(m,6H),7.06(dd,J=8.8,2.5Hz,1H),3.59-3.55(m,2H),2.70-2.66(m,2H),2.45(s,3H),2.32(s,3H),1.52-1.47(m,4H)

### Example 128

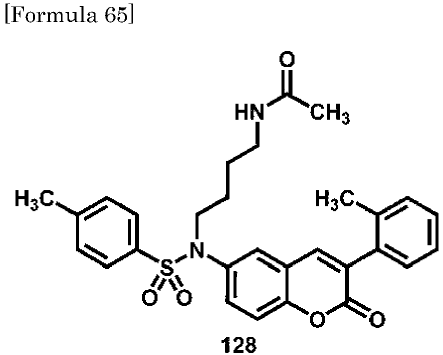

### N-(4-{N-[3-(2-methylphenyl)-2-oxo-2H-chromen-6-yl]4-methylbenzenesulfonamido}butyl)acetamide

Using N-(4-aminobutyl)-4-methyl-N-[3-(2-methylphenyl)-2-oxo-2H-chromen-6-yl]benzene-1-sulfonamide obtained in Example 127 and according to the procedure of Example 70 or a method analogous thereto, the title compound (30 mg) was obtained.

¹H NMR(500MHz,CDCl₃): δ7.59(s,1H),7.49-7.46(m,2H),7.36-7.32(m,2H),7.31-7.23(m,6H),7.06(dd,J=8.8,2.5Hz,1H),5.55-5.50(m,1H),3.57(t,J=6.9Hz,2H),3.23(td,J=7.1,6.1Hz,2H),2.44(s,3H),2.31(s,3H),1.95(s,3H ),1.63-1.55(m,2H),1.51-1.44(m,2H)

### Example 129

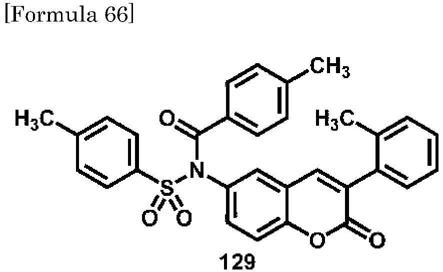

### 4-methyl-N-(4-methylbenzenesulfonyl)-N-[3-(2-methylphenyl)-2-oxo-2H-chromen-6-yl]benzamide

To a solution of 4-methyl-N-[3-(2-methylphenyl)-2-oxo-2H-chromen-6-yl]benzene-1-sulfonamide (40 mg) obtained in Reference Example 5 in dichloromethane (1 mL), triethylamine (21 µL), N,N-dimethyl-4-aminopyridine (1 mg), and 4-methylbenzoyl chloride (20 µL) were added at 0°C, and the mixture was stirred at room temperature for 1 day. The reaction mixture was concentrated, and the residue was purified by silica gel column chromatography (ethyl acetate/hexane=20%) to obtain the title compound (47 mg) as a white solid.

¹H NMR(500MHz,CDCl₃): δ7.82(d,J=8.4Hz,2H),7.55(s,1H),7.45(d,J=2.5Hz,1H),7.39(d,J=8.4Hz,2H),7.35-7.31(m,3H),7.29-7.21(m,4H),7.18(dd,J=8.8,2.5Hz,1H),7.02(d,J=8.0Hz,2H),2.45(s,3H),2.29(s,3H),2.27(s,3 H)

### Examples 130 to 145

The compounds shown in Tables 8-1 to 8-4 were synthesized according to Example 129 or a method similar thereto.

### Example 146

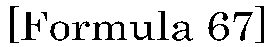

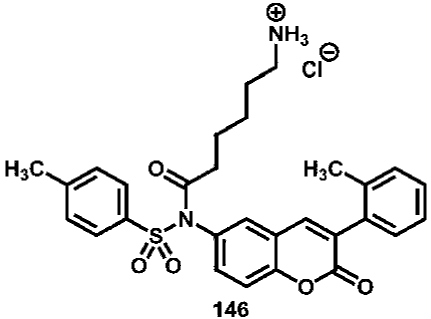

### 6-amino-N-(4-methylbenzenesulfonyl)-N-[3-(2-methylphenyl)-2-oxo-2H-chromen-6-yl]hexanamide hydrochloride

(1) To a solution of 4-methyl-N-[3-(2-methylphenyl)-2-oxo-2H-chromen-6-yl]benzene-1-sulfonamide (40 mg) obtained in Reference Example 5 in dichloromethane (1 mL), triethylamine (0.069 mL), tert-butoxycarbonylaminohexanoic acid, and HATU (57 mg) were added, and the mixture was stirred at room temperature for 2 days. A saturated aqueous solution of sodium hydrogen carbonate (10 mL) was added to the reaction mixture, which was then extracted with ethyl acetate (50 mL). The organic layer was washed with water (20 mL) and saturated brine (20 mL), dried over sodium sulfate, and then concentrated. The residue was purified by silica gel chromatography (ethyl acetate/hexane=20%) to give N-(6-{N'-(4-methylbenzenesulfonyl)-N'-[3-(2-methylphenyl-2-oxo-2H-chromen-6-yl)]amino}-6-oxohexyl)carbamic acid tert-butyl ester.
(2) Using the compound obtained above, the title compound (30 mg) was obtained according to the method of Example 69-(2) or a method analogous thereto.
   ¹H NMR(500MHz: DMSO-d₆): δ 8.08(s,1H),7.91-7.88(m,3H),7.72-7.58(m,3H),7.69(dd,J=8.8,2.5Hz,1H),7.63(d,J=8.8Hz,1H),7.50-7.47(m,2H),7.39-7.27(m,4H),7.27-6.62(m,2H),2.44(s,3H),2.45(s,3H),2.09(d,J=7.2Hz,2H),1.42-1.33(m,2H),1.31-1.24(m,2H),1.15-1.09(m,2H)

### Example 147

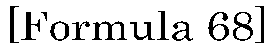

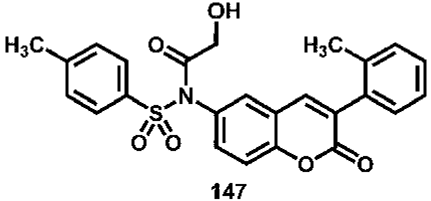

### 2-Hydroxy-N-(4-methylbenzenesulfonyl)-N-[3-(2-methylphenyl)-2-oxo-2H-chromen-6-yl]acetamide

(1) Using 4-methyl-N-[3-(2-methylphenyl)-2-oxo-2H-chromen-6-yl]benzene-1-sulfonamide obtained in Reference Example 5, 2-benzyloxy-N-(4-methylbenzenesulfonyl)-N-[3-(2-methylphenyl)-2-oxo-2H-chromen-6-yl]acetamide was obtained in accordance with the method of Example 146-(1) or a method analogous thereto.
(2) Using the compound obtained above, the title compound (7 mg) was obtained according to the method of Example 65 or a method analogous thereto.
   ¹H NMR(500MHz,CDCl₃): δ7.93-7.90(m,2H),7.63(s,1H),7.48(d,J=8.6Hz,1H),7.47(d,J=2.4Hz,1H),7.42-7.34(m,3H),7.32-7.24(m,4H),3.98(d,J=5.1Hz,2H),2.85(t,J=5.1Hz,1H),2.49(s,3H),2.32(s,3H)

### Example 148

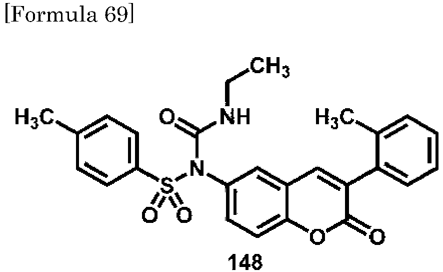

### 3-ethyl-1-(4-methylbenzenesulfonyl)-1-[3-(2-methylphenyl)-2-oxo-2H-chromen-6-yl]urea

To a solution of 4-methyl-N-[3-(2-methylphenyl)-2-oxo-2H-chromen-6-yl]benzene-1-sulfonamide (40 mg) obtained in Reference Example 5 in dichloromethane (1 mL), triethylamine (0.14 mL) and ethyl isocyanate (0.080 mL) were added and stirred at room temperature for 3.5 hours. After adding water (10 mL) to the reaction mixture. Extraction was carried out with dichloromethane (20 mL x 2). The extract was dried over sodium sulfate and filtered, and the filtrate was concentrated. The residue was purified by silica gel column chromatography (ethyl acetate/hexane=20%) to obtain the title compound (31 mg) as a white solid.

¹H NMR(500MHz,DMSO-d₆): δ8.13(s,1H),7.78-7.76(m,3H),7.54(d,J=8.7Hz,1H),7.44-7.40(m,3H),7.37-7.26(m,4H),6.87(t,J=5.6Hz,1H),2.97-2.92(m,2H),2.42(s,3H),2.22(s,3H),0.90(t,J=7.1Hz,3H)

### Examples 149 to 151

The compounds shown in Table 9 were synthesized according to the method of Example 148 or a method analogous thereto.

### Example 152

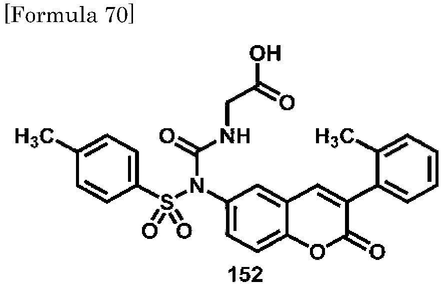

### 2-{[(4-methylbenzenesulfonyl)[3-(2-methylphenyl)-2-oxo-2H-chromen-6-yl]carbamoyl]amino}acetic acid

The compound obtained in Example 150 was treated in the manner described in Example 111-(2) or a similar method to give the title compound.

¹H NMR(500MHz,DMSO-d₆): δ12.54(brs,1H),8.15(s,1H),7.81-7.79(m,3H),7.57(d,J=8.8Hz,1H),7.44-7.40(m,3H),7.37-7.27(m,4H),7.06(t,J=5.8Hz,1H),3.57(d,J=5.8Hz,2H),2.42(s,3H),2.22(s,3H)

### Example 153

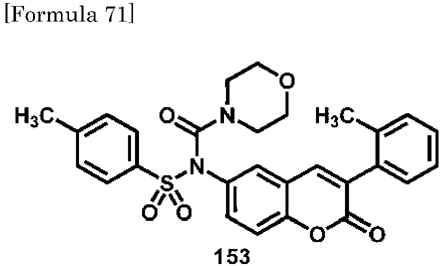

### N-(4-methylbenzenesulfonyl)-N-[3-(2-methylphenyl)-2-oxo-2H-chromen-6-yl]morpholine-4-carboxamide

(1) To a solution of triphosgene (0.24 g) in dichloromethane (2 mL), 4-methyl-N-[3-(2-methylphenyl)-2-oxo-2H-chromen-6-yl]benzene-1-sulfonamide (0.160 g) obtained in Reference Example 5 and triethylamine (0.10 mL) were added at 0°C, and the mixture was stirred at room temperature for 16 hours. After adding water (15 mL) to the reaction mixture. Extraction was carried out with dichloromethane (30 mL x 2). The extract was dried over sodium sulfate and filtered, and the filtrate was concentrated. The residue was purified by silica gel column chromatography (ethyl acetate/hexane=25%) to give N-(4-methylbenzenesulfonyl)-N-[3-(2-methylphenyl)-2-oxo-2H-chromen-6-yl]carbamoyl chloride (0.140 g) as a white solid.
(2) To a solution of the compound (19 mg) obtained above in dichloromethane (1 mL), triethylamine (0.011 mL) and morpholine (4 µL) were added. The mixture was stirred at room temperature for 1 hour. The reaction mixture was concentrated, and the residue was purified by silica gel column chromatography (ethyl acetate/hexane=30%) to obtain the title compound (0.010 g) as a white solid.
   ¹H NMR(500MHz,CDCl₃): δ7.57(d,J=8.4Hz,2H),7.54(s,1H),7.49(d,J=2.5Hz,1H),7.40(m,1H),7.36-7.23(m,7H),3.67-3.64(m,4H),3.61-3.59(m,4H),2.41(s,3H),2.29(s,3H)

### Examples 154 to 164

The compounds shown in Tables 10-1 to 10-3 were synthesized according to the method of Example 153-(1) and (2) or a method analogous thereto.

### Example 165

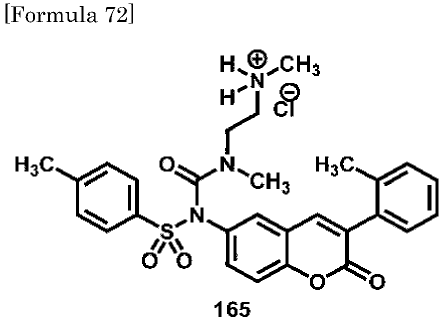

### 3-methyl-3-[2-(methylamino)ethyl]-1-(4-methylbenzenesulfonyl)-1-[3-(2-methylphenyl)-2-oxo-2H-chromen-6-yl]urea hydrochloride

(1) Using N-(4-methylbenzenesulfonyl)-N-[3-(2-methylphenyl)-2-oxo-2H-chromen-6-yl]carbamoyl chloride obtained in Example 153-(1), and according to the method of Example 86 or in a similar manner thereto, N-methyl-N-(2-{methyl[(4-methylbenzenesulfonyl)[3-(2-methylphenyl)-2-oxo-2H-chromen-6-yl]carbamoyl]amino}ethyl)carbamic acid tert-butyl ester was obtained.
(2) Using the compound obtained above (35 mg), the title compound (29 mg) was obtained according to the method of Example 69-(2) or a method analogous thereto.
   ¹H NMR(500MHz,CDCl₃): δ9.75(brs,2H),7.60(s,1H),7.52(d,J=8.0Hz,2H),7.36-7.31(m,2H),7.30-7.26(m,5H),7.25-7.21(m,2H),3.84(brs,2H),3.29(brs,2H),3.10(s,3H),2.80(s,3H),2.42(s,3H),2.29(s,3H).

### Example 166

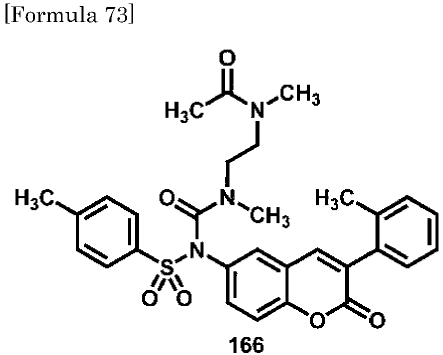

### N-methyl-N-(2-{methyl[(4-methylbenzenesulfonyl)[3-(2-methylphenyl)-2-oxo-2H-chromen-6-yl]carbamoyl]amino}ethyl)acetamide

Using 3-methyl-3-[2-(methylamino)ethyl]-1-(4-methylbenzenesulfonyl)-1-[3-(2-methylphenyl)-2-oxo-2H-chromen-6-yl]urea hydrochloride obtained in Example 165-(2), and according to the method of Example 70 or in a similar manner thereto, the title compound (12 mg) was obtained.

¹H NMR(500MHz,CDCl₃(Contains 4:1 rotamers): δ7.58-7.52(m,4H),7.40-7.27(m,5H),7.25-7.22(m,3H),3.58-3.54(m,2H),3.51-3.49(m,2H),3.09(brs,2.4H),3.07(brs,0.6H),3.05(brs,2.4H),2.94(brs,0.6H),2.42(brs,0.6H),2 .41(brs,2.4H),2.30(s,3H),2.05(s,3H).

### Example 167

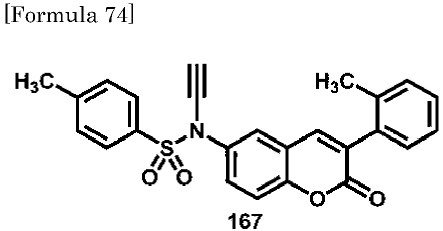

### 4-methyl-N-[3-(2-methylphenyl)-2-oxo-2H-chromen-6-yl]-N-(ethynyl)benzene-1-sulfonamide

(1) To a suspension of 4-methyl-N-[3-(2-methylphenyl)-2-oxo-2H-chromen-6-yl]benzene-1-sulfonamide (40 mg) obtained in Reference Example 5 in 1,4-dioxane, copper(II) sulfate pentahydrate (2.5 mg), 1,10-phenanthroline (36 mg), potassium carbonate (28 mg), and trimethylsilylbromoacetylene (19 µL) were added, and the mixture was stirred at 100°C for 6 hours. The reaction mixture was filtered through Celite, and the filtrate was concentrated. The residue was purified by silica gel column chromatography (ethyl acetate/hexane=25%) to give 4-methyl-N-[3-(2-methylphenyl)-2-oxo-2H-chromen-6-yl]-N-(trimethylsilylethynyl)benzene-1-sulfonamide (12 mg).
(2) To a solution of the compound (43 mg) obtained above in methanol (1 mL) was added potassium carbonate (35 mg), and the mixture was stirred at room temperature for 30 minutes. The reaction mixture was concentrated, and the residue was purified by silica gel column chromatography (ethyl acetate/hexane=20%) to obtain the title compound (25 mg).

¹H NMR(500MHz,CDCl₃): δ7.62(d,J=8.4Hz,2H),7.59(s,1H),7.51(d,J=2.0Hz,1H),7.34-7.31(m,5H),7.30-7.28(m,2H),7.25-7.23(m,1H),2.89(s,1H),2.46(s,3H),2.29(s,3H)

### Example 168

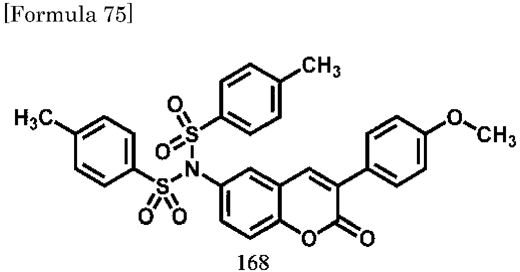

### N-[3-(4-methoxyphenyl)-2-oxo-2H-chromen-6-yl]-4-methyl-N-(4-methylbenzenesulfonyl)benzene-1-sulfonamide

A solution of N-(3-bromo-2-oxo-2H-chromen-6-yl)-4-methyl-N-(4-methylbenzenesulfonyl)benzene-1-sulfonamide (30 mg) obtained in Reference Example 6, (4-methoxyphenyl)boronic acid (13 mg), potassium carbonate (15 mg), and bis(diphenylphosphinoferrocene)dichloropalladium(II) dichloromethane adduct (2 mg) in DMF/water (500 µL/50 µL) was stirred at 70°C for 1 hour under a nitrogen atmosphere. To the reaction mixture was added saturated aqueous ammonium chloride solution, and the mixture was extracted with ethyl acetate. The organic layer was washed with water and saturated brine, dried over sodium sulfate, and then concentrated. The residue was purified by silica gel column chromatography (hexane:ethyl acetate=75:25) to obtain the title compound (21 mg) as a white solid.

¹H NMR(500MHz,CDCl₃): δ7.81(d,J=8.4Hz,4H),7.66(d,J=9.0Hz,2H),7.63(s,1H),7.35(d,J=8.4Hz,4H),7.30(d,J=8.8Hz ,1H),7.26(d,J=2.5Hz,1H),7.08(dd,J=8.8,2.5Hz,1H),6.98(d,J=9.0Hz,2H),3.86(s,3H),2.48(s, 6H)

### Examples 169 to 198

The compounds shown in Tables 11-1 to 11-8 were synthesized according to the method of Example 168 or a method similar thereto.

### Example 199

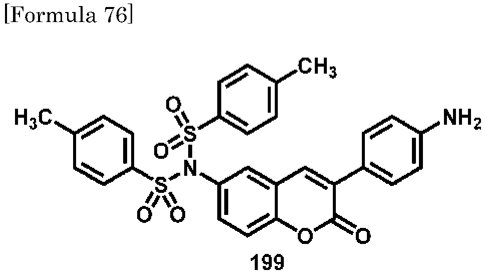

### N-[3-(4-aminophenyl)-2-oxo-2H-chromen-6-yl]-4-methyl-N-(4-methylbenzenesulfonyl)benzene-1-sulfonamide

The title compound was obtained using tert-butyl N-(4-{6-[N-(4-methylbenzenesulfonyl)4-methylbenzenesulfonamido]-2-oxo-2H-chromen-3-yl}phenyl)carbamate obtained in Example 175 and according to the method of Example 69-(2) or a method analogous thereto.

¹H NMR(500MHz,CDCl₃): δ7.81(d,J=8.4Hz,4H),7.59(s,1H),7.53(d,J=8.6Hz,2H),7.35(d,J=8.4Hz,4H),7.28(d,J=8.8Hz ,1H),7.23(d,J=2.4Hz,1H),7.05(dd,J=8.8,2.4Hz,1H),6.72(d,J=8.6Hz,2H),3.89(br-s,2H),2.48(s,6H)

### Example 200

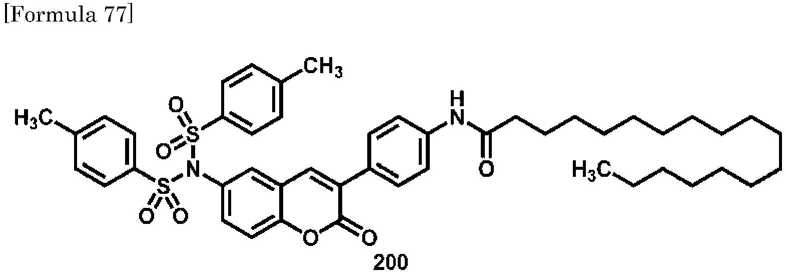

### N-(4-{6-[N-(4-methylbenzenesulfonyl)4-methylbenzenesulfonamido]-2-oxo-2H-chromen-3-yl}phenyl)octadecanamide

The title compound was obtained using N-[3-(4-aminophenyl)-2-oxo-2H-chromen-6-yl]-4-methyl-N-(4-methylbenzenesulfonyl)benzene-1-sulfonamide obtained in Example 199 and according to the method of Example 146 or an analogous method.

¹H NMR(500MHz,CDCl₃): δ7.81(d,J=8.1Hz,4H),7.66(s,1H),7.66(d,J=8.5Hz,2H),7.61(d,J=8.5Hz,2H),7.36(d,J=8.1Hz ,4H),7.30(d,J=8.8Hz,1H),7.27(d,J=2.5Hz,1H),7.10(dd,J=8.8,2.5Hz,1H),2.48(s,6H),2.38(t, J=7.7Hz,2H),1.74(tt,J=7.7,7.7Hz,2H),1.42-1.20(m,28H),0.88(t,J=7.1Hz,3H)

### Example 201

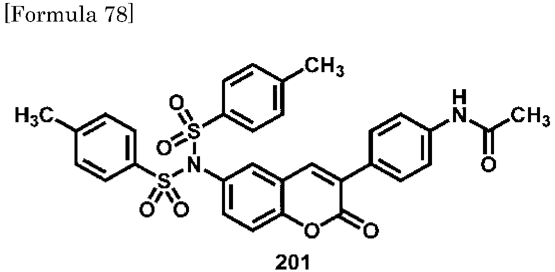

### N-(4-{6-[N-(4-methylbenzenesulfonyl)4-methylbenzenesulfonamido]-2-oxo-2H-chromen-3-yl}phenyl)acetamide

The title compound was obtained using N-[3-(4-aminophenyl)-2-oxo-2H-chromen-6-yl]-4-methyl-N-(4-methylbenzenesulfonyl)benzene-1-sulfonamide obtained in Example 199 and according to the method of Example 70 or an analogous method.

¹H NMR(500MHz,CDCl₃): δ7.81(d,J=8.4Hz,4H),7.67(d,J=8.5Hz,2H),7.67(s,1H),7.59(d,J=8.5Hz,2H),7.36(d,J=8.4Hz ,4H),7.32(br-s,1H),7.30(d,J=8.8Hz,1H),7.27(d,J=2.4Hz,1H),7.10(dd,J=8.8,2.4Hz,1H),2.48(s,6H),2.21( s,3H)

### Example 202

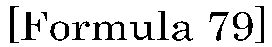

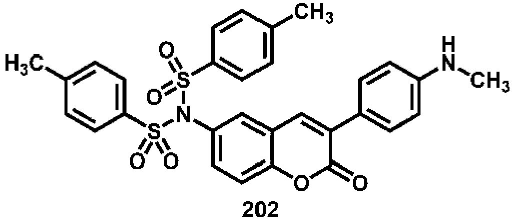

### 4-methyl-N-{3-[4-(methylamino)phenyl]-2-oxo-2H-chromen-6-yl}-N-(4-methylbenzenesulfonyl)benzene-1-sulfonamide

(1) Using tert-butyl N-(4-{6-[N-(4-methylbenzenesulfonyl)4-methylbenzenesulfonamido]-2-oxo-2H-chromen-3-yl}phenyl)carbamate obtained in Example 175, tert-butyl N-methyl-N-[N'-(4-{6-[N'-(4-methylbenzenesulfonyl)4-methylbenzenesulfonamido]-2-oxo-2H-chromen-3-yl}phenyl)]carbamate was obtained in accordance with the method of Example 101 or a method analogous thereto.
(2) The title compound was obtained using the above-obtained tert-butyl N-methyl-N-[N'-(4-{6-[N'-(4-methylbenzenesulfonyl)4-methylbenzenesulfonamido]-2-oxo-2H-chromen-3-yl}phenyl)]carbamate according to the method of Example 69-(2) or a method analogous thereto.
   ¹H NMR(500MHz,DMSO-d₆): δ8.12(s,1H),7.71-7.70(m,4H),7.58-7.56(m,2H),7.55(d,J=2.6Hz,1H),7.52-7.48(m,4H),7.42(d,J=8.7Hz,1H),7.03(dd,J=8.7,2.6Hz,1H),6.62-6.59(m,2H),6.08-6.05(m,1H),2.73(d,J=5.0Hz,3H),2.46(s,6H)

### Example 203

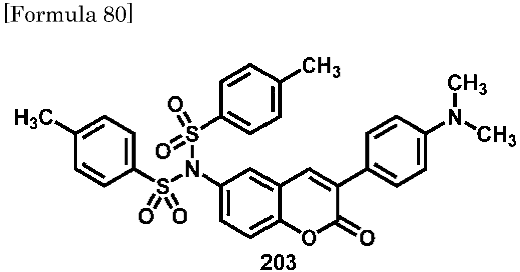

### N-{3-[4-(dimethylamino)phenyl]-2-oxo-2H-chromen-6-yl}-4-methyl-N-(4-methylbenzenesulfonyl)benzene-1-sulfonamide

The compound obtained in Example 202 was treated in the same manner as in Example 101 or a method analogous thereto to give the title compound.

¹H NMR(500MHz,DMSO-d₆): δ8.17(s,1H),7.72-7.70(m,4H),7.66-7.64(m,2H),7.57(d,J=2.6Hz,1H),7.50-7.49(m,4H),7.43(d,J=8.7Hz,1H),7.04(dd,J=8.8,2.7Hz,1H),6.79-6.78(m,2H),2.97(s,6H),2.46(s,6H)

### Example 204

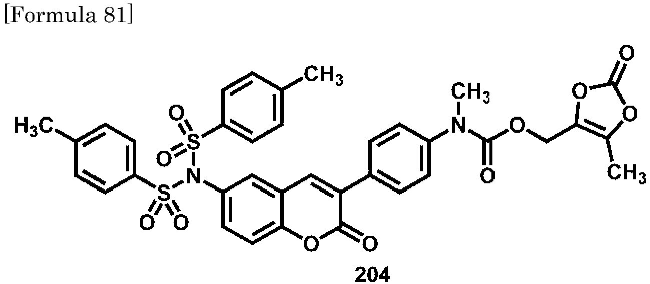

### N-methyl-N-(4-{6-[N-(4-methylbenzenesulfonyl)4-methylbenzenesulfonamido]-2-oxo-2H-chromen-3-yl}phenyl)carbamic acid (5-methyl-2-oxo-2H-1,3-dioxol-4-yl)methyl ester

(1) The compound obtained in Example 202 was treated in the same manner as in Example 85-(1) or a method analogous thereto to give N-methyl-N-(4-{6-[4-methylbenzenesulfonyl)4-methylbenzenesulfonamido]-2-oxo-2H-chromen-3-yl}phenyl) carbamoyl chloride.
(2) The compound obtained above was treated in the same manner as in Example 86 or a method analogous thereto to obtain the title compound.
   ¹H NMR(500MHz,DMSO-d₆): δ8.31(s,1H),7.74-7.70(m,6H),7.61(d,J=2.6Hz,1H),7.51-7.47(m,6H),7.44(d,J=8.8Hz,1H),7.12(dd,J=8.8,2.6Hz,1H),4.98(s,2H),2.46(s,6H),2.16(s,3 H),2.10(s,3H)

### Example 205

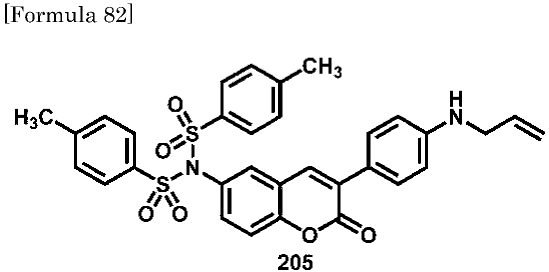

### 4-methyl-N-(4-methylbenzenesulfonyl)-N-(2-oxo-3-{4-[(2-propenyl)amino]phenyl}-2H-chromen-6-yl)benzene-1-sulfonamide

(1) Using tert-butyl N-(4-{6-[N-(4-methylbenzenesulfonyl)4-methylbenzenesulfonamido]-2-oxo-2H-chromen-3-yl}phenyl)carbamate obtained in Example 175, tert-butyl N-(2-propenyl)-N-[N'-(4-{6-[N'-(4-methylbenzenesulfonyl)4-methylbenzenesulfonamido]-2-oxo-2H-chromen-3-yl}phenyl)]carbamate was obtained in accordance with the method of Example 101 or a method analogous thereto.
(2) The compound obtained above was treated in the same manner as in Example 69-(2) or a method analogous thereto to give the title compound.
   ¹H NMR(500MHz,DMSO-d₆): δ8.11(s,1H),7.72-7.70(m,4H),7.55-7.53(m,3H),7.50-7.49(m,4H),7.42(d,J=8.8Hz,1H),7.03(dd,J=8.8,2.5Hz,1H),6.65-6.63(m,2H),6.30-6.25(m,1H),5.95-5.86(m,1H),5.26-5.11(m,1H),5.13-5.10(m,1H),3.78-3.74(m,2H),2.46(s,6H)

### Example 206

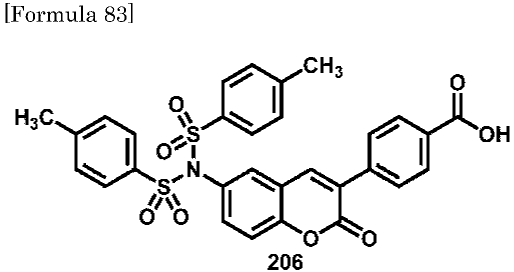

### 4-{6-[N-(4-methylbenzenesulfonyl)4-methylbenzenesulfonamido]-2-oxo-2H-chromen-3-yl}benzoic acid

The title compound was obtained using methyl 4-{6-[N-(4-methylbenzenesulfonyl)4-methylbenzenesulfonamido]-2-oxo-2H-chromen-3-yl}benzoate obtained in Example 170 and according to the method of Example 111 or an analogous method.

¹H NMR(500MHz,DMSO-d₆): δ8.38(s,1H),7.79(d,J=8.5Hz,2H),7.71(d,J=8.4Hz,4H),7.64(d,J=2.6Hz,1H),7.51-7.48(m,5H),7.45(d,J=8.5Hz,2H),7.13(dd,J=8.8,2.6Hz,1H),3.45(brs,2H),3.22(brs,2H),1.16 (brs,3H),1.08(brs,3H)

### Example 207

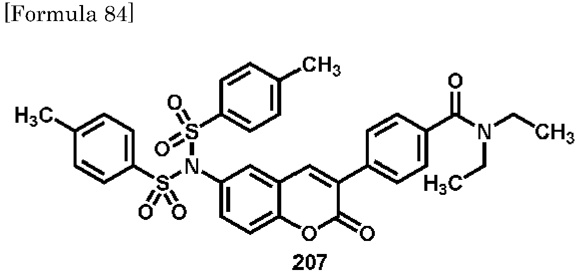

### N,N-Diethyl-4-{6-[N'-(4-methylbenzenesulfonyl)-N'-(4-methylbenzenesulfonylamino)]-2-oxo-2H-chromen-3-yl}benzoic acid carboxamide

Using 4-{6-[N-(4-methylbenzenesulfonyl)4-methylbenzenesulfonamido]-2-oxo-2H-chromen-3-yl}benzoic acid obtained in Example 206 and according to the method of Example 146 or an analogous method, the title compound was obtained.

¹H NMR(500MHz,DMSO-d₆): δ13.09(brs,1H),8.42(s,1H),8.04(d,J=8.7Hz,2H),7.86(d,J=8.7Hz,2H),7.71(d,J=8.4Hz,4H),7 .66(d,J=2.6Hz,1H),7.51-7.49(m,5H),7.14(dd,J=8.8,2.6Hz,1H),2.46(s,6H)

### Example 208

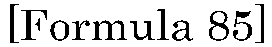

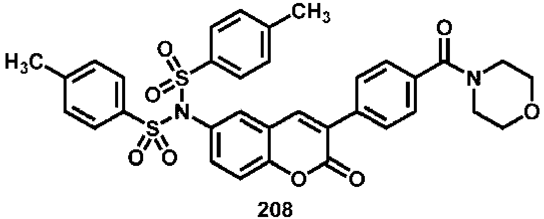

### 4-methyl-N-(4-methylbenzenesulfonyl)-N-{3-[4-(morpholine-4-carbonyl)phenyl]-2-oxo-2H-chromen-6-yl}benzene-1-sulfonamide

Using 4-{6-[N-(4-methylbenzenesulfonyl)4-methylbenzenesulfonamido]-2-oxo-2H-chromen-3-yl}benzoic acid obtained in Example 206 and according to the method of Example 146 or an analogous method, the title compound was obtained.

¹H NMR(500MHz,DMSO-d₆): δ8.37(s,1H),7.80(d,J=8.5Hz,2H),7.71(d,J=8.3Hz,4H),7.64(d,J=2.6Hz,1H),7.53-7.48(m,7H),7.14(dd,J=8.8,2.5Hz,1H),3.68-3.54(m,8H),2.46(s,6H)

### Example 209

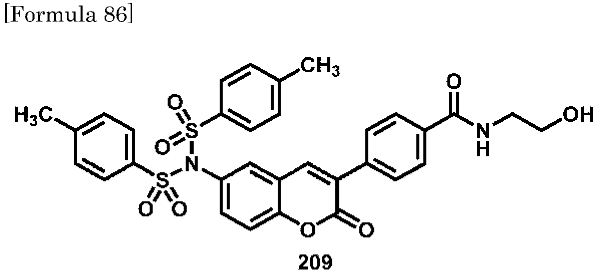

### N-(2-hydroxyethyl)-4-{6-[N-(4-methylbenzenesulfonyl)4-methylbenzenesulfonamido]-2-oxo-2H-chromen-3-yl}benzamide

Using 4-{6-[N-(4-methylbenzenesulfonyl)4-methylbenzenesulfonamido]-2-oxo-2H-chromen-3-yl}benzoic acid obtained in Example 206 and according to the method of Example 146 or an analogous method, the title compound was obtained.

¹H NMR(500MHz,CDCl₃): δ7.88(d,J=8.4Hz,2H),7.82-7.77(m,7H),7.37-7.32(m,6H),7.13(dd,J=9.0,2.7Hz,1H),6.62-6.60(m,1H),3.89-3.86(m,2H),3.70-3.66(m,2H),2.49(s,6H),2.38-2.35(m,1H)

### Example 210

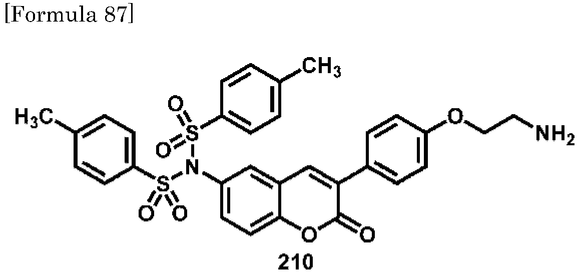

### 4-(2-aminoethoxy)-N-(4-methylbenzenesulfonyl)-N-[3-(2-methylphenyl)-2-oxo-2H-chromen-6-yl]benzene-1-sulfonamide

(1) Using N-[3-(4-hydroxyphenyl)-2-oxo-2H-chromen-6-yl]-4-methyl-N-(4-methylbenzenesulfonyl)benzene-1-sulfonamide obtained in Example 174, N-[2-(4-{6-[N',N'-bis(methylbenzenesulfonyl)amino]}-2-oxo-2H-chromen-3-ylphenoxy)ethyl]carbamic acid tert-butyl ester was obtained in accordance with the method of Example 69-(1) or a method analogous thereto.
(2) The compound obtained above was treated in the same manner as in Example 69-(2) or a method analogous thereto to give the title compound.
   ¹H NMR(500MHz,CDCl³): δ8.26(s,1H),7.99(br-s,2H),7.73(d,J=8.9Hz,2H),7.71(d,J=8.4Hz,4H),7.63(d,J=2.6Hz,1H),7.50(d,J=8.4Hz,4H),7 .47(d,J=8.9Hz,1H),7.11-7.08(m,3H),4.23(t,J=5.1Hz,2H),3.27(brs,2H),2.46(s,6H)

### Example 211

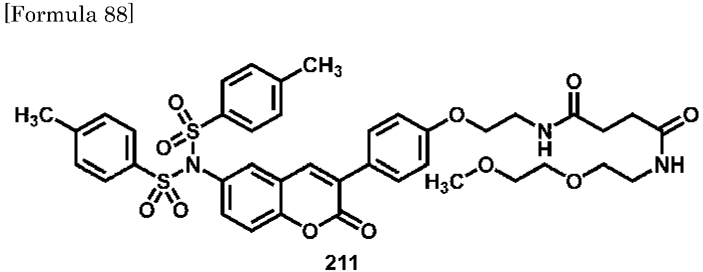

### N-[2-(2-methoxyethoxy)ethyl]-N'-[2-(4-{6-[N-(4-methylbenzenesulfonyl)4-methylbenzenesulfonamido]-2-oxo-2H-chromen-3-yl}phenoxy)ethyl]butanediamide

The title compound was obtained using 4-(2-aminoethoxy)-N-(4-methylbenzenesulfonyl)-N-[3-(2-methylphenyl)-2-oxo-2H-chromen-6-yl]benzene-1-sulfonamide obtained in Example 210 and according to the method of Example 146 or an analogous method.

¹H NMR(500MHz,CDCl₃): δ7.82(d,J=8.4Hz,4H),7.65(d,J=8.9Hz,2H),7.65(s,1H),7.36(d,J=8.4Hz,4H),7.31(d,J=8.8Hz ,1H),7.27(d,J=2.5Hz,1H),7.10(dd,J=8.8,2.5Hz,1H),6.98(d,J=8.9Hz,2H),6.51(br-s,1H),6.27(br-s,1H),4.09(t,J=5.2Hz,2H),3.69(t,J=5.3Hz,2H),3.62(t,J=6.3Hz,2H),3.55(t,J=6.3Hz,2H),3.5 5(t,J=5.2Hz,2H),3.45(t,J=5.3Hz,2H),3.40(s,3H),2.55(t,J=2.1Hz,2H),2.55(t,J=2.1Hz,2H), 2.49(s,6H)

### Example 212

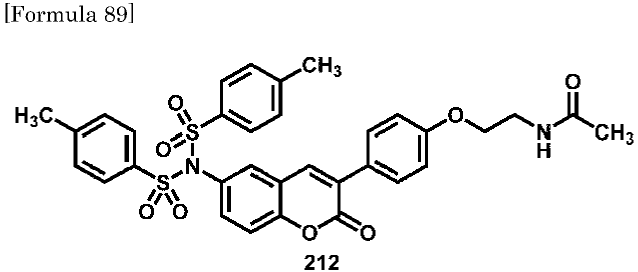

### N-[2-(4-{6-[N-(4-methylbenzenesulfonyl)4-methylbenzenesulfonamido]-2-oxo-2H-chromen-3-yl}phenoxy)ethyl]acetamide

The title compound was obtained using 4-(2-aminoethoxy)-N-(4-methylbenzenesulfonyl)-N-[3-(2-methylphenyl)-2-oxo-2H-chromen-6-yl]benzene-1-sulfonamide obtained in Example 210 and according to the method of Example 70 or an analogous method.

¹H NMR(500MHz,CDCl₃): δ7.81(d,J=8.4Hz,4H),7.65(d,J=9.0Hz,2H),7.65(s,1H),7.36(d,J=8.4Hz,4H),7.30(d,J=8.8Hz ,1H),7.27(d,J=2.5Hz,1H),7.09(dd,J=8.8,2.5Hz,1H),6.98(d,J=9.0Hz,2H),5.91(br-s,1H),4.10(t,J=5.0Hz,2H),3.69(dt,J=5.0,5.0Hz,2H),2.48(s,6H),2.03(s,3H)

### Example 213

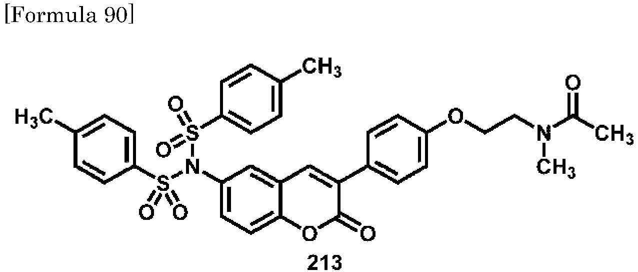

### N-methyl-N-[2-(4-{6-[N-(4-methylbenzenesulfonyl)4-methylbenzenesulfonamido]-2-oxo-2H-chromen-3-yl}phenoxy)ethyl]acetamide

(1) Using N-[3-(4-hydroxyphenyl)-2-oxo-2H-chromen-6-yl]-4-methyl-N-(4-methylbenzenesulfonyl)benzene-1-sulfonamide obtained in Example 174, N-methyl-N-[2-(4-{6-[N',N'-bis(methylbenzenesulfonyl)amino]}-2-oxo-2H-chromen-3-ylphenoxy)ethyl]carbamic acid tert-butyl ester was obtained in accordance with the method of Example 69-(1) or a method analogous thereto.
(2) The compound obtained above was treated in the same manner as in Example 69-(2) or a method analogous thereto to give 4-methyl-N-(3-{4-[2-(methylamino)ethoxy]phenyl}-2-oxo-2H-chromen-6-yl)-N-(4-methylbenzenesulfonyl)benzene-1-sulfonamide.
(3) The compound obtained above was treated in the same manner as in Example 70 to obtain the title compound.
   ¹H NMR(500MHz,CDCl₃),(70:30 rotamer mixture): δ7.81(d,J=8.4Hz,4H),7.67-7.64(m,3H),7.35(d,J=8.4Hz,4H),7.30(d,J=8.8Hz,1H),7.27(d,J=2.5Hz,1H),7.08(dd,J=8.8,2 .5Hz,1H),6.98-6.94(m,2H),4.19(t,J=5.3Hz,1.4H),4.15(t,J=5.3Hz,0.6H),3.77(t,J=5.3,1.4H),3.74(t,J=5.3,0 .6H),3.17(s,2.1H),3.02(s,0.9H),2.49(s,6H),2.21(s,0.9H),2.11(s,2.1H)

### Example 214

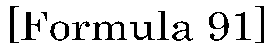

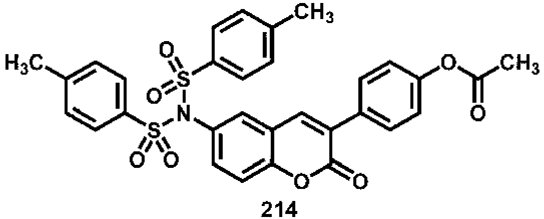

### 4-{6-[N-(4-methylbenzenesulfonyl)4-methylbenzenesulfonamido]-2-oxo-2H-chromen-3-yl}phenyl acetate

To a solution of N-[3-(4-hydroxyphenyl)-2-oxo-2H-chromen-6-yl]-4-methyl-N-(4-methylbenzenesulfonyl)benzene-1-sulfonamide (14 mg) obtained in Example 174 in dichloromethane (0.5 mL), 4-dimethylaminopyridine (1 mg), triethylamine (7 µL), and acetic anhydride (5 µL) were added, and the mixture was stirred at room temperature for 5 hours. The reaction mixture was concentrated, and the residue was purified by silica gel column chromatography (hexane:ethyl acetate=75:25) to obtain the title compound (4 mg) as a white solid.

¹H NMR(500MHz,CDCl₃): δ7.81(d,J=8.4Hz,4H),7.71(d,J=8.9Hz,2H),7.68(s,1H),7.36(d,J=8.4Hz,4H),7.32(d,J=8.8Hz ,1H),7.26(d,J=2.5Hz,1H),7.19(d,J=8.9Hz,2H),7.13(dd,J=8.8,2.5Hz,1H),2.49(s,6H),2.33(s, 3H)

### Example 215

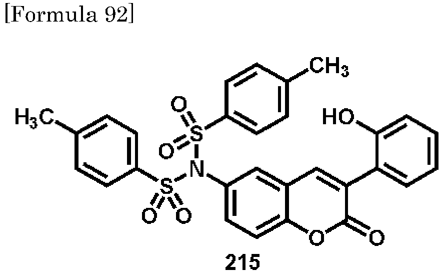

### N-[3-(2-hydroxyphenyl)-2-oxo-2H-chromen-6-yl]-4-methyl-N-(4-methylbenzenesulfonyl)benzene-1-sulfonamide

The title compound was obtained in accordance with the method of Example 65 or a method analogous thereto and using N-{3-[2-(benzyloxy)phenyl]-2-oxo-2H-chromen-6-yl}-4-methyl-N-(4-methylbenzenesulfonyl)benzene-1-sulfonamide obtained in Example 186.

¹H NMR(500MHz,DMSO-d₆): δ9.63(s,1H),8.07(s,1H),7.72-7.70(m,4H),7.63(d,J=2.6Hz,1H),7.51-7.46(m,4H),7.46(d,J=8.8Hz,1H),7.28-7.23(m,2H),7.08(dd,J=8.8,2.6Hz,1H),6.91(dd,J=8.3,1.0Hz,1H),6.88(td,J=7.4,1.1Hz,1H),2 .46(s,6H)

### Example 216

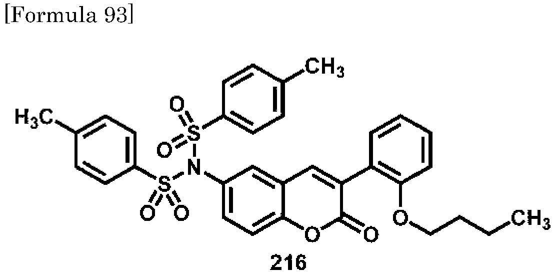

### N-[3-(2-butoxyphenyl)-2-oxo-2H-chromen-6-yl]-4-methyl-N-(4-methylbenzenesulfonyl)benzene-1-sulfonamide

The title compound was obtained using N-[3-(2-hydroxyphenyl)-2-oxo-2H-chromen-6-yl]-4-methyl-N-(4-methylbenzenesulfonyl)benzene-1-sulfonamide obtained in Example 215 and according to the method described in Example 69-(1) or a method analogous thereto.

¹H NMR(500MHz,DMSO-d₆): δ8.07(s,1H),7.73-7.70(m,4H),7.60(d,J=2.6Hz,1H),7.52-7.46(m,5H),7.44-7.39(m,1H),7.35(dd,J=7.5,1.8Hz,1H),7.12-7.08(m,2H),7.04-7.00(m,1H),3.99(t,J=6.4Hz,2H),2.46(s,6H),1.63-1.58(m,2H),1.36-1.32(m,2H),0.84(t,J=7.5Hz,3H)

### Example 217

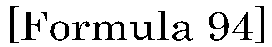

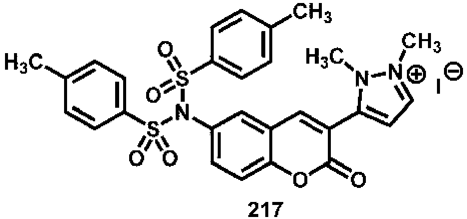

### 1,2-dimethyl-5-{6-[N-(4-methylbenzenesulfonyl)4-methylbenzenesulfonamido]-2-oxo-2H-chromen-3-yl]}-1H-pyrazole-2-ium iodide

The title compound was obtained using 4-methyl-N-[3-(1-methyl-1H-pyrazol-5-yl)-2-oxo-2H-chromen-6-yl]-N-(4-methylbenzenesulfonyl)benzene-1-sulfonamide obtained in Example 185 and according to the method of Example 101 or a method analogous thereto.

¹H NMR(500MHz,DMSO-d₆): δ8.59(d,J=2.9Hz,1H),8.49(s,1H),7.81(d,J=2.6Hz,1H),7.72-7.69(m,4H),7.57(d,J=8.9Hz,1H),7.52-7.50(m,4H),7.25(dd,J=8.8,2.6Hz,1H),7.06(d,J=2.9Hz,1H),4.22(s,3H),4.06(s,3H),2.47(s,6 H)

### Example 218

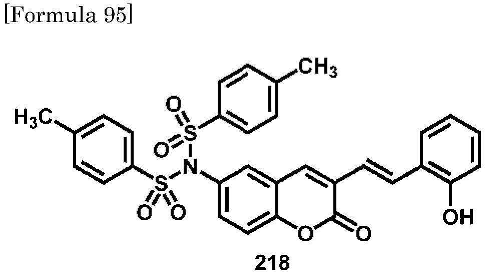

### N-{3-[(E)-2-(2-hydroxyphenyl)ethynyl]-2-oxo-2H-chromen-6-yl}-4-methyl-N-(4-methylbenzenesulfonyl)benzene-1-sulfonamide

(1) N-(3-bromo-2-oxo-2H-chromen-6-yl)-4-methyl-N-(4-methylbenzenesulfonyl)benzene-1-sulfonamide (0.10 g) obtained in Reference Example 6, 2-methoxystyrene (37 mg), N,N-diisopropylethylamine (0.048 mL), silver acetate (2 mg), tris(4-methylphenyl)phosphine (4 mg), and tris(dibenzylideneacetone)dipalladium(0) (8 mg) were suspended in 1,4-dioxane and stirred at 90°C for 18 hours under a nitrogen atmosphere. A saturated aqueous solution of ammonium chloride (10 mL) was added to the reaction mixture, which was then extracted with ethyl acetate (60 mL). The extract was washed with water (10 mL) and saturated brine (10 mL), and then dried over anhydrous sodium sulfate. The filtrate was concentrated, and the residue was purified by silica gel chromatography (ethyl acetate/hexane=0 to 50%) to give N-{3-[(E)-2-(2-methoxyphenyl)ethynyl]-2-oxo-2H-chromen-6-yl}-4-methyl-N-(4-methylbenzenesulfonyl)benzene-1-sulfonamide (60 mg) as a pale yellow solid.
(2) The compound obtained above was treated in the same manner as in Example 66 or a method analogous thereto to obtain the title compound.
   ¹H NMR(500MHz,DMSO-d₆): δ9.96(s,1H),8.30(s,1H),7.83(d,J=16.6Hz,1H),7.73-7.70(m,4H),7.57(d,J=2.6Hz,1H),7.56-7.54(m,1H),7.52-7.49(m,1H),7.45(d,J=8.7Hz,1H),7.20(d,J=16.6Hz,1H),7.17-7.13(m,1H),7.07(dd,J=8.7,2.6Hz,1H),6.91-6.89(m,1H),6.87-6.83(m,1H),2.46(s,6H)

### Example 219

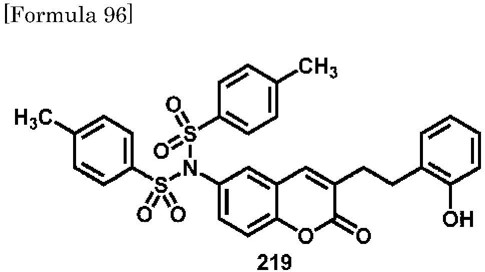

### N-{3-[2-(2-hydroxyphenyl)ethyl]-2-oxo-2H-chromen-6-yl}-4-methyl-N-(4-methylbenzenesulfonyl)benzene-1-sulfonamide

The compound obtained in Example 218 was treated in the same manner as in Example 65 or a method analogous thereto to give the title compound.

¹H NMR(500MHz,DMSO-d₆): δ9.31(s,1H),7.78(s,1H),7.70-7.67(m,4H),7.50-7.46(m,4H),7.43-7.40(m,2H),7.08-7.00(m,3H),6.78-6.76(m,1H),6.73-6.70(m,1H),2.85-2.82(m,2H),2.73-2.70(m,2H),2.46(s,6H)

### Example 220

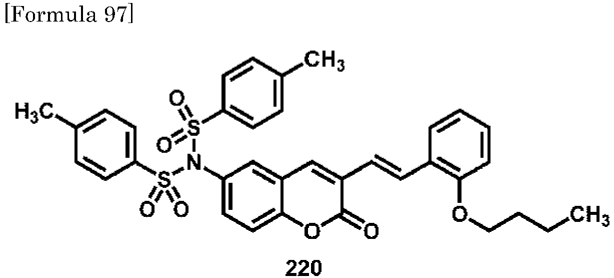

### N-{3-[(E)-2-(2-butoxyphenyl)ethenyl]-2-oxo-2H-chromen-6-yl}-4-methyl-N-(4-methylbenzenesulfonyl)benzene-1-sulfonamide

The compound obtained in Example 218 was treated in the manner described in Example 69-(1) or a method analogous thereto to give the title compound.

¹H NMR(500MHz,DMSO-d₆): δ8.25(s,1H),7.89(d,J=16.3Hz,1H),7.74-7.70(m,4H),7.63-7.60(m,1H),7.56(d,J=2.6Hz,1H),7.52-7.49(m,1H),7.45(d,J=8.7Hz,1H),7.32-7.29(m,1H),7.25(d,J=16.6Hz,1H),7.09-7.06(m,2H),7.02-6.97(m,1H),4.07(t,J=6.4Hz,2H),2.46(s,3H),1.82-1.77(m,2H),1.58-1.50(m, 2H),0.98(t,J=7.5Hz,3H)

### Example 221

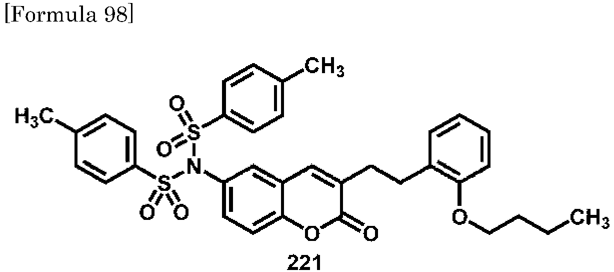

### N-{3-[2-(2-butoxyphenyl)ethyl]-2-oxo-2H-chromen-6-yl}-4-methyl-N-(4-methylbenzenesulfonyl)benzene-1-sulfonamide

The compound obtained in Example 220 was treated in the same manner as in Example 65 or a method analogous thereto to give the title compound.

¹H NMR(500MHz,DMSO-d₆): δ7.70-7.64(m,5H),7.51-7.46(m,4H),7.42(d,J=8.8Hz,1H),7.38(d,J=2.5Hz,1H),7.20-7.14(m,1H),7.05(dd,J=8.7,2.5Hz,1H),6.91(dd,J=8.0,0.8Hz,1H),6.87(td,J=7.5,0.9Hz,1H),3 .85(t,J=6.5Hz,2H),2.90-2.85(m,2H),2.75-2.70(m,2H),2.46(s,6H),1.70-1.60(m,2H),1.48-1.39(m,2H),0.98(t,J=7.5Hz,3H)

### Example 222

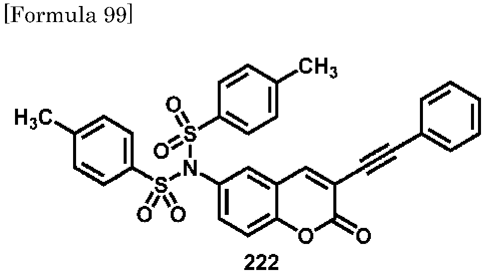

### 4-methyl-N-(4-methylbenzenesulfonyl)-N-[2-oxo-3-(2-phenylethynyl)2H-chromen-6-yl]benzene-1-sulfonamide

N-(3-bromo-2-oxo-2H-chromen-6-yl)-4-methyl-N-(4-methylbenzenesulfonyl)benzene-1-sulfonamide (0.10 g) obtained in Reference Example 6, phenylacetylene (5 µL), N,N-diisopropylethylamine (7 µL), triphenylphosphine (0.5 mg), bis(triphenylphosphine)palladium(II) chloride (1.3 mg), and copper iodide (0.3 mg) were suspended in 1,4-dioxane (0.5 mL), and the suspension was stirred at 50°C under a nitrogen atmosphere for 20 hours. The reaction mixture was concentrated, and the residue was purified by silica gel chromatography (ethyl acetate/hexane=25 to 50%) to obtain the title compound (11 mg) as a pale yellow solid.

¹H NMR(500MHz,CDCl₃): δ7.82(s,1H),7.81(d,J=8.3Hz,4H),7.60-7.58(m,2H),7.39-7.36(m,7H),7.31(d,J=8.8Hz,1H),7.20(d,J=2.5Hz,1H),7.13(dd,J=8.8,2.5Hz,1H),2.50(s,6H)

### Example 223

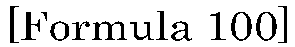

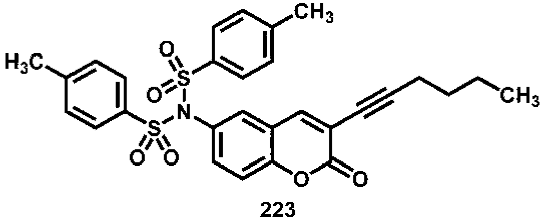

### 4-methyl-N-(4-methylbenzenesulfonyl)-N-[2-oxo-3-(2-phenylethynyl)2H-chromen-6-yl]benzene-1-sulfonamide

Using N-(3-bromo-2-oxo-2H-chromen-6-yl)-4-methyl-N-(4-methylbenzenesulfonyl)benzene-1-sulfonamide obtained in Reference Example 6 and according to the method of Example 222 or a method analogous thereto, the title compound was obtained.

¹H NMR(500MHz,CDCl₃): δ7.80(d,J=8.3Hz,4H),7.66(s,1H),7.36(d,J=8.3Hz,4H),7.27(d,J=8.8Hz,1H),7.15(d,J=2.5Hz ,1H),7.09(dd,J=8.8,2.5Hz,1H),2.49(s,6H),2.48(t,J=7.3Hz,2H),1.62(tt,J=7.6,7.3Hz,2H),1.4 9(tq,J=7.6,7.4Hz,2H),0.96(t,J=7.4Hz,2H)

### Example 224

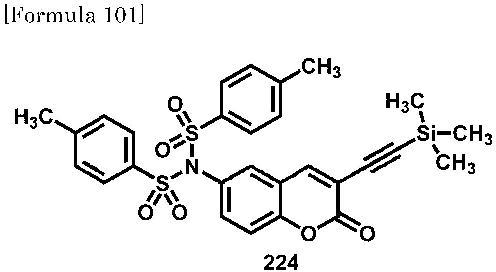

### 4-methyl-N-(4-methylbenzenesulfonyl)-N-{2-oxo-3-[2-(trimethylsilyl)ethynyl]-2H-chromen-6-yl}benzene-1-sulfonamide

Using N-(3-bromo-2-oxo-2H-chromen-6-yl)-4-methyl-N-(4-methylbenzenesulfonyl)benzene-1-sulfonamide obtained in Reference Example 6 and according to the method of Example 222 or a method analogous thereto, the title compound was obtained.

¹H NMR(500MHz,CDCl₃): δ7.79(d,J=8.2Hz,4H),7.76(s,1H),7.35(d,J=8.2Hz,4H),7.27(d,J=8.8Hz,1H),7.16(d,J=2.2Hz ,1H),7.11(dd,J=8.8,2.2Hz,1H),2.48(s,6H),0.27(s,9H)

### Example 225

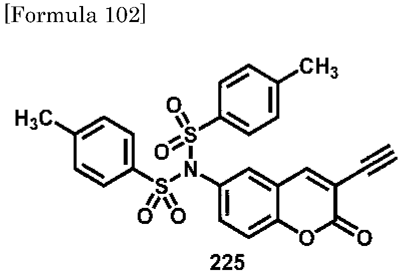

### N-(3-ethynyl-2-oxo-2H-chromen-6-yl)-4-methyl-N-(4-methylbenzenesulfonyl)benzene-1-sulfonamide

Acetic acid (3 µL) was added to a solution of 4-methyl-N-(4-methylbenzenesulfonyl)-N-{2-oxo-3-[2-(trimethylsilyl)ethynyl]-2H-chromen-6-yl}benzene-1-sulfonamide (15 mg) obtained in Example 224 in a methanol/tetrahydrofuran (1/1) mixture (1 mL), and the mixture was stirred at room temperature for 1 hour. The reaction mixture was concentrated, and the residue was purified by silica gel chromatography (ethyl acetate/hexane=25 to 50%) to obtain the title compound (7 mg) as a dark yellow solid.

¹H NMR(500MHz,CDCl₃): δ7.83(s,1H),7.80(d,J=8.4Hz,4H),7.36(d,J=8.4Hz,4H),7.30(d,J=8.9Hz,1H),7.20(d,J=2.4Hz ,1H),7.15(dd,J=8.9,2.4 Hz,1H),3.43(s,1H),2.49(s,6H)

### Example 226

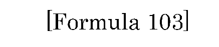

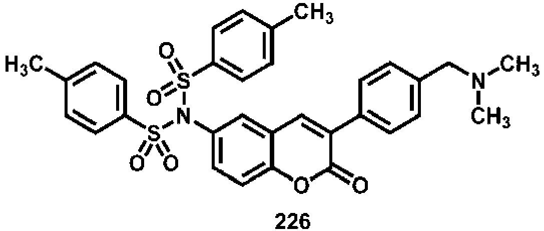

### N-[3-(4-methoxyphenyl)-2-oxo-2H-chromen-6-yl]-4-methyl-N-(4-methylbenzenesulfonyl)benzene-1-sulfonamide

(1) Using N-(3-bromo-2-oxo-2H-chromen-6-yl)-4-methyl-N-(4-methylbenzenesulfonyl)benzene-1-sulfonamide obtained in Reference Example 6, and according to the method of Example 168 or a method analogous thereto, N-[3-(4-formylphenyl)-2-oxo-2H-chromen-6-yl]-4-methyl-N-(4-methylbenzenesulfonyl)benzene-1-sulfonamide was obtained.
(2) The compound obtained above was treated in the same manner as in Example 82 or a method analogous thereto to obtain the title compound.
   ¹H NMR(500MHz,DMSO-d₆): δ8.31(s,1H),7.72-7.69(m,6H),7.62(d,J=2.6Hz,1H),7.51-7.47(m,5H),7.40-7.38(m,2H),7.11(dd,J=8.8,2.6Hz,1H),3.49(s,2H),2.46(s,6H),2.17(s,6H)

### Example 227

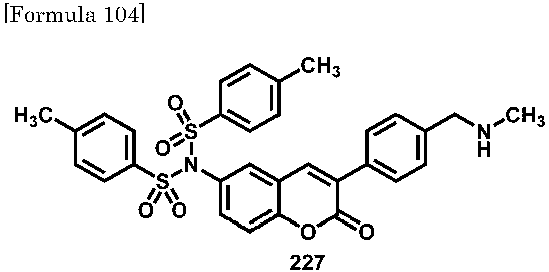

### N-[3-(4-methoxyphenyl)-2-oxo-2H-chromen-6-yl]-4-methyl-N-(4-methylbenzenesulfonyl)benzene-1-sulfonamide

The title compound was obtained according to the method of Example 82 or a method analogous thereto and using N-[3-(4-formylphenyl)-2-oxo-2H-chromen-6-yl]-4-methyl-N-(4-methylbenzenesulfonyl)benzene-1-sulfonamide obtained in Example 226-(1).

¹H NMR(500MHz,DMSO-d₆): δ8.87-8.73(brs,1H),8.35(s,1H),7.80-7.78(m,2H),7.71-7.69(m,4H),7.66(d,J=2.6Hz,1H),7.59-7.57(m,2H),7.52-7.48(m,5H),7.13(dd,J=8.8,2.6Hz,1H),4.18(s,2H),2.60(s,3H),2.46(s,6H)

### Example 228

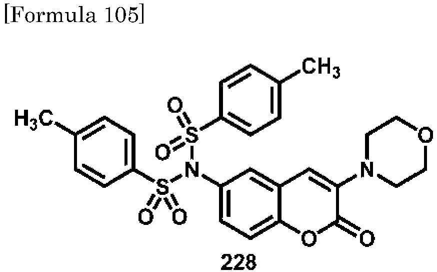

### 4-methyl-N-(4-methylbenzenesulfonyl)-N-[3-(morpholin-4-yl)-2-oxo-2H-chromen-6-yl]benzene-1-sulfonamide

N-(3-bromo-2-oxo-2H-chromen-6-yl)-4-methyl-N-(4-methylbenzenesulfonyl)benzene-1-sulfonamide (30 mg) obtained in Reference Example 6, morpholine (9.5 µL), bis[tri(tert-butyl)phosphine]palladium(0) (8.4 mg), and potassium tert-butoxide (12 mg) were suspended in toluene (0.55 mL) and stirred at 110°C for 19 hours under a nitrogen atmosphere. A saturated aqueous solution of ammonium chloride (3 mL) was added to the reaction mixture, and the mixture was then extracted with ethyl acetate (3 mL x 2). The extract was dried over anhydrous sodium sulfate and then concentrated, and the residue was purified by silica gel chromatography (ethyl acetate/hexane=0 to 35%) to obtain the title compound (1.1 mg) as a pale yellow solid.

¹H NMR(500MHz,CDCl₃): δ7.80(d,J=8.3Hz,4H),7.14(d,J=8.3Hz,4H),7.21(d,J=8.7Hz,1H),7.16(d,J=2.5Hz,1H),6.90(d d,J=8.7,2.5Hz,1H),6.68(s,1H),3.89(t,J=4.6Hz,4H),3.22(t,J=4.6Hz,4H),2.49(s,6H)

### Example 229

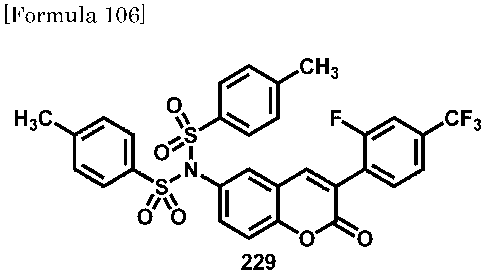

### N-{3-[2-fluoro-4-(trifluoromethyl)phenyl]-2-oxo-2H-chromen-6-yl}-4-methyl-N-(4-methylbenzenesulfonyl)benzene-1-sulfonamide

A suspension of 4-methyl-N-(4-methylbenzenesulfonyl)-N-[2-oxo-3-(tetramethyl-1,3,2-dioxaborolan-2-yl)-2H-chromen-6-yl]benzene-1-sulfonamide (30 mg) obtained in Reference Example 7, 2-fluoro-4-trifluoromethylbromobenzene (7 µL), potassium phosphate (21 mg) and bis[1,1'-bis(diphenylphosphino)ferrocene]dichloropalladium dichloromethane complex (II) (4 mg) in N,N-dimethylformamide / water (10/1) mixed solvent (1.1 mL) was stirred at 50 °C for 1 hour. A saturated aqueous solution of ammonium chloride (10 mL) was added to the reaction mixture, which was then extracted with ethyl acetate (50 mL). The extract was dried over sodium sulfate and concentrated, and the residue was purified by silica gel chromatography (ethyl acetate/hexane=0 to 50%) to obtain the title compound (7 mg) as a white solid.

¹H NMR(500MHz,DMSO-d₆): δ8.36(s,1H),7.88-7.80(m,2H),7.75-7.73(m,1H),7.72-7.70(m,4H),7.68(d,J=2.5Hz,1H),7.53(d,J=8.8Hz,1H),7.50-7.49(m,4H),7.17(dd,J=8.8,2.6Hz,1H),2.46(s,6H)

### Examples 230 to 232

The compounds shown in Table 12 were synthesized according to the method of Example 229 or a method analogous thereto.

### Example 233

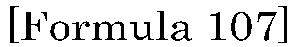

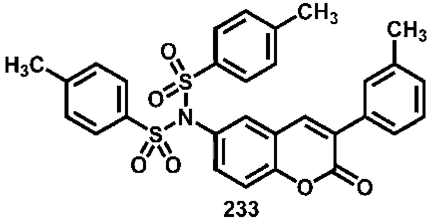

### 4-methyl-N-(4-methylbenzenesulfonyl)-N-[3-(3-methylphenyl)-2-oxo-2H-chromen-6-yl]benzene-1-sulfonamide

(1) 3-(3-methylphenyl)-6-nitro-2H-chromen-2-one was obtained from 5-nitrosalicylaldehyde according to the method described in Reference Example 2 or a method analogous thereto.
(2) The compound obtained above was treated in the manner described in Reference Example 4-(1) or a method analogous thereto to give 6-amino-3-(4-methylphenyl)-2H-chromen-2-one.
(3) The compound obtained above was treated according to the method of Reference Example 4-(2) or a method analogous thereto to give the title compound.
   ¹H NMR(500MHz,CDCl₃): δ7.83-7.80(m,4H),7.68(s,1H),7.49-7.45(m,2H),7.37-7.30(m,6H),7.28-7.24(m,2H),7.11(dd,J=8.8,2.5Hz,1H),2.49(s,6H),2.43(s,3H)

### Examples 234 to 253

The compounds shown in Tables 13-1 to 13-5 were synthesized according to the method of Example 233 or a method similar thereto.

### Example 254

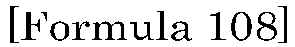

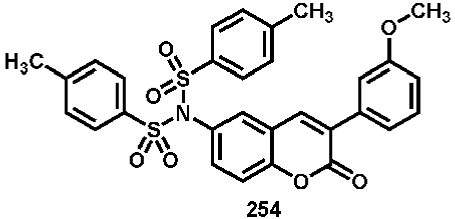

### N-[3-(3-methoxyphenyl)-2-oxo-2H-chromen-6-yl]-4-methyl-N-(4-methylbenzenesulfonyl)benzene-1-sulfonamide

(1) The compound obtained in Example 234 was treated in the same manner as in Example 68 or a method analogous thereto to give N-[3-(3-hydroxyphenyl)-2-oxo-2H-chromen-6-yl]-4-methyl-N-(4-methylbenzenesulfonyl)benzene-1-sulfonamide.
(2) The compound obtained above was treated in the same manner as in Example 101 or a method analogous thereto to obtain the title compound.
   ¹H NMR(500MHz,CDCl₃): δ7.81(d,J=8.4Hz,4H),7.70(s,1H),7.37(dd,J=8.1,8.1Hz,1H),7.36(d,J=8.4Hz,4H),7.32(d,J=8 .8Hz,1H),7.27(d,J=2.5Hz,1H),7.26-7.23(m,2H),7.11(dd,J=8.8,2.4Hz,1H),6.97(ddd,J=8.8,2.5,1.1Hz,1H),3.68(s,3H),2.48(s,6H)

### Example 255

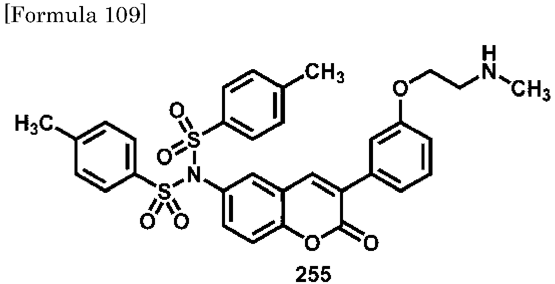

### 4-methyl-N-(3-{3-[2-(methylamino)ethoxy]phenyl}-2-oxo-2H-chromen-6-yl)-N-(4-methylbenzenesulfonyl)benzene-1-sulfonamide

(1) The compound obtained in Example 254-(1) was treated in the same manner as in Example 69-(1) or a method analogous thereto to give N-methyl-N-(2-{3-[6-N',N'-bis(4-methylbenzenesulfonyl)amino-2-oxo-2H-chromen-3-yl]phenoxy}ethyl)carbamic acid tert-butyl ester.
(2) The compound obtained above was treated in the same manner as in Example 69-(2) or a method analogous thereto to give the title compound.
   ¹H NMR(500MHz,CDCl₃): δ7.80(d,J=8.4Hz,4H),7.72(s,1H),7.35(d,J=8.4Hz,4H),7.33-7.28(m,3H),7.25-7.23(m,2H),7.09(dd,J=8.8,2.5Hz,1H),7.01(ddd,J=8.1,2.6,0.9Hz,1H),4.37(t,J=4.9Hz,2H),3 .30(t,J=4.9Hz,2H),2.75(s,3H),2.47(s,6H)

### Example 256

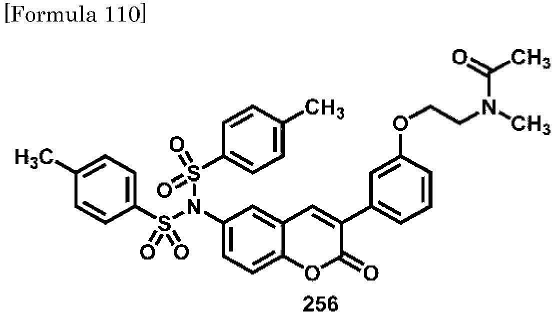

### N-methyl-N-[2-(3-{6-[N-(4-methylbenzenesulfonyl)4-methylbenzenesulfonamido]-2-oxo-2H-chromen-3-yl}phenoxy)ethyl]acetamide

The compound obtained in Example 255 was treated in the same manner as in Example 70 or a method analogous thereto to obtain the title compound.

¹H NMR(500MHz,CDCl₃),(66:34 mixture of rotamers): δ7.81(d,J=8.3Hz,4H),7.72(s,0.34H),7.71(s,0.66H),7.38-7.35(m,5H),7.33-7.22(m,5H),7.13-7.10(m,1H),6.97-6.93(m,1H),6.98-6.94(m,2H),4.19(t,J=5.3Hz,1.4H),4.16(t,J=5.3Hz,0.6H),3.78(t,J=5.3,1.4H),3.74(t,J=5.3,0 .6H),3.18(s,2H),3.03(s,1H),2.48(s,6H),2.22(s,1H),2.11(s,2H)

### Example 257

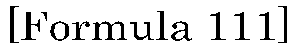

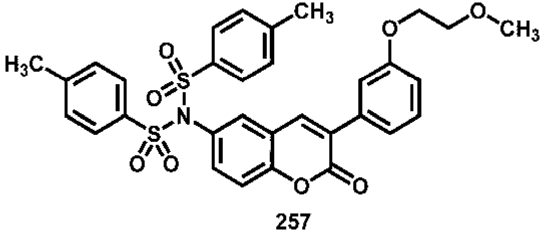

### N-{3-[3-(2-methoxyethoxy)phenyl]-2-oxo-2H-chromen-6-yl}-4-methyl-N-(4-methylbenzenesulfonyl)benzene-1-sulfonamide

The compound obtained in Example 254-(1) was treated in the same manner as in Example 69-(1) or a method analogous thereto to give the title compound.

¹H NMR(500MHz,CDCl₃): δ7.84-7.80(m,4H),7.69(s,1H),7.39-7.35(m,5H),7.32(d,J=8.8Hz,1H),7.28-7.25(m,3H),7.12(dd,J=8.7,2.4Hz,1H),7.02-6.99(m,1H),4.20-4.17(m,2H),3.80-3.77(m,2H),3.47(s,3H),2.49(s,6H)

### Example 258

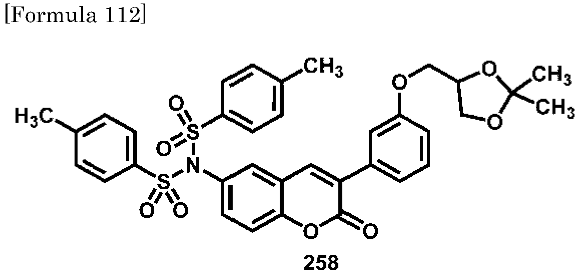

### N-{3-[3-(2,2-dimethyl-1,3-dioxolan-4-ylmethoxy)phenyl]-2-oxo-2H-chromen-6-yl}-4-methyl-N-(4-methylbenzenesulfonyl)benzene-1-sulfonamide

The compound obtained in Example 254-(1) was treated in the same manner as in Example 69-(1) or a method analogous thereto to give the title compound.

¹H NMR(500MHz,CDCl₃): δ7.84-7.81(m,4H),7.71(s,1H),7.40-7.35(m,5H),7.33(d,J=8.8Hz,1H),7.29-7.26(m,3H),7.13(dd,J=8.8,2.5Hz,1H),7.02-6.99(m,1H),4.55-4.49(m,1H),4.20(dd,J=8.5,6.5Hz,1H),4.12(dd,J=9.4,5.6Hz,1H),4.02(dd,J=9.4,5.9Hz,1H), 3.94(dd,J=8.5,5.9Hz,1H)2.50(s,6H),1.49(s,3H),1.43(s,3H)

### Example 259

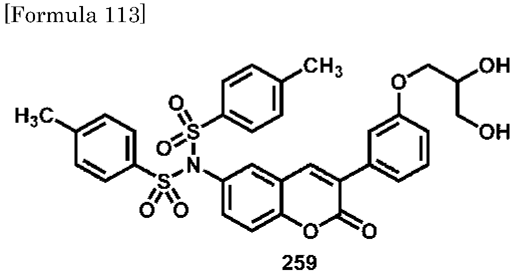

### N-{3-[3-(2,3-dihydroxypropoxy)phenyl]-2-oxo-2H-chromen-6-yl}-4-methyl-N-(4-methylbenzenesulfonyl)benzene-1-sulfonamide

The compound obtained in Example 258 was treated in the same manner as in Example 81 or a method analogous thereto to obtain the title compound.

¹H NMR(500MHz,CDCl₃): δ7.85-7.80(m,4H),7.72(s,1H),7.41-7.35(m,5H),7.33(d,J=8.8Hz,1H),7.30-7.26(m,3H),7.13(dd,J=8.8,2.5Hz,1H),7.02-6.99(m,1H),4.19-4.08(m,3H),3.91-3.85(m,1H),3.82-3.76(m,1H),2.57(d,J=4.5Hz,1H),2.50(s,6H),1.96(t,J=6.0Hz,1H)

### Example 260

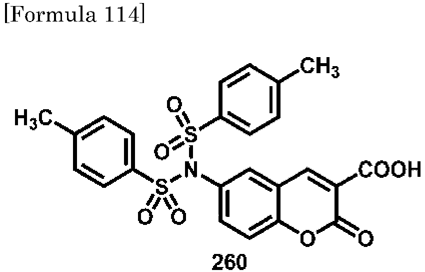

### 6-[N,N-bis(4-methylbenzenesulfonyl)amino]-2-oxo-2H-chromene-3-carboxylic acid

(1) Meldrum's acid (0.173 g) and iron(III) chloride were added to a solution of 5-nitrosalicylaldehyde (0.170 g) in benzyl alcohol (3 mL), and the mixture was stirred at 70°C for one day. The reaction mixture was diluted with ethyl acetate, and the precipitate was collected by filtration to obtain 6-nitro-2-oxo-2H-chromene-3-carboxylic acid benzyl ester (0.168 g) as a white solid.
(2) The compound obtained above was treated in the manner described in Reference Example 4-(1) or a method analogous thereto to give 6-amino-2-oxo-2H-chromene-3-carboxylic acid benzyl ester.
(3) The compound obtained above was treated in the manner described in Reference Example 4-(2) or a method analogous thereto to give 6-[N,N-bis(4-methylbenzenesulfonyl)amino]-2-oxo-2H-chromene-3-carboxylic acid benzyl ester.
(4) The compound obtained above was treated in accordance with the procedure of Reference Example 65 or a method analogous thereto to give the title compound.
   ¹H NMR(500MHz,CDCl₃): δ12.01(brs,1H),8.81(s,1H),7.82-7.78(m,4H),7.47(d,J=8.9Hz,1H),7.42-7.36(m,6H),2.51(s,6H)

### Example 261

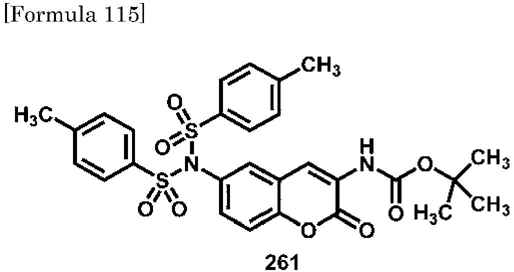

### N-{6-[N-(4-methylbenzenesulfonyl)4-methylbenzenesulfonamido]-2-oxo-2H-chromen-3-yl}carbamic acid tert-butyl ester

To a solution of 6-[N,N-bis(4-methylbenzenesulfonyl)amino]-2-oxo-2H-chromene-3-carboxylic acid (26 mg) obtained in Example 260 in tert-butanol (0.3 mL) were added triethylamine (0.010 mL) and diphenylphosphoryl azide (0.016 mL), and the mixture was stirred at 80°C for 30 minutes. The reaction mixture was diluted with ethyl acetate (30 mL) and washed with 1M hydrochloric acid (10 mL), saturated aqueous sodium hydrogen carbonate solution (10 mL), water (10 mL) and saturated brine (10 mL). The organic layer was dried over sodium sulfate and then concentrated, and the residue was purified by silica gel column chromatography (ethyl acetate/hexane 20%) to obtain the title compound (3 mg) as a white solid.

¹H NMR(500MHz,DMSO-d₆): δ8.66(s,1H),8.21(s,1H),7.72-7.68(m,4H),7.58(d,J=2.6Hz,1H),7.51-7.47(m,4H),7.42(d,J=8.8Hz,1H),6.97(dd,J=8.8,2.6Hz,1H),2.46(s,6H),1.49(s,9H)

### Example 262

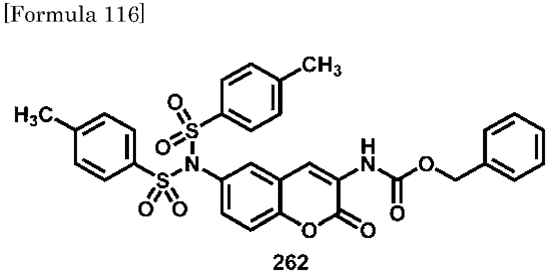

### N-{6-[N,N-bis(4-methylbenzenesulfonyl)amino]-2-oxo-2H-chromen-6-yl}carbamic acid benzyl ester

Using 6-[N,N-bis(4-methylbenzenesulfonyl)amino]-2-oxo-2H-chromene-3-carboxylic acid obtained in Example 260 and according to the method of Example 261 or an analogous method, the title compound was obtained.

¹H NMR(500MHz: DMSO-d₆): δ9.43(s,1H),8.26(s,1H),7.72-7.68(m,4H),7.59(d,J=2.6Hz,1H),7.51-7.33(m,10H),6.97(dd,J=8.8,2.6Hz,1H),5.22(s,2H),2.46(s,6H)

### Example 263

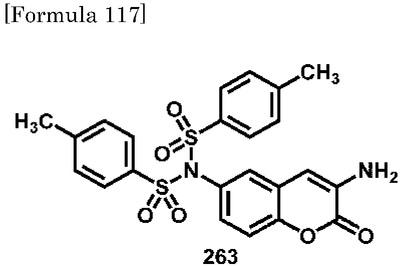

### N-(3-amino-2-oxo-2H-chromen-6-yl)-4-methyl-N-(4-methylbenzenesulfonyl)benzene-1-sulfonamide

The compound obtained in Example 261 was used to obtain the title compound according to the method of Example 69-(2) or a method analogous thereto.

¹H NMR(500MHz: DMSO-d₆): δ7.72-7.68(m,4H),7.51-7.47(m,4H),7.30(d,J=8.7Hz,1H),7.16(d,J=2.5Hz,1H),6.71(dd,J=8.7,2.5 Hz,1H),6.65(s,1H),5.93(brs,2H),2.46(s,6H)

### Example 264

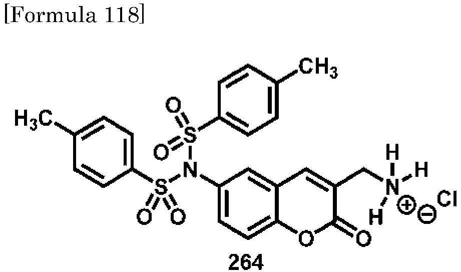

### N-[3-(aminomethyl)-2-oxo-2H-chromen-3-yl]-4-methyl-N-(4-methylbenzenesulfonyl)benzene-1-sulfonamide hydrochloride

(1) To a solution of 5-nitrosalicylaldehyde (89 mg) in acetic anhydride (0.53 mL), 3-[(tert-butoxycarbonyl)amino]propionic acid (0.100 g) and triethylamine (0.19 mL) were added, and the mixture was stirred at 140°C for 18 hours. The reaction mixture was diluted with water (10 mL) and extracted with ethyl acetate (30 mL). The extract was washed with saturated aqueous ammonium chloride solution (20 mL) and saturated brine (20 mL), dried over sodium sulfate, and then concentrated. The residue was purified by silica gel column chromatography (ethyl acetate/hexane 0-50%) to give 3-[3-N,N-bis(acetyl)aminomethyl]-6-nitro-2H-chromen-2-one (25 mg) as a yellow solid.
(2) The compound obtained above was treated in the manner described in Reference Example 4-(1) or a method analogous thereto to give 6-amino-3-[3-N,N-bis(acetyl)aminomethyl]-2H-chromen-2-one.
(3) The compound obtained above was treated in the manner described in Reference Example 4-(2) or a method analogous thereto to give N-acetyl-N-({6-[N',N'-bis(4-methylbenzenesulfonyl)amino]-2-oxo-2H-chromen-3-yl}methyl)acetamide.
(4) The compound obtained above was treated in the same manner as in Reference Example 111 or a method analogous thereto to obtain the title compound.
   ¹H NMR(500MHz,DMSO-d₆): δ8.16(s,1H),8.10(brs,1H),7.70(d,J=8.4Hz,4H),7.64(d,J=2.6Hz,1H),7.53(d,J=8.9Hz, 1H),7.50(d,J=8.1Hz,4H),7.18(d,J=8.9,2.6Hz,1H),3.91(s,2H),2.46(s,6H)

### Example 265

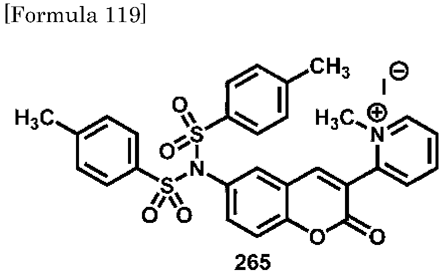

### 1-methyl-2-{6-[N-(4-methylbenzenesulfonyl)4-methylbenzenesulfonamido]-2-oxo-2H-chromen-3-yl}-pyridinium iodide

The title compound was obtained using 4-methyl-N-(4-methylbenzenesulfonyl)-N-[2-oxo-3-(pyridin-2-yl)-2H-chromen-6-yl]benzene-1-sulfonamide obtained in Example 235 and according to the method of Example 101 or an analogous method.

¹H NMR(500MHz,DMSO-d₆): δ9.19(d,J=6.0Hz,1H),8.76-8.72(m,1H),8.52(s,1H),8.27-8.24(m,2H),7.83(d,J=2.6Hz,1H),7.73-7.70(m,4H),7.62(d,J=8.9Hz,1H),7.52-7.49(m,4H),7.26(dd,J=8.8,2.6Hz,1H),4.31(s,3H),2.46(s,6H)

### Example 266

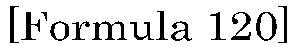

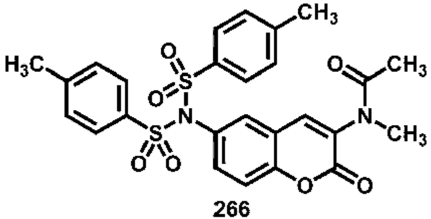

### N-methyl-{6-[N-(4-methylbenzenesulfonyl)4-methylbenzenesulfonamido]-2-oxo-2H-chromen-3-yl}acetamide

Using N-{6-[N-(4-methylbenzenesulfonyl)4-methylbenzenesulfonamido]-2-oxo-2H-chromen-3-yl}acetamide obtained in Example 241 and according to the method of Example 101 or an analogous method, the title compound was obtained.

¹H NMR(500MHz,CDCl₃): δ7.81(d,J=8.5Hz,4H),7.76(s,1H),7.74(d,J=8.5Hz,4H),7.33(d,J=9.0Hz),7.27(d,J=2.5Hz),7. 13(dd,J=2.5Hz,9.0Hz),3.24(s,3H),2.49(6H,s),2.10(brs,3H)

### Example 267

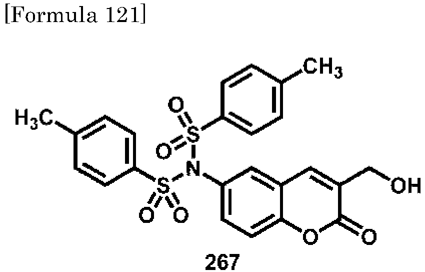

### 4-methyl-N-(4-methylbenzenesulfonyl)-N-[2-oxo-3-(hydroxymethyl)-2H-chromen-6-yl]benzene-1-sulfonamide

Palladium(II) chloride (33 mg) and sodium acetate (30 mg) were added to a solution of 4-methyl-N-(4-methylbenzenesulfonyl)-N-{2-oxo-3-[(prop-2-en-1-yloxy)methyl]-2H-chromen-6-yl}benzene-1-sulfonamide (50 mg) obtained in Example 242 in a mixed solvent of acetic acid/water (10/1) (1.1 mL), and the mixture was stirred at 75°C for 30 minutes. The reaction mixture was diluted with ethyl acetate and filtered through Celite, and the filtrate was washed with water (50 mL), an aqueous solution of sodium hydrogen carbonate (50 mL), and saturated brine (50 mL). The organic layer was dried over sodium sulfate and concentrated, and the solid residue was collected by filtration with ethyl acetate/hexane to obtain the title compound (36 mg) as a pale brown solid.

¹H NMR(500MHz,CDCl₃): δ7.80(d,J=8.4Hz,4H),7.67(s,1H),7.36(d,J=8.4Hz,4H),7.31(d,J=9.0Hz,1H),7.22(d,J=2.5Hz ,1H),7.13(dd,J=9.0,2.5Hz,1H),4.64(d,J=6.2Hz,2H),2.49(s,6H),2.36(t,J=1.7Hz,2H)

### Example 268

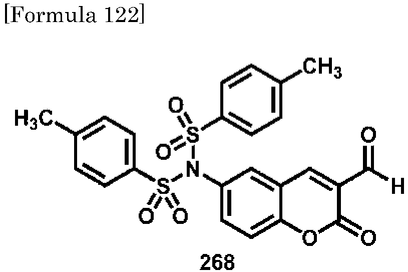

### N-(3-formyl-2-oxo-2H-chromen-6-yl)-4-methyl-N-(4-methylbenzenesulfonyl)benzene-1-sulfonamide

Manganese dioxide (600 mg) was added to a solution of 4-methyl-N-(4-methylbenzenesulfonyl)-N-[2-oxo-3-(hydroxymethyl)-2H-chromen-6-yl]benzene-1-sulfonamide (100 mg) obtained in Example 267 in dichloroethane (2 mL), and the mixture was stirred at room temperature for 95 hours. The reaction mixture was diluted with dichloromethane and filtered through Celite. The filtrate was concentrated and the residue was purified by silica gel column chromatography (ethyl acetate/hexane 0-25%) to obtain the title compound (45 mg) as a white solid.

¹H NMR(500MHz,CDCl₃): δ10.23(s,1H),8.28(s,1H),7.79(d,J=8.4Hz,4H),7.37-7.35(m,6H),7.28(dd,J=8.9,2.5Hz,1H),2.49(s,6H)

### Example 269

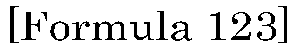

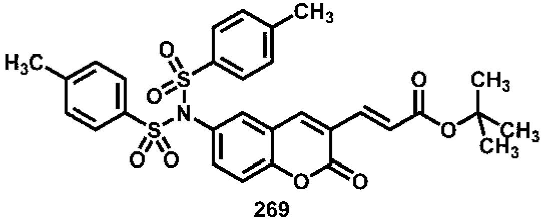

(2E)-3-{6-[N-(4-methylbenzenesulfonyl)4-methylbenzenesulfonamido]-2-oxo-2H-chromen-3-yl}prop-2-enoic acid tert-butyl ester

To a solution of diethylphosphonoacetic acid tert-butyl ester (0.026 mL) in tetrahydrofuran (0.5 mL) was added 60% sodium hydride (4.1 mg) at 0°C, and the mixture was stirred at room temperature for 30 minutes. To the reaction mixture was added a solution of N-(3-formyl-2-oxo-2H-chromen-6-yl)-4-methyl-N-(4-methylbenzenesulfonyl)benzene-1-sulfonamide (37 mg) obtained in Example 268 in tetrahydrofuran (1 mL), and the mixture was stirred at room temperature for 2 hours. A saturated aqueous solution of ammonium chloride (30 mL) was added to the reaction mixture, which was then extracted with ethyl acetate (50 mL). The extract was washed with water (50 mL) and saturated brine (50 mL), dried over sodium sulfate, and then concentrated. The residue was purified by silica gel column chromatography (ethyl acetate/hexane 0-25%) to obtain the title compound (25 mg) as a white solid.

¹H NMR(500MHz,CDCl₃): δ7.81(d,J=8.4Hz,4H),7.71(s,1H),7.43(d,J=15.9Hz,1H),7.36(d,J=8.4Hz,4H),7.30(d,J=8.8H z,1H),7.24(d,J=2.5Hz,1H),7.16(dd,J=8.8,2.5Hz,1H),7.01(d,J=15.9Hz,1H),2.50(s,6H),1.54 (s 9H)

### Example 270

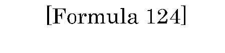

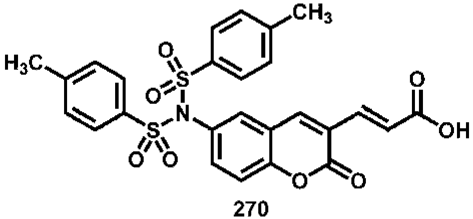

### (2E)-3-{6-[N-(4-methylbenzenesulfonyl)4-methylbenzenesulfonamido]-2-oxo-2H-chromen-3-yl}prop-2-enecarboxylic acid

The title compound was obtained using (2E)-3-{6-[N-(4-methylbenzenesulfonyl)4-methylbenzenesulfonamido]-2-oxo-2H-chromen-3-yl}prop-2-enoic acid tert-butyl ester obtained in Example 269 and according to the method of Example 69-(2) or a method analogous thereto.

¹H NMR(500MHz,CDCl₃): δ7.80(d,J=8.4Hz,4H),7.78(s,1H),7.61(d,J=15.5Hz,1H),7.36(d,J=8.4Hz,4H),7.31(d,J=8.6H z,1H),7.28(d,J=2.3Hz,1H),7.18(dd,J=8.6,2.3Hz,1H),7.13(d,J=15.5Hz,1H),2.49(s,6H)

### Example 271

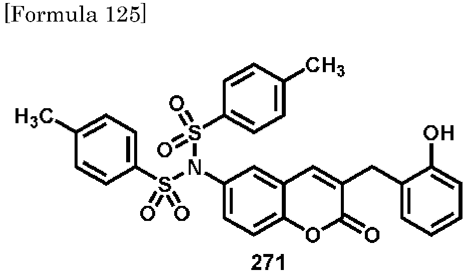

### N-{3-[(2-hydroxyphenyl)methyl]-2-oxo-2H-chromen-6-yl}-4-methyl-N-(4-methylbenzenesulfonyl)benzene-1-sulfonamide

The title compound was obtained using N-{3-[(2-methoxyphenyl)methyl]-2-oxo-2H-chromen-6-yl}-4-methyl-N-(4-methylbenzenesulfonyl)benzene-1-sulfonamide obtained in Example 245 and according to the method of Example 66 or an analogous method.

¹H NMR(500MHz,DMSO-d₆): δ9.47(s,1H),7.67(d,J=8.4Hz,4H),7.60(brs,1H),7.59(d,J=2.6Hz,1H),7.46(d,J=7.7Hz,4H),7. 40(d,J=8.8Hz,1H),7.14(dd,J=7.5,1.7Hz,1H),7.10(td,J=7.9,1.6Hz,1H),6.98(dd,J=8.8,2.4H z,1H),6.85(dd,J=8.1,1.1Hz,1H),6.77(td,J=7.3,1.1Hz,1H),3.72(s,2H),2.44(s,6H)

### Example 272

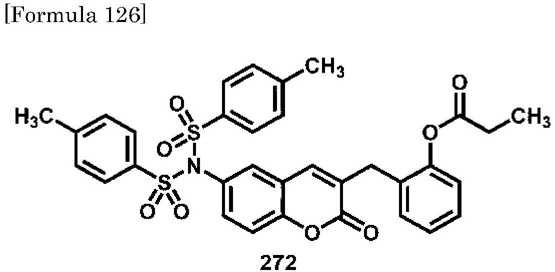

### Propionic acid 2-({6-[N-(4-methylbenzenesulfonyl)4-methylbenzenesulfonamido]-2-oxo-2H-chromen-3-yl}methyl)phenyl ester

To a solution of N-{3-[(2-hydroxyphenyl)methyl]-2-oxo-2H-chromen-6-yl}-4-methyl-N-(4-methylbenzenesulfonyl)benzene-1-sulfonamide (57 mg) obtained in Example 271 in an N,N-dimethylformamide (1 mL), 1-hydroxybenzotriazole (HOBt) monohydrate (18 mg), propionic acid (7.4 µL), 1-ethyl-3-(3-dimethylaminopropyl)carbodiimide hydrochloride (EDC.HCl) (29 mg), and triethylamine (0.028 mL) were added, and the mixture was stirred at room temperature for 24 hours. Water (10 mL) was added to the reaction mixture, which was then extracted with ethyl acetate (30 mL x 2). The extract was washed with saturated aqueous ammonium chloride solution (30 mL), water (30 mL), and saturated brine (30 mL), then dried over sodium sulfate and concentrated. The residue was purified by silica gel column chromatography (ethyl acetate/hexane 0-50%) to obtain the title compound (27 mg) as a white solid.

¹H NMR(500MHz,DMSO-d₆): δ7.75(s,1H),7.69-7.66(m,4H),7.54(d,J=2.6Hz,1H),7.49-7.46(m,4H),7.44-7.40(m,2H),7.33(td,J=7.8,1.9Hz,1H),7.25(td,J=7.5,1.2Hz,1H),7.11(dd,J=8.0,1.2Hz,1H),7. 02(dd,J=8.7,2.6Hz,1H),3.74(s,2H),2.58(q,J=7.5Hz,2H),2.45(s,6H),1.04(t,J=7.5Hz,3H)

### Example 273

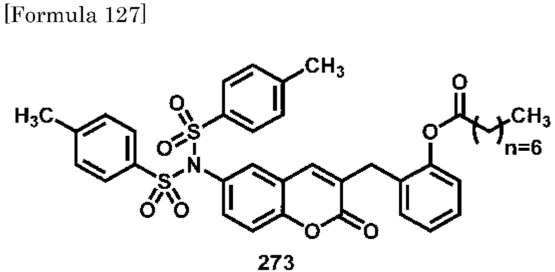

### 2-({6-[N-(4-methylbenzenesulfonyl)4-methylbenzenesulfonamido]-2-oxo-2H-chromen-3-yl}methyl)phenyl octanoate

The title compound was obtained in the same manner as in Example 272 using N-{3-[(2-hydroxyphenyl)methyl]-2-oxo-2H-chromen-6-yl}-4-methyl-N-(4-methylbenzenesulfonyl)benzene-1-sulfonamide obtained in Example 271.

¹H NMR(500MHz,DMSO-d₆): δ7.68-7.66(m,4H),7.63(s,1H),7.50(d,J=2.5Hz,1H),7.48-7.45(m,4H),7.43-7.39(m,2H),7.35(td,J=7.8,1.7Hz,1H),7.26(td,J=7.5,1.3Hz,1H),7.12(dd,J=8.0,1.1Hz,1H),7. 05(dd,J=8.8,2.6Hz,1H),3.73(s,2H),2.54(t,J=7.3Hz,2H),2.45(s,6H),1.56-1.50(m,2H),1.28-1.14(m,8H),0.83(t,J=6.8Hz,3H)

### Example 274

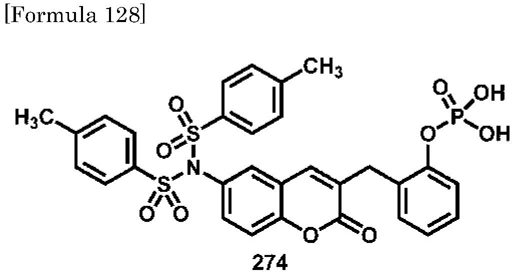

### [2-({6-[N-(4-methylbenzenesulfonyl)4-methylbenzenesulfonamido]-2-oxo-2H-chromen-3-yl}methyl)phenoxy]phosphoric acid

The title compound was obtained in accordance with the method of Example 94 and Example 95 or a method analogous thereto and using N-{3-[(2-hydroxyphenyl)methyl]-2-oxo-2H-chromen-6-yl}-4-methyl-N-(4-methylbenzenesulfonyl)benzene-1-sulfonamide obtained in Example 271.

¹H NMR(500MHz,DMSO-d₆): δ7.68-7.67(m,4H),7.58(s,1H),7.48-7.46(m,5H),7.42(d,J=8.6Hz,1H),7.35-7.32(m,1H),7.31-7.27(m,2H),7.14-7.11(m,1H),7.02(dd,J=8.7,2.5Hz,1H),3.85(s,2H),2.45(s,6H)

### Example 275

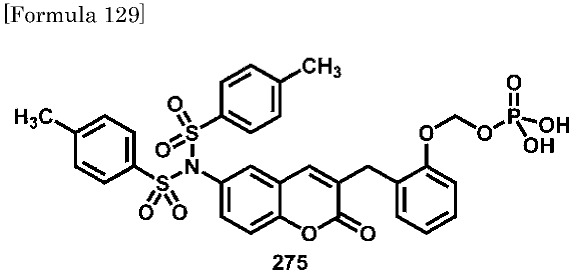

### [2-({6-[N-(4-methylbenzenesulfonyl)4-methylbenzenesulfonamido]-2-oxo-2H-chromen-3-yl}methyl)phenoxymethoxy]phosphoric acid

(1) To a solution of N-{3-[(2-hydroxyphenyl)methyl]-2-oxo-2H-chromen-6-yl}-4-methyl-N-(4-methylbenzenesulfonyl)benzene-1-sulfonamide (0.114 mg) obtained in Example 271 in N,N-dimethylformamide (2 mL) were added potassium carbonate (41 mg) and dibenzyl (chloromethyl) phosphate (0.19 mL) at 0°C, and the mixture was stirred at room temperature for 3 hours. Water (10 mL) was added to the reaction mixture, which was then extracted with ethyl acetate (30 mL x 2). The extract was washed with water (30 mL), saturated brine (20 mL x 3 times), and saturated brine (20 mL), then dried over sodium sulfate and concentrated. The residue was purified by silica gel column chromatography (ethyl acetate/hexane 0-50%) to give [2-({6-[N-(4-methylbenzenesulfonyl)4-methylbenzenesulfonamido]-2-oxo-2H-chromen-3-yl}methyl)phenoxymethoxy]phosphate dibenzyl ester (84 mg) as a white amorphous solid.
(2) The compound obtained above was treated in the same manner as in Example 65 or a method analogous thereto to obtain the title compound.
   ¹H NMR(500MHz,DMSO-d₆): δ7.69-7.66(m,4H),7.58(s,1H),7.48-7.39(m,6H),7.35-7.33(m,1H),7.30-7.27(m,2H),7.14-7.12(m,1H),7.01(dd,J=8.6,2.6Hz,1H),3.85(s,2H),2.45(s,6H),2.40(s,2H)

### Example 276

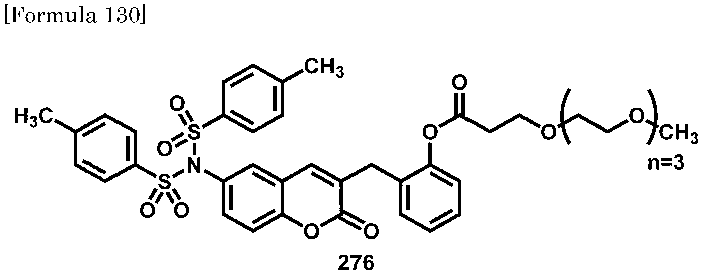

### 2,5,8,11-Tetraoxatetradecane-14-carboxylic acid 2-({6-[N-(4-methylbenzenesulfonyl)4-methylbenzenesulfonamido]-2-oxo-2H-chromen-3-yl}methyl)phenyl ester

(1) The title compound was obtained in accordance to a method in Example 272 or a method analogous thereto and using N-{3-[(2-hydroxyphenyl)methyl]-2-oxo-2H-chromen-6-yl}-4-methyl-N-(4-methylbenzenesulfonyl)benzene-1-sulfonamide obtained in Example 271.
   ¹H NMR(500MHz,DMSO-d₆): δ7.73(s,1H),7.69-7.67(m,4H),7.51(d,J=2.6Hz,1H),7.48-7.46(m,4H),7.42(d,J=8.8Hz,1H),7.40-7.38(m,1H),7.35-7.31(m,1H),7.27-7.23(m,1H),7.12-7.10(m,1H),7.04(dd,J=8.8,2.6Hz,1H),3.73(s,2H),3.71(t,J=6.2Hz,2H),3.52-3.43(m,10H),3.38-3.36(m,2H),2.82(t,J=6.1Hz,2H),2.45(s,6H)

### Example 277

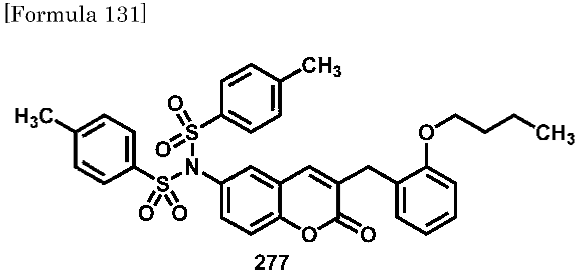

### N-{3-[(2-butoxyphenyl)methyl]-2-oxo-2H-chromen-6-yl}-4-methyl-N-(4-methylbenzenesulfonyl)benzene-1-sulfonamide

(1) The title compound was obtained using N-{3-[(2-hydroxyphenyl)methyl]-2-oxo-2H-chromen-6-yl}-4-methyl-N-(4-methylbenzenesulfonyl)benzene-1-sulfonamide obtained in Example 271 and according to the method described in Example 69-(1) or a method analogous thereto.
   ¹H NMR(500MHz,DMSO-d₆): δ7.70-7.65(m,4H),7.57(s,1H),7.52(d,J=2.5Hz,1H),7.48-7.44(m,4H),7.42(d,J=8.8Hz,1H),7.27-7.22(m,2H),7.02-6.98(m,2H),6.91(td,J=7.5,0.9Hz,1H),3.96(t,J=6.1Hz,2H),3.76(s,2H),2.44(s,6H),1.66-1.60(m,2H),1.34-1.28(m,2H),0.79(t,J=7.4Hz,3H)

### Example 278

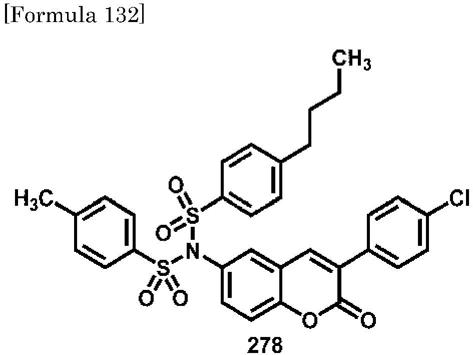

### 4-butyl-N-[3-(4-chlorophenyl)-2-oxo-2H-chromen-6-yl]-N-(4-methylbenzenesulfonyl)benzene-1-sulfonamide

(1) Using N-[3-(4-chlorophenyl)-2-oxo-2H-chromen-6-yl]-4-methyl-N-(4-methylbenzenesulfonyl)benzene-1-sulfonamide obtained in Example 193, N-[3-(4-chlorophenyl)-2-oxo-2H-chromen-6-yl]-4-methylbenzenesulfonamide was obtained according to the method of Reference Example 4-(3) or a method analogous thereto.
(2) Using the compound obtained above, the title compound was obtained according to the method of Reference Example 4-(2) or a method analogous thereto.
   ¹H NMR(500MHz,DMSO-d₆): δ8.33(s,1H),7.76-7.74(m,2H),7.73-7.71(m,2H),7.70-7.68(m,2H),7.60(d,J=2.6Hz,1H),7.57-7.55(m,2H),7.52-7.47(m,5H),7.13(dd,J=8.8,2.6Hz,1H),2.73(t,J=7.7Hz,2H),2.46(s,3H),1.64-1.56(m,2H),1.36-1.28(m,2H),0.90(t,J7.4 Hz,3H)

### Example 279

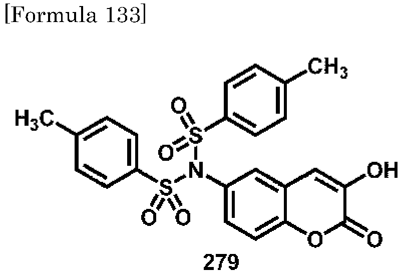

### N-(3-hydroxy-2-oxo-2H-chromen-6-yl)-4-methyl-N-(4-methylbenzenesulfonyl)benzene-1-sulfonamide

(1) Using 5-[N,N-bis(4-methylbenzenesulfonyl)amino]salicylaldehyde obtained in Reference Example 8, N-(3-benzyloxy-2-oxo-2H-chromen-6-yl)-4-methyl-N-(4-methylbenzenesulfonyl)benzene-1-sulfonamide was obtained according to the method of Reference Example 2 or a similar method.
(2) Using the compound obtained above, the title compound was obtained according to the method of Example 65 or a method analogous thereto.
   ESI-MS [M+Na]⁺: 486.0

### Example 280

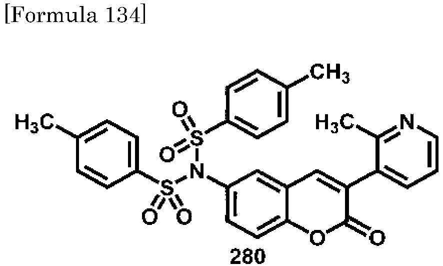

### 4-methyl-N-(4-methylbenzenesulfonyl)-N-[3-(2-methylpyridin-3-yl)-2-oxo-2H-chromen-6-yl]benzene-1-sulfonamide

Using 5-[N,N-bis(4-methylbenzenesulfonyl)amino]salicylaldehyde obtained in Reference Example 8 and according to the method of Reference Example 2 or a method analogous thereto, the title compound was obtained.

¹H NMR(500MHz,CDCl₃): δ8.59(dd,J=4.9,1.7Hz,1H),7.84-7.81(m,4H),7.58-7.56(m,2H),7.38-7.34(m,5H),7.27-7.25(m,1H),7.29(d,J=2.4Hz,1H),7.23(dd,J=7.7,4.9Hz,1H),7.15(d,J=8.8,2.4Hz,1H),2.55(s, 3H),2.49(s,6H)

### Example 281

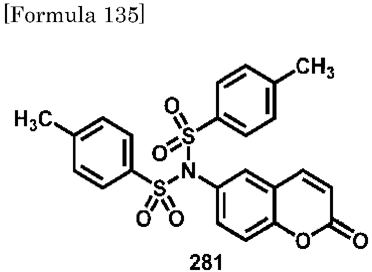

### 4-methyl-N-(4-methylbenzenesulfonyl)-N-(2-oxo-2H-chromen-6-yl)benzene-1-sulfonamide

The title compound was obtained using 6-amino-2H-chromen-2-one according to the method of Reference Example 4-(2) or a method analogous thereto.

¹H NMR(500MHz,CDCl₃): δ7.82-7.79(m,4H),7.61(d,J=9.6Hz,1H),7.38-7.35(m,4H),7.29(d,J=8.8Hz,1H),7.22(d,J=2.5Hz,1H),7.13(dd,J=8.8,2.5Hz,1H),6.47(d,J=9 .6Hz,1H),2.49(s,6H)

### Example 282

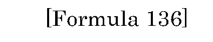

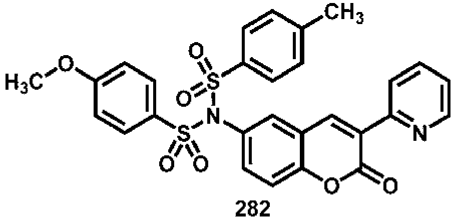

### 4-Methoxy-N-(4-methylbenzenesulfonyl)-N-[2-oxo-3-(pyridin-2-yl)-2H-chromen-6-yl]benzene-1-sulfonamide

(1) Using 4-methyl-N-(4-methylbenzenesulfonyl)-N-[2-oxo-3-(pyridin-2-yl)-2H-chromen-6-yl]benzene-1-sulfonamide obtained in Example 235, 4-methyl-N-[3-(pyridin-2-yl)-2-oxo-2H-chromen-6-yl]benzene-1-sulfonamide was obtained in accordance with the method of Reference Example 4-(3) or a method analogous thereto.
(2) Using the compound obtained above, the title compound was obtained according to the method of Reference Example 4-(2) or a method analogous thereto.
   ¹H NMR(500MHz,CDCl₃): δ8.70-8.67(m,1H),8.67(s,1H),8.40(d,J=8.0Hz,1H),7.88-7.85(m,2H),7.83-7.79(m,3H),7.38-7.31(m,5H),7.17(dd,J=8.8,2.5Hz,1H),7.03-7.00(m,2H),3.93(s,3H),2.49(s,3H)

### Example 283

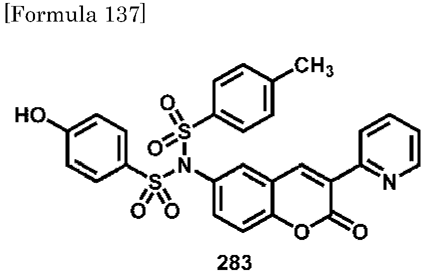

### 4-Hydroxy-N-(4-methylbenzenesulfonyl)-N-[2-oxo-3-(pyridin-2-yl)-2H-chromen-6-yl]benzene-1-sulfonamide

The title compound was obtained using 4-methoxy-N-(4-methylbenzenesulfonyl)-N-[2-oxo-3-(pyridin-2-yl)-2H-chromen-6-yl]benzene-1-sulfonamide obtained in Example 282 and according to the method of Example 66 or an analogous method.

¹H NMR(500MHz,CDCl₃): δ8.69(ddd,J=5.0,1.8,1.0Hz,1H),8.64(s,1H),8.39(dt,J=8.0,1.0Hz,1H),7.84-7.80(m,5H),7.38-7.33(m,5H),7.18(dd,J=8.8,2.5Hz,1H),6.97-6.94(m,2H),2.49(s,3H)

### Example 284

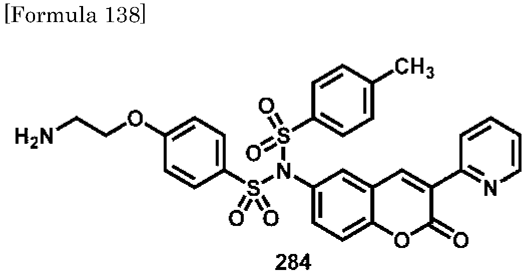

### 4-(2-aminoethoxy)-N-(4-methylbenzenesulfonyl)-N-[2-oxo-3-(pyridin-2-yl)-2H-chromen-6-yl]benzene-1-sulfonamide

(1) Using 4-hydroxy-N-(4-methylbenzenesulfonyl)-N-[2-oxo-3-(pyridin-2-yl)-2H-chromen-6-yl]benzene-1-sulfonamide obtained in Example 283, N-{N'-(4-methylbenzenesulfonyl)-N'-[2-oxo-3-(pyridin-2-yl)-2H-chromen-6-ylaminosulfonyl]phenoxyethylcarbamic acid tert-butyl ester was obtained in accordance to the method of Example 69-(1) or a method analogous thereto.
(2) Using the compound obtained above, the title compound was obtained according to the method of Example 69-(2).
   ¹H NMR(500MHz,CDCl₃): δ8.68-8.66(m,1H),8.60(s,1H),8.34-8.31(m,1H),7.84-7.84(m,5H),7.47(d,J=2.0Hz,1H),7.34-7.27(m,4H),7.12(dd,J=8.8,2.5Hz,1H),7.09-7.05(m,2H),4.41-4.36(m,1H),3.49-3.44(m,1H),2.46(s,3H)

### Example 285

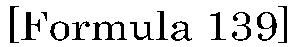

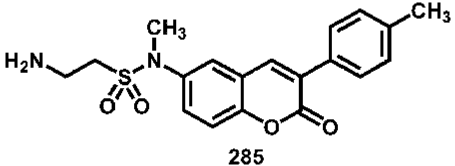

### 2-amino-N-methyl-N-[3-(4-methylphenyl)-2-oxo-2H-chromen-6-yl]ethane-1-sulfonamide

(1) Using 6-amino-3-(4-methylphenyl)-2H-chromen-2-one obtained in Reference Example 4-(1), 2-(1,3-dioxo-2,3-dihydro-1H-isoindol-2-yl)-N-[3-(4-methylphenyl)-2-oxo-2H-chromen-6-yl]ethane-1-sulfonamide was obtained in accordance to a method in Reference Example 5-(2) or a method analogous thereto.
(2) Using the compound obtained above, the title compound 2-(1,3-dioxo-2,3-dihydro-1H-isoindol-2-yl)-N-methyl-N-[3-(4-methylphenyl)-2-oxo-2H-chromen-6-yl]ethane-1-sulfonamide was obtained according to the method of Example 101 or a method analogous thereto.
(3) Using the compound obtained above, the title compound was obtained according to the method of Example 127-(2) or a method analogous thereto.
   ¹H NMR(500MHz,CDCls): δ12.29 (brs,1H),7.75(s,1H),7.72-7.69(m,6H),7.54-7.48(m,8H),7.10(d,J=8.8Hz,1H),6.95(dd,J=8.8,2.6Hz,1H),2.45(s,6H),7.77(s,1H),7.62-7.58(m,3H),7.51(dd,J=8.9,2.6Hz,1H),7.37(d,J=8.9,1H),7.29-7.25(m,2H),3.39(s,3H),3.24-3.20(m,2H),3.17-3.13(m,2H),2.41(s,3H)

### Example 286

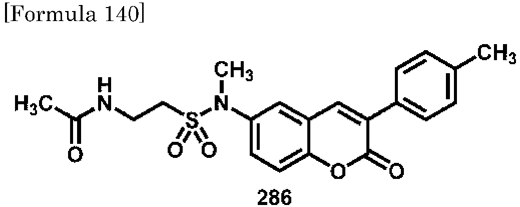

### N-(2-{N'-methyl-N'-[3-(4-methylphenyl)-2-oxo-2H-chromen-6-yl]amino}sulfonyl)acetamide

Using 2-amino-N-methyl-N-[3-(4-methylphenyl)-2-oxo-2H-chromen-6-yl]ethane-1-sulfonamide obtained in Example 285 and according to the method of Example 70 or an analogous method, the title compound was obtained.

¹H NMR(500MHz,CDCl3): δ7.75(s,1H),7.71-7.67(m,2H),7.64-7.60(m,2H),7.47-7.44(m,3H),7.36(d,J=8.9Hz,1H),7.30⁻7.27(m,2H),7.19(dd,J=8.9,2.6Hz,1H),6.42(brs,1H),4.40(s,2H),2.42(s,3H)

### Example 287

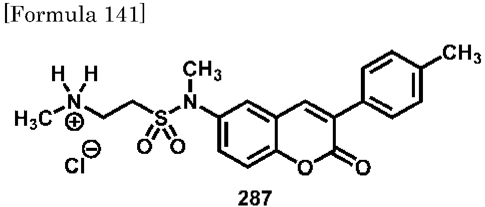

### N-methyl-2-(methylamino)-N-[3-(4-methylphenyl)-2-oxo-2H-chromen-6-yl]ethane-1-sulfonamide

(1) To a solution of 2-amino-N-methyl-N-[3-(4-methylphenyl)-2-oxo-2H-chromen-6-yl]ethane-1-sulfonamide (50 mg) obtained in Example 285 in a tetrahydrofuran/dichloroethane (2/1) mixed solvent (1.5 mL), triethylamine (0.054 mL) and di-tert-butyl dicarbonate (Boc₂O) (37 mg) were added, and the mixture was stirred at room temperature for 1 day. The reaction mixture was concentrated, and the residue was purified by silica gel column chromatography (ethyl acetate/hexane 0-35%) to give N-(2-{N'-methyl-N'-[3-(4-methylphenyl)-2-oxo-2H-chromen-6-yl]amino}sulfonyl)ethylcarbamic acid tert-butyl ester (20 mg) as a white amorphous solid.
(2) Using the compound obtained above, N-methyl-N-(2-{N'-methyl-N'-[3-(4-methylphenyl)-2-oxo-2H-chromen-6-yl]amino}sulfonyl)ethylcarbamic acid tert-butyl ester was obtained according to the method of Example 101 or an analogous method thereto.
(3) Using the compound obtained above, the title compound was obtained according to the method of Example 69-(2) or a method analogous thereto.
   ESI-MS [M+H]⁺: 387.1

### Example 288

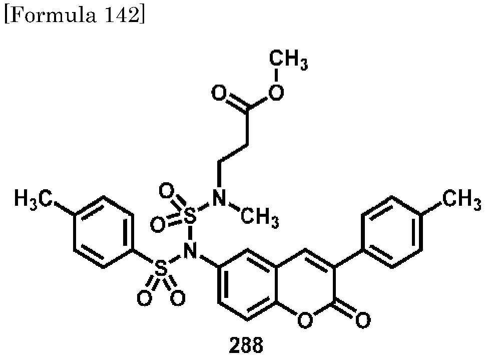

### Methyl 3-[methyl({N-[3-(4-methylphenyl)-2-oxo-2H-chromen-6-yl]4-methylbenzenesulfonamido}sulfonyl)amino]propionate

(1) Using 6-amino-3-(4-methylphenyl)-2H-chromen-2-one obtained in Reference Example 4-(1), 3-{N-methyl,N-[3-(4-methylphenyl)-2-oxo-2H-chromen-6-ylaminosulfonyl]amino}propionic acid methyl ester was obtained according to the method described in Reference Example 5-(2) or a method analogous thereto.
(2) Using the compound obtained above, the title compound was obtained according to the method of Reference Example 4-(2) or a method analogous thereto.
   ¹H NMR(500MHz,CDCl₃): δ7.72-7.69(m,3H),7.61-7.58(m,2H),7.40(d,J=2.5Hz,1H),7.33-7.28(m,4H),7.28-7.25(m,1H),7.22(dd,J=8.8,2.5Hz,1H),3.71(s,3H),3.69-3.64(m,2H),3.06(s,3H),2.68(t,J=7.1Hz,1H),2.47(s,3H),2.42(s,3H)

### Example 289

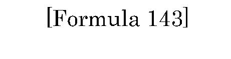

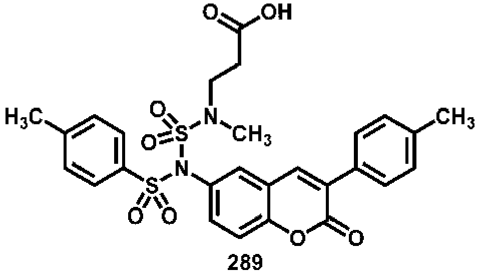

### 3-[methyl({N-[3-(4-methylphenyl)-2-oxo-2H-chromen-6-yl]4-methylbenzenesulfonamido}sulfonyl)amino]propionic acid

The title compound was obtained using methyl 3-[methyl({N-[3-(4-methylphenyl)-2-oxo-2H-chromen-6-yl]4-methylbenzenesulfonamido}sulfonyl)amino]propionate obtained in Example 288 and according to the method of Example 111 or a method analogous thereto.

ESI-MS[MH]-: 569.0

### Example 290

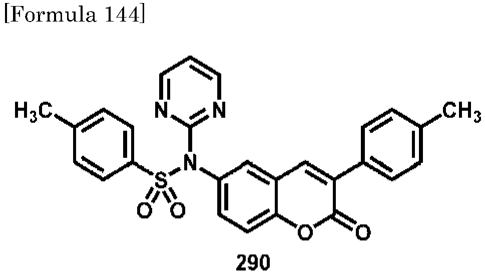

### 4-methyl-N-[3-(4-methylphenyl)-2-oxo-2H-chromen-6-yl]-N-(pyrimidin-2-yl)benzene-1-sulfonamide

(1) To a solution of 6-amino-3-(4-methylphenyl)-2H-chromen-2-one (0.163 g) obtained in Reference Example 4-(1) in a 1,4-dioxane/water (1/1) mixed solvent (9.6 mL) were added 2-bromopyrimidine (0.127 g) and concentrated hydrochloric acid (0.96 mL), and the mixture was stirred at 100°C for 2 days. A saturated aqueous solution of sodium hydrogen carbonate (30 mL) was added to the reaction mixture, which was then extracted with ethyl acetate (100 mL). The extract was washed with water (30 mL), dried over sodium sulfate, and concentrated. The residue was purified by silica gel column chromatography (ethyl acetate/hexane=45-70%) to give 3-(4-methylphenyl)-6-(pyrimidin-2-ylamino)-2H-chromen-2-one (4 mg) as a white solid.
(2) Using the compound obtained above, the title compound was obtained according to the method of Reference Example 4-(2) or a method analogous thereto.
   ESI-MS[M+Na]+: 484.3

### Example 291

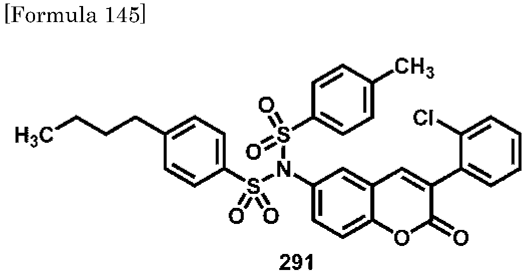

### 4-butyl-N-[3-(2-chlorophenyl)-2-oxo-2H-chromen-6-yl]-N-(4-methylbenzenesulfonyl)benzene-1-sulfonamide

(1) Using N-[3-(2-chlorophenyl)-2-oxo-2H-chromen-6-yl]-4-methyl-N-(4-methylbenzenesulfonyl)benzene-1-sulfonamide obtained in Example 189, 4-methyl-N-[3-(2-chlorophenyl)-2-oxo-2H-chromen-6-yl]benzene-1-sulfonamide was obtained in accordance to the method of Reference Example 4-(3) or a method analogous thereto.
(2) Using the compound obtained above, the title compound was obtained according to the method of Reference Example 4-(2) or a method analogous thereto.
   ¹H NMR(500MHz,DMSO-d₆): δ8.16(s,1H),7.73-7.72(m,2H),7.71-7.69(m,2H),7.65(d,J=2.6Hz,1H),7.61-7.59(m,1H),7.55-7.47(m,8H),7.15(dd,J=8.8,2.6Hz,1H),2.72(t,J=7.5Hz,2H),2.46(s,3H),1.64-1.56(m,2H),1.36-1.28(m,2H),0.90(t,J=7.2Hz,3H)

### Example 292

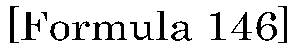

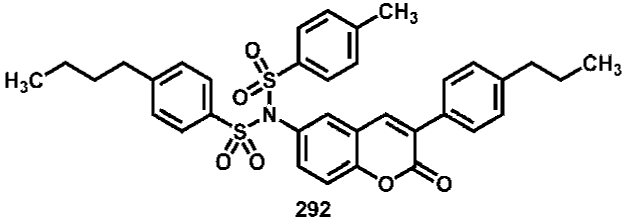

### 4-butyl-N-[3-(4-propylphenyl)-2-oxo-2H-chromen-6-yl]-N-(4-methylbenzenesulfonyl)benzene-1-sulfonamide

(1) Using N-[3-(4-propylphenyl)-2-oxo-2H-chromen-6-yl]-4-methyl-N-(4-methylbenzenesulfonyl)benzene-1-sulfonamide obtained in Example 195, 4-methyl-N-[3-(4-n-propylphenyl)-2-oxo-2H-chromen-6-yl]benzene-1-sulfonamide was obtained in accordance to the method of Reference Example 4-(3) or a method analogous thereto.
(2) Using the compound obtained above, the title compound was obtained according to the method of Reference Example 4-(2) or a method analogous thereto.
   ¹H NMR(500MHz,DMSO-d₆): δ8.33(s,1H),7.76-7.74(m,2H),7.73-7.71(m,2H),7.70-7.68(m,2H),7.60(d,J=2.6Hz,1H),7.57-7.55(m,2H),7.52-7.47(m,5H),7.13(dd,J=8.8,2.6Hz,1H),2.73(t,J=7.7Hz,2H),2.46(s,3H),1.64-1.56(m,2H),1.36-1.28(m,2H),0.90(t,J=7.4Hz,3H)

### Example 293

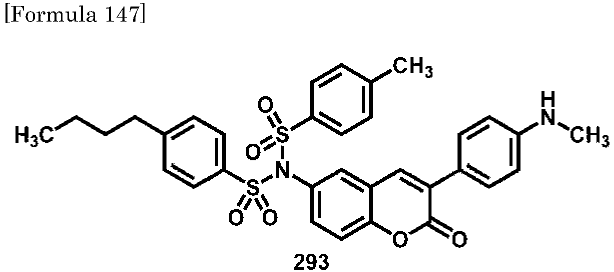

### 4-butyl-N-{3-[4-(methylamino)phenyl]-2-oxo-2H-chromen-6-yl}-N-(4-methylbenzenesulfonyl)benzene-1-sulfonamide

(1) Using N-(3-bromo-2-oxo-2H-chromen-6-yl)-4-methyl-N-(4-methylbenzenesulfonyl)benzene-1-sulfonamide obtained in Reference Example 6, and according to the method of Example 168 or an analogous method thereto, N-{3-[4-(tert-butoxycarbonyl)aminophenyl]-2-oxo-2H-chromen-6-yl}-4-methyl-N-(4-methylbenzenesulfonyl)benzene-1-sulfonamide was obtained.
(2) Using the compound obtained above, N-(3-{4-[N'-(tert-butoxycarbonyl)]-N'-methylaminophenyl}-2-oxo-2H-chromen-6-yl)-4-methyl-N-(4-methylbenzenesulfonyl)benzene-1-sulfonamide was obtained according to the method of Example 101 or a method analogous thereto.
(3) Using the compound obtained above, N-(3-{4-[N'-(tert-butoxycarbonyl)]-N'-methylaminophenyl}-2-oxo-2H-chromen-6-yl)-4-methylbenzene-1-sulfonamide was obtained according to the method of Reference Example 4-(3) or a method analogous thereto.
(4) Using the compound obtained above, N-(3-{4-[N'-(tert-butoxycarbonyl)]-N'-methylaminophenyl}-2-oxo-2H-chromen-6-yl)-4-butyl-N-(4-methylbenzenesulfonyl)benzene-1-sulfonamide was obtained according to the method of Reference Example 4-(2) or a method analogous thereto.
(5) Using the compound obtained above, the title compound was obtained according to the method of Example 69-(2) or a method analogous thereto.
   ¹H NMR(500MHz,DMSO-d₆): δ8.10(s,1H),7.73-7.71(m,2H),7.70-7.68(m,2H),7.57-7.56(m,2H),7.52-7.48(m,5H),7.42(d,J=8.8Hz,1H),7.04(dd,J=8.8,2.6Hz,1H),6.61-6.59(m,2H),6.08(q,J=5.1Hz,1H),2.74-2.72(m,5H),2.46(s,6H),1.64-1.58(m,2H),1.36-1.30(m,2H),0.91(t,J=7.4Hz,3H)

### Example 294

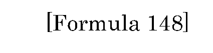

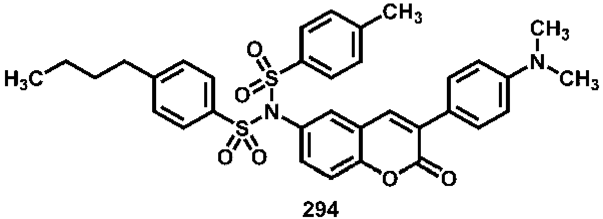

### 4-butyl-N-{3-[4-(dimethylamino)phenyl]-2-oxo-2H-chromen-6-yl}-N-(4-methylbenzenesulfonyl)benzene-1-sulfonamide

The title compound was obtained using 4-butyl-N-{3-[4-(methylamino)phenyl]-2-oxo-2H-chromen-6-yl}-N-(4-methylbenzenesulfonyl)benzene-1-sulfonamide obtained in Example 293 and according to the method of Example 101 or a method analogous thereto.

¹H NMR(500MHz,DMSO-d₆): δ8.15(s,1H),7.73-7.71(m,2H),7.70-7.68(m,2H),7.66-7.64(m,2H),7.54(d,J=2.6Hz,1H),7.51-7.48(m,4H),7.42(d,J=8.8Hz,1H),7.05(dd,J=8.8,2.6Hz,1H),6.79-6.77(m,2H),2.97(s,6H),2.74-2.72(m,2H),2.46(s,6H),1.65-1.58(m,2H),1.35-1.28(m,2H),0.91(t,J=7.4Hz,3H)

### Example 295

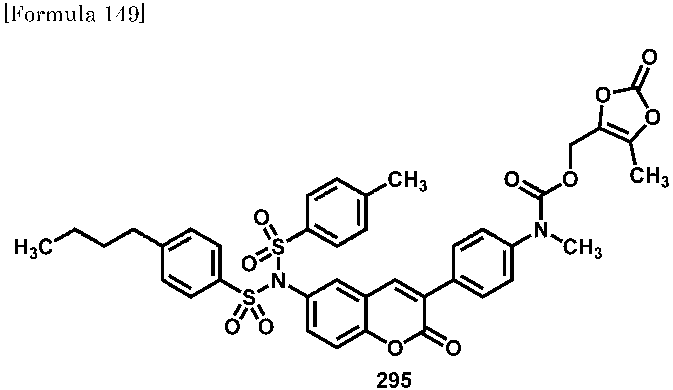

### N-methyl-N-(4-{6-[4-butylbenzenesulfonyl)4-methylbenzenesulfonamido]-2-oxo-2H-chromen-3-yl}phenyl)carbamic acid 5-methyl-2-oxo-2H-1,3-dioxol-4-ylmethyl ester

(1) Using 4-butyl-N-{3-[4-(methylamino)phenyl]-2-oxo-2H-chromen-6-yl}-N-(4-methylbenzenesulfonyl)benzene-1-sulfonamide obtained in Example 293, and according to a method analogous thereto, N-methyl-N-(4-{6-[4-methylbenzenesulfonyl)4-n-butylbenzenesulfonamido]-2-oxo-2H-chromen-3-yl}phenyl)carbamoyl chloride was obtained.
(2) Using the compound obtained above, the title compound was obtained according to the method of Example 86 or a method analogous thereto.

¹H NMR(500MHz,DMSO-d₆): δ7.99(s,1H),7.72-7.68(m,4H),7.63(d,J=2.5Hz,1H),7.51-7.48(m,4H),7.45(d,J=8.8Hz,1H),7.11-7.04(m,2H),7.01-6.99(m,1H),6.70-6.66(m,1H),6.59-6.55(m,2H),5.09(brs,2H),2.46(s,3H).

### Example 296

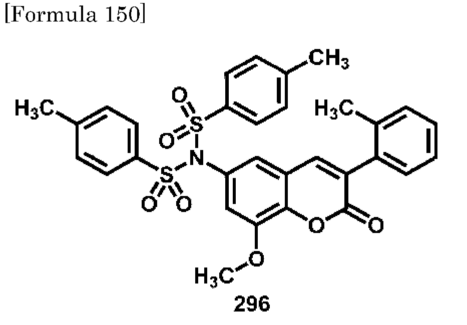

### N-[8-methoxy-3-(2-methylphenyl)-2-oxo-2H-chromen-6-yl]-4-methyl-N-(4-methylbenzenesulfonyl)benzene-1-sulfonamide

(1) 3-Methoxy-5-nitrosalicylaldehyde was used in the manner described in Reference Example 2 or a method analogous thereto to obtain 3-(3-methylphenyl)-8-methoxy-6-nitro-2H-chromen-2-one.
(2) The compound obtained above was treated in the manner described in Reference Example 4-(1) or a method analogous thereto to give 6-amino-3-(3-methylphenyl)-8-methoxy-2H-chromen-2-one.
(3) The compound obtained above was treated according to the method of Reference Example 4-(2) or a method analogous thereto to give the title compound.
   ¹H NMR(500MHz,CDCl₃): δ7.87-7.84(m, 4H),7.50(s,1H),7.39-7.35(m,4H),7.35-7.31(m,1H),7.29-7.21(m,3H),6.84(d,J=2.2Hz,1H),6.58(d,J=2.2Hz,1H),3.78(s,3H),2.49(s,6H),2.29(s,3H)

### Example 297

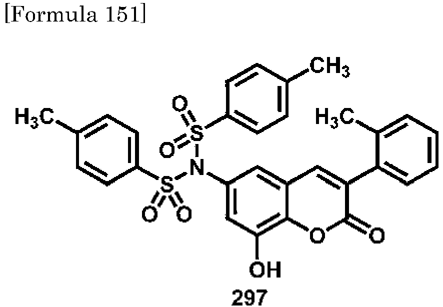

### N-[8-hydroxy-3-(2-methylphenyl)-2-oxo-2H-chromen-6-yl]-4-methyl-N-(4-methylbenzenesulfonyl)benzene-1-sulfonamide

(1) The title compound was obtained using N-[8-methoxy-3-(2-methylphenyl)-2-oxo-2H-chromen-6-yl]-4-methyl-N-(4-methylbenzenesulfonyl)benzene-1-sulfonamide obtained in Example 296 and according to the method of Example 66 or a method analogous thereto.
   ¹H NMR(500MHz,CDCl₃): δ7.86-7.83(m,4H),7.54(s,1H),7.38-7.33(m,5H),7.31-7.21(m,3H),6.82(d,J=2.3Hz,1H),680(d,J=2.3Hz,1H),6.18(brs,1H),2.48(s,6H),2.30(s,3H)

### Example 298

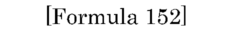

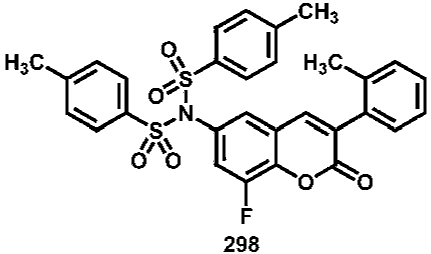

### N-[8-fluoro-3-(2-methylphenyl)-2-oxo-2H-chromen-6-yl]-4-methyl-N-(4-methylbenzenesulfonyl)benzene-1-sulfonamide

The title compound was obtained using 3-fluoro-5-nitrosalicylaldehyde according to the procedure described in Example 296 or a method analogous thereto.

¹H NMR(500MHz,CDCl₃): δ7.85-7.81(m,4H),7.53(d,J=1.0Hz,1H),7.40-7.33(m,5H),7.31-7.25(m,2H),7.24-7.21(m,1H),7.05-7.03(m,1H),6.95(dd,J=9.9,2.3Hz,1H),2.49(m,6H),2.30(m,3H)

### Example 299

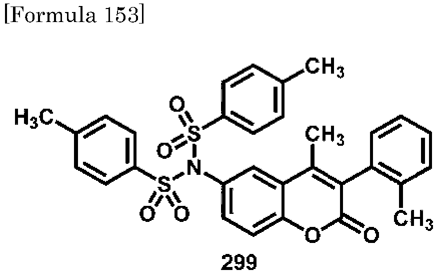

### 4-methyl-N-[4-methyl-3-(2-methylphenyl)-2-oxo-2H-chromen-6-yl]-N-(4-methylbenzenesulfonyl)benzene-1-sulfonamide

(1) To a solution of 2-hydroxy-5-nitroacetophenone (0.180 g) in n-butyl acetate (1 mL), 2-methylphenylacetic acid (0.165 g), a 1.7 M solution of propylphosphonic anhydride (T3P^{®}) in ethyl acetate (1.2 mL), and triethylamine (0.280 mL) were added, and the mixture was stirred at 120°C for 6 hours. The reaction mixture was diluted with ethyl acetate (50 mL) and then washed with saturated aqueous sodium hydrogen carbonate solution (10 mL), water (10 mL) and saturated brine (10 mL). The organic layer was dried over sodium sulfate and then concentrated, and the residue was purified by silica gel column chromatography (ethyl acetate/hexane 20%) to give 3-(2-methylphenyl)-4-methyl-6-nitro-2H-chromen-2-one (110 mg) as a white solid.
(2) The compound obtained above was treated in the manner described in Reference Example 4-(1) or a method analogous thereto to give 6-amino-3-(2-methylphenyl)-4-methyl-2H-chromen-2-one.
(3) The compound obtained above was treated according to the method of Reference Example 4-(2) or a method analogous thereto to give the title compound.
   ¹H NMR(500MHz,CDCl₃): δ7.88-7.82(m,4H),7.39-7.32(m,7H),7.30-7.28(m,2H),7.16(dd,J=8.8,2.5Hz,1H),7.12-7.09(m,1H),2.49(s,6H),2.19(s,3H),2.02(s,3H)

### Example 300

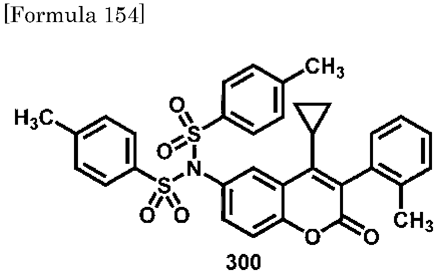

### N-[4-cyclopropyl-3-(2-methylphenyl)-2-oxo-2H-chromen-6-yl]-4-methyl-N-(4-methylbenzenesulfonyl)benzene-1-sulfonamide

(1) To a solution of 5-[N,N-bis(4-methylbenzenesulfonyl)amino]salicylaldehyde (0.450 g) obtained in Reference Example 8 in dichloromethane (7 mL), a solution of 0.7 M cyclopropylmagnesium bromide in THF (2.9 mL) was added, and the mixture was stirred under heating and reflux for 30 minutes. A saturated aqueous solution of ammonium chloride (30 mL) was added to the reaction mixture, which was then extracted with ethyl acetate (100 mL). The extract was washed with water (30 mL), dried over sodium sulfate, and concentrated. The residue was crystallized from ethyl acetate and collected by filtration to give N-[3-(1-cyclopropyl-1-hydroxy)methyl-4-hydroxyphenyl]-4-methyl-N-(4-methylbenzenesulfonyl)benzene-1-sulfonamide (0.370 g) as a white solid.
(2) The compound obtained above was treated in the manner described in Reference Example 8-(4) or a method analogous thereto to give N-[3-(1-cyclopropyl-1-oxo)methyl-4-hydroxyphenyl]-4-methyl-N-(4-methylbenzenesulfonyl)benzene-1-sulfonamide.
(3) The compound obtained above was treated according to the method of Reference Example 1-(4) or a method analogous thereto to give the title compound.
   ESI-MS[M+Na]+: 600.3

### Example 301

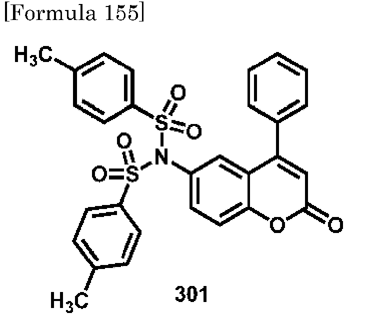

### 4-methyl-N-(4-methylbenzenesulfonyl)-N-(2-oxo-4-phenyl-2H-chromen-6-yl)benzene-1-sulfonamide

(1) To a solution of 4-hydroxycoumarin (1.6 g) in toluene (40 mL) was added tetra-n-butylammonium bromide (9.6 g) over 15 minutes, and the mixture was stirred at 100°C for 10 minutes. Next, phosphorus pentoxide (2.8 g) was added over 10 minutes, and the mixture was stirred at 100°C for 1 hour. The reaction mixture was cooled to 0°C and saturated sodium bicarbonate solution (50 mL) was added. The bilayer mixture was extracted with ethyl acetate (100 mL x 2), and the extract was dried over sodium sulfate and concentrated. The residue was purified by silica gel column chromatography (ethyl acetate/hexane=0-50%) to give 4-bromocoumarin (1.4 g).
(2) To a solution of the compound (1.4 g) obtained above in concentrated sulfuric acid (49 mL) was added concentrated nitric acid (7 mL) at -10°C, and the mixture was stirred for 30 minutes. The reaction mixture was allowed to warm to room temperature and then poured onto crushed ice. The precipitated solid was collected by filtration, and the obtained solid was washed with water (50 mL x 2). The resulting solid was purified by silica gel column chromatography (ethyl acetate/hexane=0-50%) to obtain 4-bromo-6-nitrocoumarin (0.9 g).
(3) Using the compound obtained above, 6-amino-4-bromo-coumarin was obtained according to the method described in Reference Example 4-(1) or a method analogous thereto.
(4) Using the compound obtained above, N-(4-bromo-2-oxo-2H-chromen-6-yl)-4-methyl-N-(4-methylbenzenesulfonyl)benzene-1-sulfonamide was obtained according to the method of Reference Example 4-(2) or a method analogous thereto.
(5) Using the compound obtained above, the title compound was obtained according to the method of Example 168 or a method analogous thereto.

¹H NMR(500MHz,DMSO-d₆): δ7.64(d,J=8.4Hz,4H),7.61(d, J=8.8Hz,1H),7.58-7.55(m,1H),7.51-7.48(m,2H),7.45(d,J=8.0Hz,4H),7.40(dd,J=8.8,2.5Hz,1H),7.30-7.28(m,2H),6.76(d,J=2.5Hz,1H),6.52(s,1H),2.46(s,6H).

### Example 302

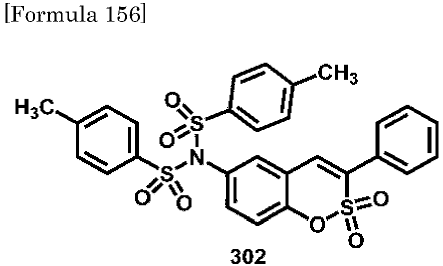

### N-(2,2-dioxo-3-phenyl-1,2-benzoxathiin-6-yl)-4-methyl-N-(4-methylbenzenesulfonyl)benzene-1-sulfonamide

(1) A solution of iron powder (120 mg) and ammonium chloride (11 mg) in a mixed solvent of ethanol/water (2/1) (4.5 mL) was stirred at 70°C for 30 minutes, and then a solution of 6-nitro-3-phenyl-1,2-benzoxathiin-2,2-dioxide (0.130 g) in tetrahydrofuran (5 mL) obtained in Reference Example 3 was added thereto, followed by stirring at 70°C for 2 hours. The reaction mixture was filtered through Celite, and the filtrate was extracted with ethyl acetate. The organic layer was washed with water (30 mL) and saturated brine (30 mL), dried over sodium sulfate, and concentrated to give crude 6-amino-3-phenyl-1,2-benzoxathiin-2,2-dioxide.
(2) To a solution of the compound obtained above in dichloromethane (4 mL), triethylamine (0.3 mL) and p-toluenesulfonyl chloride (0.210 g) were added, and the mixture was stirred at room temperature for one day. Water (10 mL) was added to the reaction mixture, which was then extracted with dichloromethane (50 mL). The organic layer was dried over sodium sulfate and concentrated, and the residue was purified by column chromatography (ethyl acetate/hexane=0-30%) to obtain the title compound (0.130 g).
   ¹H NMR(500 MHz,CDCl₃): δ7.84-7.79(m,4H),7.68-7.63(m,2H),7.52-7.47(m,3H),7.38-7.34(m,4H),7.26-7.24(m,1H),7.22(d,J=2.7Hz,1H),7.11(s,1H),7.05(dd,J=9.0,2.7Hz,1H),2.49(s,6H)

### Example 303

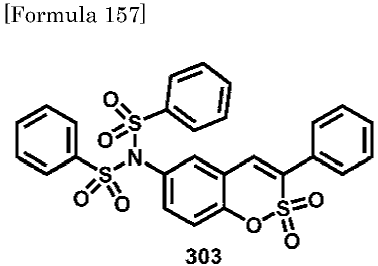

### N-(2,2-dioxo-3-phenyl-1,2-benzoxathiin-6-yl)-N-(benzenesulfonyl)benzene-1-sulfonamide

Using 6-nitro-3-phenyl-1,2-benzoxathiin-2,2-dioxide obtained in Reference Example 3, the title compound was obtained according to the method of Example 302 or a method analogous thereto.

¹H NMR(500MHz,CDCl₃): δ7.97-7.93(m,4H),7.74-7.70(m,2H),7.67-7.64(m,2H),7.61-7.57(m,4H),7.52-7.48(m,3H),7.27(d,J=8.7Hz,1H),7.20(d,J=2.6Hz,1H),7.10(s,1H),7.06(dd,J=8.7,2.6Hz,1H) ,2.49(s,6H)

### Example 304

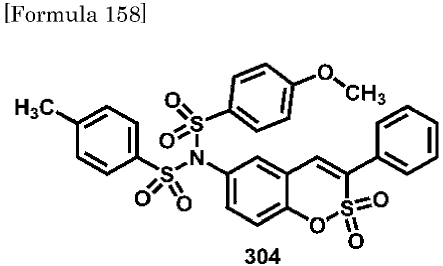

### N-(2,2-dioxo-3-phenyl-1,2-benzoxathiin-6-yl)-N-(4-methoxybenzenesulfonyl)-4-methylbenzenesulfonamide

(1) Using 6-amino-3-phenyl-1,2-benzoxathiin-2,2-dioxide obtained in Example 302-(1), N-(2,2-dioxo-3-phenyl-1,2-benzoxathiin-6-yl)-4-methylbenzenesulfonamide was obtained according to the method of Reference Example 5-(2) or a method analogous thereto.
(2) The compound obtained above was used to obtain the title compound according to the method of Example 15 or a method analogous thereto.

¹H NMR(500MHz,CDCl₃): δ7.69-7.61(m,4H),7.54-7.46(m,3H),7.33(d,J=2.5Hz,1H),7.28(s,1H),7.17(d,J=8.8Hz,1H),7.15(s,1H),7.02(dd,J=8.7 ,2.5Hz,1H),6.57(brs,1H),2.40(s,3H).

### Example 305

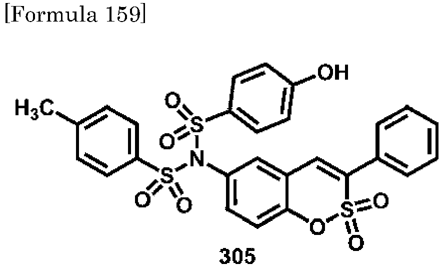

### N-(2,2-dioxo-3-phenyl-1,2-benzoxathiin-6-yl)-N-(4-hydroxybenzenesulfonyl)-4-methylbenzenesulfonamide

To a solution of N-(2,2-dioxo-3-phenyl-1,2-benzoxathiin-6-yl)-N-(4-methoxybenzenesulfonyl)-4-methylbenzenesulfonamide (30 mg) obtained in Example 304 in dichloromethane (0.5 mL), 2-methyl-2-butene (0.016 mL) and a 1 M solution of boron tribromide in dichloromethane (0.15 mL) were added at -20°C, and the mixture was stirred in the dark for 18 hours. Methanol (1 mL) was added to the reaction mixture at 0°C, and the mixture was warmed to room temperature. The reaction mixture was then poured into water (10 mL) and the organic layer and the aqueous layer were separated. The organic layer was washed with saturated brine (5 mL), dried over sodium sulfate, and then concentrated. The residue was purified by silica gel column chromatography (ethyl acetate/hexane 0-50%) to obtain the title compound (48 mg) as a pale brown oil.

¹H NMR(500MHz,CDCl₃): δ7.84-7.77(m,4H),7.68-7.61(m,2H),7.52-7.46(m,3H),7.36(d,J=8.5Hz,2H),7.28-7.24(m,1H),7.23(d,J=2.4Hz,1H),7.16-7.11(m,1H),7.06(dd,J=8.7,2.5Hz,1H),6.94(d,J=8.8Hz,2H),6.58(s,1H),2.48(s,3H).

### Example 306

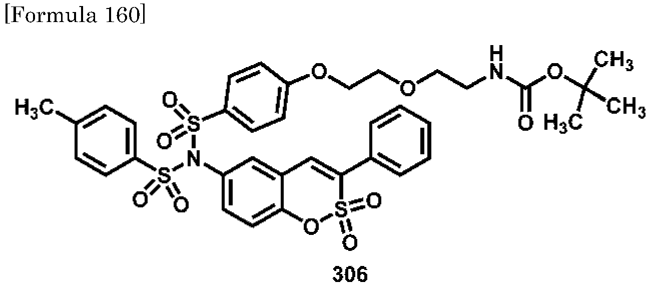

### N-{2-[2-(4-{[N-(2,2-dioxo-3-phenyl-1,2-benzoxathiin-6-yl)-4-methylbenzenesulfonylamino]sulfonyl}phenoxy)ethoxy]ethyl}carbamic acid tert-butyl ester

The title compound was obtained using N-(2,2-dioxo-3-phenyl-1,2-benzoxathiin-6-yl)-N-(4-hydroxybenzenesulfonyl)-4-methylbenzenesulfonamide obtained in Example 305 and according to the method described in Example 69-(1) or a method analogous thereto.

¹H NMR(500MHz,CDCl₃): δ7.86(d,J=9.1Hz,2H),7.82(d,J=8.4Hz,2H),7.69-7.63(m,2H),7.53-7.46(m,3H),7.36(d,J=8.1Hz,2H),7.26(d,J=8.7Hz,1H),7.22(d,J=2.5Hz,1H),7.13(s,1H),7.08 - 7.01(m,3H),4.92(brs,1H),4.21(dd,J=4.5,4.5Hz,2H),3.86(dd,J=4.6,4.6Hz,2H),3.62(t,J=5.2 Hz,2H),3.36(dt,J=5.1,5.1Hz,2H),2.48(s,3H),1.45(s,9H).

### Example 307

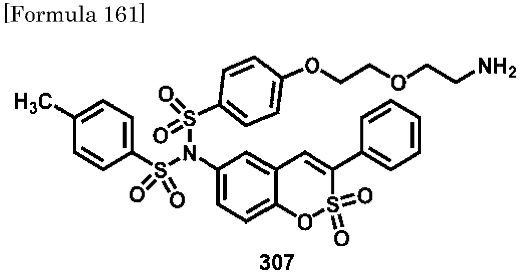

### N-{4-[2-(2-aminoethoxy)ethoxy]benzenesulfonyl}-N-(2,2-dioxo-3-phenyl-1,2-benzoxathiin-6-yl)-4-methylbenzenesulfonamide hydrochloride

The title compound was obtained using N-{2-[2-(4-{[N-(2,2-dioxo-3-phenyl-1,2-benzoxathiin-6-yl)-4-methylbenzenesulfonylamino]sulfonyl}phenoxy)ethoxy]ethyl}carbamic acid tert-butyl ester obtained in Example 306 and according to the method of Example 69-(2) or a method analogous thereto.

¹H NMR(500MHz,DMSO-d₆): δ8.00(s,1H),7.81-7.66(m,9H),7.61-7.56(m,4H),7.52(d,J=8.0Hz,2H),7.22(d,J=9.1Hz,2H),7.10(dd,J=8.7,2.6Hz,2H),4.29(dd,J= 4.1,4.1Hz,1H),3.84(d,J=4.3,4.3Hz,1H),3.67(t,J=5.1Hz,2H),3.02(d,J=5.7,5.0Hz,2H),2.49(s ,3H).

### Example 308

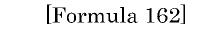

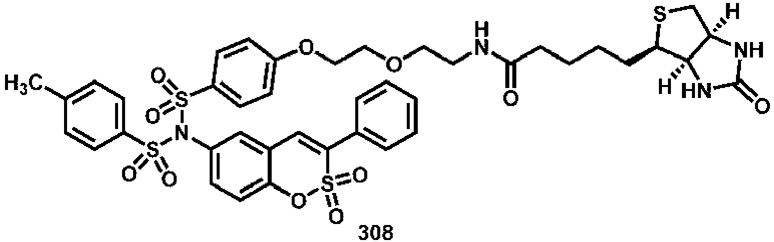

### 5-[(3aR,4R,6aS)-2-oxohexahydro-1H-thieno[3,4-d]imidazolidin-4-yl]-N-{2-[2-(4-{[N-(2,2-dioxo-3-phenyl-1,2-benzoxathiin-6-yl)-4-methylbenzenesulfonylamino]sulfonyl}phenoxy)ethoxy]ethyl}pentanamide

The title compound was obtained using N-{4-[2-(2-aminoethoxy)ethoxy]benzenesulfonyl}-N-(2,2-dioxo-3-phenyl-1,2-benzoxathiin-6-yl)-4-methylbenzenesulfonamide hydrochloride obtained in Example 307 and according to a method of Example 74-(3) or an analogous method.

¹H NMR(500MHz,CDCl₃): δ7.88(d,J=8.9Hz,2H),7.81(d,J=8.3Hz,2H),7.72-7.60(m,2H),7.54-7.45(m,3H),7.36(d,J=8.5Hz,2H),7.26(d,J=8.5Hz,1H),7.17(s,1H),7.05(d,J=8.9Hz,1H),6.29 (brs,1H),5.89(brs,1H),5.03(brs,1H),4.43(dd,J=4.9,4.9Hz,1H),4.34-4.17(m,3H),3.87(dd,J=4.4,4.4Hz,2H),3.64(t,J=5.1Hz,2H),3.54-3.39(m,3H),3.11(dt,J=7.4,4.4Hz,1H),2.87(1d,J=12.8,4.9Hz,H),2.68(d,J=12.8Hz,1H),2.48( s,3H),2.26-2.11(m,2H),1.77-1.64(m,4H),1.42(tt,J=7.7,7.7Hz,2H),

### Example 309

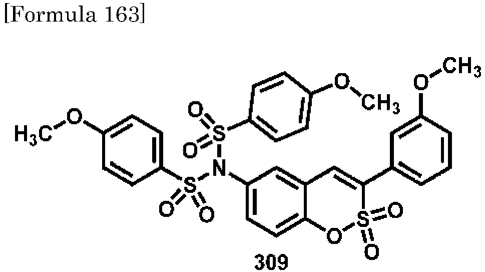

### N-[2,2-dioxo-3-(3-methoxyphenyl)-1,2-benzoxathiin-6-yl]-4-methoxy-N-(4-methoxybenzenesulfonyl)benzene-1-sulfonamide

(1) Using 5-nitrosalicylaldehyde, 6-nitro-3-(3-methoxyphenyl)-1,2-benzoxathiin-2,2-dioxide was obtained according to the method described in Reference Example 3 or a method analogous thereto.
(2) Using the compound obtained above, the title compound was obtained according to the method of Example 302 or a method analogous thereto.
   ¹H NMR(500MHz,CDCl₃): δ7.88-7.84(m,4H),7.40(t,J=8.0Hz,1H),7.27-7.22(m,2H),7.22(d,J=2.5Hz,1H),7.17-7.16(m,1H),7.12(s,1H),7.06(dd,J=8.7,2.5Hz,1H),7.05-7.00(m,5H),3.92(s,6H),3.87(s,3H)

### Example 310

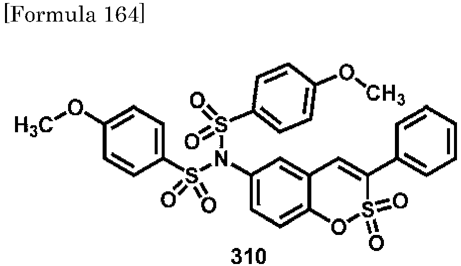

### N-(2,2-dioxo-3-phenyl-1,2-benzoxathiin-6-yl)-4-methoxy-N-(4-methoxybenzenesulfonyl)benzene-1-sulfonamide

Using 6-nitro-3-phenyl-1,2-benzoxathiin-2,2-dioxide obtained in Reference Example 3, the title compound was obtained according to the method of Example 302 or a method analogous thereto.

¹H NMR(500MHz,CDCl₃): δ7.88-7.84(m,4H),7.67-7.64(m,2H),7.51-7.48(m,2H),7.26(d,J=8.8Hz,1H),7.22(d,J=2.5Hz,1H),7.12(s,1H),7.06(dd,J=8.8,2.5Hz,1H) ,7.04-7.00(m,4H),3.92(s,6H)

### Example 311

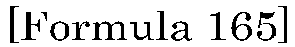

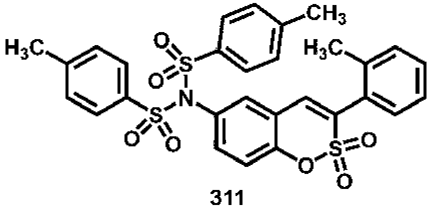

### N-[2,2-dioxo-3-(2-methylphenyl)-1,2-benzoxathiin-6-yl]-4-methyl-N-(4-methylbenzenesulfonyl)benzene-1-sulfonamide

(1) Using 5-nitrosalicylaldehyde, 6-nitro-3-(2-methylphenyl)-1,2-benzoxathiin-2,2-dioxide was obtained according to the method described in Reference Example 3 or a method analogous thereto.
(2) Using the compound obtained above, the title compound was obtained according to the method of Example 302 or a method analogous thereto.
   ¹H NMR(500MHz,CDCl₃): δ7.82(d,J=8.5Hz,4H),7.44-7.42(m,1H),7.40-7.32(m,6H),7.29-7.26(m,2H),7.16(d,J=2.5Hz,1H),7.05(dd,J=8.8,2.5Hz,1H),6.91(s,1H),2.48(s,6H),2.44(s,3 H)

### Example 312

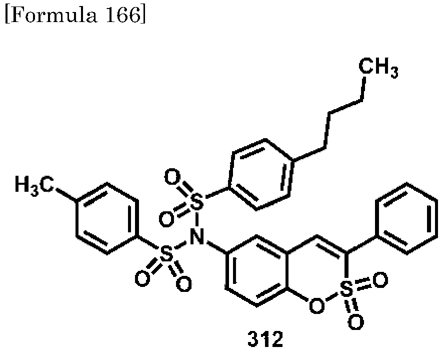

### 4-butyl-N-(2,2-dioxo-3-phenyl-1,2-benzoxazin-6-yl)-N-(4-methylbenzenesulfonyl)benzene-1-sulfonamide

(1) Using N-(2,2-dioxo-3-phenyl-1,2-benzoxazin-6-yl)-4-methyl-N-(4-methylbenzenesulfonyl)benzene-1-sulfonamide obtained in Example 302, N-(2,2-dioxo-3-phenyl-1,2-benzoxazin-6-yl)4-methylbenzenesulfonamide was obtained according to the method of Reference Example 4-(3) or a method analogous thereto.
(2) The compound obtained above was treated in the same manner as in Example 15 or an analogous method to obtain the title compound.
   ¹H NMR(500MHz,DMSO-d₆): δ7.98(s,1H),7.76-7.70(m,6H),7.65(d,J=2.6Hz,1H),7.60-7.57(m,4H),7.52-7.49(m,4H),7.13(dd,J=8.8,2.6Hz,1H),2.75-2.71(m,2H),2.46(s,3H),1.64-1.56(m,2H),1.36-1.29(m,2H),0.90(t,J=7.4Hz,3H)

### Example 313

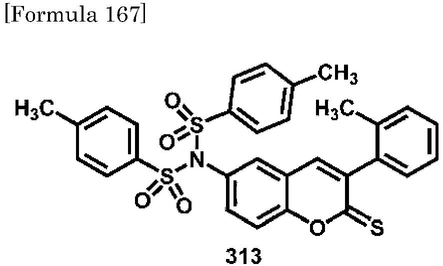

### 4-methyl-N-(4-methylbenzenesulfonyl)-N-[3-(2-methylphenyl)-2-sulfanylidene-2H-chromen-6-yl]benzene-1-sulfonamide

(1) A solution of 4-methyl-N-(4-methylbenzenesulfonyl)-N-[3-(2-methylphenyl)-2-oxo-2H-chromen-6-yl]benzene-1-sulfonamide (0.170 g) obtained in Example 1 in toluene (10 mL) was stirred with Lawesson's reagent (80 mg) under heating to reflux under a nitrogen atmosphere for 1 day. The reaction mixture was passed through a short column of silica gel using a solvent of ethyl acetate/hexane=2/1, and the filtrate was concentrated. The residue was purified by silica gel column chromatography (ethyl acetate/hexane 20%) to obtain the title compound (54 mg) as an orange solid.
   ¹H NMR(500MHz,CDCl₃): δ7.86-7.80(m,4H),7.45(d,J=8.9Hz,1H),7.38-7.32(m,6H),7.29-7.25(m,3H),7.16-7.13(m,2H),2.49(s,6H),2.26(s,3H)

### Example 314

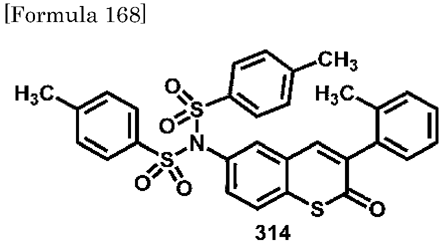

### 4-methyl-N-(4-methylbenzenesulfonyl)-N-[3-(2-methylphenyl)-2-oxo-2H-thiochromen-6-yl]benzene-1-sulfonamide

(1) Using 3-(2-methylphenyl)-6-nitro-2H-thiochromen-2-one obtained in Reference Example 1, 6-amino-3-(2-methylphenyl)-2H-thiochromen-2-one was obtained according to the method described in Reference Example 4-(1) or a method analogous thereto.
(2) The compound obtained above was treated in the manner described in Reference Example 4-(2) or a method analogous thereto to give the title compound.
   ¹H NMR(500MHz,CDCl3): δ7.85-7.81(m,4H),7.55(s,1H),7.46(d,J=8.5Hz,1H),7.38-7.30(m,6H),7.29-7.24(m,2H),7.20-7.17(m,1H),7.09(dd,J=8.5,2.2Hz,1H),2.48(s,6H),2.25(s,3H)

### Example 315

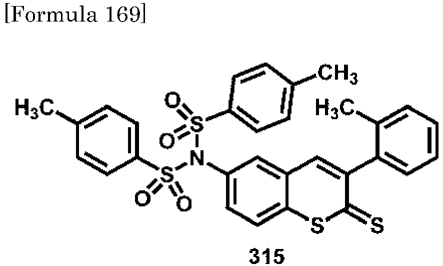

### 4-methyl-N-(4-methylbenzenesulfonyl)-N-[3-(2-methylphenyl)-2-sulfanylidene-2H-thiochromen-6-yl]benzene-1-sulfonamide

The title compound was obtained using 4-methyl-N-(4-methylbenzenesulfonyl)-N-[3-(2-methylphenyl)-2-oxo-2H-thiochromen-6-yl]benzene-1-sulfonamide obtained in Example 314 and according to the procedure of Example 313 or a method analogous thereto.

¹H NMR(500MHz,CDCl₃): δ7.85-7.81(m,4H),7.41(d,J=8.5Hz,1H),7.39(s,1H),7.38-7.31(m,6H),7.28-7.24(m,2H),7.13-7.09(m,2H),2.48(s,6H),2.21(s,3H)

### Example 316

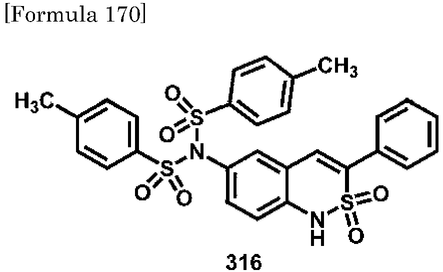

### N-(2,2-dioxo-3-phenyl-1H-2,1-benzothiazin-6-yl)-4-methyl-N-(4-methylbenzenesulfonyl)benzene-1-sulfonamide

(1) Imidazole (0.510 g) and tert-butyldimethylchlorosilane (0.497 g) were added to a solution of 2-amino-5-nitrobenzyl alcohol (0.50 g) in N,N-dimethylformamide (10 mL), and the mixture was stirred at room temperature for 3 hours. Water (20 mL) was added to the reaction mixture, which was then extracted with ethyl acetate (40 mL x 2). The extract was washed with water (50 mL x 2) and saturated brine (50 mL), dried over sodium sulfate, and then concentrated. The residue was purified by silica gel column chromatography (ethyl acetate/hexane 0-40%) to give 2-[(tertbutyldimethylsilyl)oxymethyl]-4-nitroaniline (0.812 g) as a yellow oil.
(2) To a solution of the compound (0.812 g) obtained above in tetrahydrofuran (10 mL), sodium bicarbonate (0.480 g) and benzyloxycarbonyl chloride (0.44 mL) were added at 0°C, and the mixture was stirred at room temperature for 3 days. Water (30 mL) was added to the reaction mixture, which was then extracted with ethyl acetate (50 mL x 2). The extract was washed with water (50 mL) and saturated brine (50 mL), dried over sodium sulfate, and concentrated. The residue was purified by silica gel column chromatography (ethyl acetate/hexane 0-40%) to give 2-[(tertbutyldimethylsilyl)oxymethyl]-4-nitrophenylcarbamic acid benzyl ester (0.953 g) as a white solid.
(3) Using the compound obtained above, 4-amino-2-[(tertbutyldimethylsilyl)oxymethyl]-phenylcarbamic acid benzyl ester was obtained according to the method of Reference Example 4-(1) or a method analogous thereto.
(4) Using the compound obtained above, 2-[(tert-butyldimethylsilyl)oxymethyl]-4-[bis(4-methylbenzenesulfonyl)amino]phenylcarbamic acid benzyl ester was obtained according to the method of Reference Example 4-(2) or a method analogous thereto.
(5) Using the compound obtained above, 2-hydroxymethyl-4-[bis(4-methylbenzenesulfonyl)amino]phenylcarbamic acid benzyl ester was obtained according to the method described in Reference Example 8-(3) or a method analogous thereto.
(6) Using the compound obtained above, 2-(benzyloxycarbonylamino)-5-[bis(4-methylbenzenesulfonyl)amino]benzaldehyde was obtained according to the method of Reference Example 8-(4) or a method analogous thereto.
(7) To a solution of the compound (0.175 g) obtained above in N,N-dimethylformamide (3 mL) was added 60% sodium hydride (24 mg) at 0°C, and the mixture was stirred for 30 minutes. Then, benzylsulfonyl chloride (86 mg) was added at 0°C, and the mixture was stirred at room temperature for 17 hours. Water (10 mL) was added to the reaction mixture, which was then extracted with ethyl acetate (30 mL). The extract was washed with water (40 mL x 2) and saturated brine (40 mL), dried over sodium sulfate, and then concentrated. The residue was treated with silica gel column chromatography (ethyl acetate/hexane 0-40%) to obtain a compound containing 4-hydroxy-6-[N-bis(4-methylbenzenesulfonyl)amino]-2,2-dioxo-3-phenyl-3,4-dihydro-1H-2,1-benzothiazine-1-carboxylic acid benzyl ester.
(8) To a solution of the compound obtained above in dichloromethane (1 mL), DBU (0.010 mL) was added at 0°C, and the mixture was stirred for 1 hour and then at room temperature for 4 hours. The reaction mixture was acidified with 3M hydrochloric acid, and then extracted with ethyl acetate (15 mL x 2). The extract was washed with water (20 mL), dried over sodium sulfate, and concentrated. The residue was purified by silica gel column chromatography (ethyl acetate/hexane 0-50%) to obtain the title compound (15 mg) as a white solid.
   ¹H NMR(500MHz,DMSO-d₆): δ12.29(brs,1H),7.75(s,1H),7.72-7.69(m,6H),7.54-7.48(m,8H),7.10(d,J=8.8Hz,1H),6.95(dd,J=8.8,2.6Hz,1H),2.45(s,6H)

### Example 317

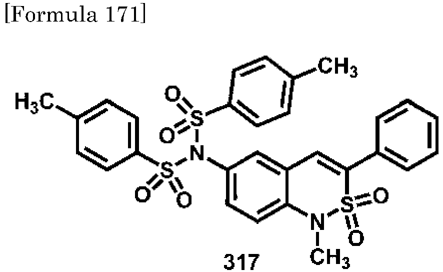

### N-(2,2-dioxo-3-phenyl-1-methyl-1H-2,1-benzothiazin-6-yl)-4-methyl-N-(4-methylbenzenesulfonyl)benzene-1-sulfonamide

The compound obtained in Example 316 was treated in the same manner as in Example 101 or a method analogous thereto to give the title compound.

¹H NMR(500MHz,CDCl3): δ7.83(d,J=8.4Hz,4H),7.66-7.64(m,2H),7.48-7.46(m,3H),7.35(d,J=8.0Hz,4H),7.20(d,J=2.3Hz,1H),7.17(s,1H),7.12(d,J=8.9Hz,1H),7.09 (dd,J=8.8,2.3Hz,1H),3.58(s,3H),2.48(s,6H)

### Example 318

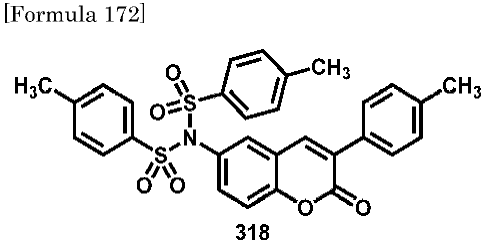

### 4-methyl-N-(4-methylbenzenesulfonyl)-N-[3-(4-methylphenyl)-2-oxo-2H-chromen-6-yl]benzene-1-sulfonamide

To a solution of 6-amino-3-(4-methylphenyl)-2H-chromen-2-one (126 mg) obtained in Reference Example 4-(1) in dichloromethane (5 mL), triethylamine (350 µL) and p-toluenesulfonyl chloride (240 mg) were added at room temperature, and the mixture was stirred at the same temperature for 2 days. Water was added to the reaction mixture, which was then extracted with dichloromethane. The organic layer was washed with water and saturated brine, dried over sodium sulfate, and then concentrated. The residue was purified by silica gel column chromatography (ethyl acetate/hexane=30%) to obtain the title compound (120 mg) as a white solid.

¹H NMR(500MHz,CDCl₃): δ7.83-7.80(m,4H),7.67(s,1H),7.58(d,J=8.2Hz,2H),7.38-7.34(m,4H),7.30(d,J=8.8Hz,1H),7.28-7.25(m,3H),7.10(dd,J=8.8,2.5Hz,1H),2.49(s,6H),2.41(s,3H)

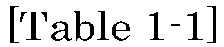

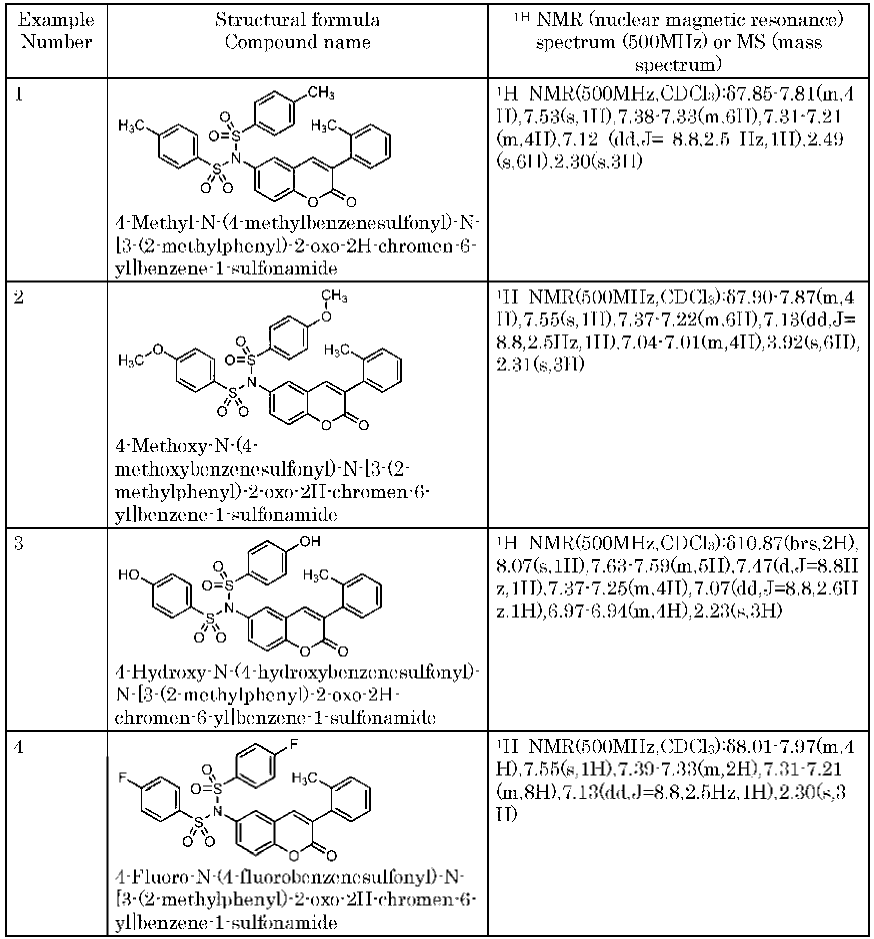

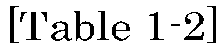

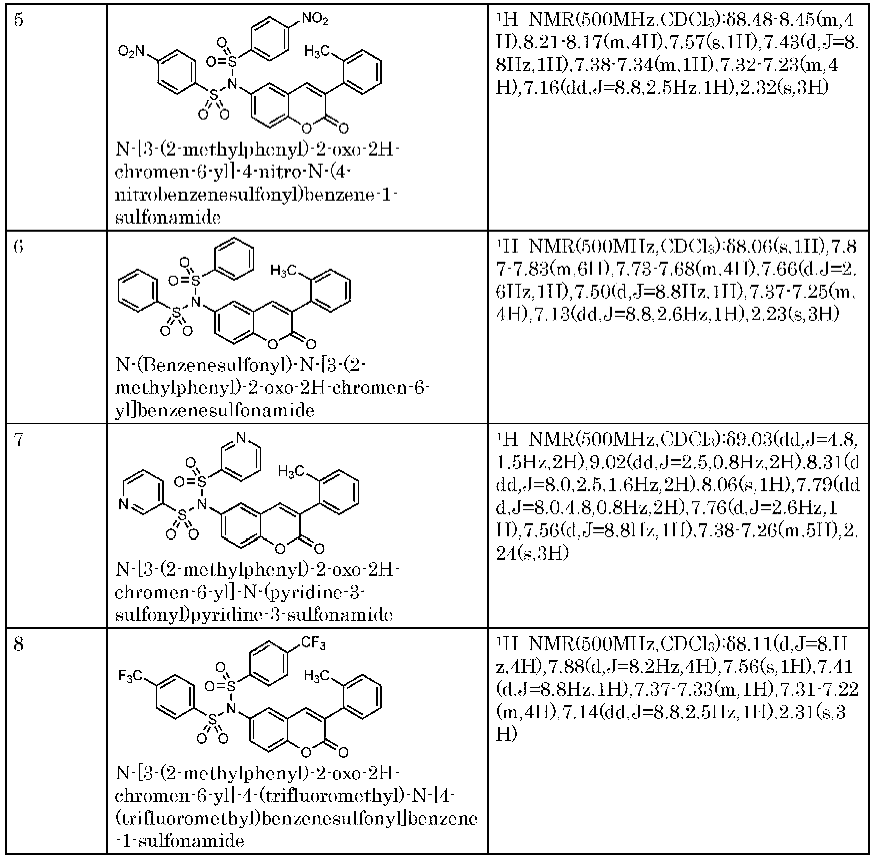

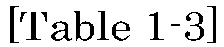

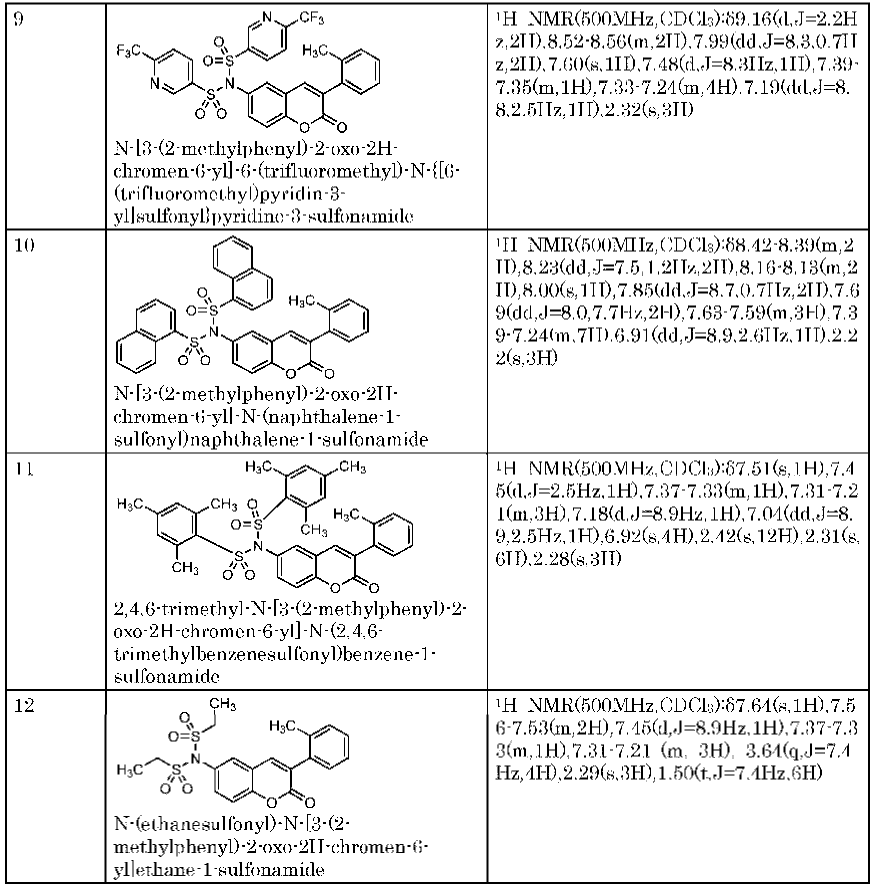

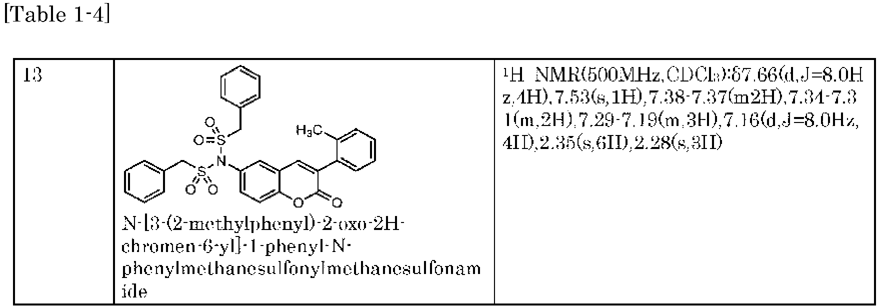

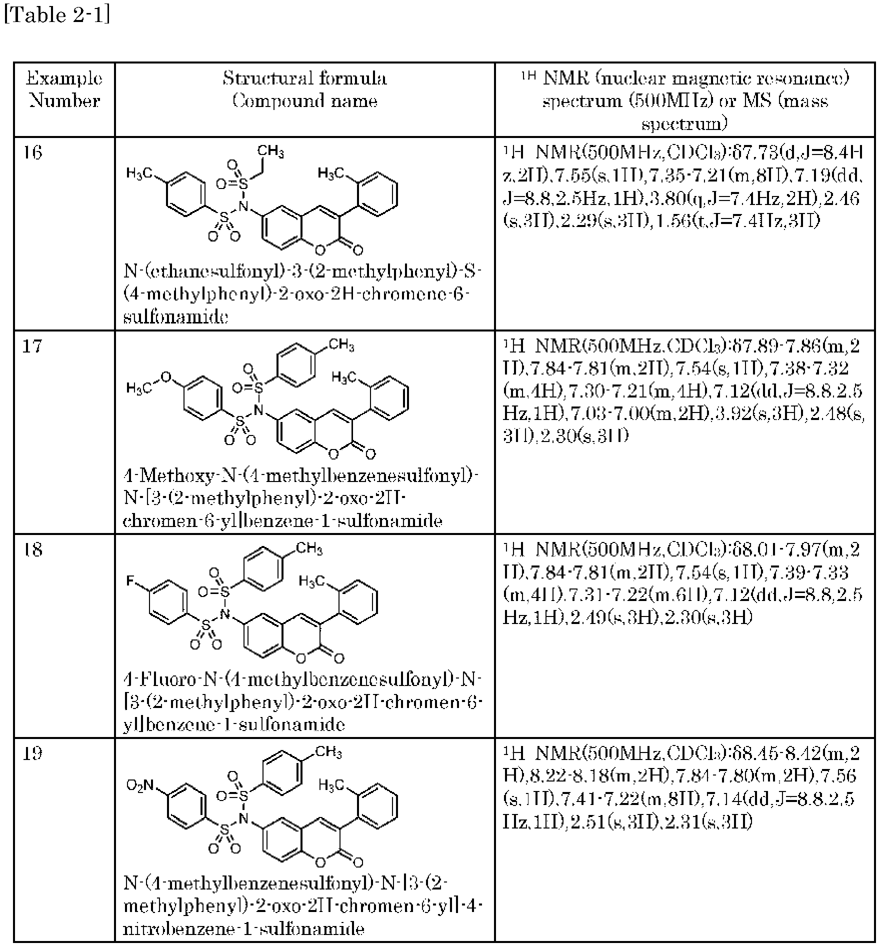

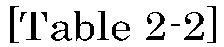

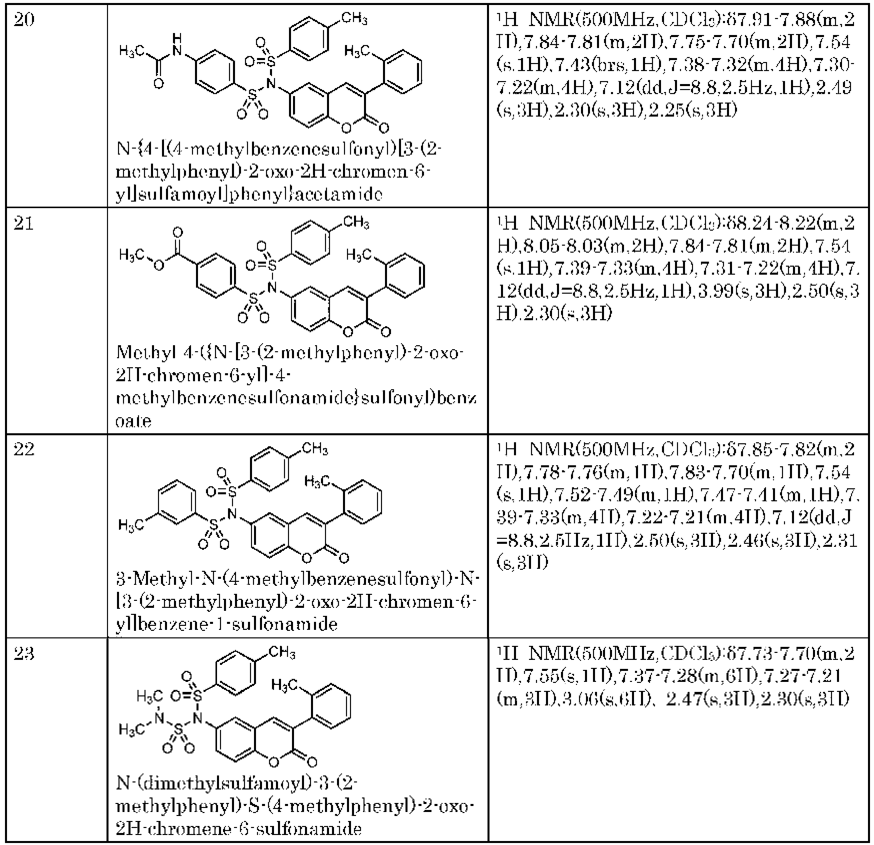

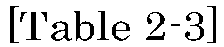

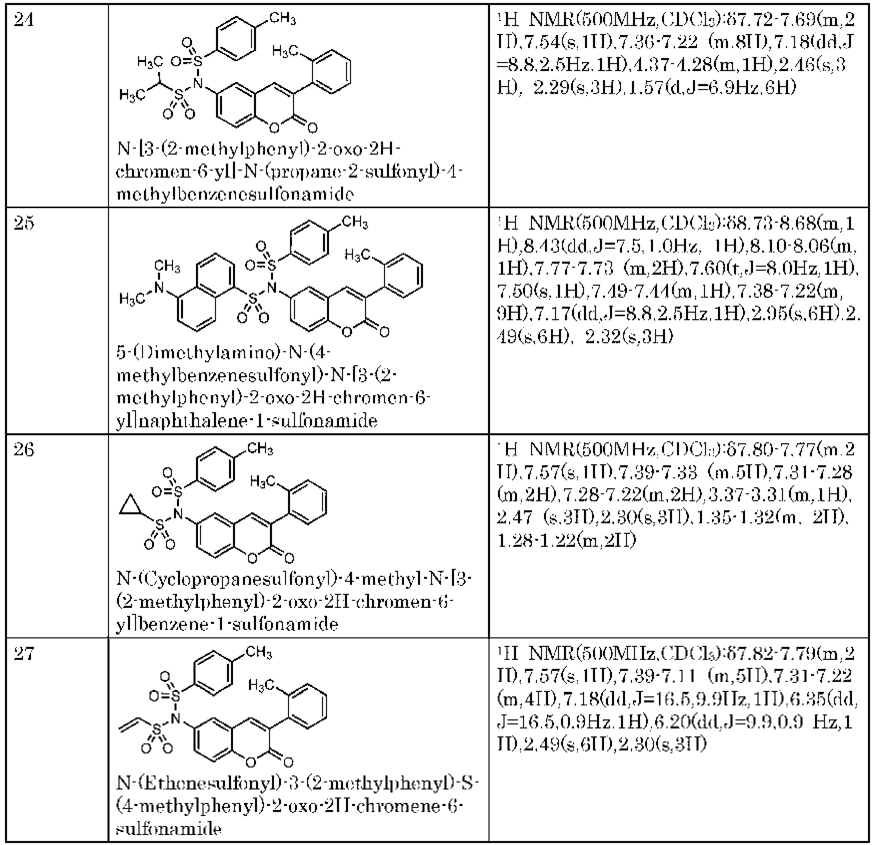

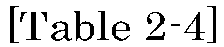

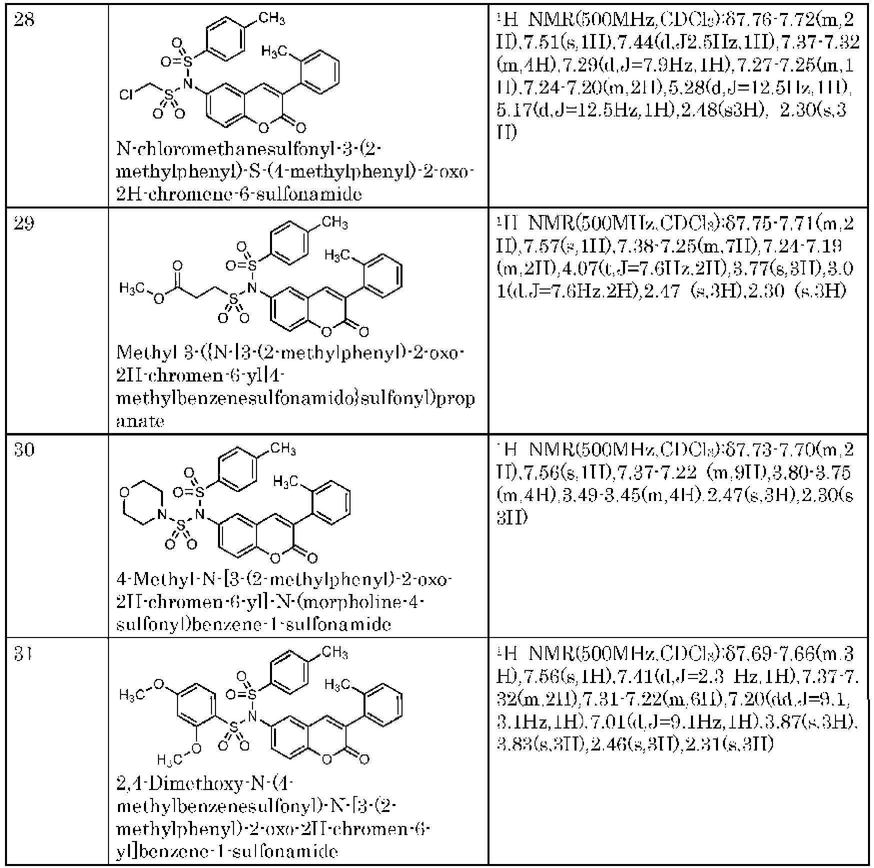

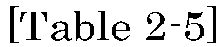

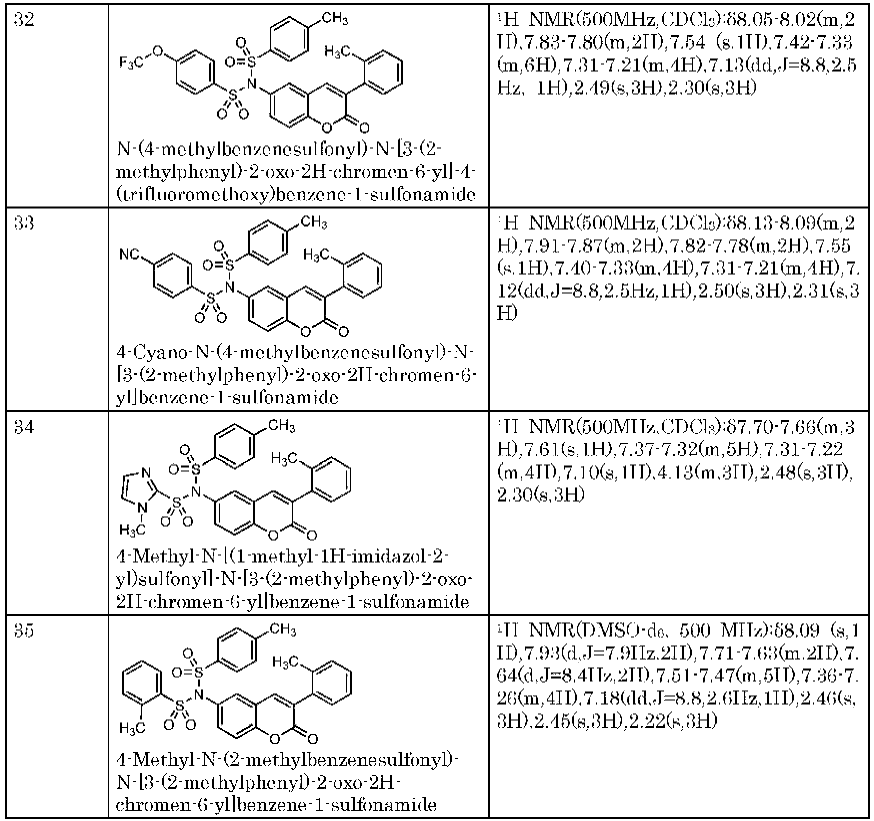

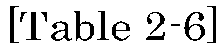

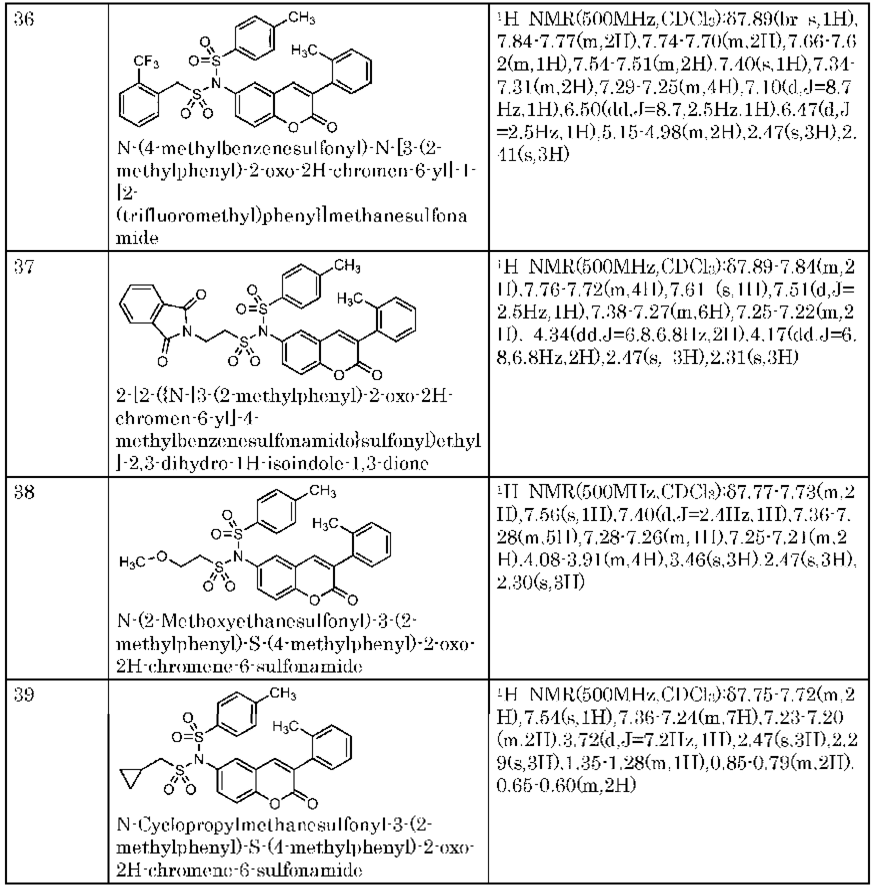

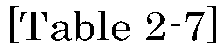

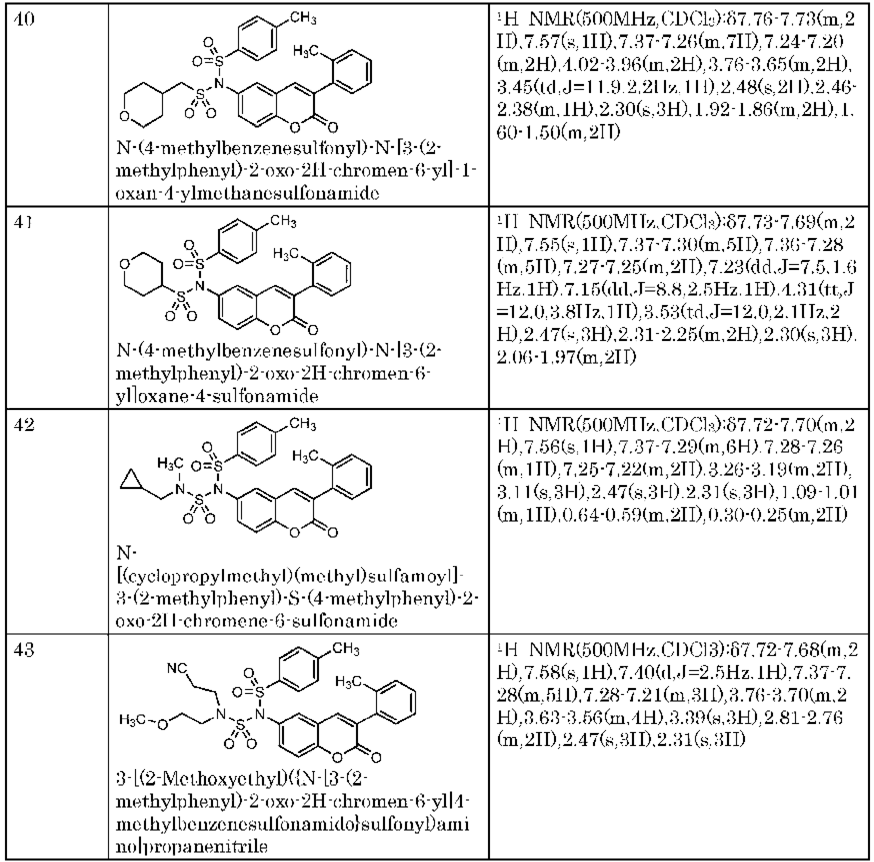

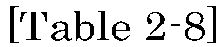

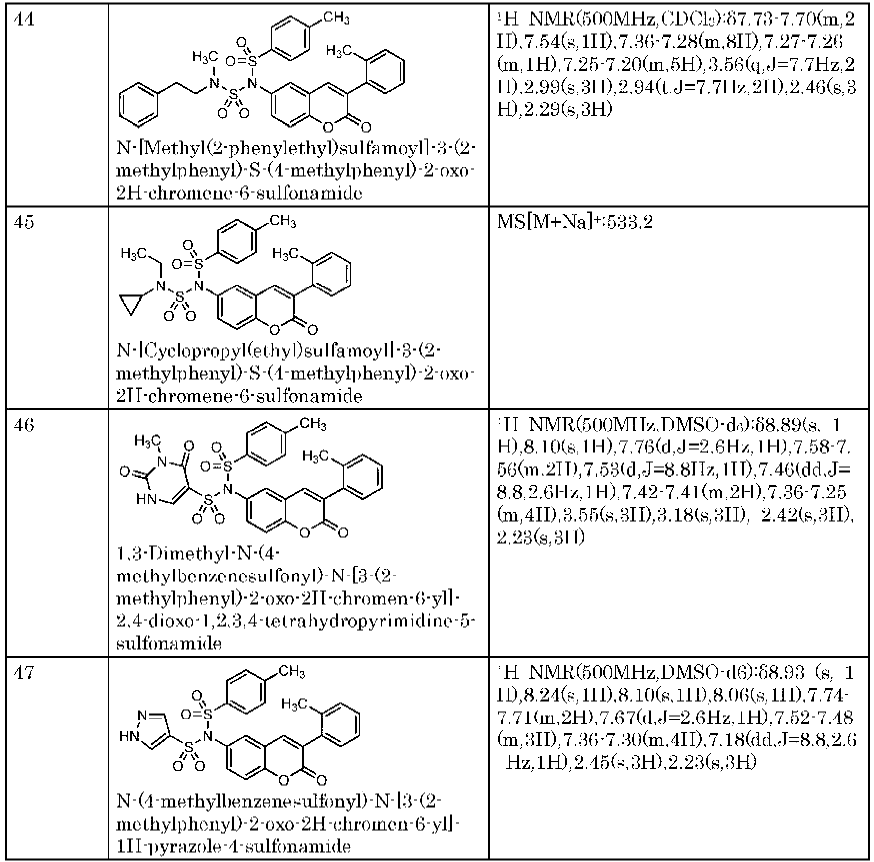

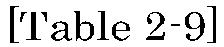

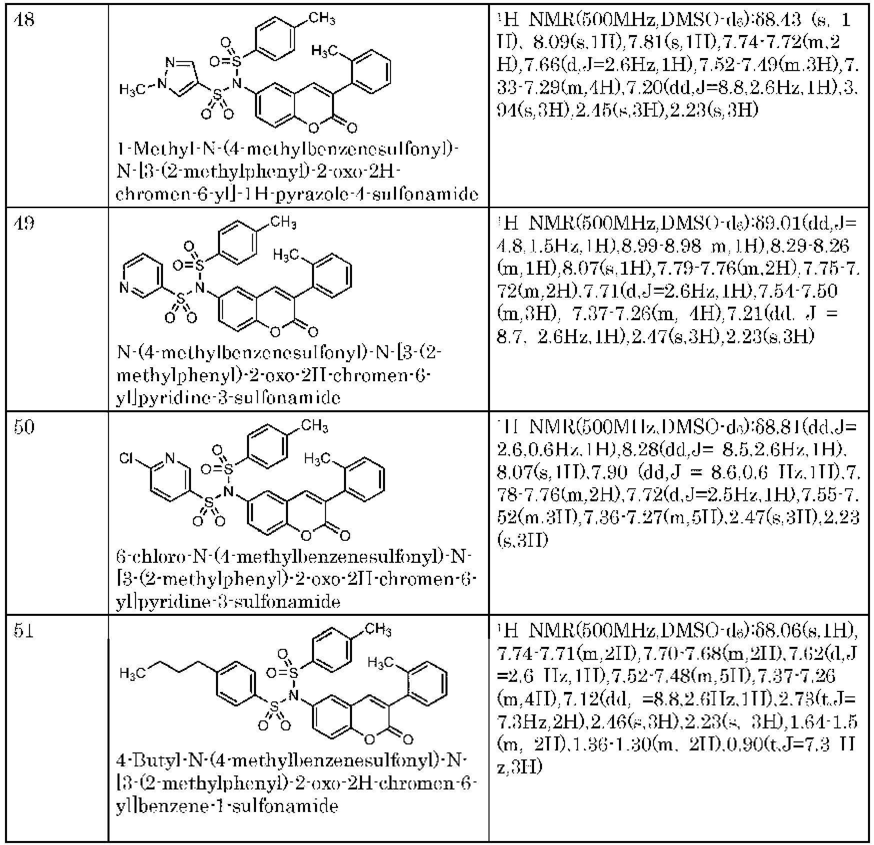

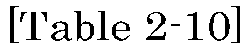

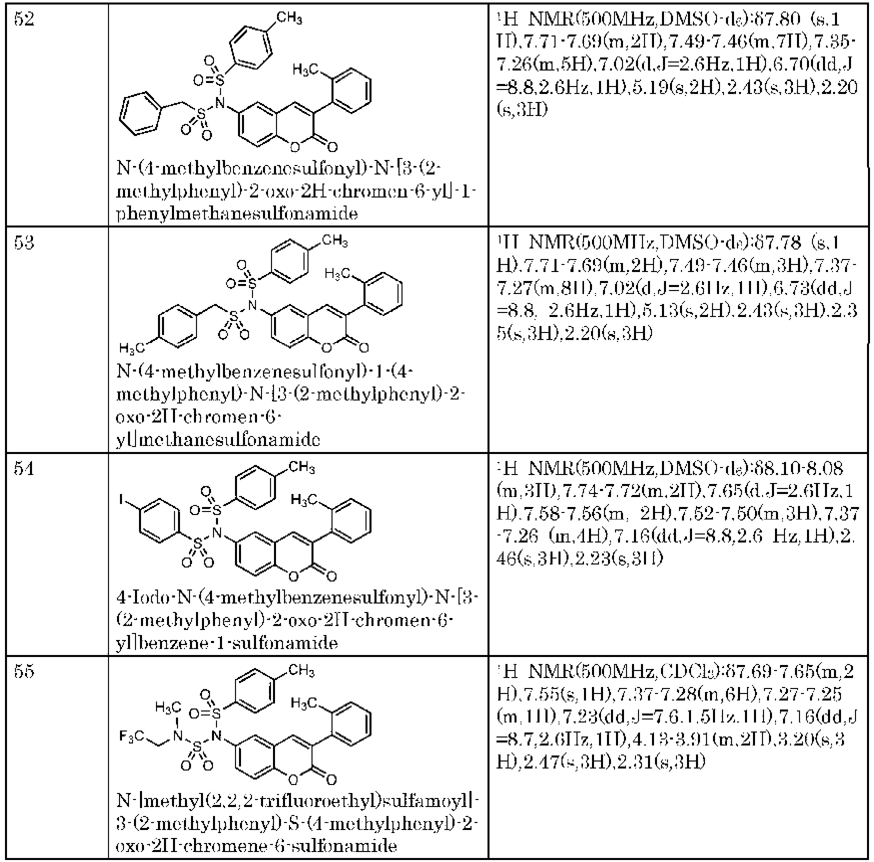

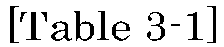

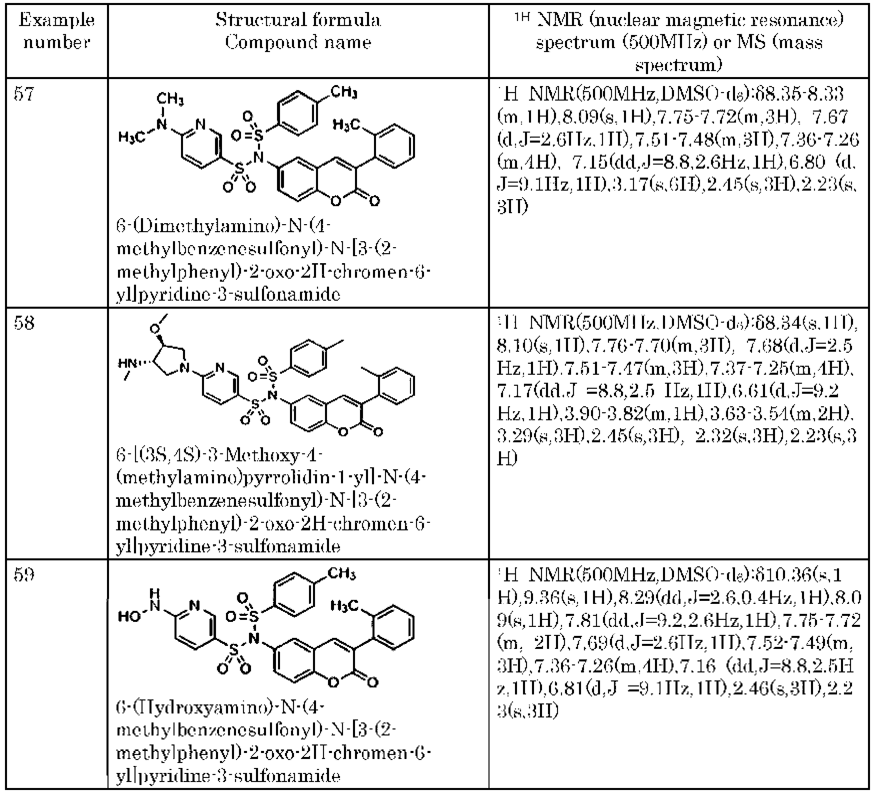

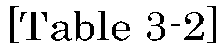

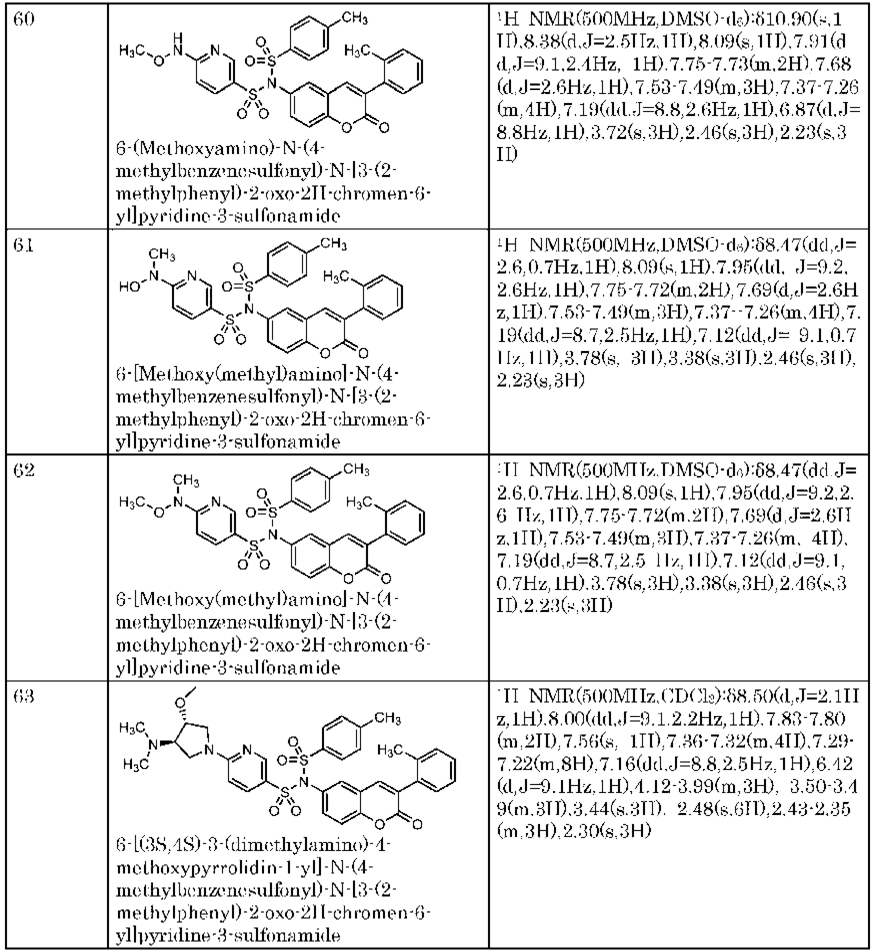

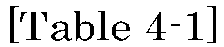

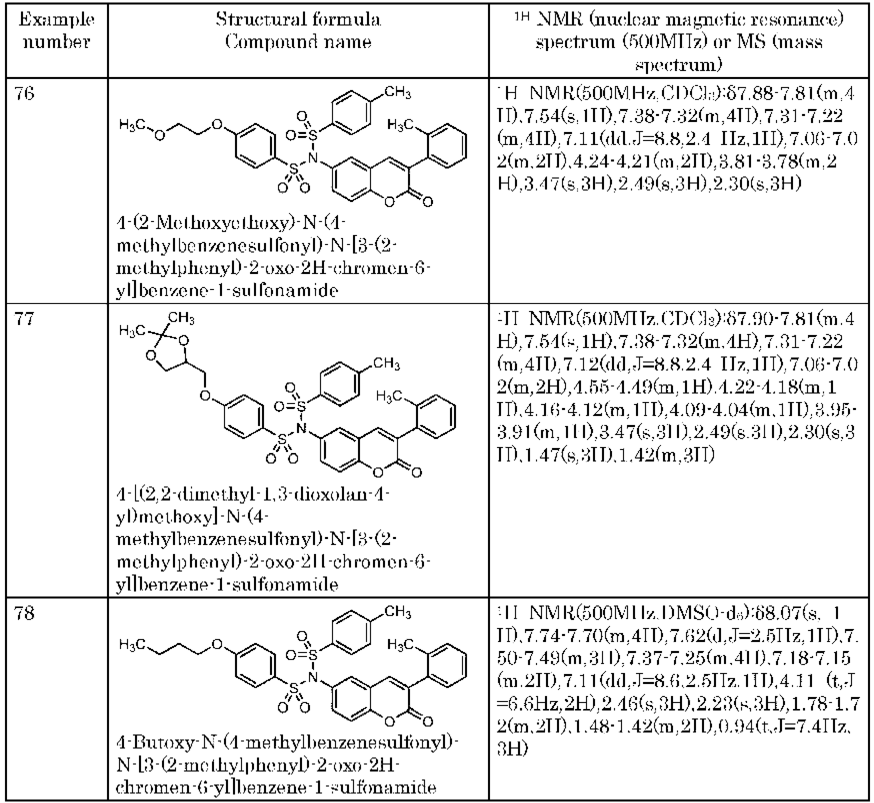

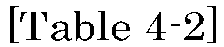

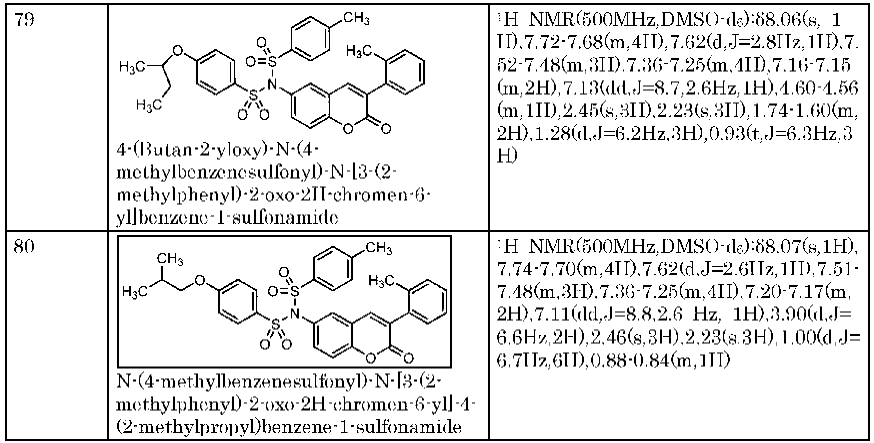

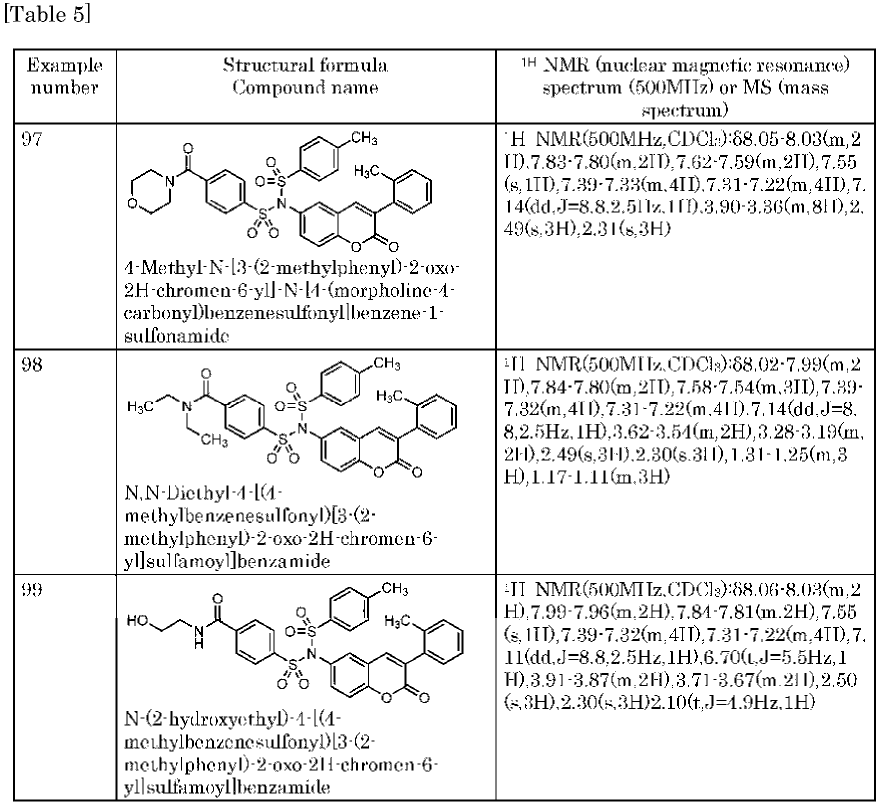

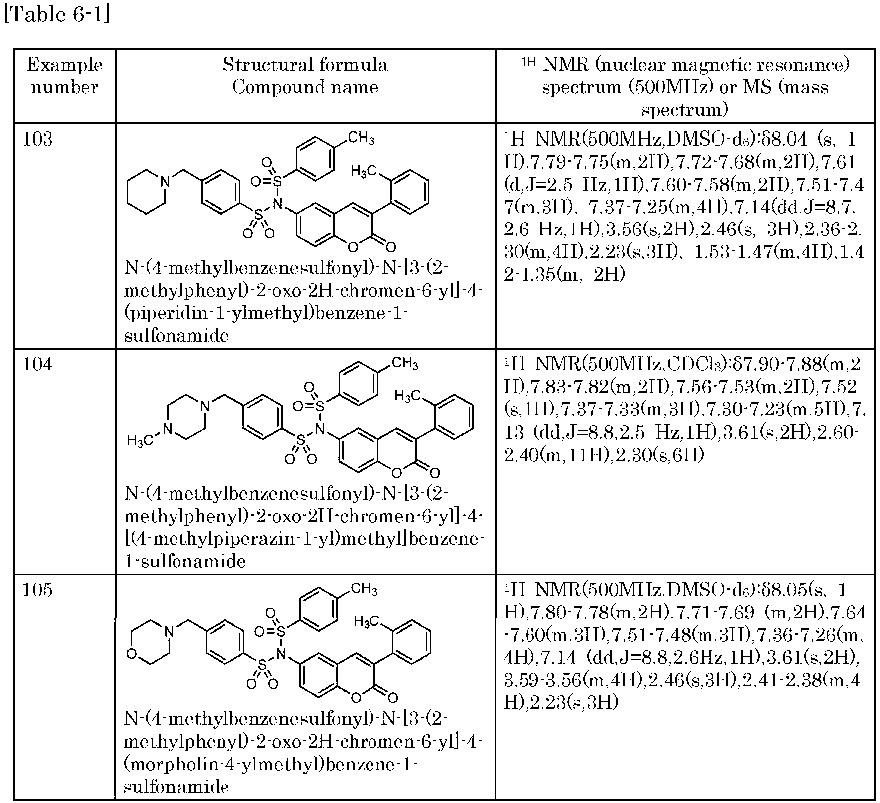

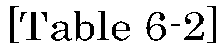

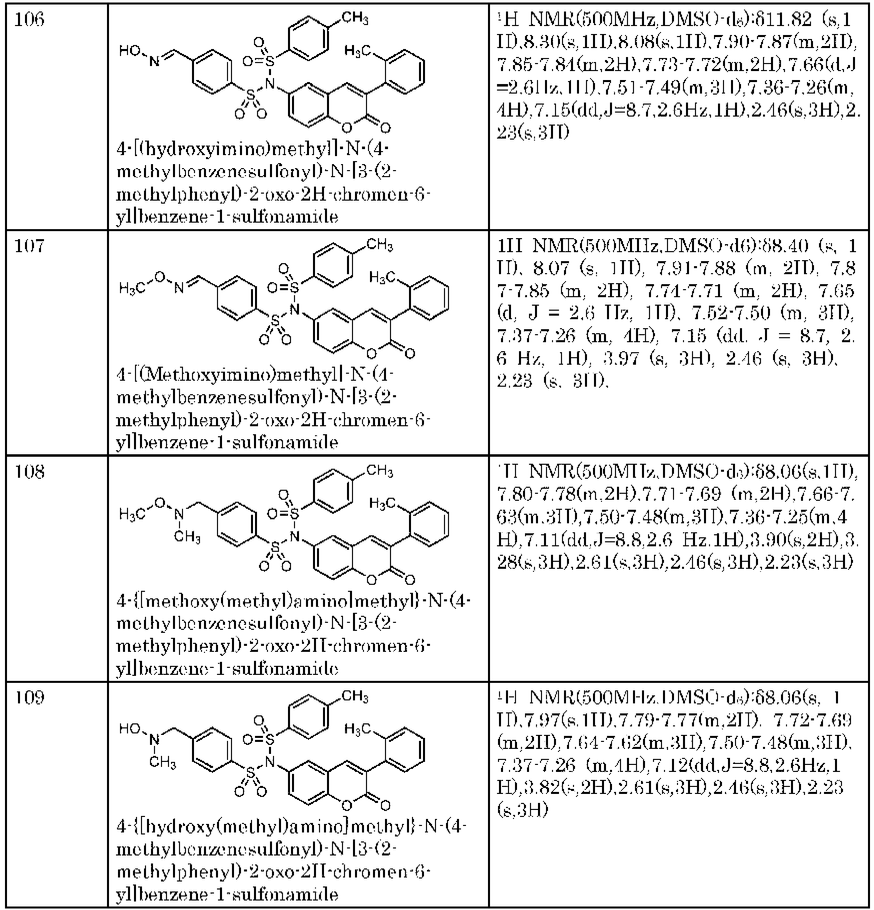

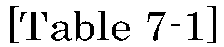

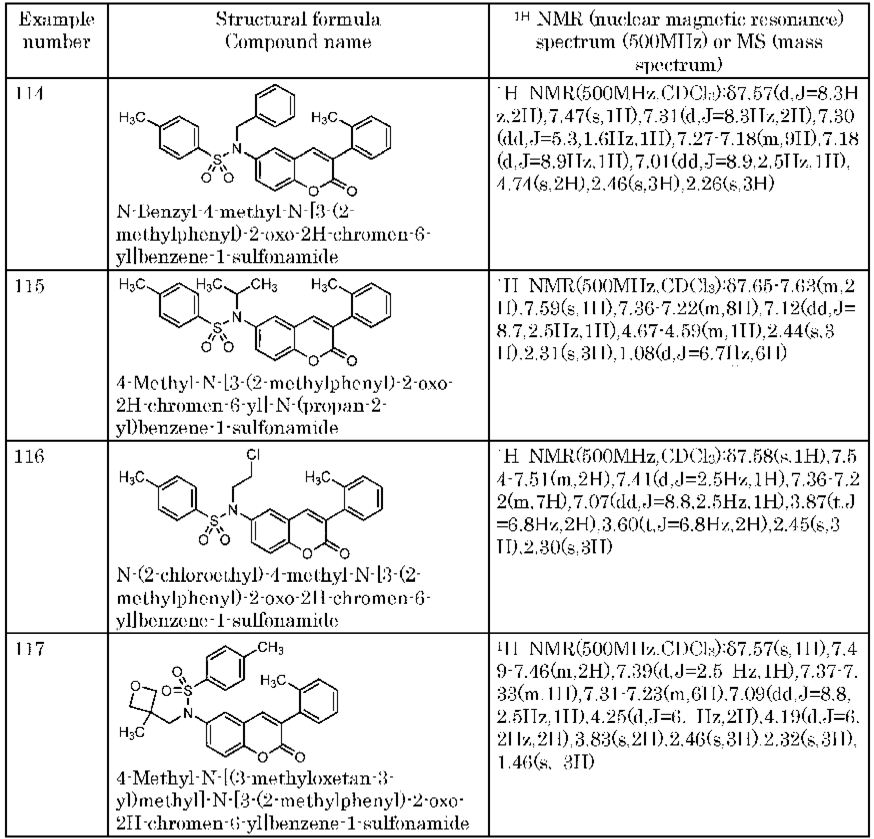

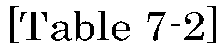

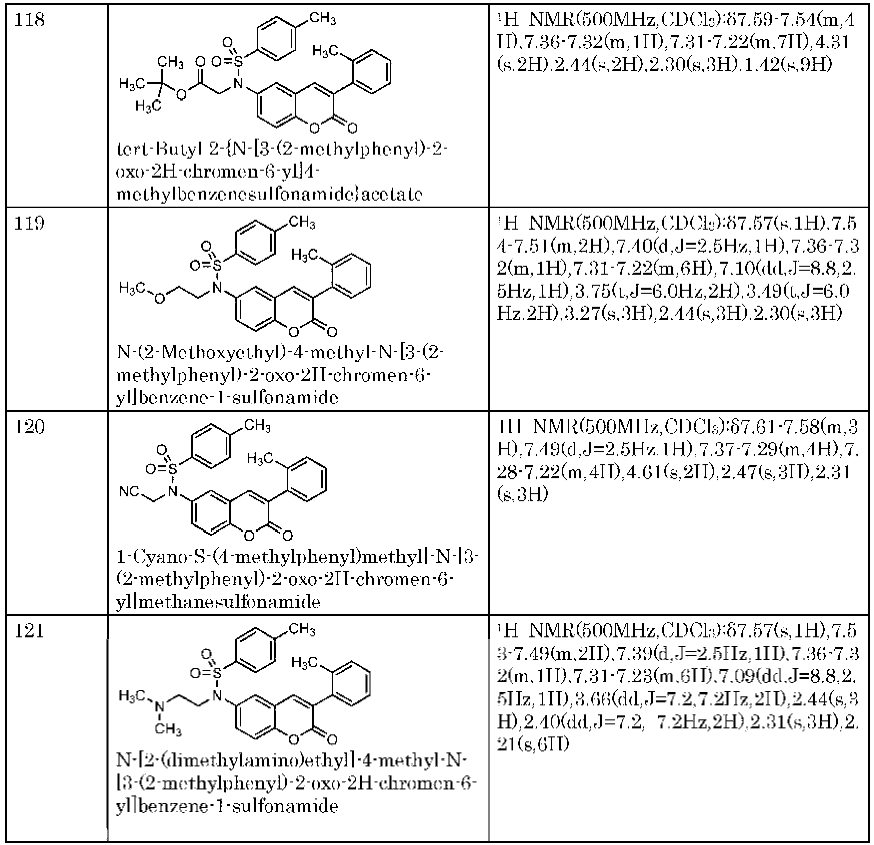

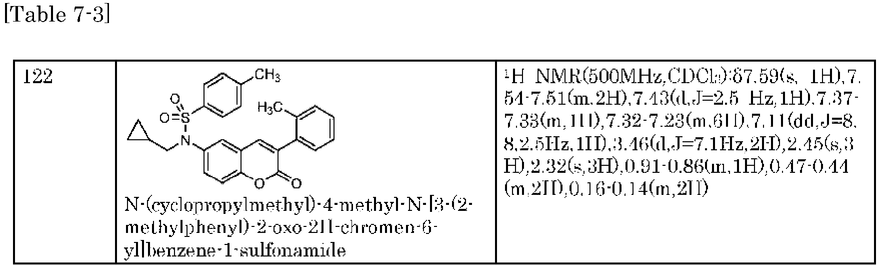

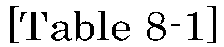

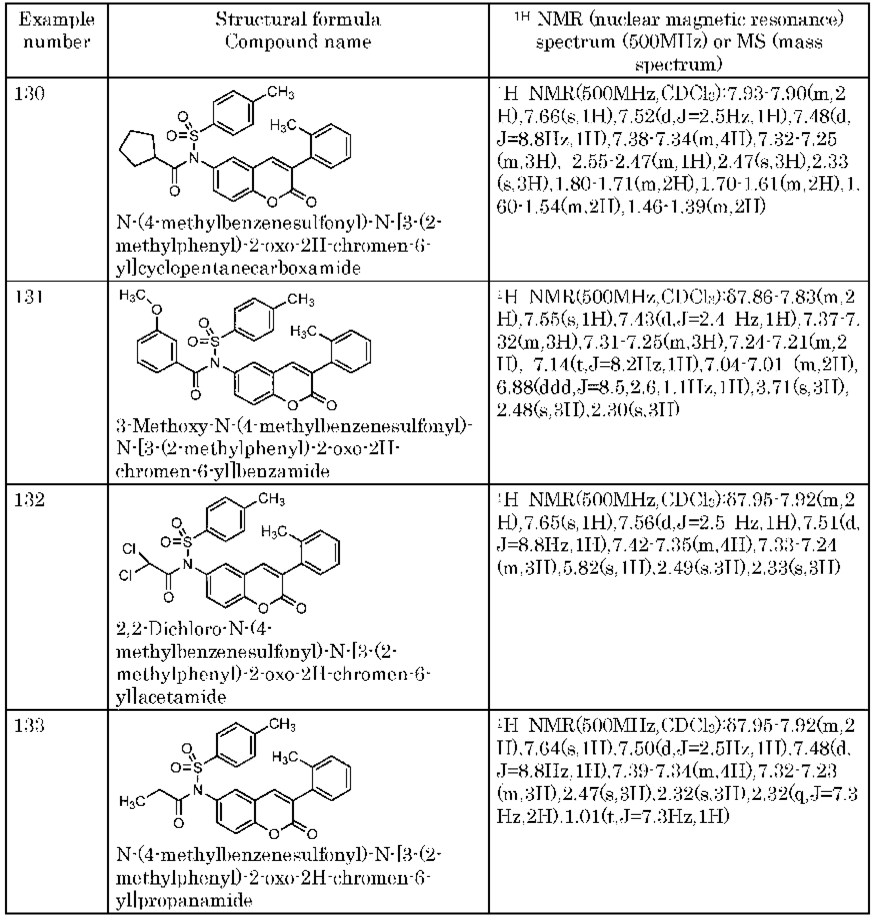

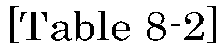

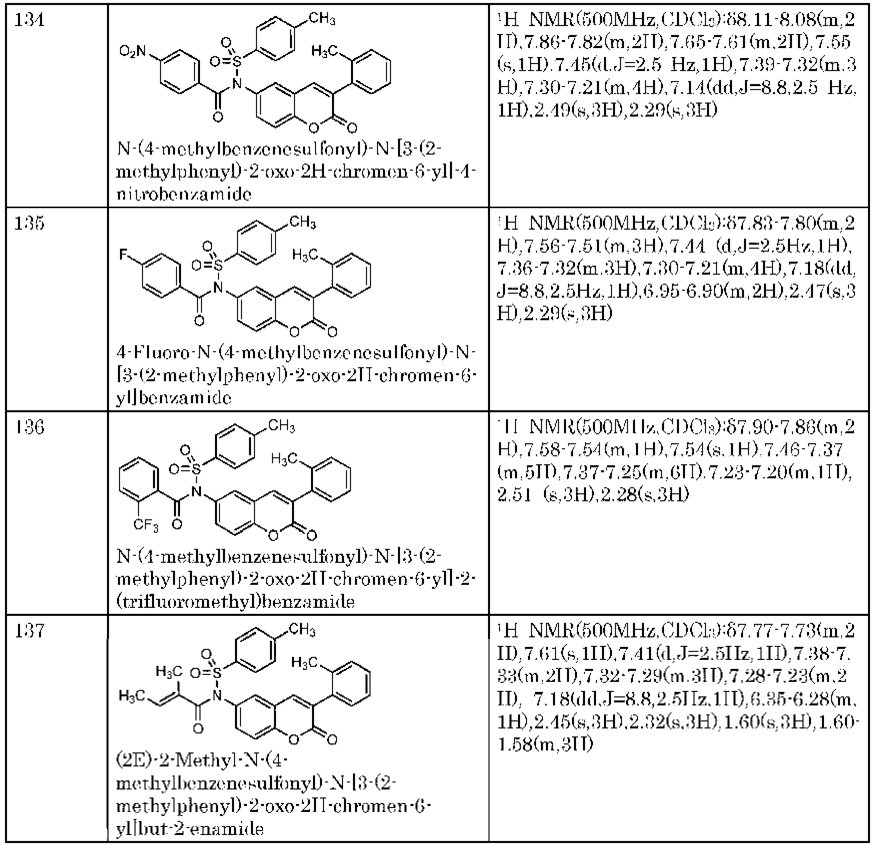

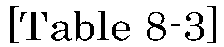

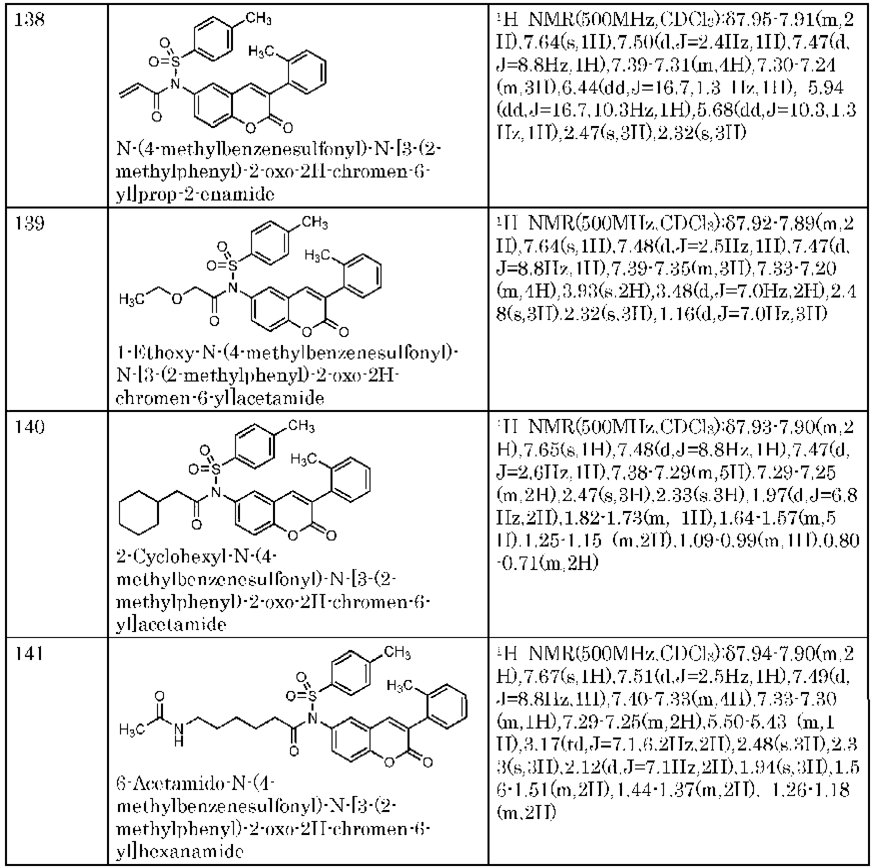

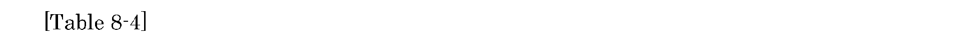

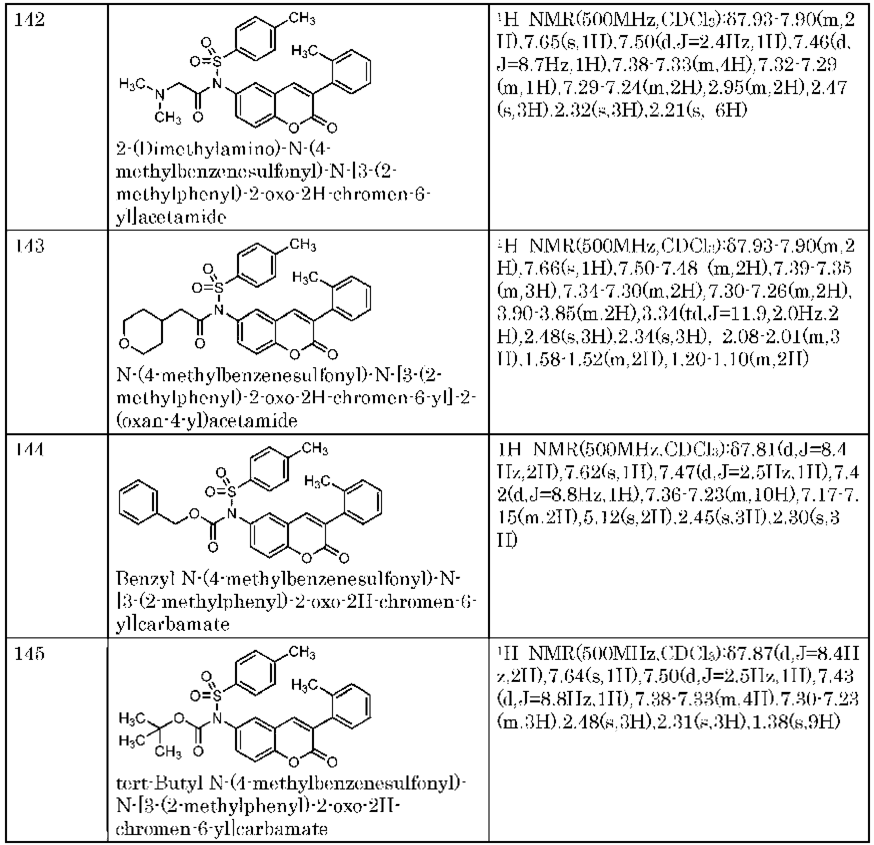

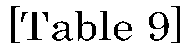

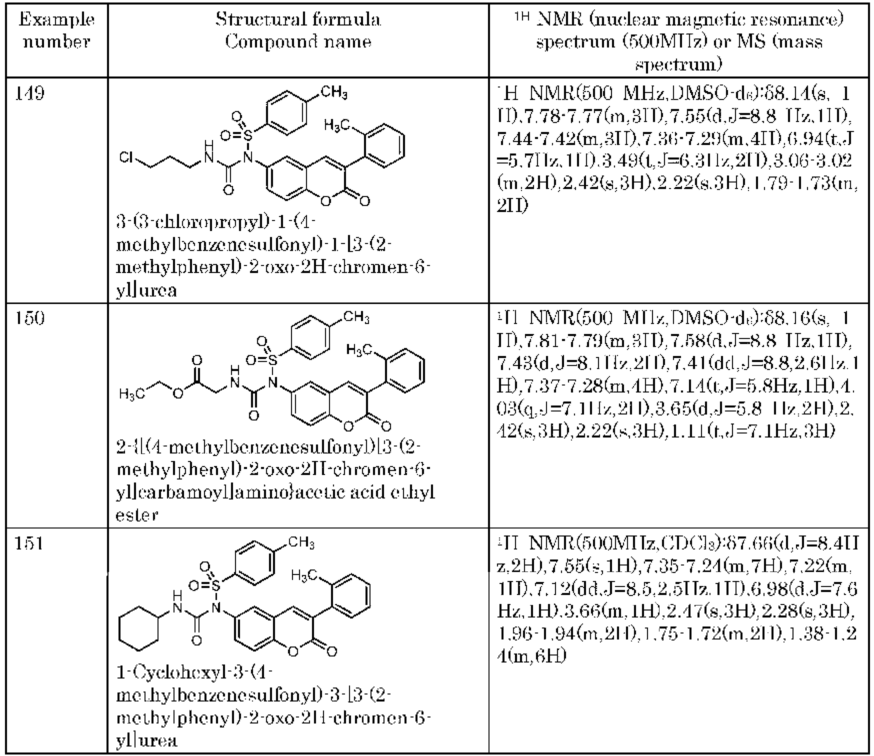

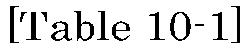

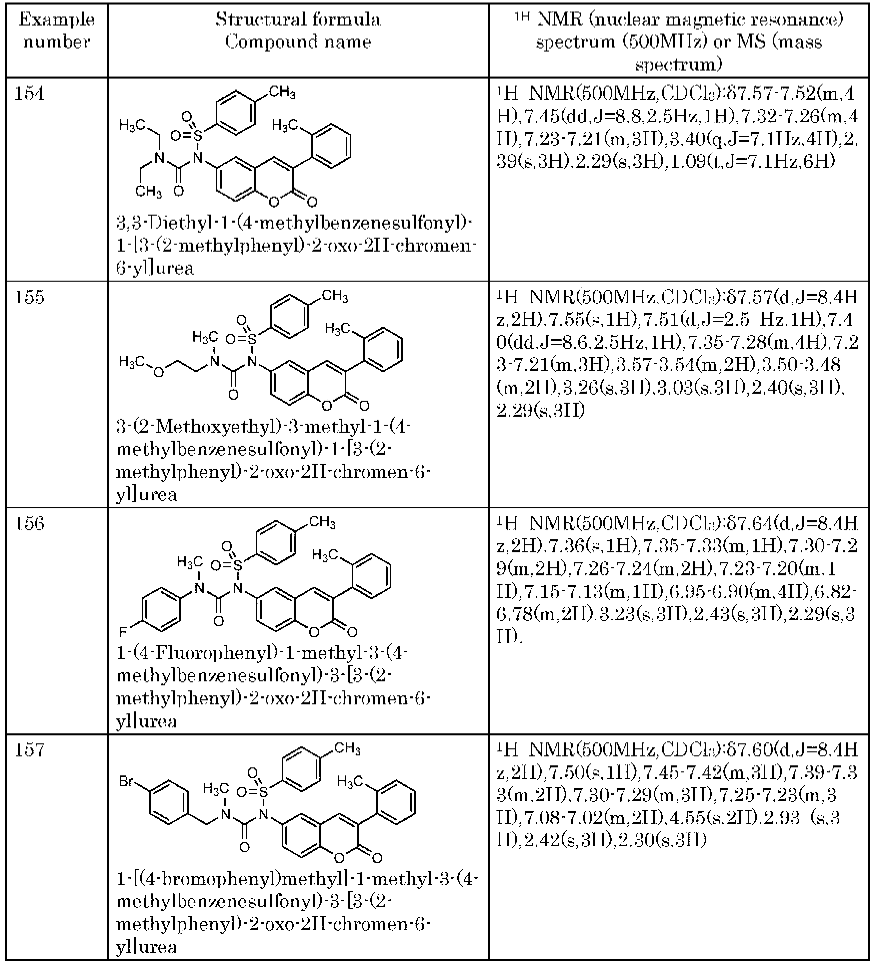

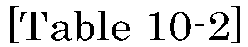

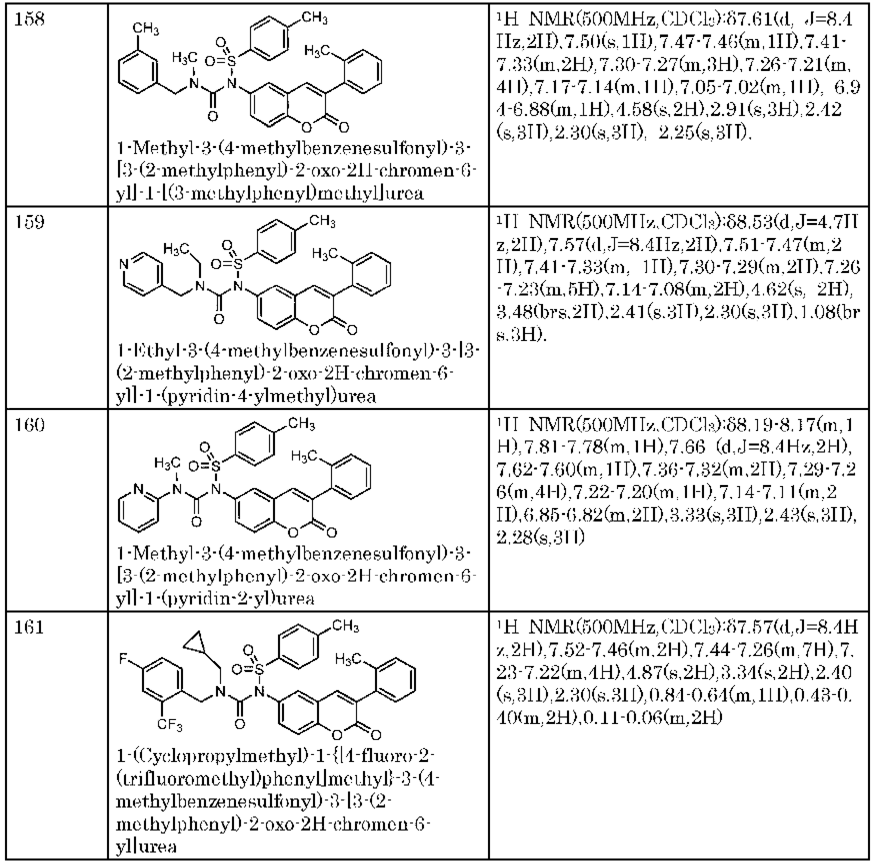

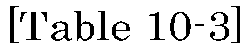

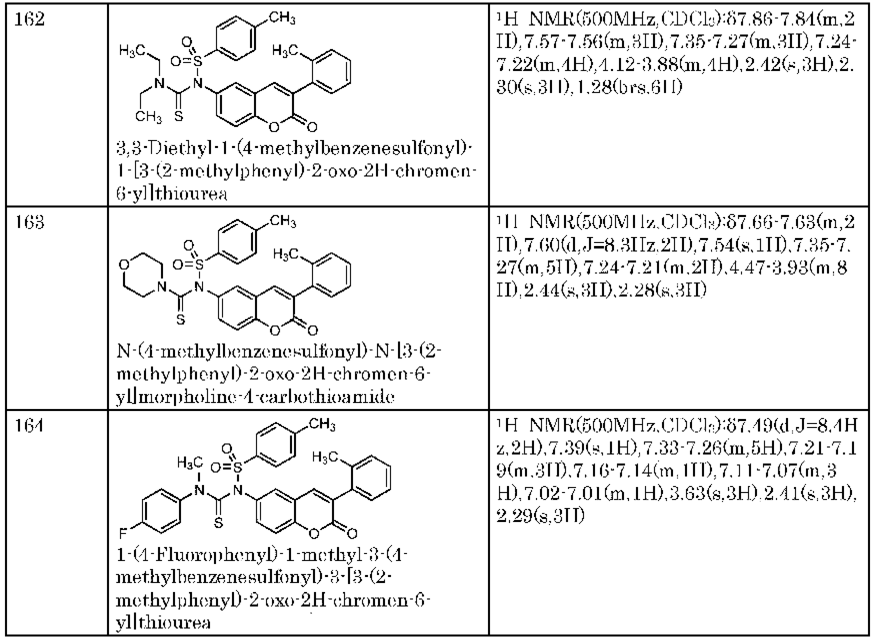

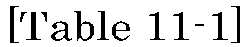

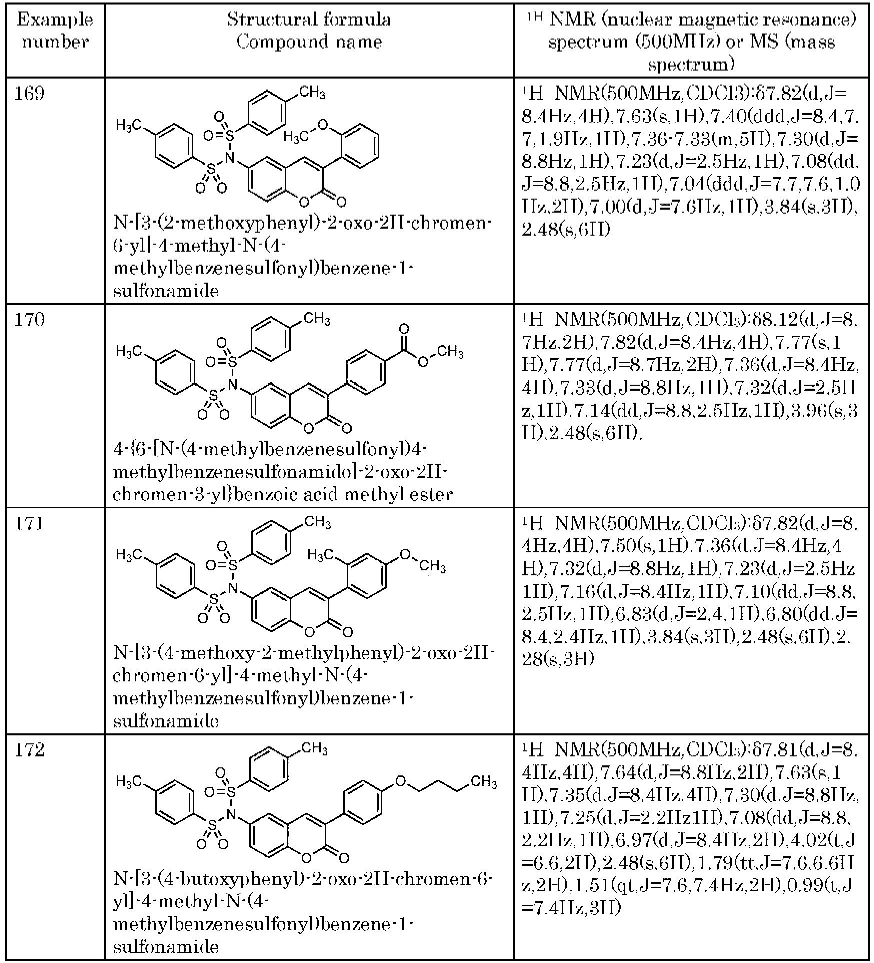

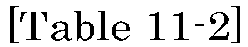

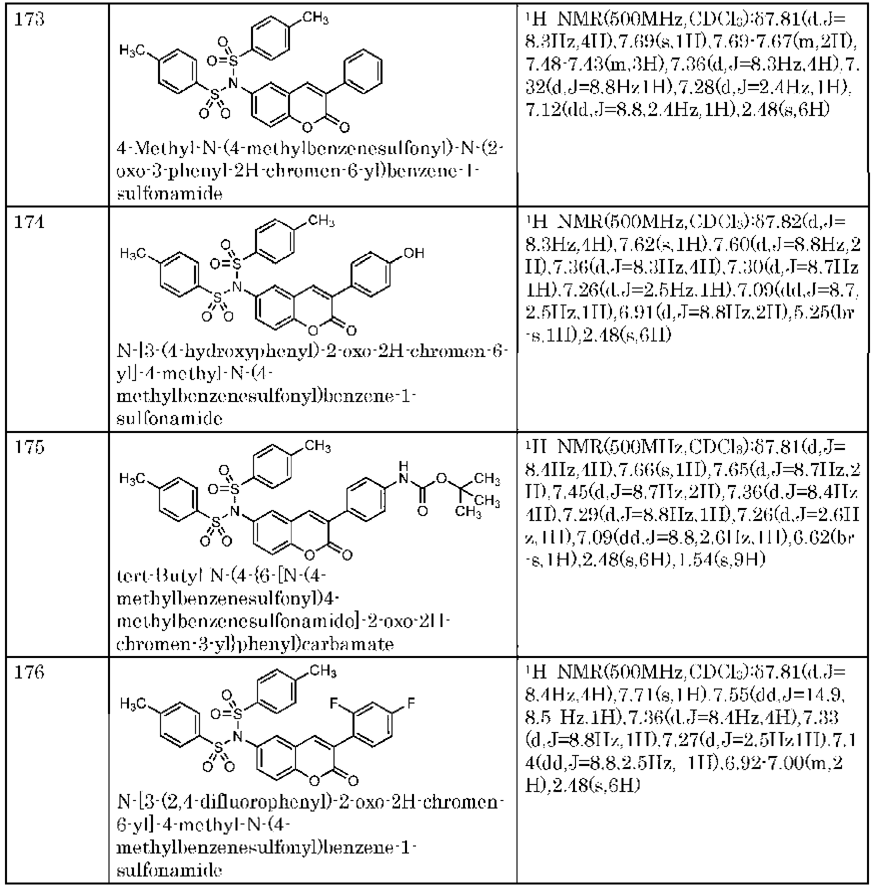

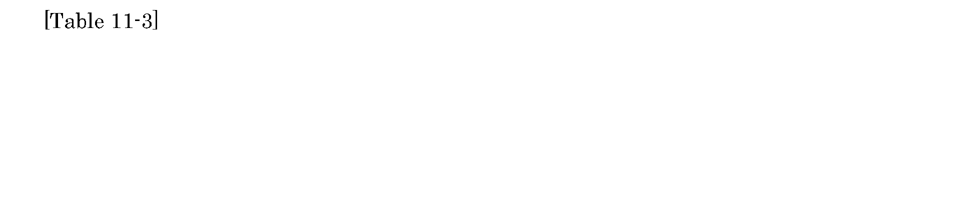

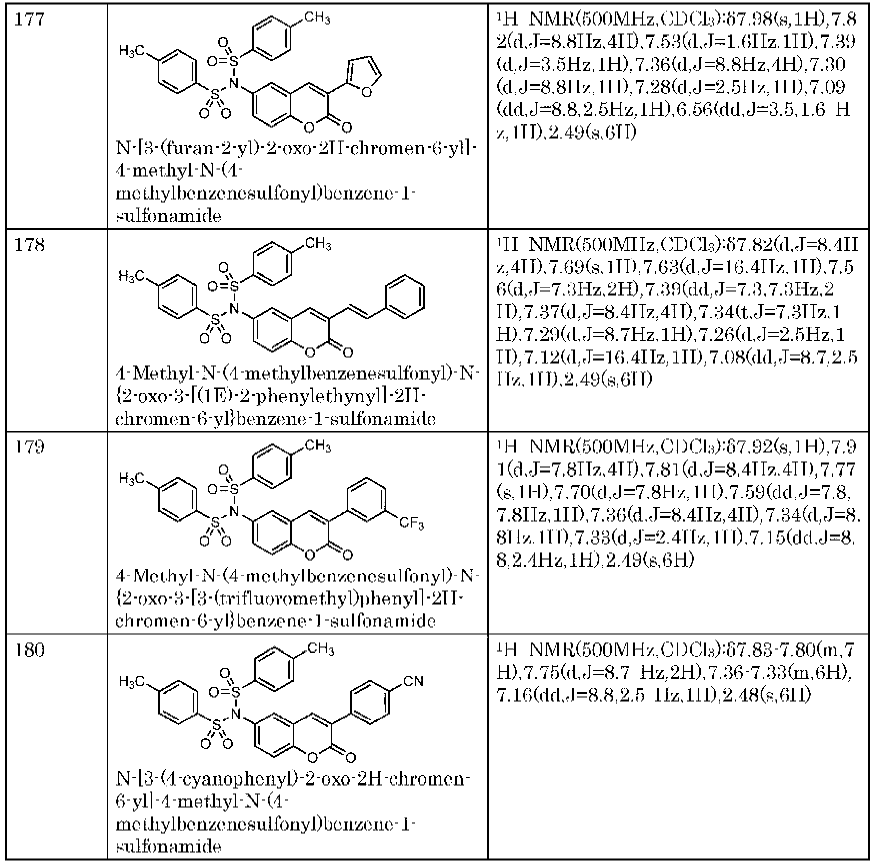

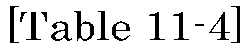

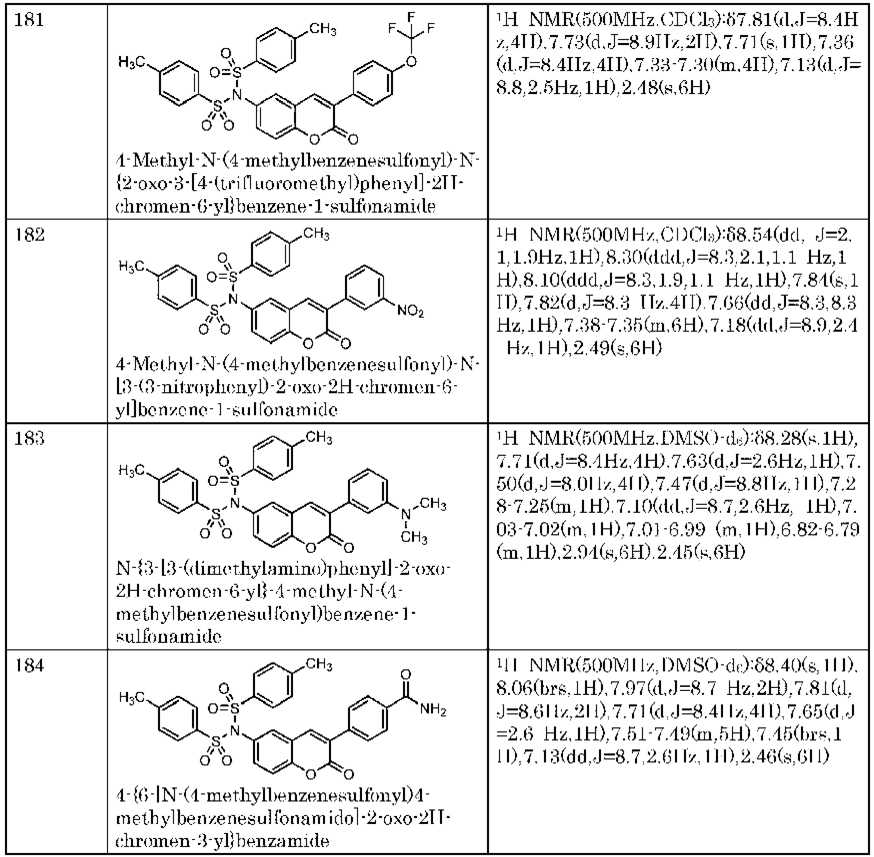

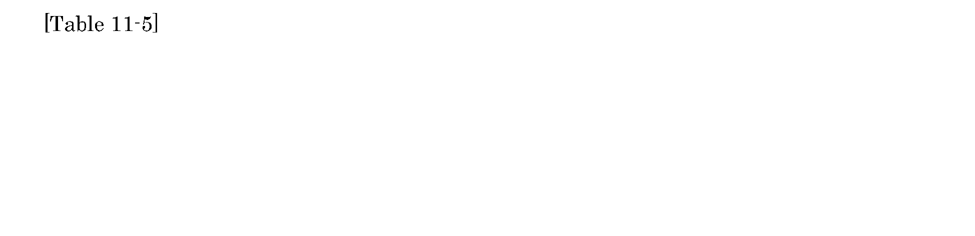

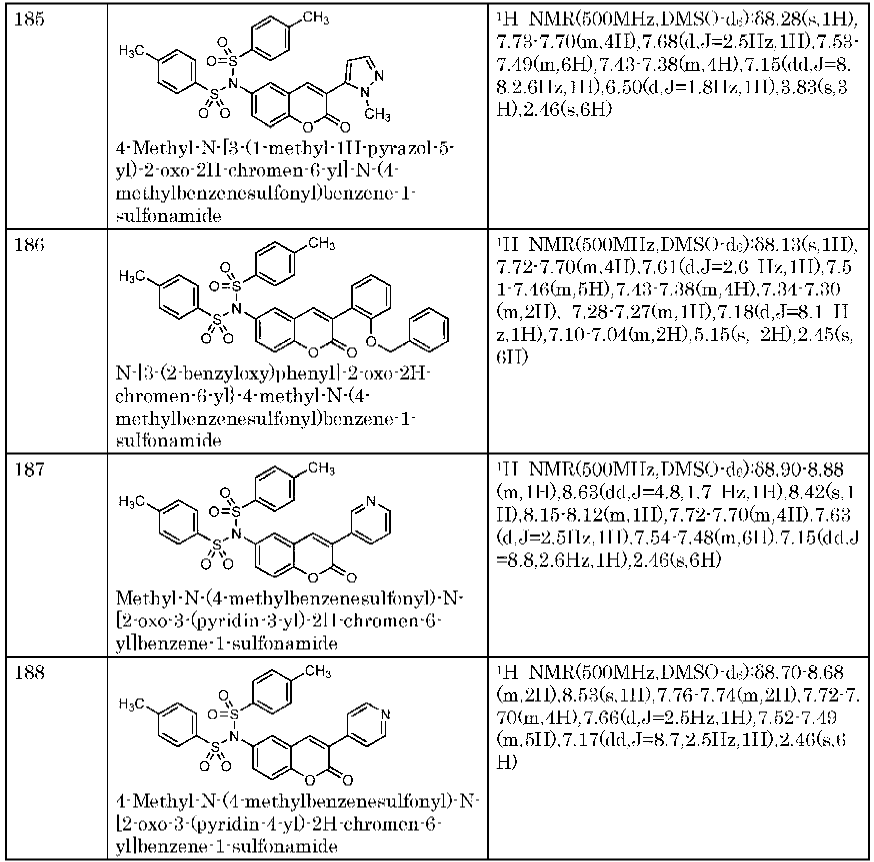

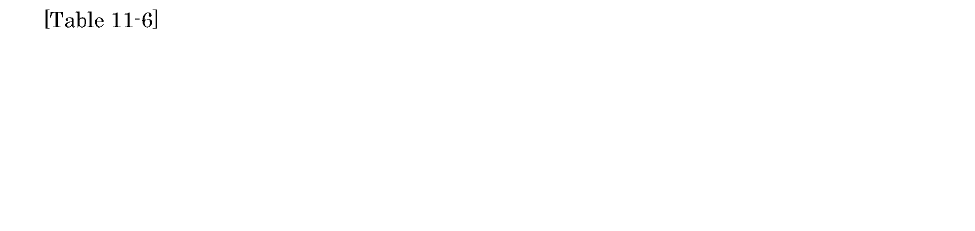

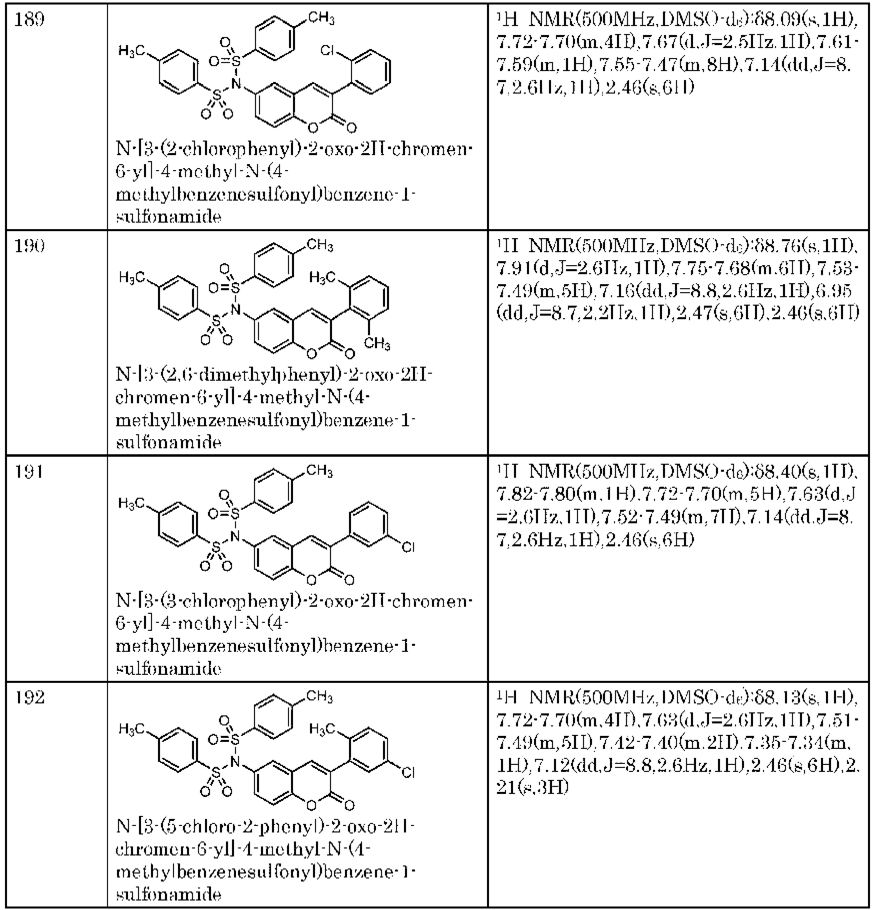

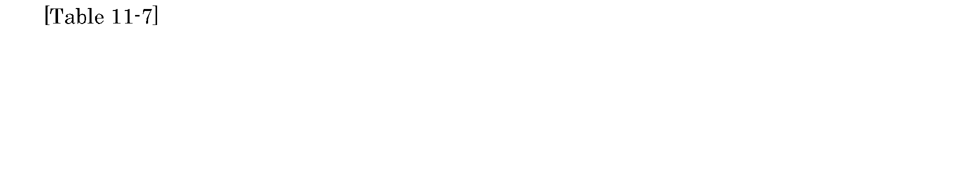

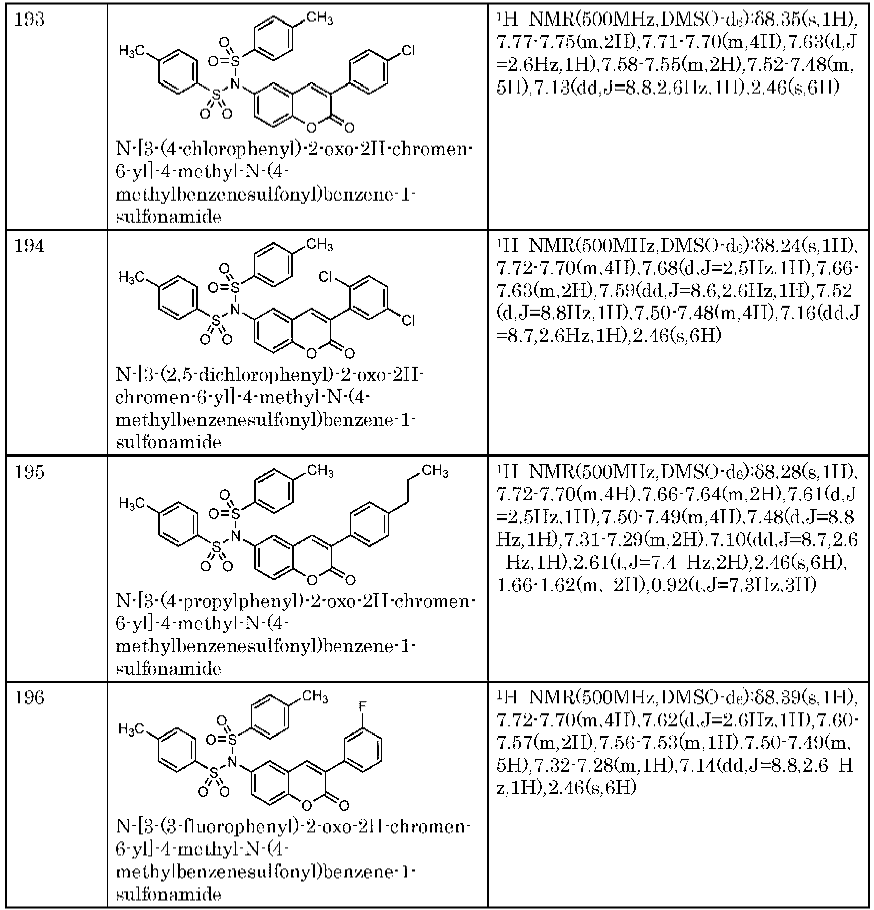

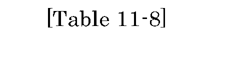

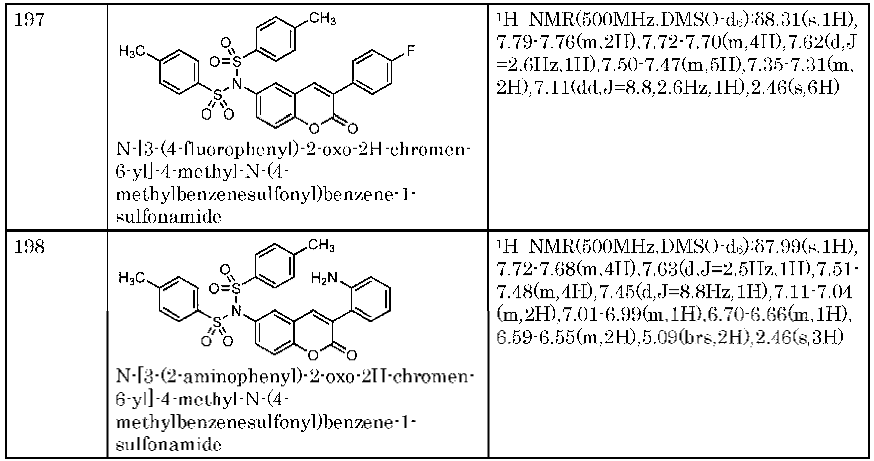

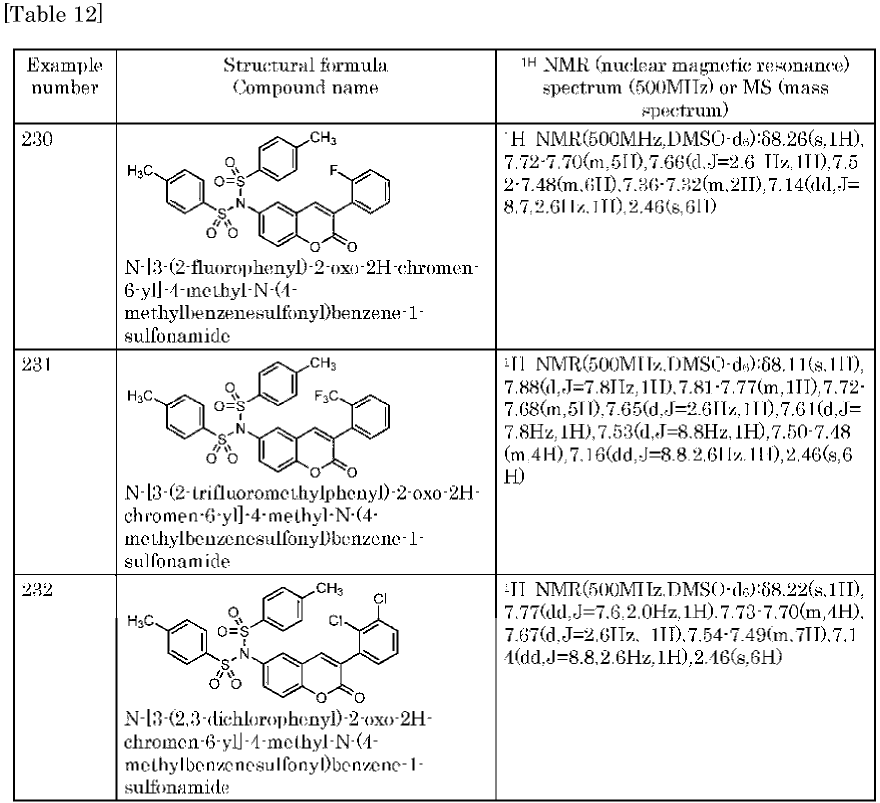

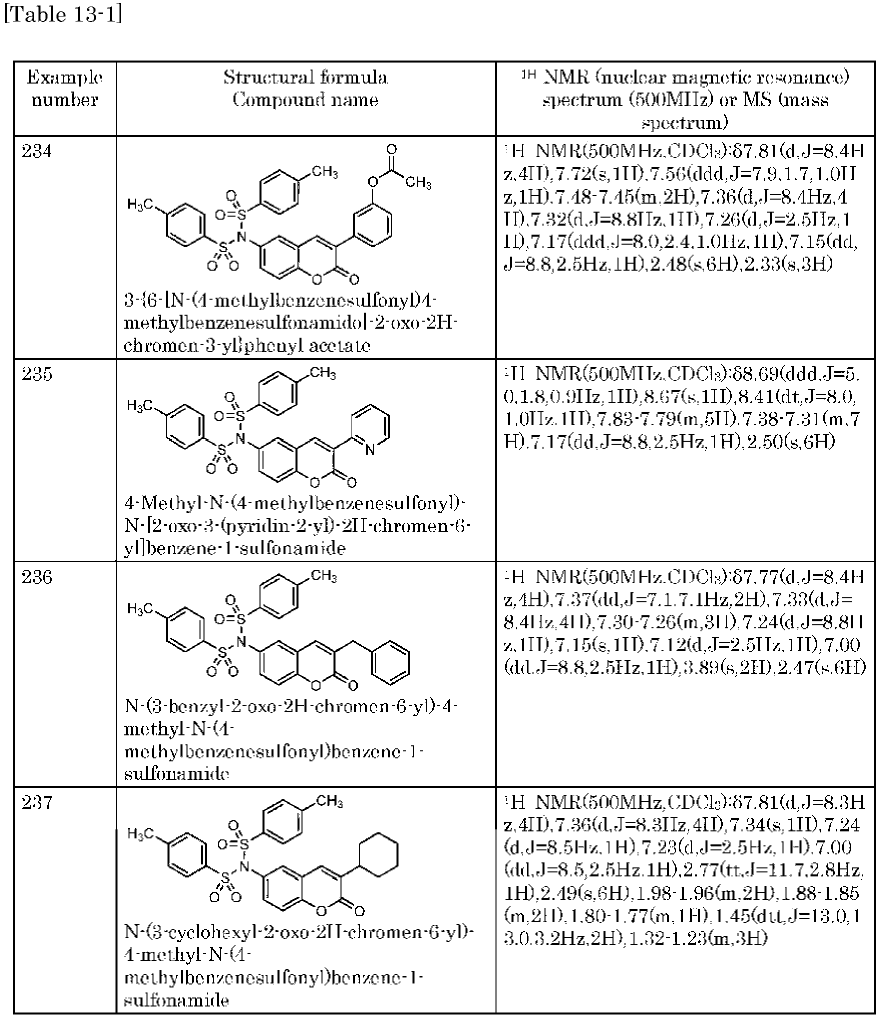

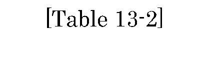

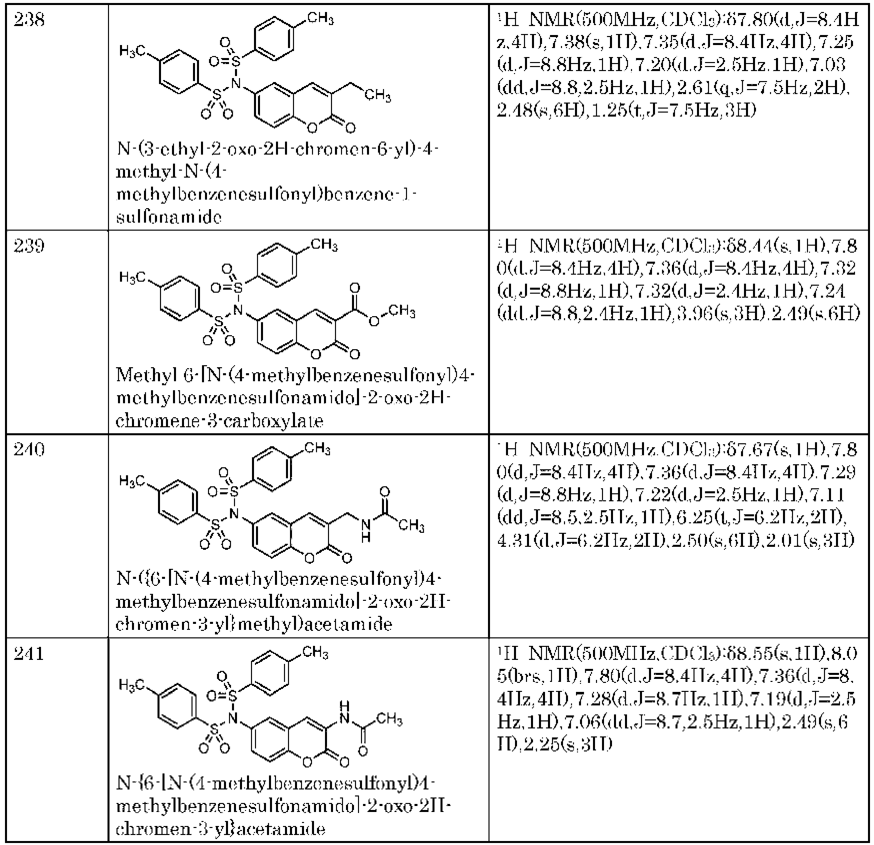

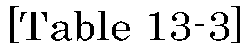

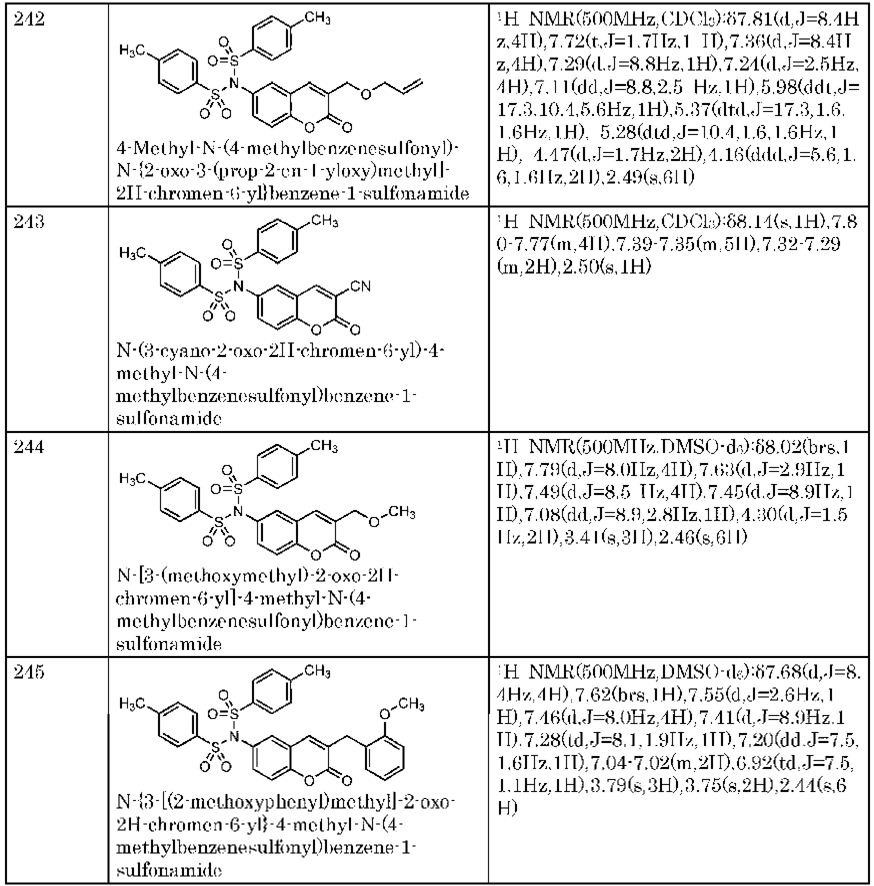

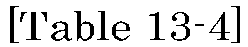

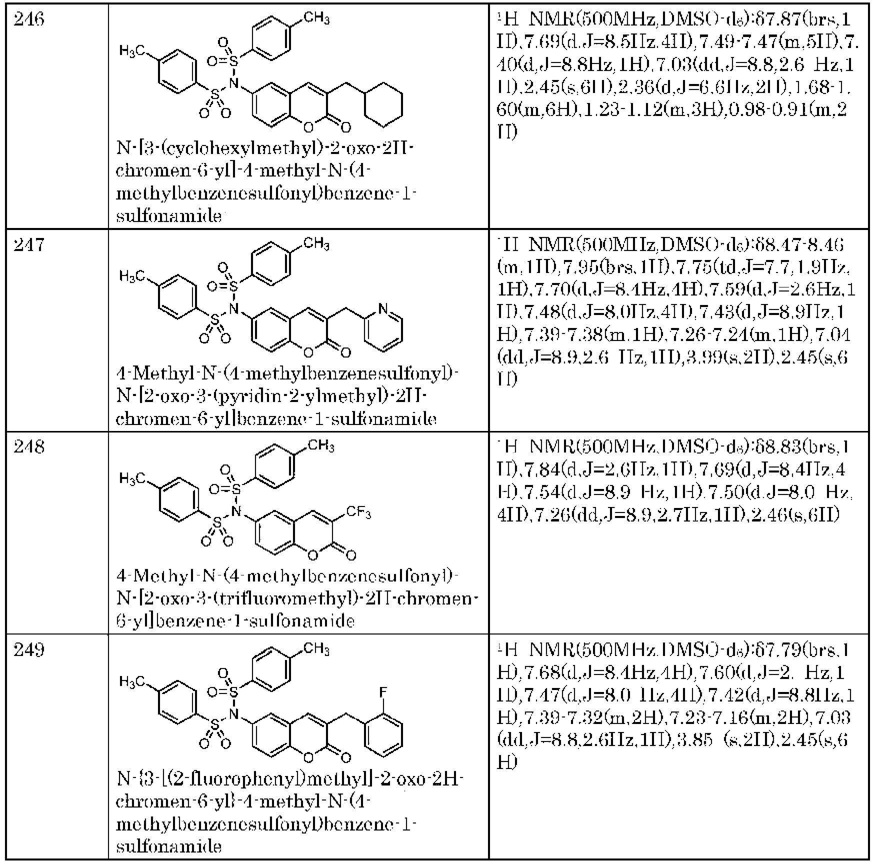

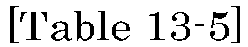

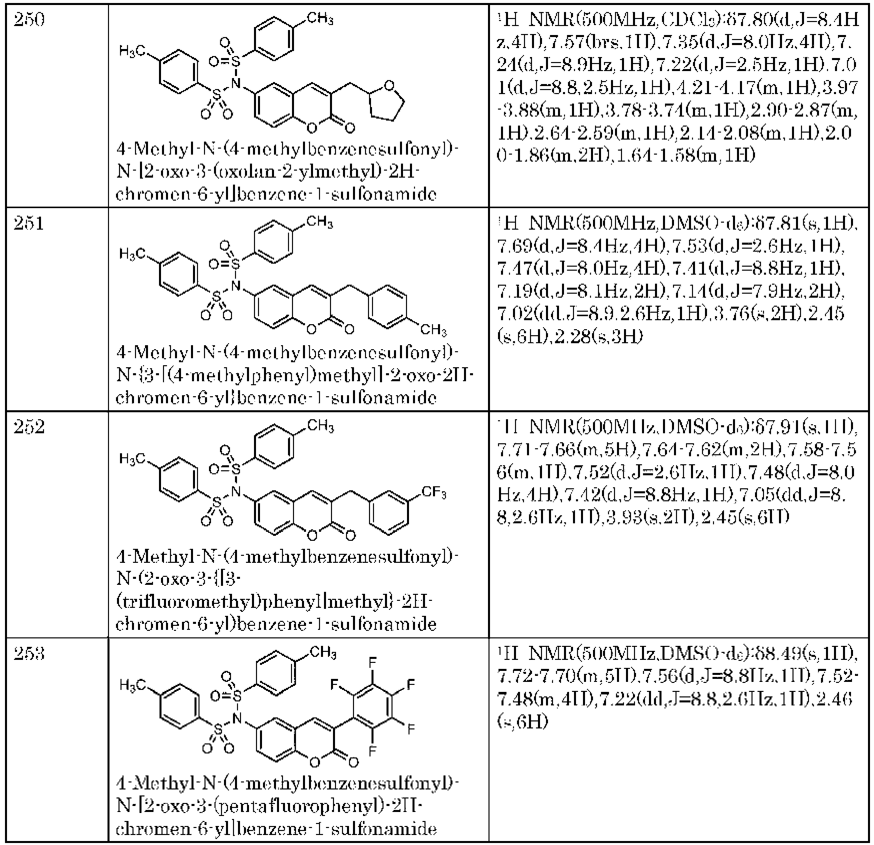

### Test example 1: Smad2/3 nuclear localization inhibitory effect

TWNT-4 human hepatic stellate cell line was suspended in Dulbecco's modified Eagle's medium (DMEM) containing 10% fetal bovine serum (FBS), seeded onto a 96-well plate at a density of 1×10³ cells/well, and cultured at 37°C in the presence of 5% CO₂ for 24 hours. The compounds of the present invention were dissolved in dimethyl sulfoxide to a concentration of 1 mM, and further diluted with dimethyl sulfoxide and 5% FBS-DMEM so that the final concentrations of the compounds of the present invention were 5000, 1667, 556, 185, 62, 21, 7, and 2 nM, respectively. The medium for the TWNT-4 cells was removed, and 0.1 mL of 1 ng/mL TGF-β1 (Peprotech) was added to each well, followed by culturing for 24 hours. Thereafter, the medium was removed, and the cells were fixed with 4% paraformaldehyde, and washed with phosphate buffered saline (PBS). Blocking was carried out for 30 minutes with PBS containing 2% bovine serum albumin and 0.1% Triton X-100, and the sections were reacted overnight at 4°C with anti-Smad2/3 antibody (Cell Signaling Technology) diluted 1/4000 in the same blocking solution. After the reaction, the plate was washed three times with PBS containing Tween-20 (PBS-T), and then an anti-rabbit immunoglobulin antibody fluorescently labeled with Alexa Fluor 647 (Thermo Fisher Scientific) and the nuclear stain Hoechst 33342 (Dojindo Chemical Industries) were each diluted 1/4000 in blocking solution, and the plate was allowed to react at room temperature for 1 hour. After the reaction was completed, the cells were washed three times with PBS-T and replaced with PBS, and then stained images were obtained using a fluorescence microscope (CellInsight CX5, manufactured by Thermo Fisher Scientific). From the acquired stained images, the nuclear Smad2/3 fluorescence intensity and the cytoplasmic Smad2/3 fluorescence intensity were quantified using analysis software (HCS Studio, manufactured by Thermo Fisher Scientific), and the nuclear localization ratio of Smad2/3 was calculated from the ratio. The 50% nuclear localization inhibitory concentration (EC₅₀ (nM)) was calculated by the least squares method, setting the nuclear localization rate of the TGF-β-treated group without compound treatment as 100% and the TGF-β-treated group with 10 µM of the ALK5 inhibitor SB431542 (Sigma) set as 0%. The results are shown in Table 14.

**[Table 14]**

| Example Number | EC₅₀ (nM) |
|---|---|
| 1 | 47 |
| 189 | 29 |
| 302 | 37 |
| 291 | 38 |
| 312 | 46 |
| 51 | 47 |
| 318 | 51 |
| 278 | 64 |
| 86 | 72 |
| 103 | 80 |
| 195 | 97 |
| 202 | 107 |
| 44 | 142 |
| 277 | 143 |
| 246 | 148 |
| 25 | 211 |
| 42 | 304 |
| 89 | 334 |
| 115 | 564 |
| 178 | 566 |
| 271 | 596 |
| 30 | 757 |

The results in Table 14 demonstrate that the compounds of the present invention significantly inhibit the nuclear localization of Smad2/3 induced by TGF-β stimulation.

### Test Example 2: Inhibitory effect on CTGF and α-SMA mRNA expression (anti-fibrotic effect)

TWNT-4 cells were suspended in 10% FBS-DMEM and seeded onto a 24-well plate at a density of 2×10⁴ cells/well, and then cultured at 37°C. in the presence of 5% CO₂ for 24 hours. The compound of the present invention was dissolved in dimethyl sulfoxide to a concentration of 1 mM, and further diluted with dimethyl sulfoxide and 1% FBS-DMEM so that the final concentrations of the compound of the present invention became 10, 1, 0.1, and 0.3 µM, respectively. The medium for the TWNT-4 cells was removed, and 1 mL of each of these was added to each well along with 2 ng/mL of TGF-β1 (Peprotech), followed by culturing for 24 hours. Thereafter, RNA was extracted using TRIzol reagent (Thermo Fisher Scientific), and the absorbance at 260 nm was measured using a spectrophotometer (Nano Drop, manufactured by Thermo Fisher Scientific) to determine the RNA concentration. Using this RNA as a template, a reverse transcription reaction was carried out using ReverTra Ace qPCR RT Kit (TOYOBO) according to the attached instructions to synthesize cDNA. Furthermore, using this cDNA as a template, real-time PCR was performed according to the attached instructions using THUNDERBIRD SYBR qPCR Mix (TOYOBO) and primers for CTGF (Connective tissue growth factor), α-SMA (α-smooth muscle actin), or GAPDH as an internal standard gene. After measuring the threshold cycle (Ct) value of each gene, the relative mRNA expression level was calculated by the ΔΔCt method. The results are shown in Figure 1.

From the results shown in Fig. 1, it was found that the compound of the present invention significantly suppressed the mRNA expression levels of CTGF and α-SMA induced by TGF-β stimulation.

## Claims

1. A compound represented by the following formula (1), or a salt thereof: 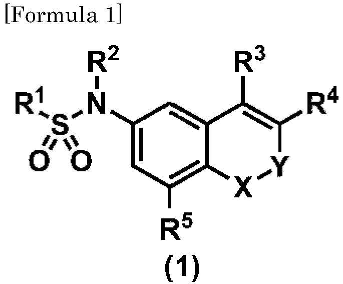
[wherein X represents an oxygen atom, a sulfur atom, or a nitrogen atom which may have a substituent;
Y represents a carbonyl group, a thiocarbonyl group, a sulfoxide group, or a sulfonyl group;
R¹ represents an optionally substituted lower alkyl group, an optionally substituted aryl group, an optionally substituted 5- or 6-membered heteroaryl group, or an optionally substituted aralkyl group;
R² represents a hydrogen atom, an optionally substituted lower alkyl group, a lower cycloalkyl group,
a lower heterocycloalkyl group, an optionally substituted lower alkynyl group, an optionally substituted aralkyl group, or any of the groups shown in 1) to 4) below;
1) The following formula (a): 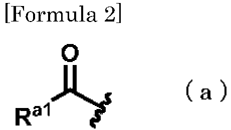 (wherein R^{a1} represents an optionally substituted lower alkyl group, a lower cycloalkyl group, an optionally substituted lower alkenyl group, an optionally substituted lower alkoxy group, an optionally substituted aralkyloxy group, or an optionally substituted aryl group, and the wavy line represents the bonding site.)
2) The following formula (b): 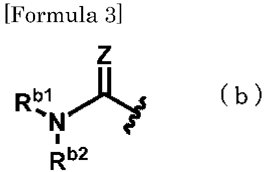 (wherein Z represents an oxygen atom or a sulfur atom; R^{b1} and R^{b2} may be the same or different and represent a hydrogen atom, an optionally substituted lower alkyl group, a lower cycloalkyl group, an optionally substituted aryl group, an optionally substituted 5- or 6-membered heteroaryl group, an optionally substituted aralkyl group or an optionally substituted heteroaralkyl group; or R^{b1} and R^{b2} together with the adjacent nitrogen atom form a 4- to 7-membered cyclic amino group which may contain a heteroatom; and the wavy line represents a bonding site.)
3) The following formula (c): 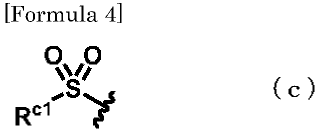 (wherein R^{c1} represents an optionally substituted lower alkyl group, a lower cycloalkyl group, a lower heterocycloalkyl group, an optionally substituted lower alkenyl group, an optionally substituted aryl group, an optionally substituted 5- or 6-membered heteroaryl group, an optionally substituted aralkyl group, or an optionally substituted heteroaralkyl group, and the wavy line represents the bonding site.)
4) The following formula (d): 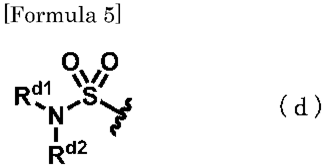 (wherein R^{d1} and R^{d2} may be the same or different and represent a hydrogen atom, an optionally substituted lower alkyl group, a lower cycloalkyl group, or an optionally substituted aralkyl group, or R^{d1} and R^{d2} together with the adjacent nitrogen atom form a 4- to 7-membered cyclic amino group which may contain a heteroatom, and the wavy line represents the bonding site.)
R³ represents a hydrogen atom, a lower alkyl group, a lower cycloalkyl group, or an aryl group;
R⁴ represents a hydrogen atom, a halogen atom, an optionally substituted lower alkyl group, a lower cycloalkyl group, an optionally substituted lower alkenyl group, an optionally substituted lower alkynyl group, a hydroxy group, an optionally substituted arylsulfinyl group, an optionally substituted lower alkoxy group, an amino group, a lower alkylamino group, a 4- to 7-membered cyclic amino group, a lower alkanoylamino group, a lower alkanoyl-lower alkylamino group, a lower alkoxycarbonylamino group, an aralkyloxycarbonylamino group, a lower alkoxycarbonyl group, a nitrile group, a carboxy group, a formyl group, an optionally substituted aryl group, an optionally substituted 5- or 6-membered heteroaryl group, an optionally substituted aralkyl group or an optionally substituted heteroaralkyl group;
R⁵ represents a hydrogen atom, a halogen atom or a lower alkoxy group].

2. The compound or salt thereof according to claim 1, wherein X is an oxygen atom, Y is a carbonyl group or a sulfonyl group, R¹ is an optionally substituted phenyl group, R² is a group represented by formula (c), R^{c1} is an optionally substituted phenyl group, R³ is a hydrogen atom, R⁴ is an optionally substituted phenyl group, and R⁵ is a hydrogen atom or a fluorine atom.

3. The compound or salt thereof according to claim 2, wherein R¹ is a phenyl group or a phenyl group substituted with a C₁₋₇ alkyl group, and R^{c1} in formula (c) represented by R² is a phenyl group substituted with a C₁₋₇ alkyl group.

4. A medicament comprising the compound of claim 1 or a salt thereof and a pharmaceutically acceptable carrier.

5. A preventive or therapeutic agent for fibrosis, comprising the compound according to claim 1 or a salt thereof as an active ingredient.

6. A TGF-β signal inhibitor comprising the compound according to claim 1 or a salt thereof as an active ingredient.

7. An inhibitor of nuclear localization of Smad2/3, comprising the compound according to claim 1 or a salt thereof as an active ingredient.

8. Use of the compound according to claim 1 or a salt thereof for the manufacture of an agent for preventing or treating fibrosis.

9. Use of the compound according to claim 1 or a salt thereof for the production of a TGF-β signal inhibitor.

10. Use of the compound according to claim 1 or a salt thereof for producing an agent for suppressing nuclear localization of Smad2/3.

11. The compound or salt thereof according to claim 1, which is used for preventing or treating fibrosis.

12. The compound or salt thereof according to claim 1, which is used to inhibit TGF-β signaling.

13. The compound or salt thereof according to claim 1, which is used to inhibit the nuclear localization of Smad2/3.

14. A method for preventing or treating fibrosis, which comprises administering the compound according to claim 1 or a salt thereof to a subject.

15. A method for inhibiting TGF-β signaling, which comprises administering the compound according to claim 1 or a salt thereof to a subject.

16. A method for inhibiting nuclear localization of Smad2/3, comprising administering the compound according to claim 1 or a salt thereof to a subject.
